(19) Europäisches Patentamt
European Patent Office
Office européen des brevets

(11) EP 4 368 618 A1

(12) EUROPEAN PATENT APPLICATION
published in accordance with Art. 153(4) EPC

(43) Date of publication:
**15.05.2024 Bulletin 2024/20**

(21) Application number: **22836740.5**

(22) Date of filing: **24.06.2022**

(51) International Patent Classification (IPC):
***C07D 401/04*** (2006.01) ***C07D 401/12*** (2006.01)
***C07D 401/14*** (2006.01) ***C07D 413/14*** (2006.01)
***C07D 403/12*** (2006.01) ***C07D 409/14*** (2006.01)
***A61K 31/165*** (2006.01) ***A61K 31/4192*** (2006.01)
***A61P 35/00*** (2006.01)

(52) Cooperative Patent Classification (CPC):
**A61K 31/165; A61K 31/4192; A61P 35/00; C07D 401/04; C07D 401/12; C07D 401/14; C07D 403/12; C07D 409/14; C07D 413/14**

(86) International application number:
**PCT/CN2022/101146**

(87) International publication number:
**WO 2023/279986 (12.01.2023 Gazette 2023/02)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **05.07.2021 PCT/CN2021/104458**
**09.09.2021 PCT/CN2021/117357**
**12.04.2022 CN 202210383102**
**22.06.2022 CN 202210715786**

(71) Applicant: **Betta Pharmaceuticals Co., Ltd**
**Yuhang**
**Hangzhou**
**Zhejiang 311100 (CN)**

(72) Inventors:
- **FU, Bang**
  **Beijing 100176 (CN)**
- **LI, Yinlong**
  **Beijing 100176 (CN)**
- **REN, Wei**
  **Beijing 100176 (CN)**
- **SHEN, Qichao**
  **Beijing 100176 (CN)**
- **DU, Guolong**
  **Beijing 100176 (CN)**
- **ZHOU, Xiaojun**
  **Beijing 100176 (CN)**
- **SUN, Yun**
  **Beijing 100176 (CN)**
- **ZHOU, Yuzhen**
  **Hangzhou, Zhejiang 311100 (CN)**
- **SUN, Xiao**
  **Beijing 100176 (CN)**
- **CAI, Weidong**
  **Hangzhou, Zhejiang 311100 (CN)**
- **LIU, Xiangyong**
  **Beijing 100176 (CN)**
- **DING, Lieming**
  **Hangzhou, Zhejiang 311100 (CN)**
- **WANG, Jiabing**
  **Beijing 100176 (CN)**

(74) Representative: **Graf von Stosch Patentanwaltsgesellschaft mbH**
**Prinzregentenstraße 22**
**80538 München (DE)**

# (54) SIX-MEMBERED ARYL OR HETEROARYL AMIDES, AND COMPOSITION AND USE THEREOF

(57) A compound of formula I and a use thereof as an MALT1 inhibitor, and a pharmaceutical composition containing the compound. The compound can be used to treat diseases or disorders such as cancer.

EP 4 368 618 A1

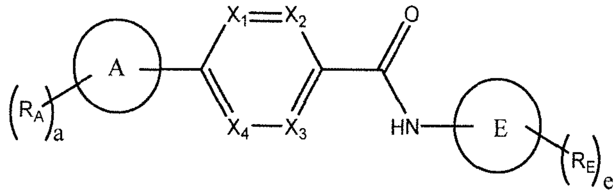

Formula I

## Description

### FIELD OF THE INVENTION

The present invention belongs to the field of medicine, and specifically relates to a six-membered aryl or heteroaryl amide, and composition and use thereof.

### BACKGROUND OF THE INVENTION

MALT1(mucosa-associated lymphoid tissue lymphoma translocation protein 1), as a key mediator of the classic NF-κB signaling pathway, is currently the only paracaspase known in humans that transduces signals from B cell receptors (BCR) and T cell receptors (TCR). The engagement of TCR/BCR results in the assembly of CARD11-BCL10-MALT1 (CBM) complex, which is composed of three proteins: CARD11 (caspase recruitment domain family member 11), BCL10 (B-cell lymphoma factor 10), and MALT1, to trigger the downstream activation. MALT1 affects NF-κB signaling through two mechanisms: first, MALT1 functions as a scaffolding protein, recruiting NF-κB signaling proteins such as TRAF6, TAB-TAK1 or NEMO-IKKα/β; second, MALT1 use its protease function to cleave and inactivate negative regulators of NF-κB signaling, such as RelB, A20 or CYLD. The caspade signaling ultimately leads to the nuclear translocation of the NF-κB transcription factor complex and the activation of genes involved in proliferation, apoptosis inhibition, and inflammation (Jaworski et al., Cell Mol Life Sci, 2016, Vol. 73, 459-473).

Sustained activation of the NF-κB signaling pathway is a hallmark of ABC-DLBCL (activated B-cell-like subtype of diffuse large B-cell lymphoma). The ABC subtype is a more aggressive form of DLBCL. DLBCL is the most common form of non-Hodgkin lymphoma (NHL), accounting for approximately 25% of lymphoma cases, while ABC-DLBCL accounts for approximately 40% of DLBCL cases. In ABC-DLBCL patients, NF- κ B pathway activation is driven by mutations in CD79A/B, CARD11, MYD88 or A20 (Staudt, ColdSpring Harb Perspect Biol, 2010, Vol. 2; Lim et al., Immunol Rev, 2012 Vol. 246, 359-378). Small molecule tool compound inhibitor of MALT1 protease has proven effective in preclinical models of ABC-DLBCL (Fontan et al., Cancer Cell, 2012, Vol. 22, 812-824; Nagel et al., Cancer ell, 2012, Vol. 22, 825-837).

In addition to lymphoma, MALT1 has also been shown to play a crutial role in both innate and adaptive immunity (Jaworski M et al., Cell Mol Life Sci., 2016). MALT1 protease inhibitors showed the anti-inflammatory activity by slowing down the disease onset and progression of experimental allergic encephalomyelitis (mouse model of multiple sclerosis) in mice (Mc Guire et al., J.Neuro inflammation, 2014, Vol. 11, 124). Furthermore, MALT1 knockout mice have fewer Tregs with Teff cell function severely impaired, and the immunity is generally compromised. In contrast, only knockout of the protease domain of MALT1 significantly diminishes Tregs but presevrs the functions of Teff cell. Therefore, MALT1 protease inhibitors can benefit patients with solid tumors by regulating Treg function and promoting the body's immune response(Arianna Bertossi et al., EMBO J, 2014, Vol. 33, 2740-2742).

Therefore, the MALT1 inhibitors of the present invention may provide beneficial therapeutic effects to patients suffering from cancer and/or immune diseases.

### SUMMARY OF INVENTION

The present invention relates to a six-membered aryl or heteroaryl amide and its composition and use, which can be used as an inhibitor of MAL T1 for the treatment of cancer and/or immune-related diseases.

A first object of the present invention is to provide a compound of Formula I, or a stereoisomer, tautomer, deuterated compound, pharmaceutically acceptable salt, prodrug, chelate, non-covalent complex or solvate thereof,

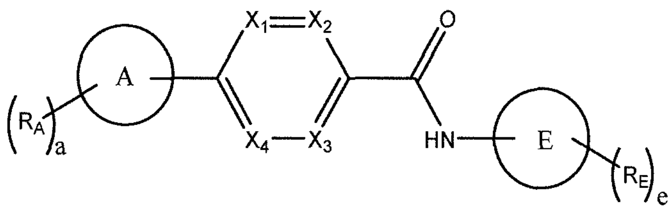

Formula I

wherein,

$X_1$ is selected from $CR_1$ or N;
$X_2$ is selected from $CR_2$ or N;

$X_3$ is selected from $CR_3$ or N;
$X_4$ is selected from $CR_4$ or N;
$R_1$, $R_2$, $R_3$, $R_4$ are the same or different, and are each independently selected from the group consisting of H, CN, $NO_2$, halogen, $-C_{1-6}$ alkyl, $-C_{2-6}$ alkenyl, $-C_{2-6}$ alkynyl, - $OR_5$, $-NR_5R_6$, $SR_5$, $-C_{0-6}$ alkyl-$C_{3-8}$ carbocyclyl, $-C_{0-6}$ alkyl-3-8 membered heterocyclyl, $-C_{0-6}$ alkyl-$C_{6-10}$ aryl, $-C_{0-6}$ alkyl-5-10 membered heteroaryl, $-C(O)R_5$, - $N(R_5)C(O)R_6$, $-C(O)NR_5R_6$, $-N(R_5)C(O)OR_6$, $-OC(O)NR_5R_6$, $-N(R_5)C(O)NR_6R_7$, - $S(O)R_5$, $-S(O)_2R_5$, $-S(O)NR_5R_6$, $-S(O)_2NR_5R_6$, $-N(R_5)S(O)R_6$, $-N(R_5)S(O)_2R_6$, - $C(O)OR_5$, $-OC(O)R_5$ and $-OC(O)OR_5$; wherein the $-C_{1-6}$ alkyl, $-C_{2-6}$ alkenyl, $-C_{2-6}$ alkynyl, $-C_{0-6}$ alkyl-$C_{3-8}$ carbocyclyl, $-C_{0-6}$ alkyl-3-8 membered heterocyclyl, $-C_{0-6}$ alkyl-$C_{6-10}$ aryl, $-C_{0-6}$ alkyl-5-10 membered heteroaryl, $R_5$, $R_6$ and $R_7$ are optionally substituted with one or more substituents selected from hydrogen, halogen, CN, $-OR_8$, $-NR_8R_9$, $-SR_8$, $-C(O)R_8$, $-N(R_8)C(O)R_9$, $-C(O)NR_8R_9$, $-N(R_8)C(O)OR_9$, $-OC(O)NR_8R_9$, $-N(R_8)C(O)NR_9R_{10}$, $-S(O)R_8$, $-S(O)_2R_8$, $-S(O)NR_8R_9$, $-S(O)_2NR_8R_9$, $-N(R_8)S(O)R_9$, - $N(R_8)S(O)_2R_9$, $-C(O)OR_8$, $-OC(O)R_8$, $-OC(O)OR_8$, oxo, $-C_{1-6}$ alkyl, $-C_{2-6}$ alkenyl, $-C_{2-6}$ alkynyl, $-C_{0-6}$ alkyl-$C_{3-8}$ carbocyclyl or $-C_{0-6}$ alkyl-3-8 membered heterocyclyl;
ring A is selected from $-C_{5-6}$ carbocyclyl, 5-6 membered heterocyclyl, $-C_6$ aryl or 5-6 membered heteroaryl;
each $R_A$ is the same or different, and is independently selected from the group consisting of hydrogen, halogen, oxo, CN, NOz, $-C_{1-6}$ alkyl, $-C_{2-6}$ alkenyl, $-C_{2-6}$ alkynyl, $-OR_5$, $-NR_5R_6$, $-SR_5$, $-C_{0-6}$ alkyl-$C_{3-8}$ carbocyclyl, $-C_{0-6}$ alkyl-3-8 membered heterocyclyl, $-C_{0-6}$ alkyl-$C_{6-10}$ aryl, $-C_{0-6}$ alkyl-5-10 membered heteroaryl, $-C(O)R_5$, - $N(R_5)C(O)R_6$, $-C(O)NR_5R_6$, $-N(R_5)C(O)OR_6$, $-OC(O)NR_5R_6$, $-N(R_5)C(O)NR_6R_7$, - $S(O)R_5$, $-S(O)_2R_5$, $-S(O)NR_5R_6$, $-S(O)_2NR_5R_6$, $-N(R_5)S(O)R_6$, $-N(R_5)S(O)_2R_6$, - C(O)ORs, $-OC(O)R_5$ and -OC(O)ORs; wherein the $-C_{1-6}$ alkyl, $-C_{2-6}$ alkenyl, $-C_{2-6}$ alkynyl, $-C_{0-6}$ alkyl-$C_{3-8}$ carbocyclyl, $-C_{0-6}$ alkyl-3-8 membered heterocyclyl, $-C_{0-6}$ alkyl-$C_{6-1ü}$ aryl, $-C_{0-6}$ alkyl-5-10 membered heteroaryl, $R_5$, $R_6$ and $R_7$ are optionally substituted with one or more substituents selected from hydrogen, halogen, CN, oxo, - $C_{1-6}$ alkyl, $-C_{2-6}$ alkenyl, $-C_{2-6}$ alkynyl, $-C_{0-6}$ alkyl-$C_{3-8}$ carbocyclyl, $-C_{0-6}$ alkyl-3-8 membered heterocyclyl, $-OR_8$, $-NR_8R_9$, $-SR_8$, $-S(O)R_8$, $-S(O)_2R_8$, $-C(O)R_8$, $-C(O)OR_8$, $-OC(O)R_8$, $-N(R_8)C(O)R_9$ or $-C(O)NR_8R_9$;
ring E is selected from 5-10 membered heterocyclyl, $C_{6-10}$ aryl or 5-10 membered heteroaryl;
each $R_E$ is the same or different, and is independently selected from the group consisting of hydrogen, halogen, oxo, CN, $NO_2$, $-C_{1-6}$ alkyl, $-C_{2-6}$ alkenyl, $-C_{2-6}$ alkynyl, $-C_{0-6}$ alkyl-5-10 membered heteroaryl, $-C_{0-6}$ alkyl-$C_{3-8}$ carbocyclyl, $-C_{0-6}$ alkyl-3-8 membered heterocyclyl, $-OR_5$, $-NR_5R_6$, $-SR_5$, $-C(O)R_5$, $-N(R_5)C(O)R_6$, - $C(O)NR_5R_6$, $-N(R_5)C(O)OR_6$, $-OC(O)NR_5R_6$, $-N(R_5)C(O)NR_6R_7$, $-S(O)R_5$, $-S(O)_2R_5$, $-S(O)NR_5R_6$, $-S(O)_2NR_5R_6$, $-N(R_5)S(O)R_6$, $-N(R_5)S(O)_2R_6$, $-C(O)OR_5$, $-OC(O)R_6$ and $-OC(O)OR_7$; wherein the $-C_{1-6}$ alkyl, $-C_{2-6}$ alkenyl, $-C_{2-6}$ alkynyl, $-C_{0-6}$ alkyl-5-10 membered heteroaryl, $-C_{0-6}$ alkyl-$C_{3-8}$ carbocyclyl, $-C_{0-6}$ alkyl-3-8 membered heterocyclyl, $R_5$, $R_6$ and $R_7$ are optionally substituted with one or more substituents selected from hydrogen, halogen, CN, oxo, $-C_{1-6}$ alkyl, $-C_{2-6}$ alkenyl, $-C_{2-6}$ alkynyl, - ORs, $-NR_8R_9$, substituted or unsubstituted $-C_{0-6}$ alkyl-$C_{3-6}$ carbocyclyl, substituted or unsubstituted $-C_{0-6}$ alkyl -3-6 membered heterocyclyl, -C(O)ORs or $-C(O)NR_8R_9$;
$R_5$, $R_6$, $R_7$, $R_8$, $R_9$, $R_{10}$ are each independently selected from the group consisting of hydrogen, halogen, CN, OH, $NH_2$, oxo, $-C_{1-6}$ alkyl, $-C_{1-6}$ alkoxy, $-C_{2-6}$ alkenyl, $-C_{2-6}$ alkynyl, $-C_{0-6}$ alkyl-$C_{3-14}$ carbocyclyl, $-C_{0-6}$ alkyl-3-14 membered heterocyclyl; wherein the $-C_{1-6}$ alkyl, $-C_{1-6}$ alkoxy, $-C_{2-6}$ alkenyl, $-C_{2-6}$ alkynyl, $-C_{0-6}$ alkyl-$C_{3-14}$ carbocyclyl, $-C_{0-6}$ alkyl-3-14 membered heterocyclyl are optionally substituted with one or more substituents selected from hydrogen, halogen, CN, OH, $NH_2$, -COOH, oxo, $-C_{1-6}$ alkyl, $-C_{1-6}$ haloalkyl, $-C_{2-6}$ alkenyl, $-C_{2-6}$ alkynyl, $-C_{1-6}$ alkoxy, $-C_{1-6}$ haloalkoxy, $-NH(C_{1-6}$ alkyl), $-N(C_{1-6}$ alkyl$)_2$, $-COO(C_{1-6}$ alkyl), $-CONH(C_{1-6}$ alkyl), - $CON(C_{1-6}$ alkyl$)_2$, substituted or unsubstituted $-C_{0-6}$ alkyl-$C_{3-6}$ carbocyclyl, substituted or unsubstituted $-C_{0-6}$ alkyl-3-6 membered heterocyclyl;
a is selected from 0, 1, 2, 3, 4, 5 or 6;
e is selected from 0, 1, 2, 3, 4, 5 or 6;
when $X_1$ is selected from CH, $X_2$ is selected from CH, $X_3$ is selected from CH, and $X_4$ is selected from CH,

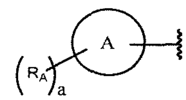

is not phenyl.

In some embodiments of Formula I,

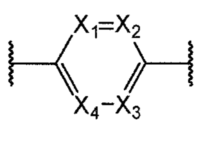

is selected from

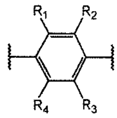,

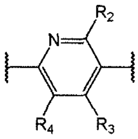, 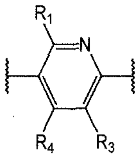, 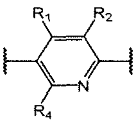, 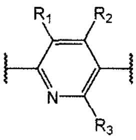, 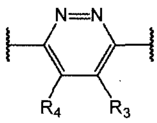, 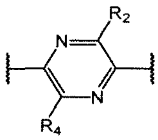, 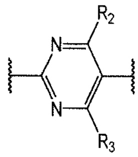,

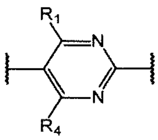, 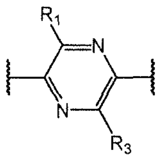 or 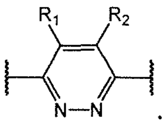.

In some embodiments of Formula I,

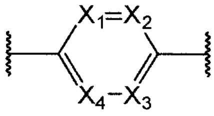

is selected from

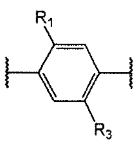,

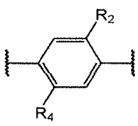, 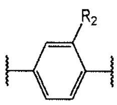, 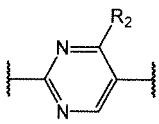, 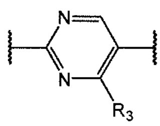, 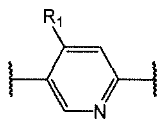, 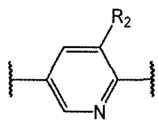,

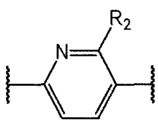, 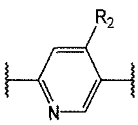 or 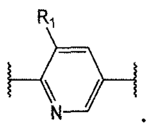.

In some embodiments of Formula I, $R_1$, $R_3$ are the same or different, and are each independently selected from the group consisting of halogen, $-C_{1-6}$ alkyl, $-OR_5$, - $NR_5R_6$, $-C_{0-6}$ alkyl $-C_{3-8}$ carbocyclyl and $-C_{0-6}$ alkyl -3-8 membered heterocyclyl; wherein the $-C_{1-6}$ alkyl, $-C_{0-6}$ alkyl $-C_{3-8}$ carbocyclyl, $-C_{0-6}$ alkyl -3-8 membered heterocyclyl, $R_5$ and $R_6$ are optionally substituted with one or more substituents selected from hydrogen, halogen, CN, OH, $NH_2$, $-C_{1-6}$ alkyl, $-C_{2-6}$ alkenyl, $-C_{2-6}$ alkynyl, $-C_{1-6}$ alkoxy, $-C_{0-6}$ alkyl $-C_{3-8}$ carbocyclyl or $-C_{0-6}$ alkyl -3-8 membered heterocyclyl.

In some embodiments of Formula I, $R_2$, $R_4$ are the same or different, and are each independently selected from the group consisting of halogen, $-C_{1-6}$ alkyl, $-OR_5$, - $NR_5R_6$, $-C_{0-6}$ alkyl $-C_{3-8}$ carbocyclyl and $-C_{0-6}$ alkyl -3-8 membered heterocyclyl; wherein the $-C_{1-6}$ alkyl, $-C_{0-6}$ alkyl $-C_{3-8}$ carbocyclyl, $-C_{0-6}$ alkyl -3-8 membered heterocyclyl, $R_5$ and $R_6$ are optionally substituted with one or more substituents selected from hydrogen, halogen, CN, OH, $NH_2$,

$-C_{1-6}$ alkyl, $-C_{2-6}$ alkenyl, $-C_{2-6}$ alkynyl, $-C_{1-6}$ alkoxy, $-C_{0-6}$ alkyl $-C_{3-8}$ carbocyclyl or $-C_{0-6}$ alkyl -3-8 membered heterocyclyl.

In some embodiments of Formula I, $R_1$, $R_2$, $R_3$, $R_4$ are the same or different, and are each independently selected from the group consisting of H, -F, -Cl, -Br, -I, -OH, $-NH_2$, -CN, $-CH_3$, $-CF_3$, $-CH_2CH_3$, $-CH(CH_3)_2$, $-CH_2CH(CH_3)_2$, $-C(CH_3)_3$, $-NHCH(CH_3)_2$, $-NHC(O)CH_3$, $-NHC(O)OCH_2CH_3$,

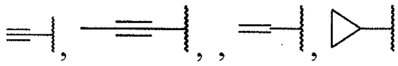 , , , , , -

$OCH_3$, $-OCH(CH_3)_2$,

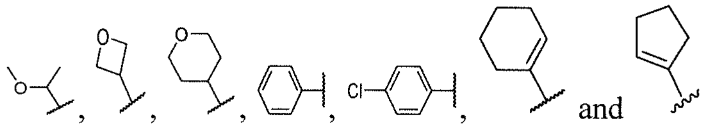 , , , , , and .

In some embodiments of Formula I,

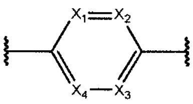

is selected from

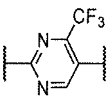,

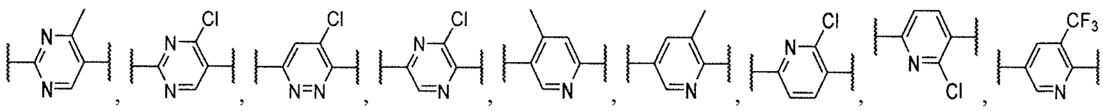 , , , , , , , , ,

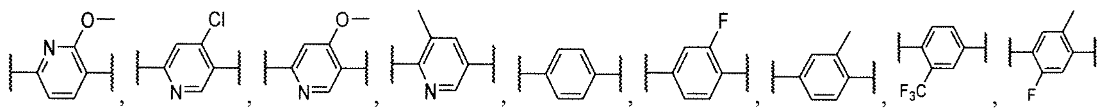 , , , , , , , , ,

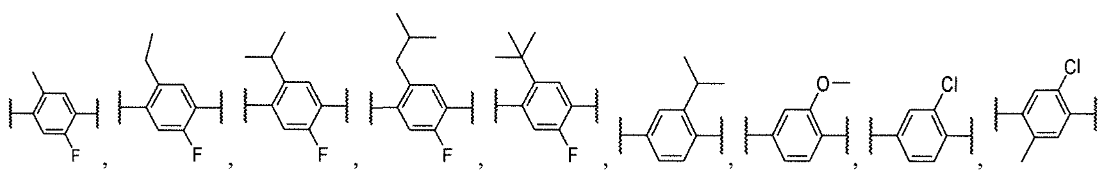 , , , , , , , , ,

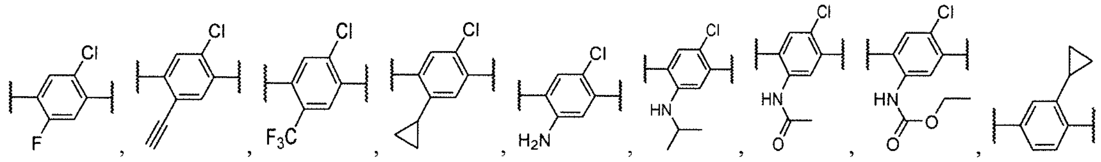 , , , , , , , , ,

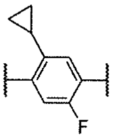, 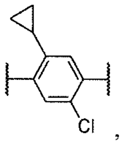, 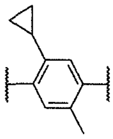, 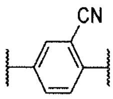, 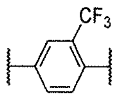, 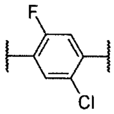, 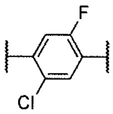, 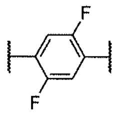, 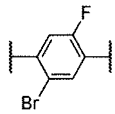,

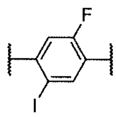, 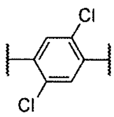, 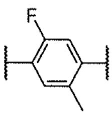, 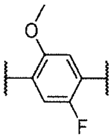, 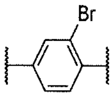, 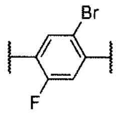, 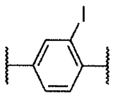, 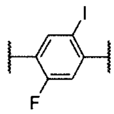,

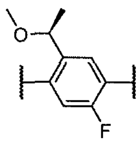, 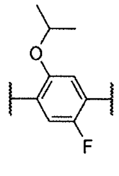, 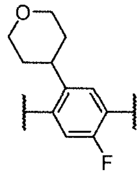, 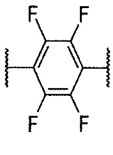, 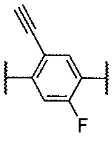, 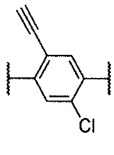, 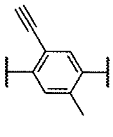, 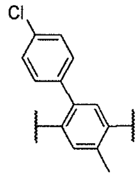,

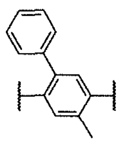, 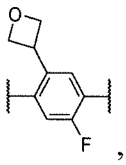, 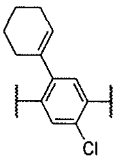, 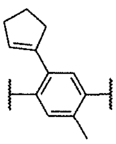, 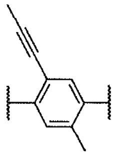, 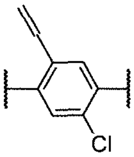, 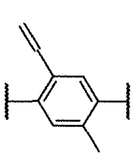, 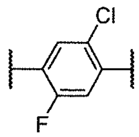,

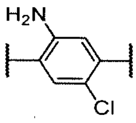, 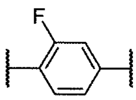 or 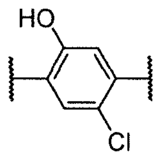.

In some embodiments of Formula I, ring A is selected from -$C_6$ carbocyclyl, 6 membered heterocyclyl, -$C_6$ aryl or 6 membered heteroaryl.

In some embodiments of Formula I, ring A is selected from Phenyl, pyridyl, furyl, thienyl, pyrrolyl, pyrazolyl, pyrimidinyl, pyridazinyl, diazinyl, cyclopentenyl, pyridin-2(1H)-one or pyrimidine-2(1H )-keto group, etc.

In some embodiments of Formula I, ring A is

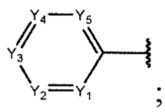;

wherein $Y_1$ is selected from $CR_A$ or N; $Y_2$ is selected from $CR_A$ or N; $Y_3$ is selected from $CR_A$ or N; $Y_4$ is selected from $CR_A$ or N; $R_A$ is defined as described for the compound represented by formula I above.

In some embodiments of Formula I, each $R_A$ is the same or different, and is independently selected from the group consisting of hydrogen, oxo, -F, -Cl, -Br, -I, - CN, -OH, $-NH_2$, $-NO_2$, $-CH_3$, $-CF_3$, $-OCF_3$, $-NHCH_3$, $-N(CH_3)_2$, $-OCH_3$, $-SCH_3$, - $CHF_2$, $-OCHF_2$, $-C(O)NH_2$, $-C(O)CH_3$, $-OC(O)NH_2$, $-NHC(O)CH_3$, $-CH_2CH_3$, - $CH(CH_3)_2$,

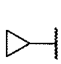, 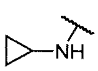,

$-SCH_3$, $-SOCH_3$, $-CH_2SO_2CH_3$, $-SO_2CH_3$, $-NHC(O)NH_2$, - $CH_2OH$, $-CH_2NH_2$, $-NHC(O)OCH_2CH_3$, -NHOH, $-CH_2OCH_3$,

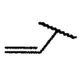, 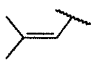,

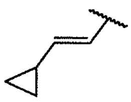, 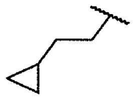, 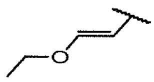, 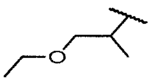, 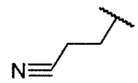, 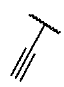, 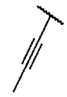, 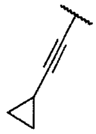,

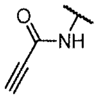, 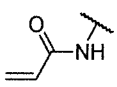, 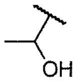, 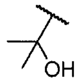, 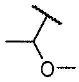, 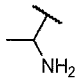, 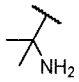, 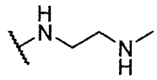, 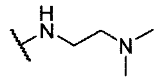,

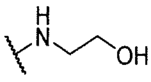, 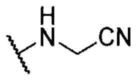, 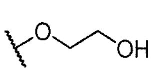, 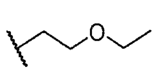, 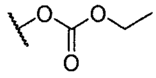, 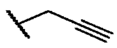, 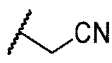,

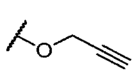, 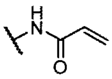, 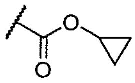, 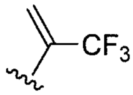, 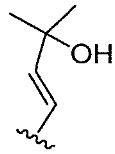, 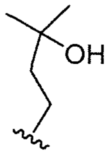, 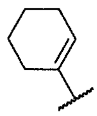, 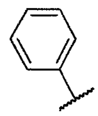,

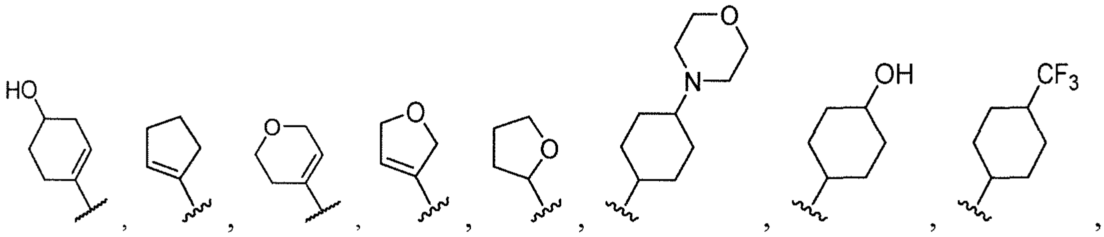
, , , , , , , ,

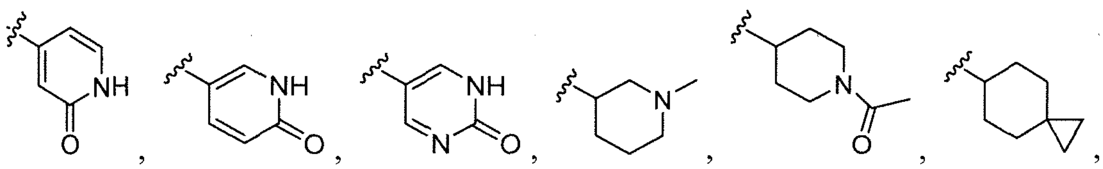
, , , , , ,

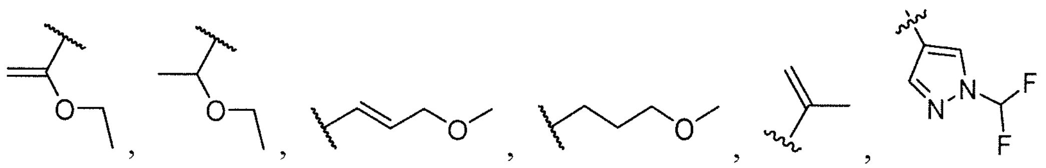
, , , , , ,

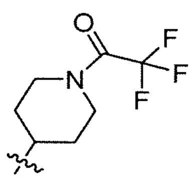
.

and

In some embodiments of Formula I,

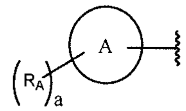

is selected from

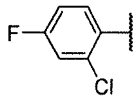,

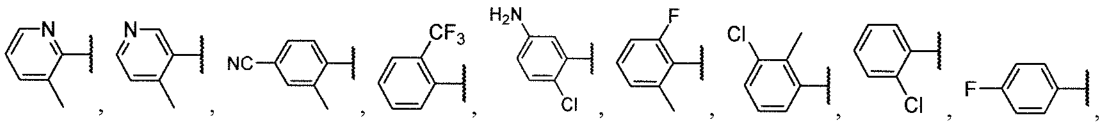,

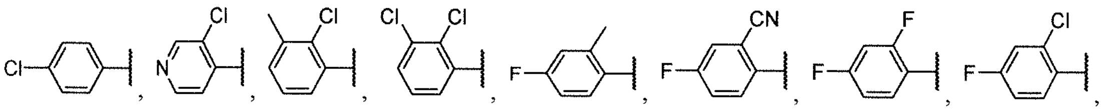,

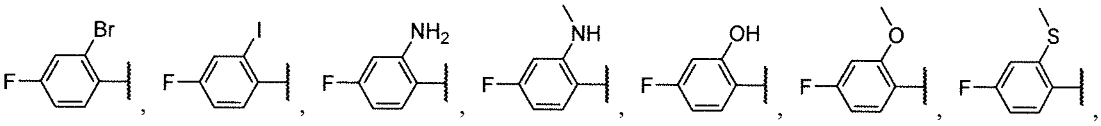,

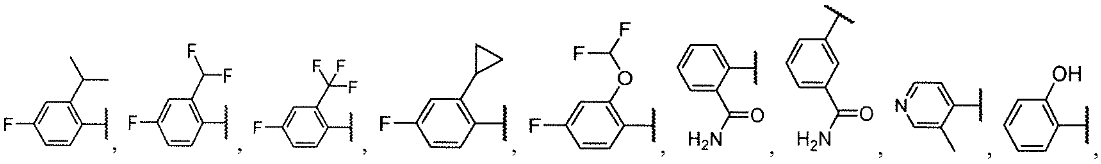,

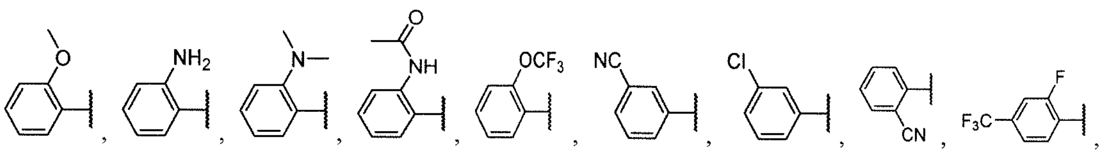,

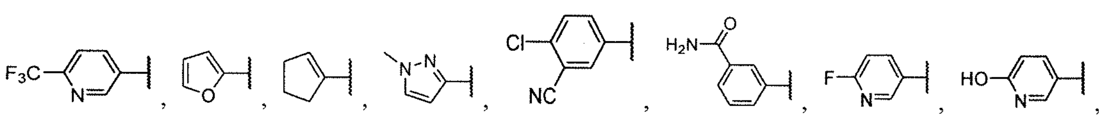,

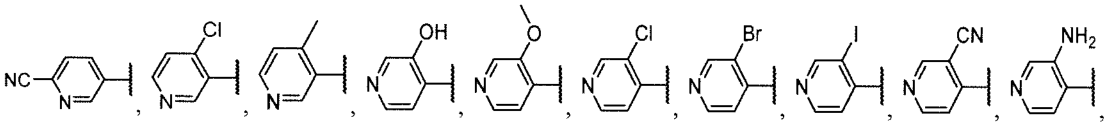,

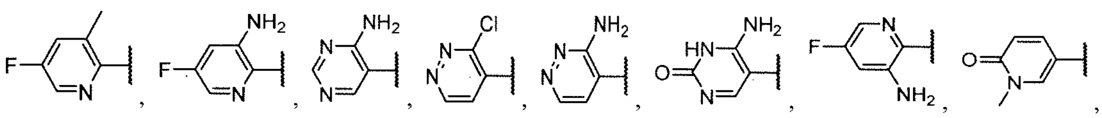,

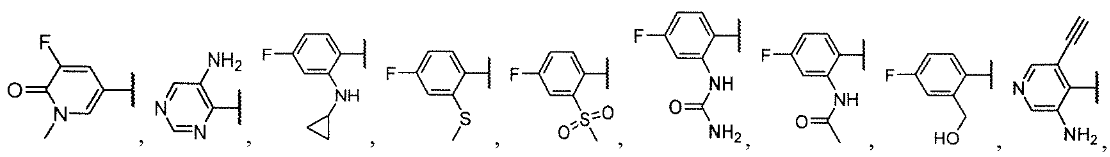
, , , , , , , , ,

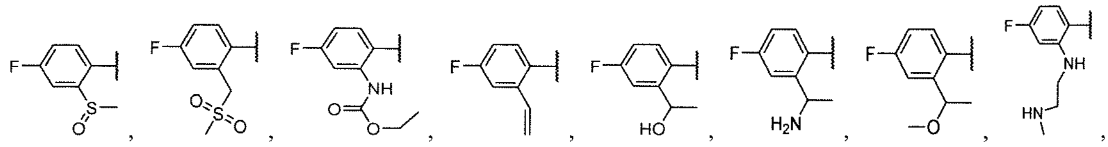
, , , , , , , , ,

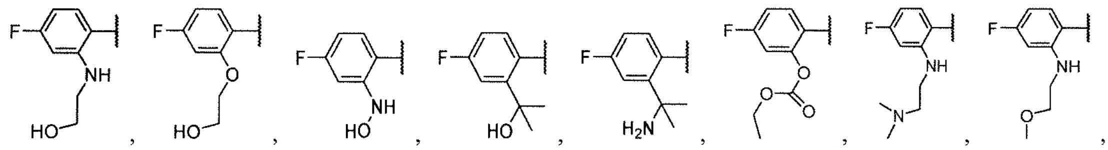
, , , , , , , , ,

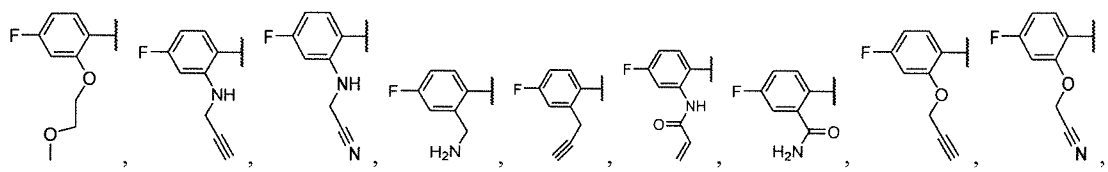
, , , , , , , , ,

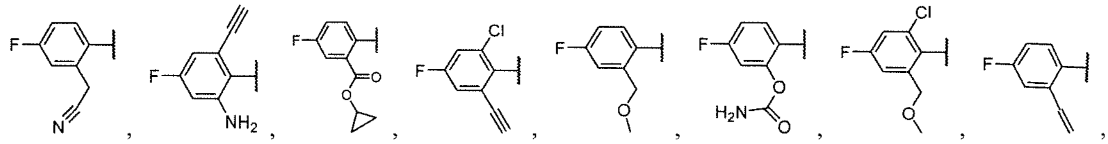
, , , , , , , ,

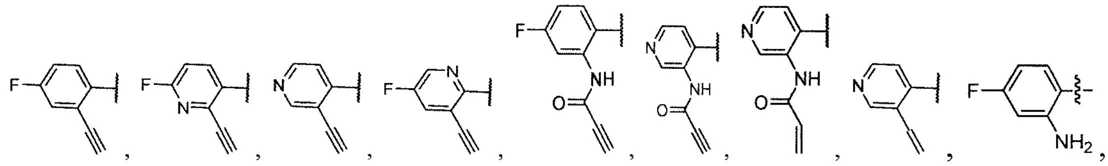
, , , , , , , , ,

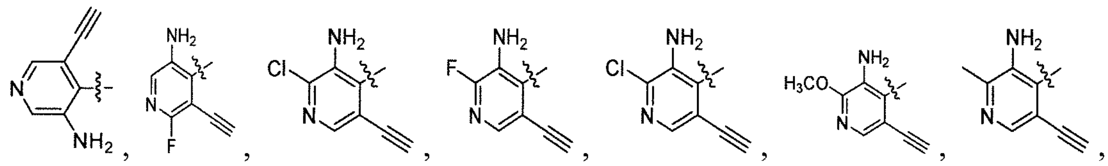
, , , , , , ,

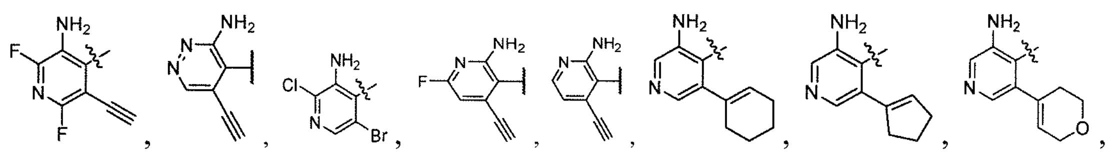
, , , , , , , ,

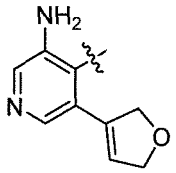, 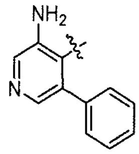, 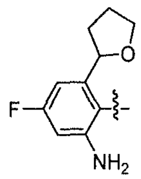, 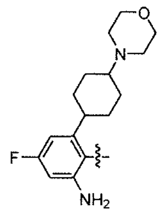, 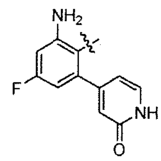, 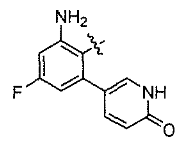,

or

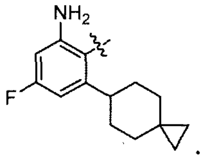.

In some embodiments of Formula I, ring E is selected from 6-10 membered heterocyclyl or 6-10 membered heteroaryl.

In some embodiments of Formula I, ring E is selected frompyridyl, benzo[d]oxazolyl, 3,4-dihydro-2H-benzo[b][1,4]oxazinyl, 1,6-naphthyridinyl, 2,3-dihydro- 1H-pyrido[2,3-b][1,4]oxazinyl, 1H-pyrazolo[4,3-b]pyridyl, 2H-pyrazolo[4,3-b]pyridyl, 1H-pyrazolo[3,4-b]pyridyl, pyrazolo[1,5-a]pyrimidinyl, imidazo[1,2-b]pyridazinyl, thiazolo[5,4-b] pyridyl or 1,5-naphthyridinyl.

In some embodiments of Formula I, each $R_E$ is the same or different, and is independently selected from the group consisting of hydrogen, halogen, oxo, CN, $-C_{1-6}$ alkyl, $-C_{0-6}$ alkyl -5-10 membered heteroaryl, $-C_{0-6}$ alkyl $-C_{3-8}$ carbocyclyl, $-C_{0-6}$ alkyl -3-8 membered heterocyclyl, $-OR_5$, $-NR_5R_6$, $-SR_5$, $-C(O)R_5$, $-S(O)R_5$, $-S(O)_2R_5$ and $-C(O)OR_5$; wherein the $-C_{1-6}$ alkyl, $-C_{0-6}$ alkyl -5-10 membered heteroaryl, $-C_{0-6}$ alkyl $-C_{3-8}$ carbocyclyl and $-C_{0-6}$ alkyl -3-8 membered heterocyclyl are optionally substituted with one or more substituents selected from hydrogen, halogen, CN, oxo, - $C_{1-6}$ alkyl, $-C_{2-6}$ alkenyl, $-C_{2-6}$ alkynyl, $-OR_8$, $-NR_8R_9$, $-C_{0-6}$ alkyl $-C_{3-8}$ carbocyclyl and $-C_{0-6}$ alkyl -3-8 membered heterocyclyl, $-C(O)OR_8$ or $-C(O)NR_8R_9$..

In some embodiments of Formula I, each $R_E$ is the same or different, is independently selected from the group consisting of hydrogen, oxo,-F, -Cl, -CN, $-CH_3$, $-OCH_3$, $-CH_2OH$, $-CH_2CN$, $-CF_3$, $-C(O)NH_2$, $-C(O)NHCH_3$, $-C(O)NHCH_2CN$, - $C(O)NHCH_2CH_3$, $-C(O)NHCH_2CF_3$, $-C(O)NHCH_2CH_2OCF_3$, $-C(O)NHCH_2CH_2CH_3$, $-OCHF_2$, $-OCF_3$, $-SO_2CH_3$, $-SCH_3$, $-SCF_3$, $-NHC(O)NH_2$, $-NHC(O)CH_3$, - $NHC(O)OCH_3$, $-NHS(O)_2CH_3$,

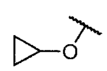, 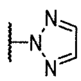, 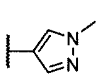, 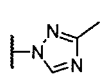, 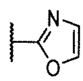, 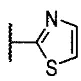, 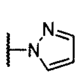,

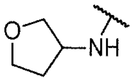, 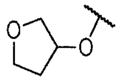, 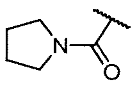, 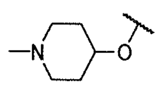, 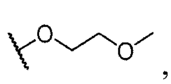, 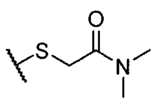, 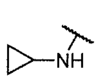,

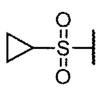, 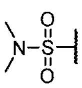, 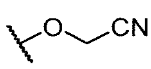, 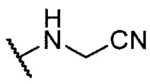, 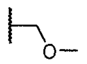, 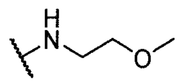, 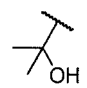, 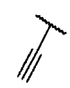,

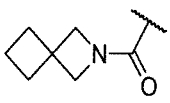, 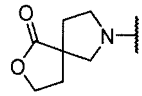, 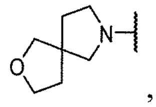, 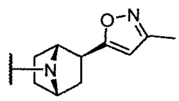, 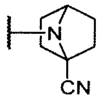, 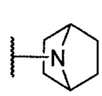, 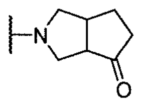,

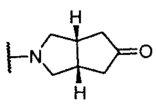, 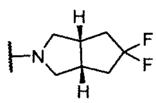, 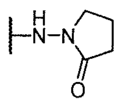, 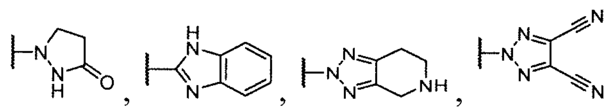,

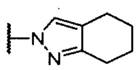, 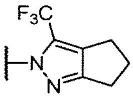, 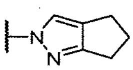, 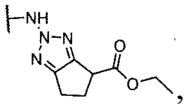, 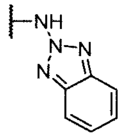, 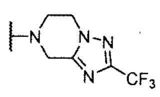, 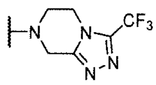,

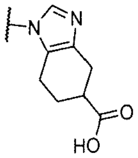, 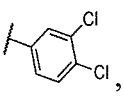, 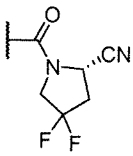, 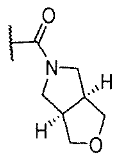, 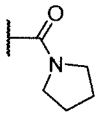, 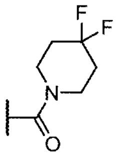, 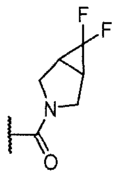, 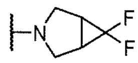,

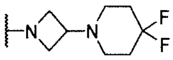, 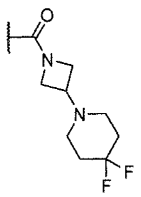, 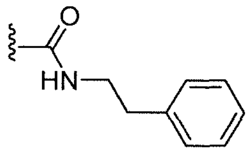, 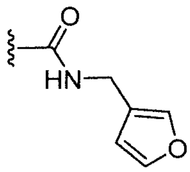, 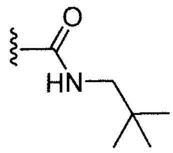,

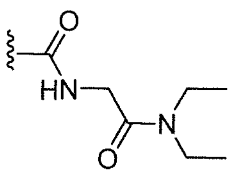, 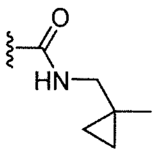, 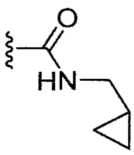, 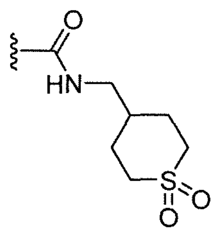, 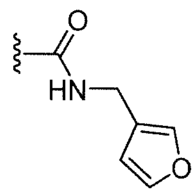,

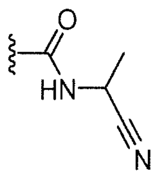, 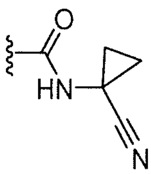, 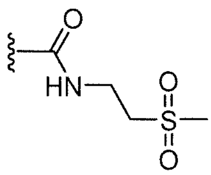, 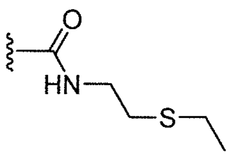 and 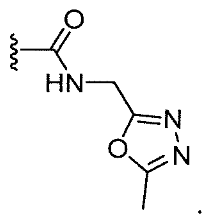.

In some embodiments of Formula I,

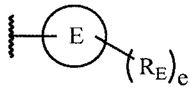

is selected from

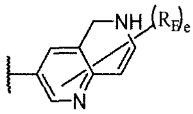, 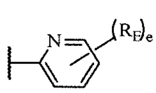, 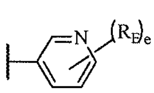, 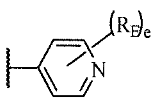, 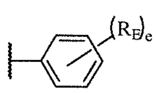, 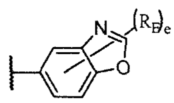,

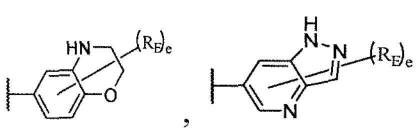, 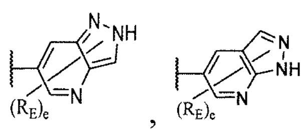, 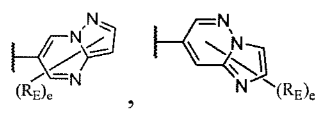,

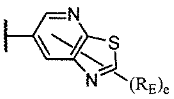, 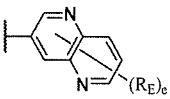 or 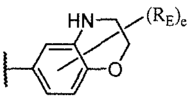 ; preferably, 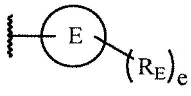 is selected from

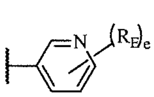, 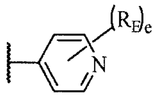

or

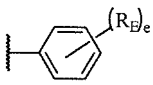;

more preferably,

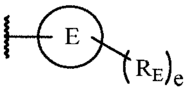 is

selected from

, 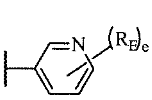 or 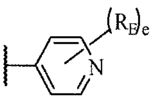.

Wherein, $R_E$ and e are defined as described in any embodiment of the present invention.

In some embodiments of Formula I,

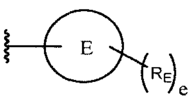

is selected from

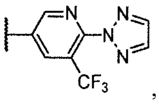,

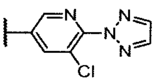, 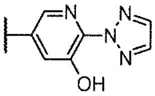, 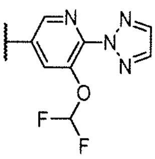, 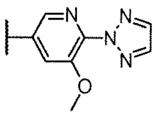, 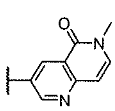, 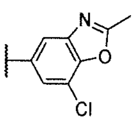, 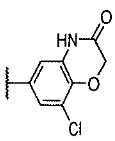,

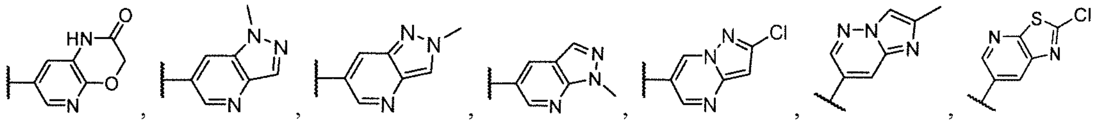
, , , , , , ,

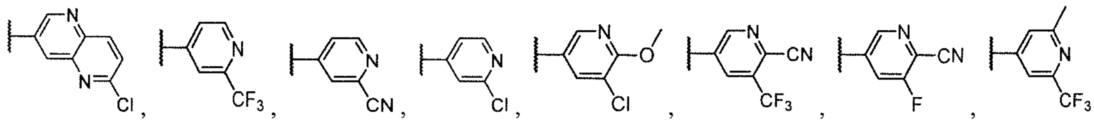
, , , , , , ,

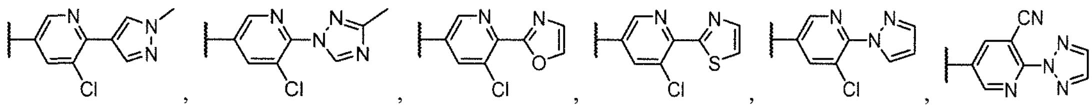
, , , , , ,

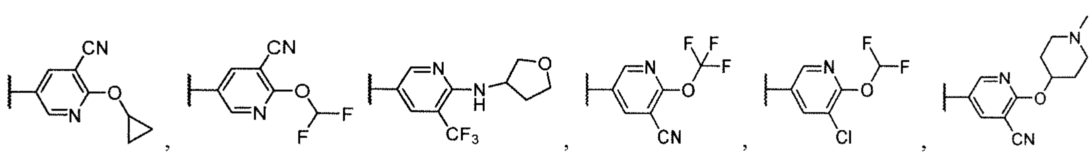
, , , , , ,

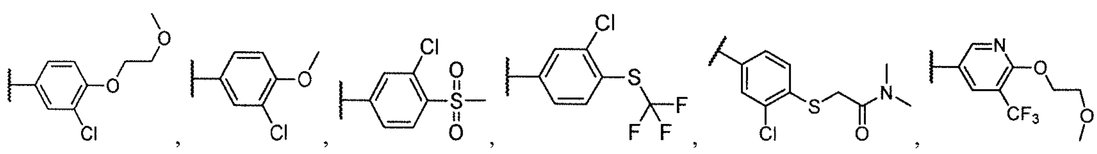
, , , , , ,

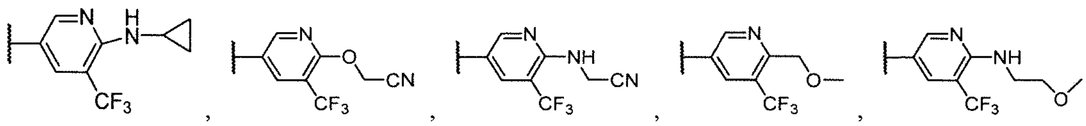
, , , , ,

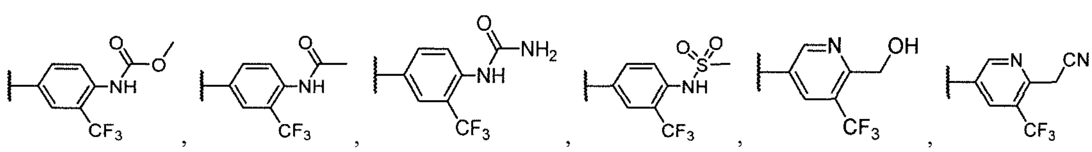
, , , , , ,

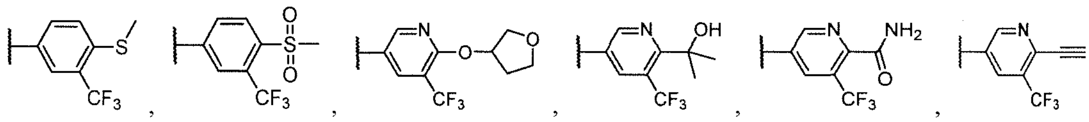
, , , , , ,

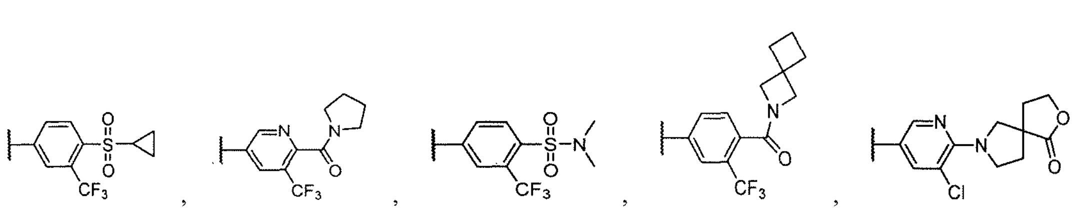
, , , , ,

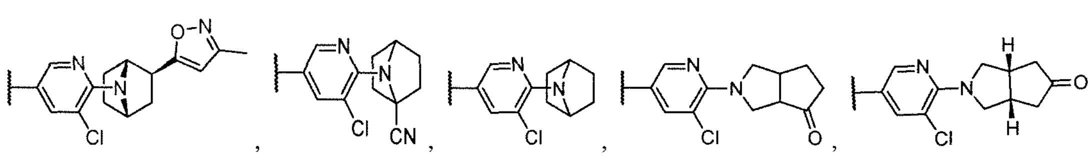
, , , , ,

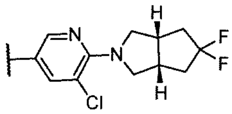, 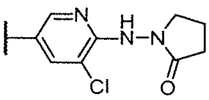, 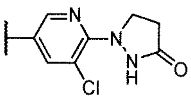, 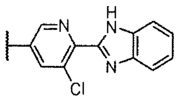, 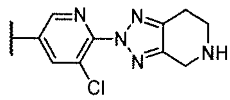,

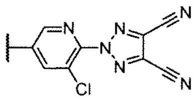, 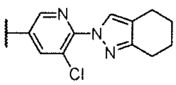, 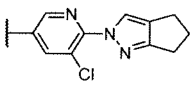, 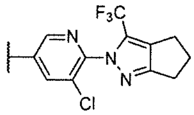, 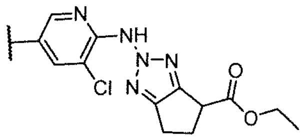,

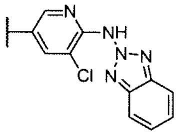, 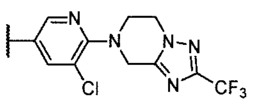, 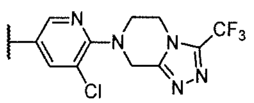, 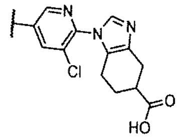,

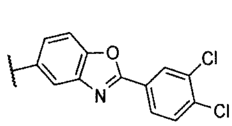, 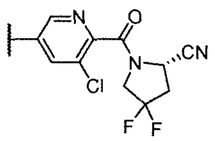, 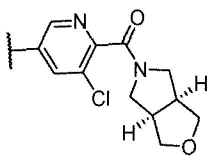, 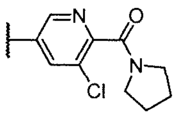, 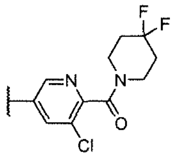,

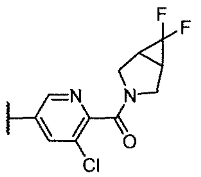, 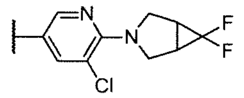, 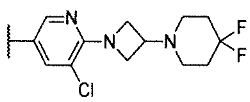, 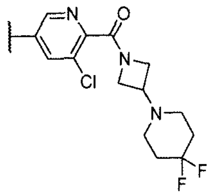,

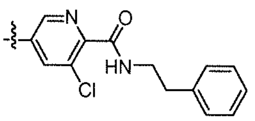, 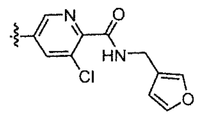, 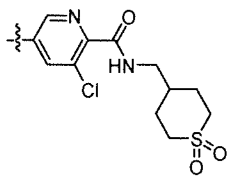, 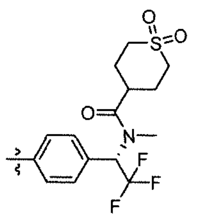,

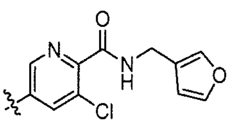, 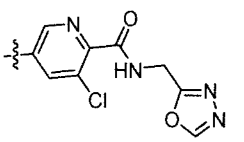, 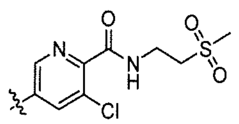, 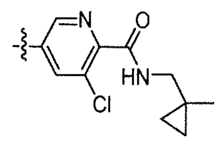,

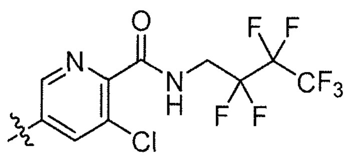 or 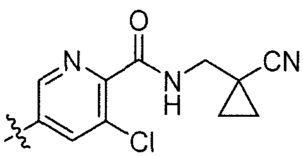.

In some embodiments of Formula I, the aforementioned -$C_{3-8}$ carbocyclyl, 3-8 membered heterocyclyl can be respectively preferably -$C_{3-6}$ carbocyclyl, 3-6 membered heterocyclyl.

In some embodiments, the compound of Formula I is selected from Formula II:

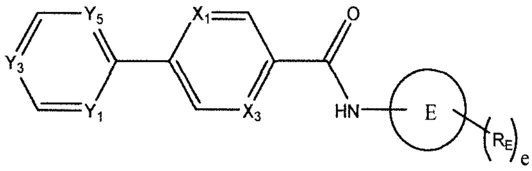

Formula II

wherein, ring E, $X_1$, $X_3$, $Y_1$, $Y_3$, $Y_5$, $R_E$, e are defined as described in any embodiment of the invention.

In some embodiments, the compound of Formula I is selected from III:

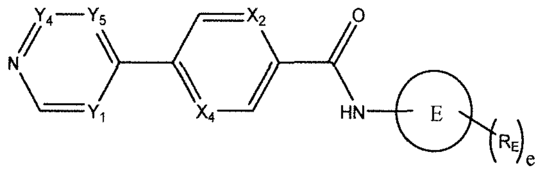

Formula III

wherein, ring E, $X_2$, $X_4$, $Y_1$, $Y_4$, $Y_5$, $R_E$, e are defined as described in any embodiment of the invention.

In some embodiments of Formula I, the compound is selected from:

N-(6-(2H-1,2,3-triazol-2-yl)-5-(trifluoromethyl)pyridin-3-yl)-5-(2-chloro-4-fluorophenyl)-4-methylpicolinamide;
N-(6-(2H-1,2,3-triazol-2-yl)-5-(trifluoromethyl)pyridin-3-yl)-2-fluoro-4-(3-methylpyridin-2-yl)benzamide;
N-(6-(2H-1,2,3-triazol-2-yl)-5-(trifluoromethyl)pyridin-3-yl)-2'-chloro-4'-fluoro-3-isopropyl-[1,1'-biphenyl]-4-carboxamide;
N-(6-(2H-1,2,3-triazol-2-yl)-5-(trifluoromethyl)pyridin-3-yl)-3,5'-dimethyl-[2,3'-bipyridine]-6'-carboxamide;
N-(6-(2H-1,2,3-triazol-2-yl)-5-(trifluoromethyl)pyridin-3-yl)-2-methoxy-4-(3-methylpyridin-2-yl)benzamide;
N-(6-(2H-1,2,3-triazol-2-yl)-5-(trifluoromethyl)pyridin-3-yl)-2'-chloro-4'-fluoro-3-methoxy-[1,1'-biphenyl]-4-carboxamide;
N-(6-(2H-1,2,3-triazol-2-yl)-5-(trifluoromethyl)pyridin-3-yl)-6-(2-chloro-4-fluorophenyl)-2-methoxynicotinamide;
N-(6-(2H-1,2,3-triazol-2-yl)-5-(trifluoromethyl)pyridin-3-yl)-2-(2-chloro-4-fluorophenyl)-4-methylpyrimidine-5-carboxamide;
N-(6-(2H-1,2,3-triazol-2-yl)-5-(trifluoromethyl)pyridin-3-yl)-4-methyl-2-(4-methylpyridin-3-yl)pyrimidine-5-carboxamide;
N-(6-(2H-1,2,3-triazol-2-yl)-5-(trifluoromethyl)pyridin-3-yl)-2'-chloro-4'-fluoro-3-methyl-[1,1'-biphenyl]-4-carboxamide;
N-(6-(2H-1,2,3-triazol-2-yl)-5-(trifluoromethyl)pyridin-3-yl)-2',3-dichloro-4'-fluoro-[1,1 '-biphenyl]-4-carboxamide;
N-(6-(2H-1,2,3-triazol-2-yl)-5-(trifluoromethyl)pyridin-3-yl)-4-chloro-6-(2-chloro-4-fluorophenyl)nicotinamide;
N-(6-(2H-1,2,3-triazol-2-yl)-5-(trifluoromethyl)pyridin-3-yl)-2'-chloro-3-cyclopropyl-4'-fluoro-[1,1'-biphenyl]-4-carboxamide;
N-(6-(2H-1,2,3-triazol-2-yl)-5-(trifluoromethyl)pyridin-3-yl)-2-(4-cyano-2-methylphenyl)-4-methylpyrimidine-5-carboxamide;
N-(6-(2H-1,2,3-triazol-2-yl)-5-(trifluoromethyl)pyridin-3-yl)-5-(2-chloro-4-fluorophenyl)-3-methylpicolinamide;
N-(6-(2H-1,2,3-triazol-2-yl)-5-(trifluoromethyl)pyridin-3-yl)-2-(2-chloro-4-fluorophenyl)-4-(trifluoromethyl)pyrimidine-5-carboxamide;
N-(6-(2H-1,2,3-triazol-2-yl)-5-(trifluoromethyl)pyridin-3-yl)-2'-chloro-3,4'-difluoro-[1,1'-biphenyl]-4-carboxamide;
N-(6-(2H-1,2,3-triazol-2-yl)-5-(trifluoromethyl)pyridin-3-yl)-4-methyl-2-(2-(trifluoromethyl)phenyl)pyrimidine-5-carboxamide;
N-(6-(2H-1,2,3-triazol-2-yl)-5-(trifluoromethyl)pyridin-3-yl)-6-(2-chloro-4-fluorophenyl)-5-methylnicotinamide;
2-(2-chloro-4-fluorophenyl)-N-(5-chloro-6-(2H-1,2,3-triazol-2-yl)pyridin-3-yl)-4-methylpyrimidine-5-carboxamide;
N-(6-(2H-1,2,3-triazol-2-yl)-5-(trifluoromethyl)pyridin-3-yl)-6-(5-amino-2-chlorophenyl)-2-methoxynicotinamide;
N-(6-(2H-1,2,3-triazol-2-yl)-5-(trifluoromethyl)pyridin-3-yl)-6-methoxy-3'-methyl-[2,2'-bipyridine]-5-carboxamide;
N-(6-(2H-1,2,3-triazol-2-yl)-5-(trifluoromethyl)pyridin-3-yl)-6-(2-chloro-4-fluorophenyl)-4-methoxynicotinamide;
3-chloro-N-(5-chloro-6-(2H-1,2,3-triazol-2-yl)pyridin-3-yl)-2'-fluoro-6'-methyl-[1,1'-biphenyl]-4-carboxamide;
3,3'-dichloro-N-(5-chloro-6-(2H-1,2,3-triazol-2-yl)pyridin-3-yl)-2'-methyl-[1,1'-biphenyl]-4-carboxamide;

5-(3-chloro-2-methylphenyl)-N-(5-chloro-6-(2H-1,2,3-triazol-2-yl)pyridin-3-yl)-3-methylpicolinamide;
5-(2-chloro-4-fluorophenyl)-N-(5-chloro-6-(2H-1,2,3-triazol-2-yl)pyridin-3-yl)-3-methylpicolinamide;
N-(6-(2H-1,2,3-triazol-2-yl)-5-(trifluoromethyl)pyridin-3-yl)-2'-chloro-3-methyl-[1,1'-biphenyl]-4-carboxamide;
N-(6-(2H-1,2,3-triazol-2-yl)-5-(trifluoromethyl)pyridin-3-yl)-2'-amino-3-methyl-[1,1'-biphenyl]-4-carboxamide;
N-(6-(2H-1,2,3-triazol-2-yl)-5-(trifluoromethyl)pyridin-3-yl)-4'-chloro-3-methyl-[1,1'-biphenyl]-4-carboxamide;
N-(6-(2H-1,2,3-triazol-2-yl)-5-(trifluoromethyl)pyridin-3-yl)-2'-chloro-3,3'-dimethyl-[1,1'-biphenyl]-4-carboxamide;
N-(6-(2H-1,2,3-triazol-2-yl)-5-(trifluoromethyl)pyridin-3-yl)-2',3'-dichloro-3-methyl-[1,1'-biphenyl]-4-carboxamide;
N-(6-(2H-1,2,3-triazol-2-yl)-5-(trifluoromethyl)pyridin-3-yl)-2'-fluoro-3,6'-dimethyl-[1,1'-biphenyl]-4-carboxamide;
N4'-(6-(2H-1,2,3-triazol-2-yl)-5-(trifluoromethyl)pyridin-3-yl)-3'-methyl-[1,1'-biphenyl]-2,4'-dicarboxamide;
2-chloro-N-(5-chloro-6-(2H-1,2,3-triazol-2-yl)pyridin-3-yl)-4-(3-methylpyridin-4-yl)benzamide;
2',3-dichloro-N-(5-chloro-6-(2H-1,2,3-triazol-2-yl)pyridin-3-yl)-[1,1'-biphenyl]-4-carboxamide;
3-chloro-N-(5-chloro-6-(2H-1,2,3-triazol-2-yl)pyridin-3-yl)-2'-(trifluoromethoxy)-[1,1'-biphenyl] -4-carboxamide;
3-chloro-N-(5-chloro-6-(2H-1,2,3-triazol-2-yl)pyridin-3-yl)-3'-cyano-[1,1'-biphenyl]-4-carboxamide;
N-(5-chloro-6-(2H-1,2,3-triazol-2-yl)pyridin-3-yl)-5-(3-chlorophenyl)-3-methylpicolinamide;
N-(6-(2H-1,2,3-triazol-2-yl)-5-(trifluoromethyl)pyridin-3-yl)-3'-chloro-3-methyl-[1,1'-biphenyl]-4-carboxamide;
N-(6-(2H-1,2,3-triazol-2-yl)-5-(trifluoromethyl)pyridin-3-yl)-2'-cyano-3-methyl-[1,1'-biphenyl]-4-carboxamide;
N-(6-(2H-1,2,3-triazol-2-yl)-5-(trifluoromethyl)pyridin-3-yl)-2'-fluoro-3-methyl-4'-(trifluoromethyl)-[1,1'-biphenyl]-4-carboxamide;
N-(6-(2H-1,2,3-triazol-2-yl)-5-(trifluoromethyl)pyridin-3-yl)-2-methyl-4-(6-(trifluoromethyl)pyridin-3-yl)benzamide;
N-(6-(2H-1,2,3-triazol-2-yl)-5-(trifluoromethyl)pyridin-3-yl)-4-(furan-2-yl)-2-methylbenzamide;
N-(6-(2H-1,2,3-triazol-2-yl)-5-(trifluoromethyl)pyridin-3-yl)-4-(cyclopent-1-en-1-yl)-2-methylbenzamide;
N-(6-(2H-1,2,3-triazol-2-yl)-5-(trifluoromethyl)pyridin-3-yl)-5-(2-cyanophenyl)-3-methylpicolinamide;
N-(6-(2H-1,2,3-triazol-2-yl)-5-(trifluoromethyl)pyridin-3-yl)-2-methyl-4-(1-methyl-1H-pyrazol-5-yl)benzamide;
N-(6-(2H-1,2,3-triazol-2-yl)-5-(trifluoromethyl)pyridin-3-yl)-2'-chloro-3-cyano-[1,1'-biphenyl]-4-carboxamide;
N-(6-(2H-1,2,3-triazol-2-yl)-5-(trifluoromethyl)pyridin-3-yl)-4'-chloro-3'-cyano-3-methyl-[1,1'-biphenyl]-4-carboxamide;
N-(6-(2H-1,2,3-triazol-2-yl)-5-(trifluoromethyl)pyridin-3-yl)-2-methyl-4-(4-methylpyridin-3-yl)benzamide;
N-(6-(2H-1,2,3-triazol-2-yl)-5-(trifluoromethyl)pyridin-3-yl)-2-methyl-4-(1-oxoisoindolin-5 -yl)benzamide;
N-(6-(2H-1,2,3-triazol-2-yl)-5-(trifluoromethyl)pyridin-3-yl)-2-methyl-4-(quinolin-4-yl)benzamide;
N4'-(6-(2H-1,2,3-triazol-2-yl)-5-(trifluoromethyl)pyridin-3-yl)-3'-methyl-[1,1'-biphenyl]-3,4'-dicarboxamide;
N-(6-(2H-1,2,3-triazol-2-yl)-5-(trifluoromethyl)pyridin-3-yl)-2'-methoxy-3-(trifluoromethyl)-[1,1'-biphenyl]-4-carboxamide;
N-(6-(2H-1,2,3-triazol-2-yl)-5-(trifluoromethyl)pyridin-3-yl)-2-methyl-4-(1H-pyrrolo[2,3-b]pyridin-5-yl)benzamide;
N-(6-(2H-1,2,3-triazol-2-yl)-5-(trifluoromethyl)pyridin-3-yl)-2'-methoxy-3-methyl-[1,1'-biphenyl]-4-carboxamide;
N-(6-(2H-1,2,3-triazol-2-yl)-5-(trifluoromethyl)pyridin-3-yl)-4-(6-fluoropyridin-3-yl)-2-methylbenzamide;
N-(6-(2H-1,2,3-triazol-2-yl)-5-(trifluoromethyl)pyridin-3-yl)-4-(6-cyanopyridin-3-yl)-2-methylbenzamide;
N-(6-(2H-1,2,3-triazol-2-yl)-5-(trifluoromethyl)pyridin-3-yl)-5-chloro-2-fluoro-2'-methoxy-[1,1'-biphenyl]-4-carboxamide;
N-(6-(2H-1,2,3-triazol-2-yl)-5-(trifluoromethyl)pyridin-3-yl)-2'-amino-3-(trifluoromethyl)-[1,1'-biphenyl]-4-carboxamide;
N-(6-(2H-1,2,3-triazol-2-yl)-5-(trifluoromethyl)pyridin-3-yl)-2'-amino-5-chloro-2-fluoro-[1,1'-biphenyl]-4-carboxamide;
N-(6-(2H-1,2,3-triazol-2-yl)-5-(trifluoromethyl)pyridin-3-yl)-2'-acetamido-3-(trifluoromethyl)-[1,1'-biphenyl]-4-carboxamide;
N-(6-(2H-1,2,3-triazol-2-yl)-5-(trifluoromethyl)pyridin-3-yl)-4-(4-chloropyridin-3-yl)-2-(trifluoromethyl)benzamide;
N-(6-(2H-1,2,3-triazol-2-yl)-5-(trifluoromethyl)pyridin-3-yl)-2'-(dimethylamino)-3-(trifluoromethyl)-[1,1'-biphenyl]-4-carboxamide;
N-(6-(2H-1,2,3-triazol-2-yl)-5-(trifluoromethyl)pyridin-3-yl)-2'-(dimethylamino)-3-methyl-[1,1'-biphenyl]-4-carboxamide;
N-(6-(2H-1,2,3-triazol-2-yl)-5-(trifluoromethyl)pyridin-3-yl)-3-chloro-2'-(dimethylamino)-[1,1'-biphenyl]-4-carboxamide;
N-(6-(2H-1,2,3-triazol-2-yl)-5-(trifluoromethyl)pyridin-3-yl)-3-cyano-2'-(dimethylamino-[1,1'-biphenyl]-4-carboxamide;
N-(6-(2H-1,2,3-triazol-2-yl)-5-(trifluoromethyl)pyridin-3-yl)-5-chloro-2'-(dimethylamino)-2-fluoro-[1,1'-biphenyl]-4-carboxamide;
N-(5-chloro-6-(2H-1,2,3-triazol-2-yl)pyridin-3-yl)-4',5-dimethyl-[3,3'-bipyridine]-6-carboxamide;
3'-chloro-N4'-(5-chloro-6-(2H-1,2,3-triazol-2-yl)pyridin-3-yl)-[1,1'-biphenyl]-3,4'-dicarboxamide;
2-chloro-N-(5-chloro-6-(2H-1,2,3-triazol-2-yl)pyridin-3-yl)-4-(1-methyl-1H-pyrazol-3-yl)benzamide;

2’-chloro-N-(5-chloro-6-(2H-1,2,3-triazol-2-yl)pyridin-3-yl)-4’-fluoro-[1,1’-biphenyl]-4-carboxamide;
2’-amino-N-(5-chloro-6-(2H-1,2,3-triazol-2-yl)pyridin-3-yl)-[1,1’-biphenyl]-4-carboxamide;
N-(6-(2H-1,2,3-triazol-2-yl)-5-(trifluoromethyl)pyridin-3-yl)-3-chloro-2’-methoxy-[1,1’-biphenyl]-4-carboxamide;
N-(6-(2H-1,2,3-triazol-2-yl)-5-(trifluoromethyl)pyridin-3-yl)-2’-amino-3-fluoro-[1,1’-biphenyl]-4-carboxamide;
N-(6-(2H-1,2,3-triazol-2-yl)-5-(trifluoromethyl)pyridin-3-yl)-2’-acetamido-3-fluoro-[1,1’-biphenyl]-4-carboxamide;
N-(6-(2H-1,2,3-triazol-2-yl)-5-(trifluoromethyl)pyridin-3-yl)-3-fluoro-2’-methoxy-[1,1’-biphenyl]-4-carboxamide;
N-(6-(2H-1,2,3-triazol-2-yl)-5-(trifluoromethyl)pyridin-3-yl)-3,4’-difluoro-2’-hydroxy-[1,1’-biphenyl]-4-carboxamide;
N-(6-(2H-1,2,3-triazol-2-yl)-5-(trifluoromethyl)pyridin-3-yl)-3-fluoro-2’-hydroxy-[1,1’-biphenyl]-4-carboxamide;
N-(6-(2H-1,2,3-triazol-2-yl)-5-(trifluoromethyl)pyridin-3-yl)-3-cyano-4’-fluoro-2’-hydroxy-[1,1’-biphenyl]-4-carboxamide;
N-(6-(2H-1,2,3-triazol-2-yl)-5-(trifluoromethyl)pyridin-3-yl)-3-cyano-2’-hydroxy-[1,1’-biphenyl]-4-carboxamide;
3-chloro-N-(5-chloro-6-(2H-1,2,3-triazol-2-yl)pyridin-3-yl)-4’-fluoro-2’-hydroxy-[1,1’-biphenyl]-4-carboxamide;
3-chloro-N-(5-chloro-6-(2H-1,2,3-triazol-2-yl)pyridin-3-yl)-4’-fluoro-[1,1’-biphenyl]-4-carboxamide;
2-chloro-N-(5-chloro-6-(2H-1,2,3-triazol-2-yl)pyridin-3-yl)-4-(3-chloropyridin-4-yl)benzamide;
3-chloro-N-(5-chloro-6-(2H-1,2,3-triazol-2-yl)pyridin-3-yl)-2’,4’-difluoro-[1,1’-biphenyl]-4-carboxamide;
2’-bromo-3-chloro-N-(5-chloro-6-(2H-1,2,3-triazol-2-yl)pyridin-3-yl)-4’-fluoro-[1,1’-biphenyl]-4-carboxamide;
3-chloro-N-(5-chloro-6-(2H-1,2,3-triazol-2-yl)pyridin-3-yl)-4’-fluoro-2’-(methylthio)-[1,1’-biphenyl]-4-carboxamide;
3-chloro-N-(5-chloro-6-(2H-1,2,3-triazol-2-yl)pyridin-3-yl)-4’-fluoro-2’-methoxy-[1,1’-biphenyl]-4-carboxamide;
N-(6-(2H-1,2,3-triazol-2-yl)-5-(trifluoromethyl)pyridin-3-yl)-2’,5-dichloro-2,4’-difluoro-[1,1’-biphenyl]-4-carboxamide;
N-(6-(2H-1,2,3-triazol-2-yl)-5-(trifluoromethyl)pyridin-3-yl)-2’,5-dichloro-2-fluoro-[1,1’-biphenyl]-4-carboxamide;
2’-amino-5-chloro-N-(5-chloro-6-(2H-1,2,3-triazol-2-yl)pyridin-3-yl)-2-fluoro-[1,1’-biphenyl]-4-carboxamide;
N-(6-(2H-1,2,3-triazol-2-yl)-5-(trifluoromethyl)pyridin-3-yl)-2’-amino-2-chloro-5-fluoro-[1,1’-biphenyl]-4-carboxamide;
N-(6-(2H-1,2,3-triazol-2-yl)-5-(trifluoromethyl)pyridin-3-yl)-2’-amino-2,5-dichloro-[1,1’-biphenyl]-4-carboxamide;
N-(6-(2H-1,2,3-triazol-2-yl)-5-(trifluoromethyl)pyridin-3-yl)-5-chloro-2,4’-difluoro-2’-methoxy-[1,1’-biphenyl]-4-carboxamide;
2’-amino-N-(5-chloro-6-(2H-1,2,3-triazol-2-yl)pyridin-3-yl)-2,4’-difluoro-5-methyl-[1,1’-biphenyl]-4-carboxamide;
2’-bromo-N-(5-chloro-6-(2H-1,2,3-triazol-2-yl)pyridin-3-yl)-2,4’-difluoro-5-methyl-[1,1’-biphenyl]-4-carboxamide;
2’-chloro-N-(5-chloro-6-(2H-1,2,3-triazol-2-yl)pyridin-3-yl)-2,4’-difluoro-5-methyl-[1,1’-biphenyl]-4-carboxamide;
N-(5-chloro-6-(2H-1,2,3-triazol-2-yl)pyridin-3-yl)-2,2’,4’-trifluoro-5-methyl-[1,1’-biphenyl]-4-carboxamide;
N-(5-chloro-6-(2H-1,2,3-triazol-2-yl)pyridin-3-yl)-2-fluoro-2’-methoxy-5-methyl-[1,1’-biphenyl]-4-carboxamide;
N-(5-chloro-6-(2H-1,2,3-triazol-2-yl)pyridin-3-yl)-2,4’-difluoro-2’-methoxy-5-methyl-[1,1’-biphenyl]-4-carboxamide;
2’-amino-2-chloro-N-(5-chloro-6-(2H-1,2,3-triazol-2-yl)pyridin-3-yl)-5-fluoro-[1,1’-biphenyl]-4-carboxamide;
2’-amino-2-chloro-N-(5-chloro-6-(2H-1,2,3-triazol-2-yl)pyridin-3-yl)-4’,5-difluoro-[1,1’-biphenyl]-4-carboxamide;
2’-amino-5-chloro-N-(5-chloro-6-(2H-1,2,3-triazol-2-yl)pyridin-3-yl)-2,4’-difluoro-[1,1’-biphenyl]-4-carboxamide;
N-(6-(2H-1,2,3-triazol-2-yl)-5-(trifluoromethyl)pyridin-3-yl)-2’-amino-5-chloro-2,4’-difluoro-[1,1’-biphenyl]-4-carboxamide;
N-(6-(2H-1,2,3-triazol-2-yl)-5-(trifluoromethyl)pyridin-3-yl)-2’-amino-2-chloro-4’,5-difluoro-[1,1’-biphenyl]-4-carboxamide;
4-(3-aminopyridin-4-yl)-5-chloro-N-(5-chloro-6-(2H-1,2,3-triazol-2-yl)pyridin-3-yl)-2-fluorobenzamide formate;
4-(3-aminopyridin-4-yl)-N-(5-chloro-6-(2H-1,2,3-triazol-2-yl)pyridin-3-yl)-5-fluoro-2-methylbenzamide;
N-(5-chloro-6-(2H-1,2,3-triazol-2-yl)pyridin-3-yl)-4-(3-chloropyridin-4-yl)-5-fluoro-2-methylbenzamide;
2-chloro-N-(5-chloro-6-(2H-1,2,3-triazol-2-yl)pyridin-3-yl)-4’,5-difluoro-2’-(methylamino)-[1,1’-biphenyl]-4-carboxamide;
N-(6-(2H-1,2,3-triazol-2-yl)-5-(trifluoromethyl)pyridin-3-yl)-4-(3-amino-5-fluoropyridin-2-yl)-2-chloro-5-fluorobenzamide;
N-(6-(2H-1,2,3-triazol-2-yl)-5-(trifluoromethyl)pyridin-3-yl)-3-chloro-2’-(difluoromethoxy)-4’-fluoro-[1,1’-biphenyl]-4-carboxamide;
4-(4-aminopyrimidin-5-yl)-2-chloro-N-(5-chloro-6-(2H-1,2,3-triazol-2-yl)pyridin-3-yl)-5-fluorobenzamide;
4-(3-aminopyridazin-4-yl)-2-chloro-N-(5-chloro-6-(2H-1,2,3-triazol-2-yl)pyridin-3-yl)-5-fluorobenzamide;
2-chloro-N-(5-chloro-6-(2H-1,2,3-triazol-2-yl)pyridin-3-yl)-4-(3-chloropyridazin-4-yl)-5-fluorobenzamide;
4-(6-amino-2-oxo-1,2-dihydropyrimidin-5-yl)-2-chloro-N-(5-chloro-6-(2H-1,2,3-triazol-2-yl)pyridin-3-yl)-5-fluorobenzamide;
2’-amino-N-(5-chloro-6-(2H-1,2,3-triazol-2-yl)pyridin-3-yl)-4’,5-difluoro-2-methoxy-[1,1’-biphenyl]-4-carboxamide;
4-(3-aminopyridin-4-yl)-N-(5-chloro-6-(2H-1,2,3-triazol-2-yl)pyridin-3-yl)-2-fluoro-5-methoxybenzamide;
2’-bromo-N-(5-chloro-6-(2H-1,2,3-triazol-2-yl)pyridin-3-yl)-4’,5-difluoro-2-methoxy-[1,1’-biphenyl]-4-carboxamide;
N-(6-(2H-1,2,3-triazol-2-yl)-5-(trifluoromethyl)pyridin-3-yl)-3-chloro-4’-fluoro-2’-iodo-[1,1’-biphenyl]-4-carboxamide;

N-(6-(2H-1,2,3-triazol-2-yl)-5-(trifluoromethyl)pyridin-3-yl)-2',3-dibromo-4'-fluoro-[1,1'-biphenyl]-4-carboxamide;
N-(6-(2H-1,2,3-triazol-2-yl)-5-(trifluoromethyl)pyridin-3-yl)-2'-amino-3-bromo-4'-fluoro-[1,1'-biphenyl]-4-carboxamide;
N-(6-(2H-1,2,3-triazol-2-yl)-5-(trifluoromethyl)pyridin-3-yl)-5-chloro-2,4'-difluoro-2'-iodo-[1,1'-biphenyl]-4-carboxamide;
N-(6-(2H-1,2,3-triazol-2-yl)-5-(trifluoromethyl)pyridin-3-yl)-3-bromo-4'-fluoro-2'-iodo-[1,1'-biphenyl]-4-carboxamide;
N-(6-(2H-1,2,3-triazol-2-yl)-5-(trifluoromethyl)pyridin-3-yl)-2'-amino-5-bromo-2,4'-difluoro-[1,1'-biphenyl]-4-carboxamide;
N-(6-(2H-1,2,3-triazol-2-yl)-5-(trifluoromethyl)pyridin-3-yl)-2-(2-amino-4-fluorophenyl)-4-methylpyrimidine-5-carboxamide;
N-(6-(2H-1,2,3-triazol-2-yl)-5-(trifluoromethyl)pyridin-3-yl)-5-bromo-2,4'-difluoro-2'-iodo-[1,1'-biphenyl]-4-carboxamide;
N-(6-(2H-1,2,3-triazol-2-yl)-5-(trifluoromethyl)pyridin-3-yl)-2'-amino-4'-fluoro-3-iodo-[1,1'-biphenyl]-4-carboxamide;
N-(6-(2H-1,2,3-triazol-2-yl)-5-(trifluoromethyl)pyridin-3-yl)-2'-bromo-5-chloro-2,4'-difluoro-[1,1'-biphenyl]-4-carboxamide;
N-(6-(2H-1,2,3-triazol-2-yl)-5-(trifluoromethyl)pyridin-3-yl)-2,4'-difluoro-2',5-diiodo-[1,1'-biphenyl]-4-carboxamide;
N-(6-(2H-1,2,3-triazol-2-yl)-5-(trifluoromethyl)pyridin-3-yl)-2'-amino-2,4'-difluoro-5-iodo-[1,1 '-biphenyl]-4-carboxamide;
N-(6-(2H-1,2,3-triazol-2-yl)-5-(trifluoromethyl)pyridin-3-yl)-2'-amino-2,4',5-trifluoro-[1,1'-biphenyl]-4-carboxamide;
N-(6-(2H-1,2,3-triazol-2-yl)-5-(trifluoromethyl)pyridin-3-yl)-2'-chloro-2,4',5-trifluoro-[1,1'-biphenyl]-4-carboxamide;
N-(6-(2H-1,2,3-triazol-2-yl)-5-(trifluoromethyl)pyridin-3-yl)-4-(3-aminopyridin-4-yl)-2,5-difluorobenzamide;
N-(6-(2H-1,2,3-triazol-2-yl)-5-(trifluoromethyl)pyridin-3-yl)-2'-amino-2-chloro-4',5-difluoro-[1,1'-biphenyl]-4-carboxamide;
2'-amino-2-bromo-N-(5-chloro-6-(2H-1,2,3-triazol-2-yl)pyridin-3-yl)-4',5-difluoro-[1,1'-biphenyl]-4-carboxamide;
2'-bromo-2-chloro-N-(5-chloro-6-(2H-1,2,3-triazol-2-yl)pyridin-3-yl)-4',5-difluoro-[1,1'-biphenyl]-4-carboxamide;
2'-amino-N-(5-chloro-6-(2H-1,2,3-triazol-2-yl)pyridin-3-yl)-4',5-difluoro-2-iodo-[1,1'-biphenyl]-4-carboxamide;
2-chloro-N-(5-chloro-6-(2H-1,2,3-triazol-2-yl)pyridin-3-yl)-4',5-difluoro-2'-iodo-[1,1'-biphenyl]-4-carboxamide;
N-(6-(2H-1,2,3-triazol-2-yl)-5-(trifluoromethyl)pyridin-3-yl)-4-(3-bromopyridin-4-yl)-2-chlorobenzamide;
N-(6-(2H-1,2,3-triazol-2-yl)-5-(trifluoromethyl)pyridin-3-yl)-4-(3-aminopyridin-4-yl)-2-bromo-5-fluorobenzamide;
N-(6-(2H-1,2,3-triazol-2-yl)-5-(trifluoromethyl)pyridin-3-yl)-4-(3-bromopyridin-4-yl)-2-chloro-5-fluorobenzamide;
N-(6-(2H-1,2,3-triazol-2-yl)-5-(trifluoromethyl)pyridin-3-yl)-4-(3-aminopyridin-4-yl)-5-fluoro-2-iodobenzamide;
N-(6-(2H-1,2,3-triazol-2-yl)-5-(trifluoromethyl)pyridin-3-yl)-2-chloro-5-fluoro-4-(3-iodopyridin-4-yl)benzamide;
N-(6-(2H-1,2,3-triazol-2-yl)-5-(trifluoromethyl)pyridin-3-yl)-4-(3-aminopyridin-4-yl)-5-fluoro-2-methylbenzamide;
N-(6-(2H-1,2,3-triazol-2-yl)-5-(trifluoromethyl)pyridin-3-yl)-4-(3-aminopyridin-4-yl)-2-chloro-5-fluorobenzamide;
N-(6-(2H-1,2,3-triazol-2-yl)-5-(trifluoromethyl)pyridin-3-yl)-2-chloro-5-fluoro-4-(3-methoxypyridin-4-yl)benzamide;
N-(6-(2H-1,2,3-triazol-2-yl)-5-(trifluoromethyl)pyridin-3-yl)-4-(3-chloropyridin-4-yl)-2,5-difluorobenzamide;
N-(6-(2H-1,2,3-triazol-2-yl)-5-(trifluoromethyl)pyridin-3-yl)-2-chloro-4-(3-chloropyridin-4-yl)-5-fluorobenzamide;
2,2'-dibromo-N-(5-chloro-6-(2H-1,2,3-triazol-2-yl)pyridin-3-yl)-4',5-difluoro-[1,1'-biphenyl]-4-carboxamide;
4-(3-aminopyridin-4-yl)-5-chloro-N-(5-chloro-6-(2H-1,2,3-triazol-2-yl)pyridin-3-yl)-2-fluorobenzamide;
4-(3-bromopyridin-4-yl)-5-chloro-N-(5-chloro-6-(2H-1,2,3-triazol-2-yl)pyridin-3-yl)-2-fluorobenzamide;
5-chloro-N-(5-chloro-6-(2H-1,2,3-triazol-2-yl)pyridin-3-yl)-4-(3-chloropyridin-4-yl)-2-fluorobenzamide;
(S)-4-(3-aminopyridin-4-yl)-N-(5-chloro-6-(2H-1,2,3-triazol-2-yl)pyridin-3-yl)-2- fluoro-5-(1-methoxyethyl)benzamide;
(S)-4-(3-amino-5-fluoropyridin-2-yl)-N-(5-chloro-6-(2H-1,2,3-triazol-2-yl)pyridin-3-yl)-2-fluoro-5-(1-methoxyethyl)benzamide;
2'-amino-N-(5-chloro-6-(2H-1,2,3-triazol-2-yl)pyridin-3-yl)-4',5-difluoro-2-isopropoxy-[1,1 '-biphenyl]-4-carboxamide;
2'-bromo-N-(5-chloro-6-(2H-1,2,3-triazol-2-yl)pyridin-3-yl)-4',5-difluoro-2-isopropoxy-1,1'-biphenyl]-4-carboxamide;
4-(3-aminopyridin-4-yl)-N-(5-chloro-6-(2H-1,2,3-triazol-2-yl)pyridin-3-yl)-2-fluoro-5 -methylbenzamide;
4-(3-aminopyridin-4-yl)-N-(5-chloro-6-(2H-1,2,3-triazol-2-yl)pyridin-3-yl)-2-fluoro-5-isopropylbenzamide;
2'-amino-N-(5-chloro-6-(2H-1,2,3-triazol-2-yl)pyridin-3-yl)-4',5-difluoro-2-isobutyl-[1,1'-biphenyl]-4-carboxamide;
2'-bromo-N-(5-chloro-6-(2H-1,2,3-triazol-2-yl)pyridin-3-yl)-4',5-difluoro-2-isobutyl-[1,1'-biphenyl]-4-carboxamide;
4-(3-aminopyridin-4-yl)-N-(5-chloro-6-(2H-1,2,3-triazol-2-yl)pyridin-3-yl)-5-cyclopropyl-2-fluorobenzamide;
4-(3-aminopyridin-4-yl)-N-(5-chloro-6-(2H-1,2,3-triazol-2-yl)pyridin-3-yl)-2-fluoro-5-isopropoxybenzamide;
2'-amino-N-(5-chloro-6-(2H-1,2,3-triazol-2-yl)pyridin-3-yl)-2-cyclopropyl-4',5-difluoro-[1,1'-biphenyl]-4-carboxamide;

2'-bromo-N-(5-chloro-6-(2H-1,2,3-triazol-2-yl)pyridin-3-yl)-2-cyclopropyl-4',5-difluoro-[1,1'-biphenyl]-4-carboxamide;
4-(3-aminopyridin-4-yl)-N-(5-chloro-6-(2H-1,2,3-triazol-2-yl)pyridin-3-yl)-2-fluoro-5-(tetrahydro-2H-pyran-4-yl)benzamide;
4-(3-aminopyridin-4-yl)-N-(5-chloro-6-(2H-1,2,3-triazol-2-yl)pyridin-3-yl)-5-ethynyl-2-fluorobenzamide;
2'-amino-N-(5-chloro-6-(2H-1,2,3-triazol-2-yl)pyridin-3-yl)-2-ethynyl-4',5-difluoro-[1,1'-biphenyl]-4-carboxamide;
2'-bromo-N-(5-chloro-6-(2H-1,2,3-triazol-2-yl)pyridin-3-yl)-2-ethynyl-4',5-difluoro-[1,1'-biphenyl]-4-carboxamide;
4-(3-aminopyridin-4-yl)-5-(tert-butyl)-N-(5-chloro-6-(2H-1,2,3-triazol-2-yl)pyridin-3-yl)-2-fluorobenzamide;
4-(3-aminopyridin-4-yl)-N-(5-chloro-6-(2H-1,2,3-triazol-2-yl)pyridin-3-yl)-2-fluoro-5 -isobutylbenzamide;
2'-bromo-N-(5-chloro-6-(2H-1,2,3-triazol-2-yl)pyridin-3-yl)-4',5-difluoro-2-(oxetan-3-yl)-[1,1'-biphenyl]-4-carboxamide;
4-(3-aminopyridin-4-yl)-N-(5-chloro-6-(2H-1,2,3-triazol-2-yl)pyridin-3-yl)-2-fluoro-5-(oxetan-3-yl)benzamide;
4-(3-aminopyridin-4-yl)-N-(5-chloro-6-(2H-1,2,3-triazol-2-yl)pyridin-3-yl)-5-ethyl-2-fluorobenzamide;
2'-amino-N-(5-chloro-6-(2H-1,2,3-triazol-2-yl)pyridin-3-yl)-4',5-difluoro-2-isopropyl-[1,1 '-biphenyl]-4-carboxamide;
2'-bromo-N-(5-chloro-6-(2H-1,2,3-triazol-2-y1)pyridin-3-yl)-4',5-difluoro-2-isopropyl-[1,1 '-biphenyl]-4-carboxamide;
5-chloro-N-(5-chloro-6-(2H-1,2,3-triazol-2-yl)pyridin-3-yl)-2'-(difluoromethyl)-2,4'-difluoro-[1,1'-biphenyl]-4-carboxamide;
5-chloro-N-(5-chloro-6-(2H-1,2,3-triazol-2-yl)pyridin-3-yl)-2'-(difluoromethoxy)-2,4'-difluoro-[1,1'-biphenyl]-4-carboxamide;
2-chloro-N-(5-chloro-6-(2H-1,2,3-triazol-2-yl)pyridin-3-yl)-5-fluoro-4-(6-oxo-1,6-dihydropyridin-3-yl)benzamide;
2-chloro-N-(5-chloro-6-(2H-1,2,3-triazol-2-yl)pyridin-3-yl)-5-fluoro-4-(1-methyl-6-oxo-1,6-dihydropyridin-3-yl)benzamide;
2-chloro-N-(5-chloro-6-(2H-1,2,3-triazol-2-yl)pyridin-3-yl)-5-fluoro-4-(5-fluoro-1-methyl-6-oxo-1,6-dihydropyridin-3-yl)benzamide;
N-(5-chloro-6-(2H-1,2,3-triazol-2-yl)pyridin-3-yl)-2,4'-difluoro-2'-hydroxy-5-methyl-[1,1 '-biphenyl]-4-carboxamide;
N-(5-chloro-6-(2H-1,2,3-triazol-2-yl)pyridin-3-yl)-5-fluoro-4-(3-hydroxypyridin-4-yl)-2-methylbenzamide;
4-(5-aminopyrimidin-4-yl)-N-(5-chloro-6-(2H-1,2,3-triazol-2-yl)pyridin-3-yl)-5-fluoro-2-methylbenzamide;
2-chloro-N-(5-chloro-6-(2H-1,2,3-triazol-2-yl)pyridin-3-yl)-5-fluoro-4-(5-fluoro-3-methylpyridin-2-yl)benzamide;
5-chloro-N-(5-chloro-6-(2H-1,2,3-triazol-2-yl)pyridin-3-yl)-2'-(difluoromethyl)-2,4'-difluoro-[1,1'-biphenyl]-4-carboxamide;
2-chloro-N-(5-chloro-6-(2H-1,2,3-triazol-2-yl)pyridin-3-yl)-5-fluoro-4-(6-oxo-1,6-dihydropyridin-3-yl)benzamide;
N-(5-chloro-6-(2H-1,2,3-triazol-2-yl)pyridin-3-yl)-2,4'-difluoro-2'-hydroxy-5-methyl-[1,1'-biphenyl]-4-carboxamide;
5-chloro-N-(5-chloro-6-(2H-1,2,3-triazol-2-yl)pyridin-3-yl)-2'-cyano-2,4'-difluoro-[1,1'-biphenyl]-4-carboxamide;
5-chloro-N-(5-chloro-6-(2H-1,2,3-triazol-2-yl)pyridin-3-yl)-2'-(difluoromethoxy)-2,4'-difluoro-[1,1'-biphenyl]-4-carboxamide;
2-chloro-N-(5-chloro-6-(2H-1,2,3-triazol-2-yl)pyridin-3-yl)-5-fluoro-4-(1-methyl-6-oxo-1,6-dihydropyridin-3-yl)benzamide;
N-(5-chloro-6-(2H-1,2,3-triazol-2-yl)pyridin-3-yl)-5-fluoro-4-(3-hydroxypyridin-4-yl)-2-methylbenzamide;
2-chloro-N-(5-chloro-6-(2H-1,2,3-triazol-2-yl)pyridin-3-y1)-4-(3-cyanopyridin-4-yl)-5-fluorobenzamide;
N-(6-(2H-1,2,3-triazol-2-yl)-5-(trifluoromethyl)pyridin-3-yl)-2'-amino-5-chloro-4'-fluoro-2-(trifluoromethyl)-[1,1'-biphenyl]-4-carboxamide;
N-(6-(2H-1,2,3-triazol-2-yl)-5-(trifluoromethyl)pyridin-3-yl)-2,2',3,4',5,6-hexafluoro-[1,1'-biphenyl]-4-carboxamide;
2'-chloro-N-(5-chloro-6-(2H-1,2,3-triazol-2-yl)pyridin-3-yl)-2,3,4',5,6-pentafluoro-[1,1'-biphenyl]-4-carboxamide;
N-(6-(2H-1,2,3-triazol-2-yl)-5-(trifluoromethyl)pyridin-3-yl)-2',5-dichloro-4'-fluoro-2-(trifluoromethyl)-[1,1'-biphenyl]-4-carboxamide;
N-(6-(2H-1,2,3-triazol-2-yl)-5-(trifluoromethyl)pyridin-3-yl)-5-chloro-2',4'-difluoro-2-(trifluoromethyl)-[1,1'-biphenyl]-4-carboxamide;
2'-amino-5-chloro-N-(5-chloro-6-(2H-1,2,3-triazol-2-yl)pyridin-3-yl)-2-(trifluoromethyl)-[1,1'-biphenyl]-4-carboxamide;
5-chloro-N-(5-chloro-6-(2H-1,2,3-triazol-2-yl)pyridin-3-yl)-2,2',4'-trifluoro-[1,1'-biphenyl]-4-carboxamide;
2'-bromo-5-chloro-N-(5-chloro-6-(2H-1,2,3-triazol-2-yl)pyridin-3-yl)-2,4'-difluoro-[1,1'-biphenyl]-4-carboxamide;
2'-amino-5-chloro-N-(5-chloro-6-(2H-1,2,3-triazol-2-yl)pyridin-3-yl)-4'-fluoro-2-(trifluoromethyl)-[1,1'-biphenyl]-4-carboxamide;
2'-amino-5-chloro-N-(5-chloro-6-(2H-1,2,3-triazol-2-yl)pyridin-3-yl)-2-cyclopropyl-4'-fluoro-[1,1'-biphenyl]-4-carboxamide;
5-chloro-N-(5-chloro-6-(2H-1,2,3-triazol-2-yl)pyridin-3-yl)-2'-(difluoromethyl)-2,4'-difluoro-[1,1'-biphenyl]-4-carboxamide;

5-chloro-N-(5-chloro-6-(2H-1,2,3-triazol-2-yl)pyridin-3-yl)-2,4'-difluoro-2'-(trifluoromethyl)-[1,1'-biphenyl]-4-carboxamide;
5-chloro-N-(5-chloro-6-(2H-1,2,3-triazol-2-yl)pyridin-3-yl)-2'-cyclopropyl-2,4'-difluoro-[1,1'-biphenyl]-4-carboxamide;
2'-amino-5-chloro-N-(5-chloro-6-(2H-1,2,3-triazol-2-yl)pyridin-3-yl)-4'-fluoro-2-methyl-[1,1'-biphenyl]-4-carboxamide;
4-(3-aminopyridin-4-yl)-2-chloro-N-(5-chloro-6-(2H-1,2,3-triazol-2-yl)pyridin-3-yl)-5-(trifluoromethyl)benzamide;
2-chloro-N-(5-chloro-6-(2H-1,2,3-triazol-2-yl)pyridin-3-yl)-4-(3-chloropyridin-4-yl)-5-(trifluoromethyl)benzamide;
4-(3-bromopyridin-4-yl)-2-chloro-N-(5-chloro-6-(2H-1,2,3-triazol-2-yl)pyridin-3-yl)-5-(trifluoromethyl)benzamide;
2'-amino-5-chloro-N-(5-chloro-6-(2H-1,2,3-triazol-2-yl)pyridin-3-yl)-2-ethynyl-4'-fluoro-[1,1'-biphenyl]-4-carboxamide;
2',5-dichloro-N-(5-chloro-6-(2H-1,2,3-triazol-2-yl)pyridin-3-yl)-4'-fluoro-2-(trifluoromethyl)-[1,1'-biphenyl]-4-carboxamide;
2'-bromo-5-chloro-N-(5-chloro-6-(2H-1,2,3-triazol-2-yl)pyridin-3-yl)-4'-fluoro-2-(trifluoromethyl)-[1,1'-biphenyl]-4-carboxamide;
5-chloro-N-(5-chloro-6-(2H-1,2,3-triazol-2-yl)pyridin-3-yl)-4'-fluoro-2'-methyl-2-(trifluoromethyl)-[1,1'-biphenyl]-4-carboxamide;
2'-amino-2,5-dichloro-N-(5-chloro-6-(2H-1,2,3-triazol-2-yl)pyridin-3-yl)-4'-fluoro-[1,1'-biphenyl]-4-carboxamide;
2',5-dichloro-2,4'-difluoro-N-(6-methyl-5-oxo-5,6-dihydro-1,6-naphthyridin-3-yl)-[1,1'-biphenyl]-4-carboxamide;
2',5-dichloro-N-(7-chloro-2-methylbenzo[d]oxazol-5-yl)-2,4'-difluoro-[1,1'-biphenyl]-4-carboxamide;
2',5-dichloro-N-(8-chloro-3-oxo-3,4-dihydro-2H-benzo[b][1,4]oxazin-6-yl)-2,4'-difluoro-[1,1'-biphenyl]-4-carboxamide;
2',5-dichloro-2,4'-difluoro-N-(1-methyl-1H-pyrazolo[4,3-b]pyridin-6-yl)-[1,1'-biphenyl]-4-carboxamide;
2',5-dichloro-2,4'-difluoro-N-(2-methyl-2H-pyrazolo[4,3-b]pyridin-6-yl)-[1,1'-biphenyl]-4-carboxamide;
2',5-dichloro-2,4'-difluoro-N-(1-methyl-1H-pyrazolo[3,4-b]pyridin-5-yl)-[1,1'-biphenyl]-4-carboxamide;
2',5-dichloro-N-(2-chloropyrazolo[1,5-a]pyrimidin-6-yl)-2,4'-difluoro-[1,1'-biphenyl]-4-carboxamide;
2',5-dichloro-2,4'-difluoro-N-(2-methylimidazo[1,2-b]pyridazin-7-yl)-[1,1'-biphenyl] -4-carboxamide;
2',5-dichloro-N-(2-chlorothiazolo[5,4-b]pyridin-6-yl)-2,4'-difluoro-[1,1'-biphenyl]-4-carboxamide;
2',5-dichloro-N-(6-chloro-1,5-naphthyridin-3-yl)-2,4'-difluoro-[1,1'-biphenyl]-4-carboxamide;
2',5-dichloro-2,4'-difluoro-N-(2-(trifluoromethyl)pyridin-4-yl)-[1,1'-biphenyl]-4-carboxamide;
2',5-dichloro-N-(2-cyanopyridin-4-yl)-2,4'-difluoro-[1,1'-biphenyl]-4-carboxamide;
2',5-dichloro-N-(2-chloropyridin-4-yl)-2,4'-difluoro-[1,1'-biphenyl]-4-carboxamide;
2',5-dichloro-N-(5-chloro-6-methoxypyridin-3-yl)-2,4'-difluoro-[1,1'-biphenyl]-4-carboxamide;
2',5-dichloro-N-(6-cyano-5-(trifluoromethyl)pyridin-3-yl)-2,4'-difluoro-[1,1'-biphenyl]-4-carboxamide;
2',5-dichloro-N-(6-cyano-5-fluoropyridin-3-yl)-2,4'-difluoro-[1,1'-biphenyl]-4-carboxamide;
2',5-dichloro-2,4'-difluoro-N-(2-methyl-6-(trifluoromethyl)pyridin-4-yl)-[1,1'-biphenyl]-4-carboxamide;
2',5-dichloro-N-(5-chloro-6-(1-methyl-1H-pyrazol-4-yl)pyridin-3-yl)-2,4'-difluoro-[1,1'-biphenyl]-4-carboxamide;
2',5-dichloro-N-(5-chloro-6-(3-methyl-1H-1,2,4-triazol-1-yl)pyridin-3-yl)-2,4'-difluoro-[1,1'-biphenyl]-4-carboxamide;
2',5-dichloro-N-(5-chloro-6-(oxazol-2-yl)pyridin-3-yl)-2,4'-difluoro-[1,1'-biphenyl]-4-carboxamide;
2',5-dichloro-N-(5-chloro-6-(thiazol-2-yl)pyridin-3-yl)-2,4'-difluoro-[1,1'-biphenyl]-4-carboxamide;
2',5-dichloro-N-(5-chloro-6-(1H-pyrazol-1-yl)pyridin-3-yl)-2,4'-difluoro-[1,1'-biphenyl]-4-carboxamide;
2',5-dichloro-N-(5-cyano-6-(2H-1,2,3-triazol-2-yl)pyridin-3-yl)-2,4'-difluoro-[1,1'-biphenyl]-4-carboxamide;
2',5-dichloro-N-(5-cyano-6-cyclopropoxypyridin-3-yl)-2,4'-difluoro-[1,1'-biphenyl]-4-carboxamide;
2',5-dichloro-N-(5-cyano-6-(difluoromethoxy)pyridin-3-yl)-2,4'-difluoro-[1,1'-biphenyl]-4-carboxamide;
4-chloro-6-(2-chloro-4-fluorophenyl)-N-(5-chloro-6-(2H-1,2,3-triazol-2-yl)pyridin-3-yl)nicotinamide;
6-(2-amino-4-fluorophenyl)-4-chloro-N-(5-chloro-6-(2H-1,2,3-triazol-2-yl)pyridin-3-yl)nicotinamide;
2-(2-amino-4-fluorophenyl)-4-chloro-N-(5-chloro-6-(2H-1,2,3-triazol-2-yl)pyridin-3-yl)pyrimidine-5-carboxamide;
6-(2-amino-4-fluorophenyl)-2-chloro-N-(5-chloro-6-(2H-1,2,3-triazol-2-yl)pyridin-3-yl)nicotinamide;
4-chloro-6-(2-chloro-4-fluorophenyl)-N-(5-chloro-6-(2H-1,2,3-triazol-2-yl)pyridin-3 -yl)pyridazine-3 -carboxamide;
3-chloro-5-(2-chloro-4-fluorophenyl)-N-(5-chloro-6-(2H-1,2,3-triazol-2-yl)pyridin-3-yl)pyrazine-2-carboxamide;
N-(6-(2H-1,2,3-triazol-2-yl)-5-(trifluoromethyl)pyridin-3-yl)-4-(3-bromopyridin-4-yl)-2-chloro-5-(trifluoromethyl)benzamide;
2-amino-2',5-dichloro-N-(5-chloro-6-(2H-1,2,3-triazol-2-yl)pyridin-3-yl)-4'-fluoro-[1,1'-biphenyl]-4-carboxamide;
2-acetamido-2',5-dichloro-N-(5-chloro-6-(2H-1,2,3-triazol-2-yl)pyridin-3-yl)-4'-fluoro-[1,1'-biphenyl]-4-carboxamide;
ethyl(2',5-dichloro-4-((5 -chloro-6-(2H-1,2,3 -triazol-2-yl)pyridin-3 - yl)aminoformyl)-4'-fluoro-[1,1'-biphenyl]-2-yl)aminoformate;

2',5-dichloro-N-(5-chloro-6-(2H-1,2,3-triazol-2-yl)pyridin-3-yl)-4'-fluoro-2-(isopropylamino)-[1,1'-biphenyl]-4-carboxamide;
N-(6-(2H-1,2,3-triazol-2-yl)-5-(trifluoromethyl)pyridin-3-yl)-5-chloro-2,2',4'-trifluoro-[1,1'-biphenyl]-4-carboxamide;
5-chloro-N-(5-chloro-6-(2H-1,2,3-triazol-2-yl)pyridin-3-yl)-2,2',4'-trifluoro-[1,1'-biphenyl]-4-carboxamide;
N-(5-chloro-6-(2H-1,2,3-triazol-2-yl)pyridin-3-yl)-2,2',4',5-tetrafluoro-[1,1'-biphenyl]-4-carboxamide;
N-(6-(2H-1,2,3-triazol-2-yl)-5-(trifluoromethyl)pyridin-3-yl)-2,2',4',5-tetrafluoro-[1,1 '-biphenyl]-4-carboxamide;
4-(3-bromopyridin-4-yl)-2-chloro-N-(5-chloro-6-(2H-1,2,3-triazol-2-yl)pyridin-3 -yl)-5 -fluorobenzamide;
4-(2-amino-6-fluoropyridin-3-yl)-N-(5-chloro-6-(2H-1,2,3-triazol-2-yl)pyridin-3-yl)- 2-Fluoro-5-isopropylbenzamide;
5-chloro-N-(5-chloro-6-(2H-1,2,3-triazol-2-yl)pyridin-3-yl)-2'-(cyclopropylamino)-2,4'-difluoro-[1,1'-biphenyl]-4-carboxamide;
5-chloro-N-(5-chloro-6-(2H-1,2,3-triazol-2-yl)pyridin-3-yl)-2,4'-difluoro-2'-(methylthio)-[1,1'-biphenyl]-4-carboxamide;
5-chloro-N-(5-chloro-6-(2H-1,2,3-triazol-2-yl)pyridin-3-yl)-2,4'-difluoro-2'-(methylsulfonyl)-[1,1'-biphenyl]-4-carboxamide;
5-chloro-N-(5-chloro-6-(2H-1,2,3-triazol-2-yl)pyridin-3-yl)-2,4'-difluoro-2'-ureido-[1,1'-biphenyl]-4-carboxamide;
2'-acetamido-5-chloro-N-(5-chloro-6-(2H-1,2,3-triazol-2-yl)pyridin-3-yl)-2,4'-difluoro-[1,1'-biphenyl]-4-carboxamide;
5-chloro-N-(5-chloro-6-(2H-1,2,3-triazol-2-yl)pyridin-3-yl)-2,4'-difluoro-2'-(hydroxymethyl)-[1,1 '-biphenyl]-4-carboxamide;
4-(3-amino-5-ethynylpyridin-4-yl)-2-chloro-N-(5-chloro-6-(2H-1,2,3-triazol-2-yl)pyridin-3-yl)-5-fluorobenzamide;
5-chloro-N-(5-chloro-6-(2H-1,2,3-triazol-2-yl)pyridin-3-yl)-2,4'-difluoro-2'-(methylsulfinyl)-[1,1 '-biphenyl]-4-carboxamide;
5-chloro-N-(5-chloro-6-(2H-1,2,3-triazol-2-yl)pyridin-3-yl)-2,4'-difluoro-2'-((methylsulfonyl)methyl)-[1,1'-biphenyl]-4-carboxamide;
ethyl (5'-chloro-4'-((5-chloro-6-(2H-1,2,3-triazol-2-yl)pyridin-3-yl)carbamoyl)-2',4-difluoro-[1,1'-biphenyl]-2-yl)carbamate;
5-chloro-N-(5-chloro-6-(2H-1,2,3-triazol-2-yl)pyridin-3-yl)-2,4'-difluoro-2'-vinyl-[1,1'-biphenyl]-4-carboxamide;
5-chloro-N-(5-chloro-6-(2H-1,2,3-triazol-2-yl)pyridin-3-yl)-2,4'-difluoro-2'-(1-hydroxyethyl)-[1,1 '-biphenyl]-4-carboxamide
5-chloro-N-(5-chloro-6-(2H-1,2,3-triazol-2-yl)pyridin-3-yl)-2,4'-difluoro-2'-(1-methoxyethyl)-[1,1 '-biphenyl]-4-carboxamide;
5-chloro-N-(5-chloro-6-(2H-1,2,3-triazol-2-yl)pyridin-3-yl)-2,4'-difluoro-2'-((2-(methylamino)ethyl)amino)-[1,1 '-biphenyl]-4-carboxamide;
5-chloro-N-(5-chloro-6-(2H-1,2,3-triazol-2-yl)pyridin-3-yl)-2,4'-difluoro-2'-((2-hydroxyethyl)amino)-[1,1'-biphenyl]-4-carboxamide;
5-chloro-N-(5-chloro-6-(2H-1,2,3-triazol-2-yl)pyridin-3-yl)-2,4'-difluoro-2'-(2-hydroxyethoxy)-[1,1'-biphenyl]-4-carboxamide;
5-chloro-N-(5-chloro-6-(2H-1,2,3-triazol-2-yl)pyridin-3-yl)-2,4'-difluoro-2'-(hydroxyamino)-[1,1 '-biphenyl]-4-carboxamide;
5-chloro-N-(5-chloro-6-(2H-1,2,3-triazol-2-yl)pyridin-3-yl)-2,4'-difluoro-2'-(2-hydroxypropan-2-yl)-[1,1'-biphenyl]-4-carboxamide;
5'-chloro-4'-((5-chloro-6-(2H-1,2,3-triazol-2-yl)pyridin-3-yl)carbamoyl)-2',4-difluoro-[1,1'-biphenyl]-2-yl ethyl carbonate;
5-chloro-N-(5-chloro-6-(2H-1,2,3-triazol-2-yl)pyridin-3-yl)-2'-((2-(dimethylamino)ethyl)amino)-2,4'-difluoro-[1,1'-biphenyl]-4-carboxamide;
5-chloro-N-(5-chloro-6-(2H-1,2,3-triazol-2-yl)pyridin-3-yl)-2,4'-difluoro-2'-((2-methoxyethyl)amino)- [1,1 '-biphenyl]-4-carboxamide;
5-chloro-N-(5-chloro-6-(2H-1,2,3-triazol-2-yl)pyridin-3-yl)-2,4'-difluoro-2'-(2-methoxyethoxy)-[1,1 '-biphenyl]-4-carboxamide;
5-chloro-N-(5-chloro-6-(2H-1,2,3-triazol-2-yl)pyridin-3-yl)-2,4'-difluoro-2'-(prop-2-yn-1-ylamino)-[1,1'-biphenyl]-4-carboxamide;
5-chloro-N-(5-chloro-6-(2H-1,2,3-triazol-2-yl)pyridin-3-yl)-2'-((cyanomethyl)amino)-2,4'-difluoro-[1,1'-biphenyl]-4-carboxamide;
2'-(aminomethyl)-5-chloro-N-(5-chloro-6-(2H-1,2,3-triazol-2-yl)pyridin-3-yl)-2,4'-difluoro-[1,1'-biphenyl]-4-carboxamide;
5-chloro-N-(5-chloro-6-(2H-1,2,3-triazol-2-yl)pyridin-3-yl)-2,4'-difluoro-2'-(prop-2-yn-1-yl)-[1,1'-biphenyl]-4-carboxamide;
2'-acrylamido-5-chloro-N-(5-chloro-6-(2H-1,2,3-triazol-2-yl)pyridin-3-yl)-2,4'-difluoro-[1,1'-biphenyl]-4-carboxamide;
5'-chloro-N4'-(5-chloro-6-(2H-1,2,3-triazol-2-yl)pyridin-3-yl)-2',4-difluoro-[1,1'-biphenyl]-2,4'-dicarboxamide;
5-chloro-N-(5-chloro-6-(2H-1,2,3-triazol-2-yl)pyridin-3-yl)-2,4'-difluoro-2'-(prop-2-yn-1-yloxy)-[1,1 '-biphenyl]-4-carboxamide;
5-chloro-N-(5-chloro-6-(2H-1,2,3-triazol-2-yl)pyridin-3-yl)-2'-(cyanomethoxy)-2,4'-difluoro-[1,1'-biphenyl]-4-carboxamide;
2'-(1-aminoethyl)-5-chloro-N-(5-chloro-6-(2H-1,2,3-triazol-2-yl)pyridin-3-yl)-2,4'-difluoro-[1,1'-biphenyl]-4-carboxamide;
5-chloro-N-(5-chloro-6-(2H-1,2,3-triazol-2-yl)pyridin-3-yl)-2'-(cyanomethyl)-2,4'-difluoro-[1,1'-biphenyl]-4-carboxamide;
2'-amino-5-chloro-N-(5-chloro-6-(2H-1,2,3-triazol-2-yl)pyridin-3-yl)-6'-ethynyl-2,4'-difluoro-[1,1'-biphenyl]-4-carboxamide;
cyclopropyl 5'-chloro-4'-((5-chloro-6-(2H-1,2,3-triazol-2-yl)pyridin-3-yl)carbamoyl)-2',4-difluoro-[1,1'-biphenyl]-2-carboxylate;
2',5-dichloro-N-(5-chloro-6-(2H-1,2,3-triazol-2-yl)pyridin-3-yl)-6'-ethynyl-2,4'-difluoro-[1,1'-biphenyl]-4-carboxamide;
2'-(2-aminopropan-2-yl)-5-chloro-N-(5-chloro-6-(2H-1,2,3-triazol-2-yl)pyridin-3-yl)-2,4'-difluoro-[1,1'-biphenyl]-4-carboxamide;
5-chloro-N-(5-chloro-6-(2H-1,2,3-triazol-2-yl)pyridin-3-yl)-2,4'-difluoro-2'-(methoxymethyl)-[1,1'-biphenyl]-4-carboxamide;
5'-chloro-4'-((5-chloro-6-(2H-1,2,3-triazol-2-yl)pyridin-3-yl)carbamoyl)-2',4-difluoro-[1,1'-biphenyl]-2-yl carbamate;
2',5-dichloro-N-(5-chloro-6-(2H-1,2,3-triazol-2-yl)pyridin-3-yl)-2,4'-difluoro-6'-(methoxymethyl)-[1,1'-biphenyl]-4-carboxamide;
N-(6-(2H-1,2,3-triazol-2-yl)-5-(trifluoromethyl)pyridin-3-yl)-5-chloro-2,4'-difluoro-2'-vinyl-[1,1'-biphenyl]-4-carboxamide;
N-(6-(2H-1,2,3-triazol-2-yl)-5-(trifluoromethyl)pyridin-3-yl)-5-chloro-2'-ethynyl-2,4'-difluoro-[1,1'-biphenyl]-4-carboxamide;
2-chloro-N-(5-chloro-6-(2H-1,2,3-triazol-2-yl)pyridin-3-yl)-4-(2-ethynyl-6-fluoropyridin-3-yl)-5-fluorobenzamide;
2-chloro-N-(5-chloro-6-(2H-1,2,3-triazol-2-yl)pyridin-3-yl)-4-(3-ethynylpyridin-4-yl)-5-fluorobenzamide;
5-chloro-2'-ethynyl-2,4'-difluoro-N-(2-(trifluoromethyl)pyridin-4-yl)-[1,1'-biphenyl] -4-carboxamide;
5-chloro-N-(8-chloro-3-oxo-3,4-dihydro-2H-benzo[b][1,4]oxazin-6-yl)-2'-ethynyl-2,4'-difluoro-[1,1'-biphenyl]-4-carboxamide;
5-chloro-N-(6-cyano-5-(trifluoromethyl)pyridin-3-yl)-2'-ethynyl-2,4'-difluoro-[1,1'-biphenyl]-4-carboxamide;
N-(6-(2H-1,2,3-triazol-2-yl)-5-(trifluoromethyl)pyridin-3-yl)-2-chloro-4-(2-ethynyl-6-fluoropyridin-3-yl)-5-fluorobenzamide;
2-chloro-4-(3-ethynylpyridin-4-yl)-5-fluoro-N-(6-((tetrahydrofuran-3-yl)amino)-5-(trifluoromethyl)pyridin-3-yl)benzamide;
2-chloro-4-(3-ethynyl-5-fluoropyridin-2-yl)-5-fluoro-N-(2-(trifluoromethyl)pyridin-4-yl)benzamide;
2-chloro-N-(8-chloro-3-oxo-3,4-dihydro-2H-benzo[b][1,4] oxazin-6-yl)-4-(3 - ethynyl-5-fluoropyridin-2-yl)-5-fluorobenzamide;
2-chloro-N-(6-cyano-5-(trifluoromethyl)pyridin-3-yl)-4-(3-ethynyl-5-fluoropyridin-2-yl)-5-fluorobenzamide;
N-(6-(2H-1,2,3-triazol-2-yl)-5-(trifluoromethyl)pyridin-3-yl)-2-chloro-4-(3-ethynyl-5-fluoropyridin-2-yl)-5-fluorobenzamide;
5-chloro-N-(5-cyano-6-(trifluoromethoxy)pyridin-3-yl)-2'-ethynyl-2,4'-difluoro-[1,1'-biphenyl]-4-carboxamide;
5-chloro-N-(5-chloro-6-(difluoromethoxy)pyridin-3-yl)-2'-ethynyl-2,4'-difluoro-[1,1'-biphenyl]-4-carboxamide;
5-chloro-N-(5-cyano-6-((1-methylpiperidin-4-yl)oxy)pyridin-3-yl)-2'-ethynyl-2,4'-difluoro-[1,1'-biphenyl]-4-carboxamide;
N-(6-(2H-1,2,3 -triazol-2-yl)-5 -(trifluoromethyl)pyridin-3 -yl)-2-chloro-4-(3 - ethynylpyridin-4-yl)-5-fluorobenzamide;
N-(5-chloro-6-(2H-1,2,3-triazol-2-yl)pyridin-3-yl)-2,3,4',5,6-pentafluoro-2'-propiolamido-[1,1 '-biphenyl]-4-carboxamide;
N-(5-chloro-6-(2H-1,2,3-triazol-2-yl)pyridin-3-yl)-2,3,4',5,6-pentafluoro-2'-propiolamido-[1,1 '-biphenyl] -4-carboxamide;
N-(5-chloro-6-(2H-1,2,3-triazol-2-yl)pyridin-3-yl)-4-(3-ethynylpyridin-4-yl)-2,3,5,6-tetrafluorobenzamide;
2-chloro-N-(3-chloro-4-(2-methoxyethoxy)phenyl)-4-(3-ethynylpyridin-4-yl)-5-fluorobenzamide;
2-chloro-N-(5-chloro-6-(2H-1,2,3-triazol-2-yl)pyridin-3-yl)-4-(3-ethynyl-5-fluoropyridin-2-yl)-5-fluorobenzamide;
6-chloro-N-(5-chloro-6-(2H-1,2,3-triazol-2-yl)pyridin-3-yl)-3'-ethynyl-[2,4'-bipyridine]-5-carboxamide;
2-chloro-N-(5-chloro-6-(2H-1,2,3-triazol-2-yl)pyridin-3-yl)-5-fluoro-4-(3-propiolamidopyridin-4-yl)benzamide;
2-chloro-N-(3-chloro-4-methoxyphenyl)-4-(3-ethynylpyridin-4-yl)-5-fluorobenzamide;

2-chloro-4-(3-ethynyl-5-fluoropyridin-2-yl)-5-fluoro-N-(6-((tetrahydrofuran-3-yl)amino)-5-(trifluoromethyl)pyridin-3-yl)benzamide;
N-(5-chloro-6-(2H-1,2,3-triazol-2-yl)pyridin-3-yl)-4-(3-ethynyl-5-fluoropyridin-2-yl)-2,3,5,6-tetrafluorobenzamide;
N-(5-chloro-6-(2H-1,2,3-triazol-2-yl)pyridin-3-yl)-2,3,5,6-tetrafluoro-4-(5-fluoro-3-propiolamidopyridin-2-yl)benzamide;
4-(3-acrylamidopyridin-4-yl)-2-chloro-N-(5-chloro-6-(2H-1,2,3-triazol-2-yl)pyridin-3-yl)-5-fluorobenzamide;
N-(3-chloro-4-(methylsulfonyl)phenyl)-4-(3-ethynylpyridin-4-yl)-2,3,5,6-tetrafluorobenzamide;
N-(3-chloro-4-((trifluoromethyl)thio)phenyl)-4-(3-ethynylpyridin-4-yl)-2,3,5,6-tetrafluorobenzamide;
N-(3-chloro-4-((2-(dimethylamino)-2-oxoethyl)thio)phenyl)-4-(3-ethynylpyridin-4-yl)-2,3,5,6-tetrafluorobenzamide;
N-(6-(2H-1,2,3-triazol-2-yl)-5-(trifluoromethyl)pyridin-3-yl)-2-chloro-5-fluoro-4-(3-vinylpyridin-4-yl)benzamide;
2'-amino-5-chloro-2,4'-difluoro-N-(6-(2-methoxyethoxy)-5-(trifluoromethyl)pyridin-3-yl)-[1,1'-biphenyl]-4-carboxamide;
2'-amino-5-chloro-N-(6-(cyclopropylamino)-5-(trifluoromethyl)pyridin-3-yl)-2,4'-difluoro-[1,1'-biphenyl]-4-carboxamide;
2'-amino-5-chloro-N-(6-(cyanomethoxy)-5-(trifluoromethyl)pyridin-3-yl)-2,4'-difluoro-[1,1'-biphenyl]-4-carboxamide;
2'-amino-5-chloro-N-(6-((cyanomethyl)amino)-5-(trifluoromethyl)pyridin-3-yl)-2,4'-difluoro-[1,1'-biphenyl]-4-carboxamide;
2'-amino-5-chloro-2,4'-difluoro-N-(6-(methoxymethyl)-5-(trifluoromethyl)pyridin-3-yl)-[1,1'-biphenyl] -4-carboxamide;
2'-amino-5-chloro-2,4'-difluoro-N-(6-((2-methoxyethyl)amino)-5-(trifluoromethyl)pyridin-3-yl)-[1,1 '-biphenyl] -4-carboxamide;
methyl (4-(2'-amino-5-chloro-2,4'-difluoro-[1,1'-biphenyl]-4-carboxamido)-2-(trifluoromethyl)phenyl)carbamate;
N-(4-acetamido-3-(trifluoromethyl)phenyl)-2'-amino-5-chloro-2,4'-difluoro-[1,1'-biphenyl]-4-carboxamide;
2'-amino -5 -chloro-2,4'-difluoro-N-(3-(trifluoromethyl)-4-ureidophenyl)-[1,1'-biphenyl]-4-carboxamide;
2'-amino-5-chloro-2,4'-difluoro-N-(4-(methylsulfonamido)-3-(trifluoromethyl)phenyl)-[1,1 '-biphenyl]-4-carboxamide;
2'-amino-5-chloro-2,4'-difluoro-N-(6-(hydroxymethyl)-5-(trifluoromethyl)pyridin-3-yl)-[1,1'-biphenyl]-4-carboxamide;
2'-amino-5-chloro-N-(6-(cyanomethyl)-5-(trifluoromethyl)pyridin-3-yl)-2,4'-difluoro-[1,1'-biphenyl]-4-carboxamide;
2'-amino-5-chloro-2,4'-difluoro-N-(4-(methylthio)-3-(trifluoromethyl)phenyl)-[1,1'-biphenyl]-4-carboxamide;
2'-amino-5-chloro-2,4'-difluoro-N-(4-(methylsulfonyl)-3-(trifluoromethyl)phenyl)-[1,1 '-biphenyl]-4-carboxamide;
2'-amino-5-chloro-2,4'-difluoro-N-(6-((tetrahydrofuran-3-yl)oxy)-5-(trifluoromethyl)pyridin-3-yl)-[1,1'-biphenyl]-4-carboxamide;
2'-amino-5-chloro-2,4'-difluoro-N-(6-(2-hydroxypropan-2-yl)-5-(trifluoromethyl)pyridin-3-yl)-[1,1 '-biphenyl]-4-carboxamide;
5-(2'-amino-5-chloro-2,4'-difluoro-[1,1'-biphenyl]-4-carboxamido)-3-(trifluoromethyl)picolinamide;
2'-amino-5-chloro-N-(6-ethynyl-5-(trifluoromethyl)pyridin-3-yl)-2,4'-difluoro-[1,1'-biphenyl]-4-carboxamide;
2'-amino-5-chloro-2,4'-difluoro-N-(6-((tetrahydrofuran-3-yl)amino)-5-(trifluoromethyl)pyridin-3-yl)-[1,1'-biphenyl]-4-carboxamide;
2'-amino-5-chloro-N-(4-(cyclopropylsulfonyl)-3-(trifluoromethyl)phenyl)-2,4'-difluoro-[1,1'-biphenyl]-4-carboxamide;
2'-amino-5-chloro-2,4'-difluoro-N-(6-(pyrrolidine-1-carbonyl)-5-(trifluoromethyl)pyridin-3-yl)-[1,1'-biphenyl] -4-carboxamide;
2'-amino-5-chloro-N-(4-(N,N-dimethylsulfamoyl)-3-(trifluoromethyl)phenyl)-2,4'-difluoro-[1,1 '-biphenyl]-4-carboxamide;
N-(4-(2-azaspiro[3.3]heptane-2-carbonyl)-3-(trifluoromethyl)phenyl)-2'-amino-5-chloro-2,4'-difluoro-[1,1 '-biphenyl] -4-carboxamide;
2-chloro-N-(5-chloro-6-(1-oxo-2-oxa-7-azaspiro[4.4]nonan-7-yl)pyridin-3-yl)-4-(3-ethynylpyridin-4-yl)-5-fluorobenzamide;
2-chloro-N-(5-chloro-6-(2-oxa-7-azaspiro[4.4]nonan-7-yl)pyridin-3-yl)-4-(3-ethynylpyridin-4-yl)-5-fluorobenzamide;
2-chloro-N-(5-chloro-6-((1R,2S,4S)-2-(3-methylisoxazol-5-yl)-7-azabicyclo[2.2.1]heptan-7-yl)pyridin-3 -yl)-4-(3 -ethynylpyridin-4-yl)-5 - fluorobenzamide;
2-chloro-N-(5-chloro-6-(1-cyano-7-azabicyclo[2.2.1]heptan-7-yl)pyridin-3-yl)-4-(3-ethynylpyridin-4-yl)-5-fluorobenzamide;
N-(6-(7-azabicyclo[2.2.1]heptan-7-yl)-5-chloropyridin-3-yl)-2-chloro-4-(3-ethynylpyridin-4-yl)-5-fluorobenzamide;
2-chloro-N-(5-chloro-6-(4-oxohexahydrocyclopenta[c]pyrrol-2(1H)-yl)pyridin-3-yl)-4-(3-ethynylpyridin-4-yl)-5-fluorobenzamide;

2-chloro-N-(5-chloro-6-((3aR,6aS)-5-oxohexahydrocyclopenta[c]pyrrol-2(1H)-yl)pyridin-3-yl)-4-(3-ethynylpyridin-4-yl)-5-fluorobenzamide;
2-chloro-N-(5-chloro-6-((3aR,6aS)-5,5-difluorohexahydrocyclopenta[c]pyrrol-2(1H)-yl)pyridin-3-yl)-4-(3-ethynylpyridin-4-yl)-5-fluorobenzamide;
2-chloro-N-(5-chloro-6-((2-oxopyrrolidin-1-yl)amino)pyridin-3-yl)-4-(3-ethynylpyridin-4-yl)-5-fluorobenzamide;
2-chloro-N-(5-chloro-6-(3-oxopyrazolidin-1-yl)pyridin-3-yl)-4-(3-ethynylpyridin-4-yl)-5-fluorobenzamide;
N-(6-(1H-benzo[d]imidazol-2-yl)-5-chloropyridin-3-yl)-2-chloro-4-(3-ethynylpyridin-4-yl)-5-fluorobenzamide;
2-chloro-N-(5-chloro-6-(4,5,6,7-tetrahydro-2H-[1,2,3]triazolo[4,5-c]pyridin-2-yl)pyridin-3-yl)-4-(3-ethynylpyridin-4-yl)-5-fluorobenzamide;
2-chloro-N-(5-chloro-6-(4,5-dicyano-2H-1,2,3-triazol-2-yl)pyridin-3-yl)-4-(3-ethynylpyridin-4-yl)-5-fluorobenzamide;
2-chloro-N-(5-chloro-6-(4,5,6,7-tetrahydro-2H-indazol-2-yl)pyridin-3-yl)-4-(3-ethynylpyridin-4-yl)-5-fluorobenzamide;
2-chloro-N-(5-chloro-6-(5,6-dihydrocyclopenta[c]pyrazol-2(4H)-yl)pyridin-3-yl)-4-(3-ethynylpyridin-4-yl)-5-fluorobenzamide;
2-chloro-N-(5-chloro-6-(3-(trifluoromethyl)-5,6-dihydrocyclopenta[c]pyrazol-2(4H)-yl)pyridin-3-yl)-4-(3-ethynylpyridin-4-yl)-5-fluorobenzamide;
ethyl 2-((3-chloro-5-(2-chloro-4-(3-ethynylpyridin-4-yl)-5-fluorobenzamido)pyridin-2-yl)amino)-2,4,5,6-tetrahydrocyclopenta[d][1,2,3]triazole-4-carboxylate;
N-(6-((2H-benzo[d][1,2,3]triazol-2-yl)amino)-5-chloropyridin-3 -yl)-2-chloro-4-(3-ethynylpyridin-4-yl)-5-fluorobenzamide;
2-chloro-N-(5-chloro-6-(2-(trifluoromethyl)-5,6-dihydro-[1,2,4]triazolo[1,5-a]pyrazin-7(8H)-yl)pyridin-3-yl)-4-(3-ethynylpyridin-4-yl)-5-fluorobenzamide;
2-chloro-N-(5-chloro-6-(3-(trifluoromethyl)-5,6-dihydro-[1,2,4]triazolo[4,3-a]pyrazin-7(8H)-yl)pyridin-3-yl)-4-(3-ethynylpyridin-4-yl)-5-fluorobenzamide;
1-(3-chloro-5-(2-chloro-4-(3-ethynylpyridin-4-yl)-5-fluorobenzamido)pyridin-2-yl)-4,5,6,7-tetrahydro-1H-benzo[d]imidazole-5-carboxylic acid;
2-chloro-N-(2-(3,4-dichlorophenyl)benzo[d]oxazol-5-yl)-4-(3-ethynylpyridin-4-yl)-5-fluorobenzamide;
(S)-2-chloro-N-(5-chloro-6-(2-cyano-4,4-difluoropyrrolidine-1-carbonyl)pyridin-3-yl)-4-(3-ethynylpyridin-4-yl)-5-fluorobenzamide;
2-chloro-N-(5 -chloro-6-((3aR,6aS)-hexahydro-1H-furo[3,4-c]pyrrole-5 - carbonyl)pyridin-3 -yl)-4-(3 -ethynylpyridin-4-yl)-5 -fluorobenzamide;
2-chloro-N-(5-chloro-6-(pyrrolidine-1-carbonyl)pyridin-3-yl)-4-(3-ethynylpyridin-4-yl)-5-fluorobenzamide;
2-chloro-N-(5-chloro-6-(4,4-difluoropiperidine-1-carbonyl)pyridin-3-yl)-4-(3-ethynylpyridin-4-yl)-5-fluorobenzamide;
2-chloro-N-(5-chloro-6-(6,6-difluoro-3-azabicyclo[3.1.0]hexane-3-carbonyl)pyridin-3-yl)-4-(3-ethynylpyridin-4-yl)-5-fluorobenzamide;
2-chloro-N-(5-chloro-6-(6,6-difluoro-3-azabicyclo[3.1.0]hexan-3-yl)pyridin-3-yl)-4-(3-ethynylpyridin-4-yl)-5-fluorobenzamide;
2-chloro-N-(5-chloro-6-(3-(4,4-difluoropiperidin-1-yl)azetidin-1-yl)pyridin-3-yl)-4-(3-ethynylpyridin-4-yl)-5-fluorobenzamide;
2-chloro-N-(5-chloro-6-(3-(4,4-difluoropiperidin-1-yl)azetidine-1-carbonyl)pyridin-3-yl)-4-(3-ethynylpyridin-4-yl)-5-fluorobenzamide;
4,4'-dichloro-N-(5-chloro-6-(2H-1,2,3-triazol-2-yl)pyridin-3-yl)-6-(3-ethynylpyridin-4-yl)-[1,1'-biphenyl] -3 -carboxamide;
2-amino-4",5'-dichloro-N-(5-chloro-6-(2H-1,2,3-triazol-2-yl)pyridin-3-yl)-4-fluoro-[1,1 ':2',1 "-terphenyl]-4'-carboxamide;
N-(6-(2H-1,2,3-triazol-2-yl)-5-(trifluoromethyl)pyridin-3-yl)-5'-methyl-[1,1':2',1"-terphenyl]-4'-carboxamide;
2-chloro-N-(5-chloro-6-(2H-1,2,3-triazol-2-yl)pyridin-3-yl)-5-ethynyl-4-(3-ethynylpyridin-4-yl)benzamide;
N-(5-chloro-6-(2H-1,2,3-triazol-2-yl)pyridin-3-yl)-5-ethynyl-4-(3-ethynylpyridin-4-yl)-2-methylbenzamide;
N-(5-chloro-6-(2H-1,2,3-triazol-2-yl)pyridin-3-yl)-5-cyclopropyl-4-(3-ethynylpyridin-4-yl)-2-methylbenzamide;
4-(3-amino-5-fluoropyridin-2-yl)-N-(5-chloro-6-(2H-1,2,3-triazol-2-yl)pyridin-3-yl)-5-cyclopropyl-2-methylbenzamide;
2-chloro-N-(5-chloro-6-(2H-1,2,3-triazol-2-yl)pyridin-3-yl)-5-cyclopropyl-4-(3-ethynylpyridin-4-yl)benzamide;
4-(3-amino-5-fluoropyridin-2-yl)-2-chloro-N-(5-chloro-6-(2H-1,2,3-triazol-2-yl)pyridin-3 -yl)-5 -cyclopropylbenzamide;
2'-amino-N-(5-chloro-6-(2H-1,2,3-triazol-2-yl)pyridin-3-yl)-2-ethynyl-4'-fluoro-5-methyl-[1,1'-biphenyl]-4-carboxamide;
N-(6-(2H-1,2,3-triazol-2-yl)-5-(trifluoromethyl)pyridin-3-yl)-4-(3-amino-5-ethynylpyridin-4-yl)-2-chloro-5-fluorobenzamide;

4-(3-amino-5-ethynylpyridin-4-yl)-2-chloro-N-(5-chloro-6-(2H-1,2,3-triazol-2-yl)pyridin-3-yl)-5-fluorobenzamide;
2'-amino-5-chloro-N-(5-chloro-6-(2H-1,2,3-triazol-2-yl)pyridin-3-yl)-6'-ethynyl-2,4'-difluoro-[1,1'-biphenyl]-4-carboxamide;
N-(6-(2H-1,2,3-triazol-2-yl)-5-(trifluoromethyl)pyridin-3-yl)-2',5-dichloro-6'-ethynyl-2-fluoro-[1,1'-biphenyl]-4-carboxamide;
2'-chloro-N-(5-chloro-6-(2H-1,2,3-triazol-2-yl)pyridin-3-yl)-2-ethynyl-4'-fluoro-5-methyl-[1,1'-biphenyl]-4-carboxamide;
N-(5-chloro-6-(2H-1,2,3-triazol-2-yl)pyridin-3-yl)-2,2'-diethynyl-4'-fluoro-5-methyl-[1,1'-biphenyl]-4-carboxamide;
4-(2-amino-6-fluoropyridin-3-yl)-N-(5-chloro-6-(2H-1,2,3-triazol-2-yl)pyridin-3-yl)-5-ethynyl-2-methylbenzamide;
N-(5-chloro-6-(2H-1,2,3-triazol-2-yl)pyridin-3-yl)-5-ethynyl-4-(3-methoxypyridin-4-yl)-2-methylbenzamide;
2-amino-5'-chloro-N-(5-chloro-6-(2H-1,2,3-triazol-2-yl)pyridin-3-yl)-4-fluoro-2",3",4",5"-tetrahydro-[1,1':2',1"-terphenyl]-4'-carboxamide;
2-chloro-N-(5-chloro-6-(2H-1,2,3-triazol-2-yl)pyridin-3-yl)-4-fluoro-5'-methyl-[1,1 ':2',1 "-terphenyl]-4'-carboxamide;
2'-amino-N-(5-chloro-6-(2H-1,2,3-triazol-2-yl)pyridin-3-yl)-2-(cyclopent-1-en-1-yl)-4'-fluoro-5-methyl-[1,1'-biphenyl]-4-carboxamide;
2-amino-N-(5-chloro-6-(2H-1,2,3-triazol-2-yl)pyridin-3-yl)-4-fluoro-5'-methyl-[1,1':2',1"-terphenyl]-4'-carboxamide;
N-(5-chloro-6-(2H-1,2,3-triazol-2-yl)pyridin-3-yl)-2-ethynyl-4-fluoro-5'-methyl-[1,1':2',1"-terphenyl]-4'-carboxamide;
2'-amino-N-(5-chloro-6-(2H-1,2,3-triazol-2-yl)pyridin-3-yl)-4'-fluoro-5-methyl-2-(prop-1 -yn-1 -yl)-[1,1 '-biphenyl] -4-carboxamide;
2'-amino-5-chloro-N-(5-chloro-6-(2H-1,2,3-triazol-2-yl)pyridin-3-yl)-4'-fluoro-2-vinyl-[1,1'-biphenyl]-4-carboxamide;
2'-amino-N-(5-chloro-6-(2H-1,2,3-triazol-2-yl)pyridin-3-yl)-4'-fluoro-5-methyl-2-vinyl-[1,1'-biphenyl]-4-carboxamide;
3-chloro-5-(2-chloro-4-(3-ethynylpyridin-4-yl)-5-fluorobenzamido)-N-methylpicolinamide;
3-chloro-5-(2-chloro-4-(3-ethynylpyridin-4-yl)-5-fluorobenzamido)-N-(cyanomethyl)picolinamide;
3-chloro-5-(2-chloro-4-(3-ethynylpyridin-4-yl)-5-fluorobenzamido)-N-ethylpicolinamide;
3-chloro-5-(2-chloro-4-(3-ethynylpyridin-4-yl)-5-fluorobenzamido)-N-(2,2,2-trifluoroethyl)picolinamide;
3-chloro-5-(2-chloro-4-(3-ethynylpyridin-4-yl)-5-fluorobenzamido)-N-propylpicolinamide;
3-chloro-5-(2-chloro-4-(3-ethynylpyridin-4-yl)-5-fluorobenzamido)-N-(2-(trifluoromethoxy)ethyl)picolinamide;
3-chloro-5-(2-chloro-4-(3-ethynylpyridin-4-yl)-5-fluorobenzamido)-N-phenethylpicolinamide;
3-chloro-5-(2-chloro-4-(3-ethynylpyridin-4-yl)-5-fluorobenzamido)-N-(furan-3-ylmethyl)picolinamide;
3 -chloro-5 -(2-chloro-4-(3 -ethynylpyridin-4-yl)-5 -fluorobenzamido)-N-neopentylpicolinamide;
3-chloro-5-(2-chloro-4-(3-ethynylpyridin-4-yl)-5-fluorobenzamido)-N-(2-(diethylamino)-2-oxoethyl)picolinamide;
5-(2'-amino-5-chloro-2,4'-difluoro-[1,1 '-biphenyl]-4-carboxamido)-3-chloro-N-((1 -methylcyclopropyl)methyl)picolinamide;
5-(2'-amino-5-chloro-2,4'-difluoro-[1,1'-biphenyl]-4-carboxamido)-3-chloro-N-((1,1-dioxidotetrahydro-2H-thiopyran-4-yl)methyl)picolinamide;
5-(2'-amino-5-chloro-2,4'-difluoro-[1,1'-biphenyl]-4-carboxamido)-N-butyl-3-chloropicolinamide;
5-(2'-amino-5-chloro-2,4'-difluoro-[1,1'-biphenyl]-4-carboxamido)-3-chloro-N-(prop-2-yn-1-yl)picolinamide;
N-(6-(2H-1,2,3-triazol-2-yl)-5-(trifluoromethyl)pyridin-3-yl)-4-(3-bromo-5 - nitropyridin-4-yl)-2-chloro-5-fluorobenzamide;
5-(2'-amino-5-chloro-2,4'-difluoro-[1,1'-biphenyl]-4-carboxamido)-3-chloro-N-(cyanomethyl)picolinamide;
5-(2'-amino-5-chloro-2,4'-difluoro-6'-methyl-[1,1'-biphenyl]-4-carboxamido)-3-chloro-N-(cyanomethyl)picolinamide;
5-(2'-amino-5-chloro-2,4'-difluoro-[1,1'-biphenyl]-4-carboxamido)-3-chloro-N-(2,2,2-trifluoroethyl)picolinamide;
5-(2'-amino-5-chloro-2,4'-difluoro-[1,1'-biphenyl]-4-carboxamido)-3-chloro-N-ethylpicolinamide;
2-chloro-N-(5-chloro-6-(2H-1,2,3-triazol-2-yl)pyridin-3-yl)-4-(3-cyclopropylpyridin-4-yl)-5-fluorobenzamide;
5-(2'-amino-5-chloro-2,4'-difluoro-[1,1'-biphenyl]-4-carboxamido)-3-chloro-N-neopentylpicolinamide;
5-(2'-amino-5-chloro-2,4'-difluoro-[1,1'-biphenyl]-4-carboxamido)-3-chloro-N-(furan-3-ylmethyl)picolinamide;
5-(2'-amino-5-chloro-2,4'-difluoro-[1,1'-biphenyl]-4-carboxamido)-3-chloro-N-propylpicolinamide;
5 -(2'-amino-5 -chloro-2,4'-difluoro- [1,1 '-biphenyl] -4-carboxamido)-3 -chloro-N-(2-methoxyethyl)picolinamide;
5-(2'-amino-5-chloro-2,4'-difluoro-[1,1'-biphenyl]-4-carboxamido)-3-chloro-N-(1-cyanoethyl)picolinamide;
5-(2'-amino-5-chloro-2,4'-difluoro-[1,1 '-biphenyl] -4-carboxamido)-3 -chloro-N-((3,3-difluorocyclobutyl)methyl)picolinamide;
N-((1,3,4-oxadiazol-2-yl)methyl)-5-(2'-amino-5-chloro-2,4'-difluoro-[1,1'-biphenyl]-4-carboxamido)-3-chloropicolinamide;
5-(2'-amino-5-chloro-2,4'-difluoro-[1,1'-biphenyl]-4-carboxamido)-3-chloro-N-(2-(dimethylamino)ethyl)picolinamide;
5-(2'-amino-5-chloro-2,4'-difluoro-[1,1'-biphenyl]-4-carboxamido)-3-chloro-N-(cyclobutylmethyl)picolinamide;

5-(2'-amino-5-chloro-2,4'-difluoro-[1,1'-biphenyl]-4-carboxamido)-3-chloro-N-((1-methyl-1H-pyrazol-3-yl)methyl)picolinamide;
5-(4-(2-amino-6-fluoropyridin-3-yl)-2-chloro-5-fluorobenzamido)-3-chloro-N-(cyanomethyl)picolinamide;
5-(2'-amino-5-chloro-2,4'-difluoro-[1,1'-biphenyl]-4-carboxamido)-3-chloro-N-(2,2-difluoro-2X3-ethyl)picolinamide;
5-(2'-amino-5-chloro-2,4'-difluoro-[1,1'-biphenyl]-4-carboxamido)-3-chloro-N-(2,2,3,3,4,4,4-heptafluorobutyl)picolinamide;
5-(2'-amino-5-chloro-2,4'-difluoro-[1,1'-biphenyl]-4-carboxamido)-3-chloro-N-(2-(trifluoromethoxy)ethyl)picolinamide;
2,2'-diamino-5-chloro-N-(5-chloro-6-(2H-1,2,3-triazol-2-yl)pyridin-3-yl)-4'-fluoro-[1,1'-biphenyl]-4-carboxamide;
5-(2'-amino-5-chloro-2,4'-difluoro-[1,1'-biphenyl]-4-carboxamido)-3-chloro-N-(2-fluoroethyl)picolinamide;
5-(2'-amino-5-chloro-2,4'-difluoro-[1,1'-biphenyl]-4-carboxamido)-3-chloro-N-((5-methyl-1,3,4-oxadiazol-2-yl)methyl)picolinamide;
N-((1H-1,2,4-triazol-3-yl)methyl)-5-(2'-amino-5-chloro-2,4'-difluoro-[1,1'-biphenyl]-4-carboxamido)-3-chloropicolinamide;
5-(2'-amino-5-chloro-2,4'-difluoro-[1,1'-biphenyl]-4-carboxamido)-3-chloro-N-(2-(ethylthio)ethyl)picolinamide;
N-(2-amino-2-oxoethyl)-5-(2'-amino-5-chloro-2,4'-difluoro-[1,1'-biphenyl]-4-carboxamido)-3-chloropicolinamide;
5-(2'-amino-5-chloro-2,4'-difluoro-[1,1'-biphenyl]-4-carboxamido)-3-chloro-N-((1-cyanocyclopropyl)methyl)picolinamide;
5-(2'-amino-5-chloro-2,4'-difluoro-[1,1 '-biphenyl] -4-carboxamido)-3 -chloro-N-(2,2,3,3,3 -pentafluoropropyl)picolinamide;
5-(2'-amino-5-chloro-2,4'-difluoro-[1,1'-biphenyl]-4-carboxamido)-3-chloro-N-((1-(trifluoromethyl)cyclopropyl)methyl)picolinamide;
5-(2'-amino-5-chloro-2,4'-difluoro-[1,1'-biphenyl]-4-carboxamido)-3-chloro-N-(2-(methylsulfonyl)ethyl)picolinamide;
5-(4-(3-amino-5-ethynylpyridin-4-yl)-2-chloro-5-fluorobenzamido)-3-chloro-N-(2,2,2-trifluoroethyl)picolinamide;
5-(4-(3-amino-5-ethynylpyridin-4-yl)-2-chloro-5-fluorobenzamido)-3-chloro-N-(2,2-difluoroethyl)picolinamide;
5-(4-(3-amino-5-ethynylpyridin-4-yl)-2-chloro-5-fluorobenzamido)-3-chloro-N-(2-fluoroethyl)picolinamide;
5-(4-(3-amino-5-ethynylpyridin-4-yl)-2-chloro-5-fluorobenzamido)-3-chloro-N-((1,1-dioxidotetrahydro-2H-thiopyran-4-yl)methyl)picolinamide;
5-(4-(3-amino-5-ethynylpyridin-4-yl)-2-chloro-5-fluorobenzamido)-3-chloro-N-(furan-3-ylmethyl)picolinamide;
N-(6-(2H-1,2,3-triazol-2-yl)-5-(trifluoromethyl)pyridin-3-yl)-4-(3-amino-5-cyclopropylpyridin-4-yl)-2-chloro-5-fluorobenzamide;
5-(4-(3-amino-5-ethynylpyridin-4-yl)-2-chloro-5-fluorobenzamido)-3-chloro-N-(1-cyanoethyl)picolinamide;
5-(4-(3-amino-5-ethynylpyridin-4-yl)-2-chloro-5-fluorobenzamido)-3-chloro-N-(cyclopropylmethyl)picolinamide;
5-(4-(3-amino-5-ethynylpyridin-4-yl)-2-chloro-5-fluorobenzamido)-3-chloro-N-neopentylpicolinamide;
5-(4-(3-amino-5-ethynylpyridin-4-yl)-2-chloro-5-fluorobenzamido)-3-chloro-N-ethylpicolinamide;
5-(4-(3-amino-5-ethynylpyridin-4-yl)-2-chloro-5-fluorobenzamido)-3-chloro-N-(1-cyanocyclopropyl)picolinamide;
5-(4-(3-amino-5-ethynylpyridin-4-yl)-2-chloro-5-fluorobenzamido)-3-chloro-N-((1-cyanocyclopropyl)methyl)picolinamide;
5-(4-(3-amino-5-ethynylpyridin-4-yl)-2-chloro-5-fluorobenzamido)-3-chloro-N-(2-(methylsulfonyl)ethyl)picolinamide;
5-(4-(3-amino-5-ethynylpyridin-4-yl)-2-chloro-5-fluorobenzamido)-3-chloro-N-(2-(ethylthio)ethyl)picolinamide;
5-(4-(3-amino-5-ethynylpyridin-4-yl)-2-chloro-5-fluorobenzamido)-3-chloro-N-((5-methyl-1,3,4-oxadiazol-2-yl)methyl)picolinamide;
4-(3-amino-5-ethynyl-2-fluoropyridin-4-yl)-2-chloro-N-(5-chloro-6-(2H-1,2,3-triazol-2-yl)pyridin-3-yl)-5-fluorobenzamide;
4-(5-amino-3-ethynyl-2-fluoropyridin-4-yl)-2-chloro-N-(5-chloro-6-(2H-1,2,3-triazol-2-yl)pyridin-3-yl)-5-fluorobenzamide;
N-(6-(2H-1,2,3-triazol-2-yl)-5-(trifluoromethyl)pyridin-3-yl)-4-(3-amino-5-fluoropyridin-2-yl)-2-chloro-5-ethynylbenzamide;
N-(6-(2H-1,2,3-triazol-2-yl)-5-(trifluoromethyl)pyridin-3-yl)-4-(3-amino-5-(2-methylprop-1-en-1-yl)pyridin-4-yl)-2-chloro-5-fluorobenzamide;
N-(6-(2H-1,2,3-triazol-2-yl)-5-(trifluoromethyl)pyridin-3-yl)-4-(3-amino-5-(1-ethoxyvinyl)pyridin-4-yl)-2-chloro-5-fluorobenzamide;
N-(6-(2H-1,2,3-triazol-2-yl)-5-(trifluoromethyl)pyridin-3-yl)-4-(3-amino-5-(1-ethoxyethyl)pyridin-4-yl)-2-chloro-5-fluorobenzamide;
N-(6-(2H-1,2,3-triazol-2-yl)-5-(trifluoromethyl)pyridin-3-yl)-4-(3-amino-5-(1-hydroxyethyl)pyridin-4-yl)-2-chloro-5-fluorobenzamide;

N-(6-(2H-1,2,3-triazol-2-yl)-5-(trifluoromethyl)pyridin-3-yl)-4-(3-amino-5-(2-cyanoethyl)pyridin-4-yl)-2-chloro-5-fluorobenzamide;
N-(6-(2H-1,2,3-triazol-2-yl)-5-(trifluoromethyl)pyridin-3-yl)-4-(3-amino-5-(2-ethoxyethyl)pyridin-4-yl)-2-chloro-5-fluorobenzamide;
N-(6-(2H-1,2,3-triazol-2-yl)-5-(trifluoromethyl)pyridin-3-yl)-4-(3-acetyl-5-aminopyridin-4-yl)-2-chloro-5-fluorobenzamide;
N-(6-(2H-1,2,3-triazol-2-yl)-5-(trifluoromethyl)pyridin-3-yl)-4-(3-amino-5-(3,3,3-trifluoroprop-1-en-2-yl)pyridin-4-yl)-2-chloro-5-fluorobenzamide;
(E)-N-(6-(2H-1,2,3-triazol-2-yl)-5-(trifluoromethyl)pyridin-3-yl)-4-(3-amino-5-(3-hydroxy-3-methylbut-1-en-1-yl)pyridin-4-yl)-2-chloro-5-fluorobenzamide;
(E)-N-(6-(2H-1,2,3-triazol-2-yl)-5-(trifluoromethyl)pyridin-3-yl)-4-(3-amino-5-(2-cyclopropylvinyl)pyridin-4-yl)-2-chloro-5-fluorobenzamide;
N-(6-(2H-1,2,3-triazol-2-yl)-5-(trifluoromethyl)pyridin-3-yl)-4-(3-amino-5-(2-cyclopropylethyl)pyridin-4-yl)-2-chloro-5-fluorobenzamide;
N-(6-(2H-1,2,3-triazol-2-yl)-5-(trifluoromethyl)pyridin-3-yl)-4-(3-amino-5-(3-hydroxy-3-methylbutyl)pyridin-4-yl)-2-chloro-5-fluorobenzamide;
(E)-N-(6-(2H-1,2,3-triazol-2-yl)-5-(trifluoromethyl)pyridin-3-yl)-4-(3-amino-5-(3-methoxyprop-1-en-1-yl)pyridin-4-yl)-2-chloro-5-fluorobenzamide;
N-(6-(2H-1,2,3-triazol-2-yl)-5-(trifluoromethyl)pyridin-3-yl)-4-(3-amino-5-(3-methoxypropyl)pyridin-4-yl)-2-chloro-5-fluorobenzamide;
5-(4-(3-amino-5-ethynylpyridin-4-yl)-2-chloro-5-fluorobenzamido)-3-chloro-N-(2,2,2-trifluoroethyl)picolinamide;
4-(3-amino-5-(1-(difluoromethyl)-1H-pyrazol-4-yl)pyridin-4-yl)-2-chloro-N-(5-chloro-6-(2H-1,2,3-triazol-2-yl)pyridin-3-yl)-5-fluorobenzamide;
4-(3-amino-5-(prop-1-en-2-yl)pyridin-4-yl)-2-chloro-N-(5-chloro-6-(2H-1,2,3-triazol-2-yl)pyridin-3-yl)-5-fluorobenzamide;
4-(3-amino-5-ethynylpyridin-4-yl)-2-chloro-N-(5-ethynyl-6-(2H-1,2,3-triazol-2-yl)pyridin-3-yl)-5-fluorobenzamide;
4-(3-amino-5-cyclopropylpyridin-4-yl)-2-chloro-N-(5-chloro-6-(2H-1,2,3-triazol-2-yl)pyridin-3-yl)-5-fluorobenzamide;
4-(3-amino-5-vinylpyridin-4-yl)-2-chloro-N-(5-chloro-6-(2H-1,2,3-triazol-2-yl)pyridin-3-yl)-5-fluorobenzamide;
4-(3-amino-5-(prop-1-yn-1-yl)pyridin-4-yl)-2-chloro-N-(5-chloro-6-(2H-1,2,3-triazol-2-yl)pyridin-3-yl)-5-fluorobenzamide formate;
4-(3-amino-5-(cyclohex-1-en-1-yl)pyridin-4-yl)-2-chloro-N-(5-chloro-6-(2H-1,2,3-triazol-2-yl)pyridin-3-yl)-5-fluorobenzamide;
N-(6-(2H-1,2,3-triazol-2-yl)-5-(trifluoromethyl)pyridin-3-yl)-4-(3-amino-5-bromo-2-ethynylpyridin-4-yl)-2-chloro-5-fluorobenzamide;
N-(6-(2H-1,2,3-triazol-2-yl)-5-(trifluoromethyl)pyridin-3-yl)-4-(3-amino-2,5-diethynylpyridin-4-yl)-2-chloro-5-fluorobenzamide;
4-(3-amino-5-(cyclopent-1 -en-1 -yl)pyridin-4-yl)-2-chloro-N-(5-chloro-6-(2H-1,2,3-triazol-2-yl)pyridin-3-yl)-5-fluorobenzamide;
4-(3-amino-5-ethynylpyridin-4-yl)-2-chloro-5-fluoro-N-(6-(trifluoromethyl)pyridin-3-yl)benzamide;
4-(3-amino-5-(cyclopropylethynyl)pyridin-4-yl)-2-chloro-N-(5-chloro-6-(2H-1,2,3-triazol-2-yl)pyridin-3-yl)-5-fluorobenzamide;
N-(6-(2H-1,2,3-triazol-2-yl)-5-(trifluoromethyl)pyridin-3-yl)-4-(3-amino-5-ethynylpyridin-4-yl)-3-fluorobenzamide;
5-(4-(3-amino-5-cyclopropylpyridin-4-yl)-2-chloro-5-fluorobenzamido)-3-chloro-N-(2,2,2-trifluoroethyl)picolinamide;
N-(6-(2H-1,2,3-triazol-2-yl)-5-(trifluoromethyl)pyridin-3-yl)-4-(3-amino-5-bromopyridin-4-yl)-2-chloro-5-fluorobenzamide;
4-(3-amino-5-ethynylpyridin-4-yl)-2-chloro-5-fluoro-N-(2-(trifluoromethyl)pyridin-4-yl)benzamide;
5-(4-(3-amino-5-cyclopropylpyridin-4-yl)-2-chloro-5-fluorobenzamido)-3-cyclopropyl-N-(2,2,2-trifluoroethyl)picolinamide;
N-(6-(2H-1,2,3-triazol-2-yl)-5-(trifluoromethyl)pyridin-3-yl)-4-(5-amino-3-ethynyl-2-fluoropyridin-4-yl)-2-chloro-5-fluorobenzamide;
N-(6-(2H-1,2,3-triazol-2-yl)-5-(trifluoromethyl)pyridin-3-yl)-4-(5-amino-3-bromo-2-fluoropyridin-4-yl)-2-chloro-5-fluorobenzamide;
N-(6-(2H-1,2,3-triazol-2-yl)-5-(trifluoromethyl)pyridin-3-yl)-4-(3-amino-5-(3,6-dihydro-2H-pyran-4-yl)pyridin-4-yl)-2-chloro-5-fluorobenzamide;
N-(6-(2H-1,2,3-triazol-2-yl)-5-(trifluoromethyl)pyridin-3-yl)-4-(3-amino-5-(3,6-dihydro-2H-pyran-4-yl)pyridin-4-yl)-3-fluorobenzamide;

4-(3-amino-5-cyclopropylpyridin-4-yl)-2-chloro-5-fluoro-N-(2-(trifluoromethyl)pyridin-4-yl)benzamide;
2'-amino-5-chloro-N-(5-cyclopropyl-6-(2H-1,2,3-triazol-2-yl)pyridin-3-yl)-2,4'-difluoro-[1,1'-biphenyl]-4-carboxamide;
2-chloro-4-(3-cyclopropyl-5-(methylamino)pyridin-4-yl)-5-fluoro-N-(2-(trifluoromethyl)pyridin-4-yl)benzamide;
N-(6-(2H-1,2,3-triazol-2-yl)-5-(trifluoromethyl)pyridin-3-yl)-2'-amino-5-chloro-6'-cyclopropyl-2,4'-difluoro-[1,1'-biphenyl]-4-carboxamide;
N-(6-(2H-1,2,3-triazol-2-yl)-5-(trifluoromethyl)pyridin-3-yl)-4-(3-amino-5-(2,5-dihydrofuran-3-yl)pyridin-4-yl)-2-chloro-5-fluorobenzamide;
4-(3-acetyl-5-aminopyridin-4-yl)-2-chloro-N-(5-chloro-6-(2H-1,2,3-triazol-2-yl)pyridin-3 -yl)-5-fluorobenzamide;
N-(6-(2H-1,2,3-triazol-2-yl)-5-(trifluoromethyl)pyridin-3-yl)-4-(3-amino-5-(4-hydroxycyclohex-1 -en-1 -yl)pyridin-4-yl)-2-chloro-5-fluorobenzamide;
N-(6-(2H-1,2,3-triazol-2-yl)-5-(trifluoromethyl)pyridin-3-yl)-4-(3-amino-5-phenylpyridin-4-yl)-2-chloro-5-fluorobenzamide;
2'-amino-5-chloro-6'-(2-ethoxyethyl)-2,4'-difluoro-N-(2-(trifluoromethyl)pyridin-4-yl)-[1,1'-biphenyl]-4-carboxamide;
2'-amino-5-chloro-2,4'-difluoro-6'-(tetrahydrofuran-2-yl)-N-(2-(trifluoromethyl)pyridin-4-yl)-[1,1'-biphenyl]-4-carboxamide;
2'-amino-5-chloro-2,4'-difluoro-6'-(4-morpholinocyclohexyl)-N-(2-(trifluoromethyl)pyridin-4-yl)-[1,1'-biphenyl]-4-carboxamide;
2'-amino-5-chloro-2,4'-difluoro-6'-(4-hydroxycyclohexyl)-N-(2-(trifluoromethyl)pyridin-4-yl)-[1,1'-biphenyl]-4-carboxamide;
2'-amino-5-chloro-2,4'-difluoro-6'-(2-oxo-1,2-dihydropyridin-4-yl)-N-(2-(trifluoromethyl)pyridin-4-yl)-[1,1'-biphenyl]-4-carboxamide;
2'-amino-5-chloro-2,4'-difluoro-6'-(6-oxo-1,6-dihydropyridin-3-yl)-N-(2-(trifluoromethyl)pyridin-4-yl)-[1,1'-biphenyl]-4-carboxamide;
2'-amino-5-chloro-2,4'-difluoro-6'-(1-methylpiperidin-3-yl)-N-(2-(trifluoromethyl)pyridin-4-yl)-[1,1'-biphenyl]-4-carboxamide;
2'-(1-acetylpiperidin-4-yl)-6'-amino-5-chloro-2,4'-difluoro-N-(2-(trifluoromethyl)pyridin-4-yl)-[1,1'-biphenyl]-4-carboxamide;
2'-amino-5-chloro-2,4'-difluoro-6'-(spiro[2.5]octan-6-yl)-N-(2-(trifluoromethyl)pyridin-4-yl)-[1,1'-biphenyl]-4-carboxamide;
2'-amino-5-chloro-2,4'-difluoro-6'-(2-oxo-1,2-dihydropyrimidin-5-yl)-N-(2-(trifluoromethyl)pyridin-4-yl)-[1,1'-biphenyl]-4-carboxamide;
2'-amino-5-chloro-2,4'-difluoro-6'-(1-(2,2,2-trifluoroacetyl)piperidin-4-yl)-N-(2-(trifluoromethyl)pyridin-4-yl)-[1,1'-biphenyl]-4-carboxamide;
2'-amino-5-chloro-2,4'-difluoro-6'-(4-(trifluoromethyl)cyclohexyl)-N-(2-(trifluoromethyl)pyridin-4-yl)-[1,1'-biphenyl]-4-carboxamide;
2'-amino-5-chloro-2,4'-difluoro-6'-(1-hydroxyethyl)-N-(2-(trifluoromethyl)pyridin-4-yl)-[1,1 '-biphenyl]-4-carboxamide;
4-(3-amino-5-bromo-2-chloropyridin-4-yl)-2-chloro-5-fluoro-N-(2-(trifluoromethyl)pyridin-4-yl)benzamide;
4-(3-amino-2,5-dimethylpyridin-4-yl)-2-chloro-5-fluoro-N-(2-(trifluoromethyl)pyridin-4-yl)benzamide;
4-(3-amino-5-bromo-2-methoxypyridin-4-yl)-2-chloro-5-fluoro-N-(2-(trifluoromethyl)pyridin-4-yl)benzamide;
2-chloro-4-(2,3-diamino-5-ethynylpyridin-4-yl)-5-fluoro-N-(2-(trifluoromethyl)pyridin-4-yl)benzamide;
4-(2-amino-5-ethynyl-3-iodopyridin-4-yl)-2-chloro-5-fluoro-N-(2-(trifluoromethyl)pyridin-4-yl)benzamide;
4-(3-amino-5-ethynyl-2-methylpyridin-4-yl)-2-chloro-5-fluoro-N-(2-(trifluoromethyl)pyridin-4-yl)benzamide;
N-(6-(2H-1,2,3-triazol-2-yl)-5-(trifluoromethyl)pyridin-3-yl)-4-(3-amino-5-ethynyl-2-methylpyridin-4-yl)-2-chloro-5-fluorobenzamide;
N-(6-(2H-1,2,3-triazol-2-yl)-5-(trifluoromethyl)pyridin-3-yl)-4-(3-amino-5-ethynyl-2-fluoropyridin-4-yl)-2-chloro-5-fluorobenzamide;
N-(6-(2H-1,2,3-triazol-2-yl)-5-(trifluoromethyl)pyridin-3-yl)-4-(3-amino-2-chloro-5-ethynylpyridin-4-yl)-2-chloro-5-fluorobenzamide;
N-(6-(2H-1,2,3-triazol-2-yl)-5-(trifluoromethyl)pyridin-3-yl)-4-(3-amino-2-cyano-5-ethynylpyridin-4-yl)-2-chloro-5-fluorobenzamide;
N-(6-(2H-1,2,3-triazol-2-yl)-5-(trifluoromethyl)pyridin-3-yl)-4-(3-amino-5-ethynyl-2-methoxypyridin-4-yl)-2-chloro-5-fluorobenzamide;
N-(6-(2H-1,2,3-triazol-2-yl)-5-(trifluoromethyl)pyridin-3-yl)-4-(3-amino-5-ethynyl-6-fluoro-2-methylpyridin-4-yl)-2-chloro-5-fluorobenzamide;
N-(6-(2H-1,2,3-triazol-2-yl)-5-(trifluoromethyl)pyridin-3-yl)-4-(3-amino-5-ethynyl-2,6-difluoropyridin-4-yl)-2-chloro-5-fluorobenzamide;

N-(6-(2H-1,2,3-triazol-2-yl)-5-(trifluoromethyl)pyridin-3-yl)-4-(3-amino-5-bromo-2-chloropyridin-4-yl)-2-chloro-5-fluorobenzamide;
N-(6-(2H-1,2,3-triazol-2-yl)-5-(trifluoromethyl)pyridin-3-yl)-4-(3-amino-5-ethynyl-2-hydroxypyridin-4-yl)-2-chloro-5-hydroxybenzamide;
N-(6-(2H-1,2,3-triazol-2-yl)-5-(trifluoromethyl)pyridin-3-yl)-4-(3-amino-5-bromo-2-hydroxypyridin-4-yl)-2-chloro-5-hydroxybenzamide;
4-(4-((6-(2H-1,2,3-triazol-2-yl)-5-(trifluoromethyl)pyridin-3-yl)carbamoyl)-5-chloro-2-fluorophenyl)-3-amino-5-ethynylpicolinamide;
N-(6-(2H-1,2,3-triazol-2-yl)-5-(trifluoromethyl)pyridin-3-yl)-4-(3-amino-5-ethynylpyridazin-4-yl)-2-chloro-5-fluorobenzamide;
N-(6-(2H-1,2,3-triazol-2-yl)-5-(trifluoromethyl)pyridin-3-yl)-4-(2-amino-4-ethynyl-6-fluoropyridin-3-yl)-2-chloro-5-fluorobenzamide;
N-(6-(2H-1,2,3-triazol-2-yl)-5-(trifluoromethyl)pyridin-3-yl)-4-(2-amino-4-ethynylpyridin-3-yl)-2-chloro-5-fluorobenzamide;
N-((1S)-1-(4-(4-(3-amino-5-ethynylpyridin-4-yl)-2-chloro-5-fluorobenzamide)phenyl)-2,2,2-trifluoroethyl)-N-methyltetrahydro2H-thiopyran-4-carboxamide 1,1 -dioxide;
4-(3-amino-2-chloro-5-ethynylpyridin-4-yl)-2-chloro-N-(5-chloro-6-(2H-1,2,3-triazol-2-yl)pyridin-3-yl)-5-fluorobenzamide;
4-(3-amino-2-chloro-5-ethynylpyridin-4-yl)-2-chloro-5-fluoro-N-(2-(trifluoromethyl)pyridin-4-yl)benzamido;
4-(3-amino-5-ethynylpyridin-4-yl)-2-chloro-5-fluoro-N-(5-hydroxy -6-(2H-1,2,3-triazol-2-yl)pyridin-3-yl)benzamido;
4-(3-amino-5-ethynylpyridin-4-yl)-2-chloro-5-fluoro-N-(5-methoxy-6-(2H-1,2,3-triazol-2-yl)pyridin-3-yl)benzamido;
4-(3-amino-5-ethynylpyridin-4-yl)-2-chloro-N-(5-(difluoromethoxy)-6-(2H-1,2,3 -triazol-2-yl)pyridin-3 -yl)-5 -fluorobenzamide; or
N-(6-(2H-1,2,3-triazol-2-yl)-5-(trifluoromethyl)pyridin-3-yl)-4-(5-amino-3-ethynyl-2-fluoropyridin-4-yl)-2-chloro-5-fluorobenzamide.

A second object of the present invention is to provide a pharmaceutical composition comprising a therapeutically effective amount of at least a compound of Formula I,or a stereoisomer, tautomer, deuterated compound, pharmaceutically acceptable salt, prodrug, chelate, non-covalent complex, or solvate thereof as an active ingredient, and at least one pharmaceutically acceptable ingredient, such as carrier or excipient.

A third object of the present invention is to provide use of a compound of Formula I or a stereoisomer, tautomer, deuterated compound, pharmaceutically acceptable salt, prodrug, chelate, non-covalent complex, or solvate thereof for the manufacture of a medicament for the treatment of diseases, syndromes, disorders or obstacles.

In some embodiments, the diseases, syndromes, disorders and obstacles are affected by MALT1 inhibition.

In some embodiments, the diseases, syndromes, disorders and obstacles are selected from diffuse large B-cell lymphoma (DLBCL), mantle cell lymphoma (MCL), follicular lymphoma (FL), mucosa-associated lymphoid tissue (MALT) lymphoma, rheumatoid arthritis (RA), psoriatic arthritis (PsA), psoriasis (Pso), ulcerative colitis (UC), Crohn's disease, systemic lupus erythematosus (SLE), asthma and chronic obstructive pulmonary disease (COPD).

A forth object of the present invention is to provide a method of treating diseases, syndromes, disorders or obstacles, wherein the diseases, syndromes, disorders and obstacles are affected by MALT1 inhibition, comprising administering to a patient in need the compound of Formula I, or a stereoisomer, tautomer, deuterated compound, pharmaceutically acceptable salt, prodrug, chelate, non-covalent complex, or solvate thereof.

In some embodiments, the diseases, syndromes, disorders and obstacles are selected from diffuse large B-cell lymphoma (DLBCL), mantle cell lymphoma (MCL), follicular lymphoma (FL), mucosa-associated lymphoid tissue (MALT) lymphoma, rheumatoid arthritis (RA), psoriatic arthritis (PsA), psoriasis (Pso), ulcerative colitis (UC), Crohn's disease, systemic lupus erythematosus (SLE), asthma and chronic obstructive pulmonary disease (COPD).

**Definition and description**

The general chemical terms used in the formula above have their usual meanings. For example, the term "halogen", as used herein, unless otherwise indicated, means fluoro, chloro, bromo or iodo.

As used herein, unless otherwise indicated, alkyl includes saturated monovalent hydrocarbon radicals having straight, or branched moieties. For example, alkyl radicals include methyl, ethyl, propyl, isopropyl, n-butyl, isobutyl, sec-butyl, t-butyl, n-pentyl, 3-(2-methyl)butyl, 2-pentyl, 2-methylbutyl, neopentyl, n-hexyl, 2-hexyl, and 2-methylpentyl. The alkyl group is preferably a $C_{1-8}$ alkyl, the $C_{1-8}$ alkyl is further preferably a $C_{1-6}$ alkyl, the $C_{1-6}$ alkyl is further preferably a $C_{1-3}$ alkyl. Among them, $C_{1-8}$, as in $C_{1-8}$ alkyl is defined to identify the group as having 1, 2, 3, 4, 5, 6, 7 or 8 carbon atoms in a linear or branched arrangement.

Alkoxy radicals are oxygen ethers formed from the previously described straight, branched chain or cyclic alkyl

groups.

Alkenyl and alkynyl groups include straight, branched chain or cyclic alkenes and alkynes. Likewise, "$C_{2-8}$ alkenyl" and "$C_{2-8}$ alkynyl" means an alkenyl or alkynyl radicals having 2, 3, 4, 5, 6, 7 or 8 carbon atoms in a linear or brached arrangement. For example, alkenyl radicals include ethenyl, propenyl, etc. For example, alkynyl radicals include ethynyl, propynyl, etc.

Unless otherwise stated, the term "heteroaryl" as used herein refers to a monocyclic or polycyclic (e.g., fused bicyclic) aromatic heterocycle having at least one heteroatom ring member selected from the group consisting of N, O, and / or S. and wherein the nitrogen or sulfur heteroatom may be optionally oxidized, and the nitrogen heteroatom may be optionally quaternized. The heteroaryl group is preferably a 5-10-membered heteroaryl group, and the 5-10-membered heteroaryl group is further preferably a 5-6-membered heteroaryl group. Examples of heteroaryl groups include, but are not limited to, thienyl, furyl, imidazolyl, isoxazolyl, oxazolyl, pyrazolyl, pyrrolyl, thiazolyl, thiadiazolyl, triazolyl, pyridyl, pyridyl Azinyl, indolyl, azaindolyl, indazolyl, benzimidazolyl, benzofuranyl, benzothienyl, benzisoxazolyl, benzoxazolyl, benzopyrazolyl , benzothiazolyl, benzothiadiazolyl, benzotriazolyladenyl, quinolyl or isoquinolyl.

Unless otherwise stated, the term "carbocyclyl" refers to a stable monocyclic, bicyclic or tricyclic ring with carbon atoms; they can be cyclic saturated alkyl groups or structures with partially unsaturated bonds. The carbocyclic group is preferably a $C_{3-14}$ carbocyclyl, the $C_{3-14}$ carbocyclic group is further preferably a $C_{3-8}$ carbocyclyl,the $C_{3-8}$ carbocyclic group is further preferably a $C_{3-6}$ carbocyclic, the $C_{3-6}$ carbocyclic group is further preferably a $C_{5-6}$ carbocyclyl. Examples of carbocyclic groups include, but are not limited to, cyclopropyl, cyclobutyl, cyclobutyl, cyclopentyl, and cyclohexenyl.

The term "heterocyclyl", as used herein, unless otherwise indicated, represents stable heteroatom-containing monocyclic, bicyclic or tricyclic rings, which can be saturated or partially unsaturated. They contain carbon atoms and 1-4 heteroatoms selected from N, O or S, and wherein nitrogen or Sulfur heteroatoms can optionally be oxidized, and nitrogen heteroatoms can optionally be quaternized. Heterocyclyl groups can be attached to any heteroatom or carbon atom, resulting in a stable structure. The heterocyclyl group is preferably a 3-14-membered heterocyclyl group, the 3-14-membered heterocyclyl group is further preferably a 3-8-membered heterocyclyl group or a 5-10-membered heterocyclyl group, and the 3-8-membered heterocyclic group The ring group is further preferably a 3-6-membered heterocyclyl group, and the 3-6-membered heterocyclyl group is further preferably a 5-6-membered heterocyclyl group. Examples of heterocyclic groups include, but are not limited to, oxiranyl, piperazinyl, morpholinyl, piperidinyl, tetrahydropyrrolyl, tetrahydrofuranyl, 4,5,6,7-tetrahydro-2H-[1 ,2,3]triazolyl[4,5-c]pyridyl, 4,5,6,7-tetrahydro-2H-indazolyl, 2,4,5,6-tetrahydrocyclopentyl[c] ]pyrazolyl, 2,4,5,6-tetrahydrocyclopentyl[d][1,2,3]triazolyl, 5,6,7,8-tetrahydro-[1,2,4 ]triazole[1,5-a]pyrazinyl, 5,6,7,8-tetrahydro[1,2,4]triazole[4,3-a]pyrazinyl, 4,5, 6,7-tetrahydro-1H-phenyl[d]imidazolyl, 2-azaspiro[3.3]heptyl, 2-oxo-7-azaspiro[4.4]nonanyl, 7-aza Bicyclo[2.2.1]heptyl, octahydrocyclopenta[c]pyrrolyl, 3-azabicyclo[3.1.0]hexyl, 3,4-dihydro-2H-benzo[b] [1,4]oxazine, 2H-benzo[b][1,4]oxazine-3(4H)-one, etc.

The term "substituted" refers to a group in which one or more hydrogen atoms are each independently replaced with the same or different substituent(s). Typical substituents include, but are not limited to, halogen (F, Cl, Br or I), $C_{1-8}$ alkyl, $C_{2-12}$ cycloalkyl, $-OR_{11}$, $SR_{11}$, =O, =S, $-C(O)R_{11}$, $-C(S)R_{11}$, $=NR_{11}$, $-C(O)OR_{11}$, $-C(S)OR_{11}$, $-NR_{11}R_{12}$, $-C(O)NR_{11}R_{12}$, cyano, nitro, $-S(O)_2R_{11}$, $-OS(O_2)OR_{11}$, $-OS(O)_2R_{11}$, $- OP(O)(OR_{11})(OR_{12})$; wherein $R_{11}$ and $R_{12}$ is independently selected from -H, $C_{1-8}$ alkyl, , $C_{1-8}$ haloalkyl. In some embodiments, the substituent(s) is independently selected from the group consisting of -F, -Cl, -Br, -I, -OH, trifluromethoxy, ethoxy, propyloxy, iso-propyloxy, n-butyloxy, isobutyloxy, t-butyloxy, $-SCH_3$, $-SC_2H_5$, formaldehyde group,$-C(OCH_3)$, cyano, nitro, $CF_3$, $-OCF_3$, amino, dimethylamino, methyl thio, sulfonyl and acetyl.

Examples of substituted alkyl group include, but not limited to, 2-aminoethyl, 2-hydroxyethyl, pentachloroethyl, trifluoromethyl, methoxymethyl, pentafluoroethyl and piperazinylmethyl.

Examples of substituted alkoxy groups include, but not limited to, aminomethoxy, tetrafluoromethoxy, 2-diethylaminoethoxy, 2-ethoxycarbonylethoxy, 3 -hydroxypropoxy.

The term "one or more" or "at least one" means one, two, three, four, five, six, seven, eight, nine or more.

The present invention includes within its scope the prodrugs of the compounds of this invention. In general, such prodrugs will be functional derivatives of the compounds that are readily converted in vivo into the required compound. Thus, in the methods of treatment of the present invention, the term "administering" shall encompass the treatment of the various disorders described with the compound specifically disclosed or with a compound which may not be specifically disclosed, but which converts to the specified compound in vivo after administration to the subject. Conventional procedures for the selection and preparation of suitable prodrug derivatives are described, for example, in "Design of Prodrugs", ed. H. Bundgaard, Elsevier, 1985.

It is intended that the definition of any substituent or variable at a particular location in a molecule be independent of its definitions elsewhere in that molecule. It is understood that substituents and substitution patterns on the compounds of this invention can be selected by one of ordinary skill in the art to provide compounds that are chemically stable and that can be readily synthesized by techniques know in the art as well as those methods set forth herein.

The term "stereoisomer" refers to a compound that contains one or more asymmetric centers (or axes) in its structure and is capable of stereoisomerism, including geometric isomers, optical isomers (including atropisomers) )

and conformational isomers. The present invention includes all possible diastereoisomers and their racemic mixtures, their substantially pure resolved enantiomers, all possible geometric isomers and their pharmaceutical acceptable salt.

The above Formula I are shown without a definitive stereochemistry at certain positions. The present invention includes all stereoisomers of Formula I and pharmaceutically acceptable salts thereof. Further, mixtures of stereoisomers as well as isolated specific stereoisomers are also included. During the course of the synthetic procedures used to prepare such compounds, or in using racemization or epimerization procedures known to those skilled in the art, the products of such procedures can be a mixture of stereoisomers.

When a tautomer of the compound of Formula I exists, the present invention includes any possible tautomers and pharmaceutically acceptable salts thereof, and mixtures thereof, except where specifically stated otherwise.

When the compound of Formula I and pharmaceutically acceptable salts thereof exist in the form of solvates or polymorphic forms, the present invention includes any possible solvates and polymorphic forms. A type of a solvent that forms the solvate is not particularly limited so long as the solvent is pharmacologically acceptable. For example, water, ethanol, propanol, acetone or the like can be used.

The term "pharmaceutically acceptable salts" refers to salts prepared from pharmaceutically acceptable non-toxic bases or acids. When the compound of the present invention is acidic, its corresponding salt can be conveniently prepared from pharmaceutically acceptable non-toxic bases, including inorganic bases and organic bases. Salts derived from such inorganic bases include aluminum, ammonium, calcium, copper (ic and ous), ferric, ferrous, lithium, magnesium, manganese (ic and ous), potassium, sodium, zinc and the like salts. Particularly preferred are the ammonium, calcium, magnesium, potassium and sodium salts. Salts derived from pharmaceutically acceptable organic non-toxic bases include salts of primary, secondary, and tertiary amines, as well as cyclic amines and substituted amines such as naturally occurring and synthesized substituted amines. Other pharmaceutically acceptable organic non-toxic bases from which salts can be formed include ion exchange resins such as, for example, arginine, betaine, caffeine, choline, N',N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine and the like.

When the compound of the present invention is basic, its corresponding salt can be conveniently prepared from pharmaceutically acceptable non-toxic acids, including inorganic and organic acids. Such acids include, for example, acetic, benzenesulfonic, benzoic, camphorsulfonic, citric, ethanesulfonic, formic, fumaric, gluconic, glutamic, hydrobromic, hydrochloric, isethionic, lactic, maleic, malic, mandelic, methanesulfonic, mucic, nitric, pamoic, pantothenic, phosphoric, succinic, sulfuric, tartaric, p-toluenesulfonic acid and the like. Preferred are citric, hydrobromic, formic, hydrochloric, maleic, phosphoric, sulfuric and tartaric acids, particularly preferred are formic and hydrochloric acid. Since the compounds of Formula I are intended for pharmaceutical use they are preferably provided in substantially pure form, for example at least 60% pure, more suitably at least 75% pure, especially at least 98% pure (% are on a weight for weight basis).

The pharmaceutical compositions of the present invention comprise a compound represented by Formula I (or a pharmaceutically acceptable salt thereof) as an active ingredient, a pharmaceutically acceptable carrier and optionally other therapeutic ingredients or adjuvants. The compositions include compositions suitable for oral, rectal, topical, and parenteral (including subcutaneous, intramuscular, and intravenous) administration, although the most suitable route in any given case will depend on the particular host, and nature and severity of the conditions for which the active ingredient is being administered. The pharmaceutical compositions may be conveniently presented in unit dosage form and prepared by any of the methods well known in the art of pharmacy.

In practice, the compounds represented by Formula I, or a prodrug, or a metabolite, or pharmaceutically acceptable salts thereof, of this invention can be combined as the active ingredient in intimate admixture with a pharmaceutical carrier according to conventional pharmaceutical compounding techniques. The carrier may take a wide variety of forms depending on the form of preparation desired for administration, e.g., oral or parenteral (including intravenous). Thus, the pharmaceutical compositions of the present invention can be presented as discrete units suitable for oral administration such as capsules, cachets or tablets each containing a predetermined amount of the active ingredient. Further, the compositions can be presented as a powder, as granules, as a solution, as a suspension in an aqueous liquid, as a non-aqueous liquid, as an oil-in-water emulsion, or as a water-in- oil liquid emulsion. In addition to the common dosage forms set out above, the compound represented by Formula I, or a pharmaceutically acceptable salt thereof, may also be administered by controlled release means and/or delivery devices. The compositions may be prepared by any of the methods of pharmacy. In general, such methods include a step of bringing into association the active ingredient with the carrier that constitutes one or more necessary ingredients. In general, the compositions are prepared by uniformly and intimately admixing the active ingredient with liquid carriers or finely divided solid carriers or both. The product can then be conveniently shaped into the desired presentation.

Thus, the pharmaceutical compositions of this invention may include a pharmaceutically acceptable carrier and a compound, or a stereoisomer, tautomer, deuterated compound, pharmaceutically acceptable salt, prodrug, chelate,

noncovalent complex, or solvate thereof. The compounds of Formula I, or pharmaceutically acceptable salts thereof, can also be included in pharmaceutical compositions in combination with one or more other therapeutically active compounds.

The pharmaceutical carrier employed can be, for example, a solid, liquid, or gas. Examples of solid carriers include such as lactose, terra alba, sucrose, talc, gelatin, agar, pectin, acacia, magnesium stearate, and stearic acid. Examples of liquid carriers include such as sugar syrup, peanut oil, olive oil, and water. Examples of gaseous carriers include such as carbon dioxide and nitrogen. In preparing the compositions for oral dosage form, any convenient pharmaceutical media may be employed. For example, water, glycols, oils, alcohols, flavoring agents, preservatives, coloring agents, and the like may be used to form oral liquid preparations such as suspensions, elixirs and solutions; while carriers such as starches, sugars, microcrystalline cellulose, diluents, granulating agents, lubricants, binders, disintegrating agents, and the like may be used to form oral solid preparations such as powders, capsules and tablets. Because of their ease of administration, tablets and capsules are the preferred oral dosage units whereby solid pharmaceutical carriers are employed. Optionally, tablets may be coated by standard aqueous or nonaqueous techniques.

A tablet containing the composition of this invention may be prepared by compression or molding, optionally with one or more accessory ingredients or adjuvants. Compressed tablets may be prepared by compressing, in a suitable machine, the active ingredient in a free-flowing form such as powder or granules, optionally mixed with a binder, lubricant, inert diluent, surface active or dispersing agent. molded tablets may be made by molding in a suitable machine, a mixture of the powdered compound moistened with an inert liquid diluent. Each tablet preferably contains from about 0.05mg to about 5g of the active ingredient and each cachet or capsule preferably containing from about 0.05mg to about 5g of the active ingredient. For example, a formulation intended for the oral administration to humans may contain from about 0.5mg to about 5g of active agent, compounded with an appropriate and convenient amount of carrier material which may vary from about 5 to about 95 percent of the total composition. Unit dosage forms will generally contain between from about 1 mg to about 2g of the active ingredient, typically 25mg, 50mg, 100mg, 200mg, 300mg, 400mg, 500mg, 600mg, 800mg, or 1000mg.

Pharmaceutical compositions of the present invention suitable for injectable use include sterile aqueous solutions or dispersions. Furthermore, the compositions can be in the form of sterile powders for the extemporaneous preparation of such sterile injectable solutions or dispersions. In all cases, the final injectable form must be sterile and must be effectively fluid for easy syringability. The pharmaceutical compositions must be stable under the conditions of manufacture and storage; thus, preferably should be preserved against the contaminating action of microorganisms such as bacteria and fungi. The carrier can be a solvent or dispersion medium containing, for example, water, ethanol, polyol (e.g., glycerol, propylene glycol and liquid polyethylene glycol), vegetable oils, and suitable mixtures thereof.

Pharmaceutical compositions of the present invention can be in a form suitable for topical use such as, for example, an aerosol, cream, ointment, lotion, dusting powder, or the like. Further, the compositions can be in a form suitable for use in transdermal devices. These formulations may be prepared, utilizing a compound represented by Formula I of this invention, or a pharmaceutically acceptable salt thereof, via conventional processing methods. As an example, a cream or ointment is prepared by admixing hydrophilic material and water, together with about 5wt% to about 10wt% of the compound, to produce a cream or ointment having a desired consistency.

Pharmaceutical compositions of this invention can be in a form suitable for rectal administration wherein the carrier is a solid. It is preferable that the mixture forms unit dose suppositories. Suitable carriers include cocoa butter and other materials commonly used in the art. The suppositories may be conveniently formed by first admixing the composition with the softened or melted carrier(s) followed by chilling and shaping in molds.

In addition to the aforementioned carrier ingredients, the pharmaceutical formulations described above may include, as appropriate, one or more additional carrier ingredients such as diluents, buffers, flavoring agents, binders, surface-active agents, thickeners, lubricants, preservatives (including antioxidants) and the like. Furthermore, other adjuvants can be included to render the formulation isotonic with the blood of the intended recipient. Compositions containing a compound described by Formula I, or pharmaceutically acceptable salts thereof, may also be prepared in powder or liquid concentrate form.

Generally, dosage levels on the order of from about 0.01mg/kg to about 150mg/kg of body weight per day are useful in the treatment of the above-indicated conditions, or alternatively about 0.5mg to about 7g per patient per day. For example, colon cancer, rectal cancer, mantle cell lymphoma, multiple myeloma, breast cancer, prostate cancer, glioblastoma, squamous cell esophageal cancer, liposarcoma, T-cell lymphoma melanoma, pancreatic cancer, or lung cancer, may be effectively treated by the administration of from about 0.01 to 50mg of the compound per kilogram of body weight per day, or alternatively about 0.5mg to about 3.5g per patient per day.

It is understood, however, that lower or higher doses than those recited above may be required. Specific dose level and treatment regimens for any particular subject will depend upon a variety of factors, including the activity of the specific compound employed, the age, body weight, general health, sex, diet, time of administration, route of administration, rate of excretion, drug combination, the severity and course of the particular disease undergoing therapy, the subject disposition to the disease, and the judgment of the treating physician.

These and other aspects will become apparent from the following written description of the invention.

The following Examples are provided to better illustrate the present invention. All parts and percentages are by weight and all temperatures are degrees Celsius, unless explicitly stated otherwise.

The invention will be described in greater detail by way of specific examples. The following examples are offered for illustrative purposes, and are not intended to limit the invention in any manner. Those of skill in the art will readily recognize a variety of non-critical parameters which can be changed or modified to yield essentially the same results.

**EXAMPLE**

It is to be understood that both the foregoing general description and the following detailed description are exemplary and explanatory only and are not limiting of any subject matter claimed. All parts and percentages are by weight unless expressly stated otherwise. All temperatures are in degrees Celsius. The raw materials for the synthesis method are not given in this invention, and the reagents can be obtained from commercial sources or synthesized by conventional methods as shown below using commercially available raw materials and reagents.

The following abbreviations have been used in the examples:

$POCl_3$: Phosphorus oxychloride;
DCM: Dichloromethane;
Dioxane: 1 ,4-dioxane;
$Pd(dppf)Cl_2$:1,1'-1,1'-bis(diphenylphosphine)ferrocenepalladium dichloride;
$K_2CO_3$: Potassium carbonate;
$Na_2CO_3$: Sodium carbonate;
PE: Petroleum ether;
EA: Ethyl acetate;
h or hrs: hour or hours;
Pd/C: Palladium carbon;
MeOH: Methanol;
$Pd(PPh_3)_4$: Tetrakis triphenylphosphine palladium;
NaOH: Sodium hydroxide;
LCMS: Liquid chromatography-mass spectrometry;
$NaNO_2$: Sodium nitrite;
KI: Potassium iodide;
$Na_2S_2O_3$: Sodium thiosulfate;
$H_2O$: water;
PTLC: Preparative thin layer chromatography;
TLC: Thin layer chromatography.

Examples: Synthesis of Compound A05

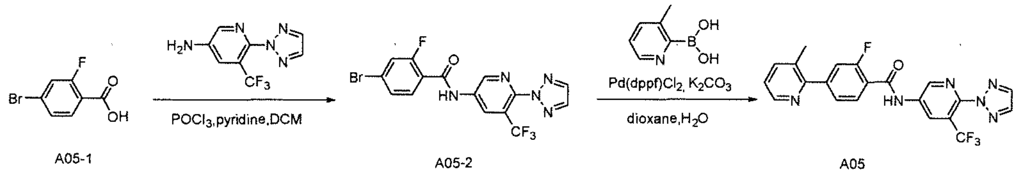


A05-1 A05-2 A05

Step1: Synthesis of Compound A05-2

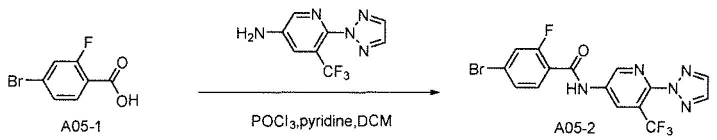


A05-1 A05-2

To a solution of A05-1 (4-bromo-2-fluorobenzoic acid) (2.19 g, 10.0 mmol) and 6-(2H-1,2,3-triazol-2-yl)-5-(trifluoromethyl)pyridin-3-amine (2.52 g, 11.0 mmol) in dichloromethane (25 mL) was added pyridine (1.19 g, 15.0 mmol) at room temperature. The mixture was cooled to 0 °C in an ice bath, and then phosphorus oxychloride was added dropwise (4.59 g, 30.0 mmol). The reaction was stirred for 1 hour and quenched by water, extracted with dichloromethane (250 mL). The organic layer was washed with brine, dried over anhydrous $Na_2SO_4$ and concentrated in vacuum. The residue was purified by flash column chromatography over silica gel to afford compound A05-2 (N-(6-(2H-1,2,3- triazol-2-yl)-5-(trifluoromethyl)pyridin-3-ly)-4-bromo-2-fluorobenzamide) (2.5 g). LC-MS ($ES^+$): *m/z* 430 $(M+H)^+$

Step2: Synthesis of Compound A05

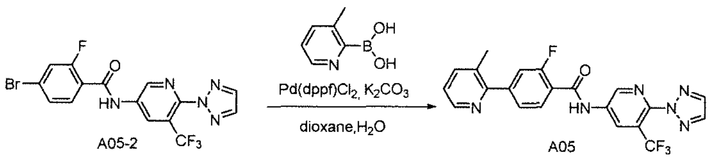


A05-2 A05

Into a 100-mL round-bottom flask, was placed A05-2 (215 mg, 0.5 mmol), (3-methylpyridin-2-yl)boronic acid (82 mg, 0.6mmol), Pd(dppf)$Cl_2$ (36.6 mg, 0.05 mmol), $K_2CO_3$(414mg, 3.0 mmol),dioxane (3 mL) and $H_2O$ (0.5 mL). The resulting mixture was stirred at 100°C for 2 hours under nitrogen. Removing of the solvent and purifying by column chromatography (PE / EtOAc =1 / 1) to give compound A05 (47 mg). LC-MS ($ES^+$): *m/z* 443 $[M+H]^+$

Example 12: Synthesis of Compound A12

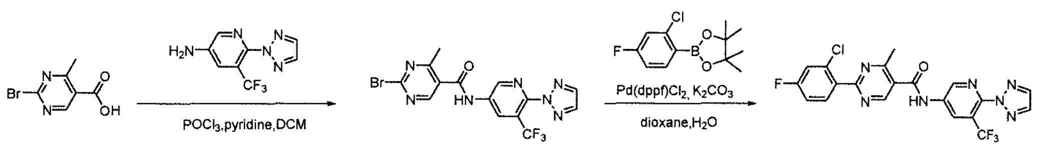


A12-1 A12-2 A12

Step 1: Synthesis of Compound A12-2

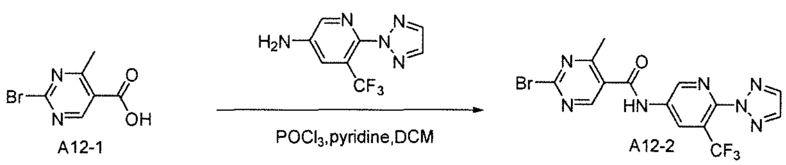


A12-1 A12-2

To a solution of A12-1 (2.17 g, 10.0 mmol) and 6-(2H-1,2,3-triazol-2-yl)-5-(trifluoromethyl)pyridin-3-amine (2.52 g, 11.0 mmol) in dichloromethane (25 mL), was added pyridine (1.19 g, 15.0 mmol) at room temperature. The mixture was cooled to 0°C in an ice bath, and then phosphorus oxychloride (4.59 g, 30.0 mmol) was added dropwise. The reaction was stirred at room temperature for 1 hour and quenched by water, extracted with dichloromethane (250 mL). The organic layer was washed with brine, dried over anhydrous $Na_2SO_4$ and concentrated in vacuum. The residue was purified by flash column chromatography over silica gel to afford compound A12-2(2.1 g). LC-MS ($ES^+$): *m/z* 428,430 $[M+H]^+$

Step2: Synthesis of Compound A12

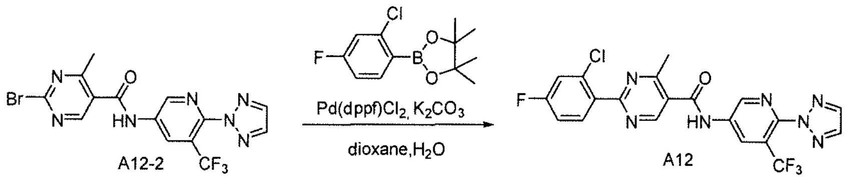

A12-2 A12

Into a 50-mL round-bottom flask, was placed A12-2 (215 mg, 0.5 mmol), 2-(2-chloro-4-fluorophenyl)-4,4,5,5-tetramethyl-1,3,2-dioxaborane (154 mg , 0.6mmol), Pd(dppf)$Cl_2$, (36.6 mg, 0.05 mmol), $K_2CO_3$(414mg, 3.0 mmol), dioxane (3 mL) and $H_2O$(0.5 mL). The resulting mixtue was stirred at 100°C for 2 hours under nitrogen. Removing of the solvent and purifying by column chromatography(PE / EtOAc =1 / 1) to give compound A12 (41 mg), LC-MS ($ES^+$): *m/z* 478 $[M+H]^+$

Example 35: Synthesis of Compound A35

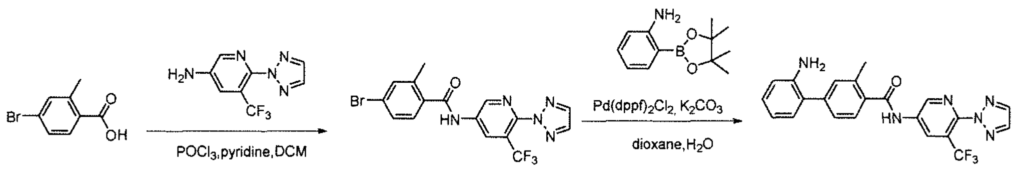

A35-1 A35-2 A35

Step1: Synthesis of Compound A35-2

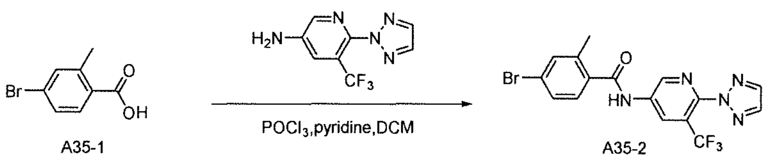

A35-1 A35-2

To a solution of A35-1 (2.15 g, 10.0 mmol) and 6-(2H-1,2,3-triazol-2-yl)-5-(trifluoromethyl)pyridin-3-amine (2.52 g, 11.0 mmol) in dichloromethane (25 mL), was added pyridine (1.19 g, 15.0 mmol) at room temperature. The mixture was cooled to 0°C in an ice bath, and then phosphorus oxychloride was added dropwise (4.59 g, 30.0 mmol). The reaction was stirred at room temperature for 1 hour and quenched by water, extracted with dichloromethane (250 mL). The organic layer was washed with brine, dried over anhydrous $Na_2SO_4$ and concentrated in vacuum. The residue was purified by flash column chromatography over silica gel to afford compound A35-2(1.8 g), LC-MS ($ES^+$): *m/z* 426 $[M+H]^+$

Step2: Synthesis of Compound A35

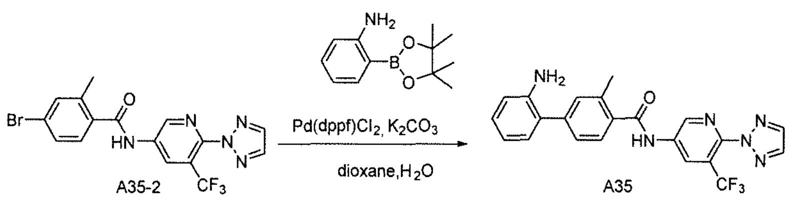

A35-2 A35

Into a 50-mL round-bottom flask, was placed A35-2 (213 mg, 0.5 mmol), 2-(4,4,5,5-Tetramethyl-1,3,2-dioxaborolan-2-yl)aniline (154 mg, 0.6mmol), Pd(dppf)$Cl_2$, (36.6 mg, 0.05 mmol), $K_2CO_3$(414mg, 3.0 mmol), dioxane (3 mL) and $H_2O$(0.5 mL). The resulting mixture was stirred at 100°C for 2 hours under nitrogen. Removing of the solvent and purifying by column chromatography to give compound A35 (60 mg), LC-MS ($ES^+$): *m/z* 439 $[M+H]^+$

Example41: Synthesis of Compound A41

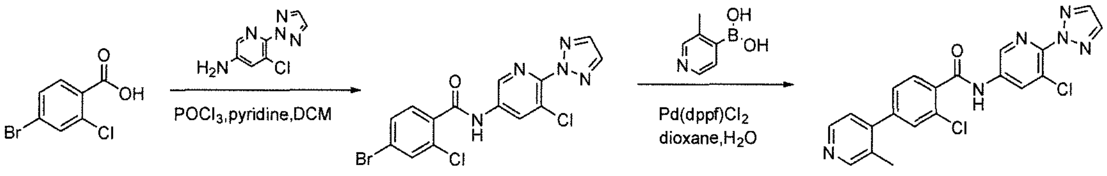


A41-1 A41-2 A41

Step 1: Synthesis of Compound A41-2

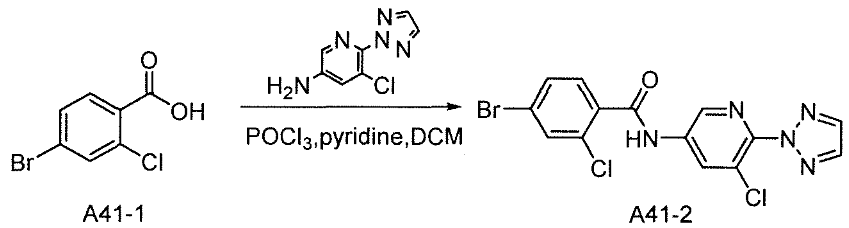


A41-1 A41-2

To a solution of A41-1 (2.35 g, 10.0 mmol) and 5-chloro-6-(2H-1,2,3-triazol-2-yl)pyridin-3-amine (2.15 g, 11.0 mmol) in dichloromethane (25 mL), was added pyridine (1.19 g, 15.0 mmol) at room temperature. The mixture was cooled to 0°C in an ice bath, and then phosphorus oxychloride was added dropwise (4.59 g, 30.0 mmol). The reaction was stirred at room temperature for 1 hour and quenched by water, extracted with dichloromethane (250 mL). The organic layer was washed with brine, dried over anhydrous $Na_2SO_4$ and concentrated in vacuum. The residue was purified by flash column chromatography over silica gel to afford compound A41-2 4-bromo-2-chloro-N-(5-chloro-6-(2H-1,2,3-triazol-2-yl)pyridin-3-yl)benzamide(3.1 g, yellow solid). LC-MS ($ES^+$): *m/z* 412,414 $[M+H]^+$

Step2: Synthesis of Compound A41

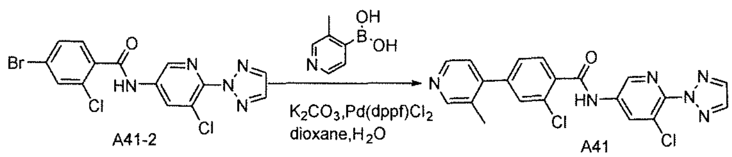


A41-2 A41

Into a 50-mL round-bottom flask, was placed A41-2(314 mg, 0.76 mmol), 3-methylpyridine-4-boronic acid (158 mg, 1.15 mmol), $Pd(dppf)Cl_2$(58.5 mg, 0.08 mmol), $K_2CO_3$(476 mg, 3.45 mmol), dioxane (3 mL) and $H_2O$(0.5 mL). The resulting mixture was stirred at 75°C for 2 hours under nitrogen. Removing of the solvent and purifying by column chromatography (PE / EtOAc =1 / 1) to give compound A41(50 mg, off-white solid). LC-MS ($ES^+$): *m/z* 425 $[M+H]^+$

Example42: Synthesis of Compound A42

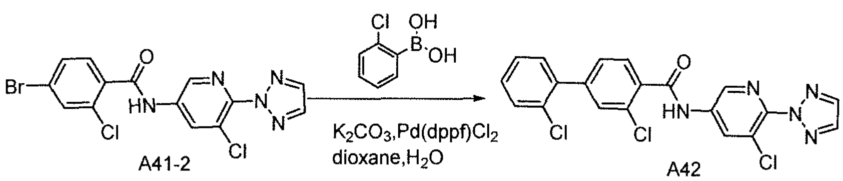


A41-2 A42

Into a 100-mL round-bottom flask, was placed A41-2 (314 mg, 0.76 mmol), 2-chlorophenylboronic acid (179.8 mg, 1.15 mmol), Pd(dppf)$Cl_2$(58.5 mg, 0.08 mmol), $K_2CO_3$ (476 mg, 3.45 mmol), dioxane (3 mL) and $H_2O$(0.5 mL). The resulting mixture was stirred at 75°C for 2 hours under nitrogen. Removing of the solvent and purifying by column chromatography (PE / EtOAc =1 / 1) to give compound A42 (40 mg off-white solid). LC-MS ($ES^+$): *m/z* 444 $[M+H]^+$

Example64: Synthesis of Compound A64

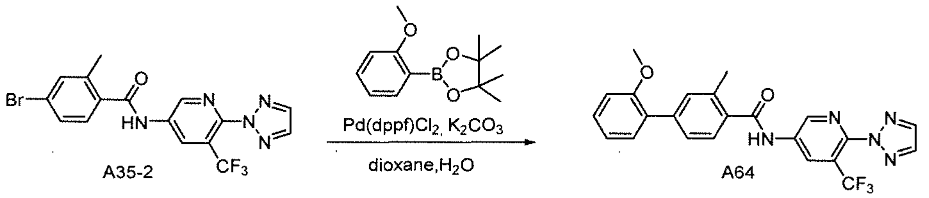


A35-2 A64

Into a 100-mL round-bottom flask, was placed A35-2 (213 mg, 0.5 mmol), 2-(2-methoxyphenyl)-4,4,5,5-tetramethyl-1,3,2-dioxaborane (140 mg, 0.6 mmol), Pd(dppf)$Cl_2$, (36.6 mg, 0.05 mmol), $K_2CO_3$(414mg, 3.0 mmol), dioxane(3 mL) and $H_2O$(0.5 mL). The resulting mixture was stirred at 100°C for 2 hours under nitrogen. Removing of the solvent and purifying by column chromatography(PE / EtOAc =1/1) to give compound A64 (50 mg), LC-MS ($ES^+$): *m/z* 454 $[M+H]^+$

Example98: Synthesis of Compound A98

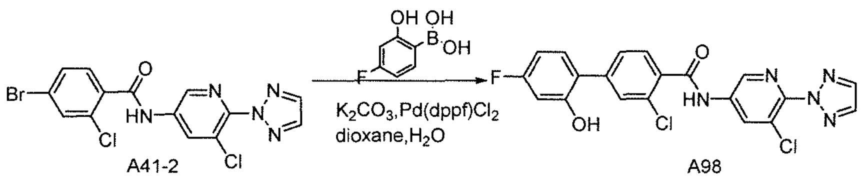


A41-2 A98

Into a 100-mL round-bottom flask, was placed A41-2 (314 mg, 0.76 mmol), 4-fluoro-2-hydroxyphenylboronic acid (179 mg, 1.15 mmol), Pd(dppf)$Cl_2$ (58.5 mg, 0.08 mmol), $K_2CO_3$ (476 mg, 3.45 mmol), dioxane(3 mL) and $H_2O$ (0.5 mL). The resulting mixture was stirred at 75°C for 2 hours under nitrogen. Removing of the solvent and purifying by column chromatography (PE / EtOAc =1 / 1) to give compound A98 (49 mg off-white solid). LC-MS ($ES^+$): *m/z* 444 $[M+H]^+$

Example 100: Synthesis of Compound A100

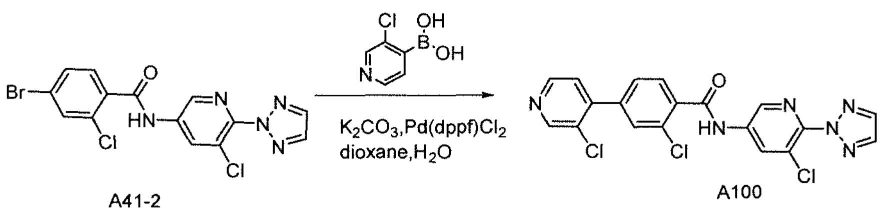


A41-2 A100

Into a 100-mL round-bottom flask, was placed A41-2 (314mg, 0.76 mmol), 3-chloropyridine-4-boronic acid (180mg, 1.15 mmol), Pd(dppf)$Cl_2$ (58.5 mg, 0.08 mmol), $K_2CO_3$(476 mg, 3.45 mmol), dioxane(3 mL) and $H_2O$(0.5 mL). The resulting mixture was stirred at 75°C for 2 hours under nitrogen. Removing of the solvent and purifying by column chromatography(PE / EtOAc =1 / 1) to give compound A100 (59 mg off-white solid) LC-MS ($ES^+$): *m/z* 445 $[M+H]^+$

Example 102: Synthesis of Compound A 102

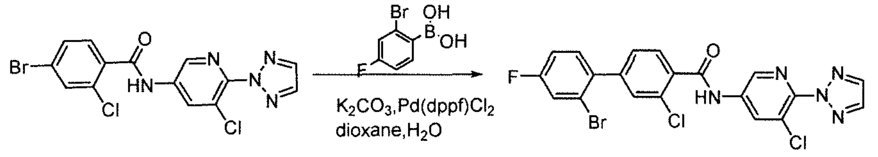


A41-2 A102

Into a 100-mL round-bottom flask, was placed A41-2 (314 mg, 0.76 mmol), 2-Bromo-4-fluorophenylboronic acid (252 mg, 1.15 mmol), Pd(dppf)$Cl_2$ (58.5 mg, 0.08 mmol), $K_2CO_3$(476 mg, 3.45 mmol), dioxane(3 mL) and $H_2O$(0.5 mL). The resulting mixture was stirred at 75°C for 2 hours. Removing of the solvent and purifying by column chromatography (PE / EtOAc =1 / 1) to give compound A102 (53 mg off-white solid). LC-MS ($ES^+$): *m/z* 506,508 $[M+H]^+$

A102:

$^1H$ NMR (500 MHz, DMSO-$d_6$) $\delta$ 11.41 (s, 1H), 8.76 (d, *J* = 87.2 Hz, 2H), 8.19 (s, 2H), 7.60 (dd, *J* = 126.7, 61.0 Hz, 6H).

Example 104: Synthesis of Compound A104

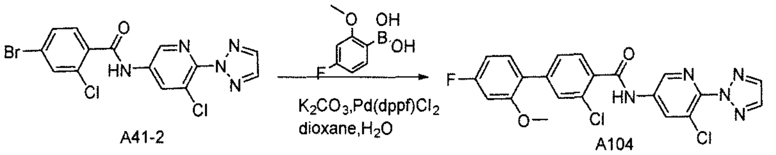


A41-2 A104

Into a 100-mL round-bottom flask, was placed A41-2 (314 mg, 0.76 mmol), 4-fluoro-2-methoxyphenylboronic acid (196 mg, 1.15 mmol), Pd(dppf)$Cl_2$ (58.5 mg, 0.08 mmol), $K_2CO_3$ (476 mg, 3.45 mmol), dioxane(3 mL) and $H_2O$(0.5 mL). The resulting mixture was stirred at 75°C for 2 hours under nitrogen. Removing of the solvent and purifying by column chromatography(PE / EtOAc =1 / 1) to give compound A104 (57 mg off-white solid). LC-MS ($ES^+$): *m/z* 458 $[M+H]^+$

Example 105: Synthesis of Compound A105

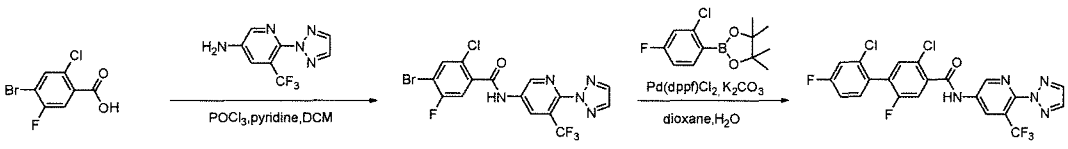


A105-1 A105-2 A105

Step1: Synthesis of Compound A105-2

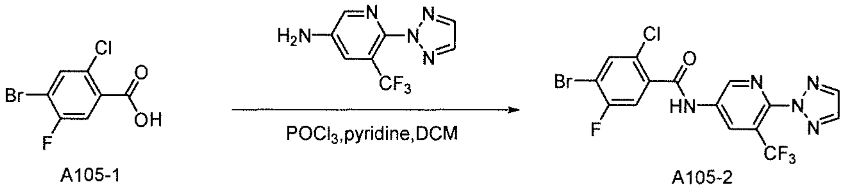

A105-1

A105-2

To a solution of A105-1 (2.54 g, 10.0 mmol) and 6-(2H-1,2,3-triazol-2-yl)-5-(trifluoromethyl)pyridin-3-amine (2.52 g, 11.0 mmol) in dichloromethane (25 mL) , was added pyridine (1.19 g, 15.0 mmol) at room temperature . The mixture was cooled to 0°C in an ice bath, and then phosphorus oxychloride was added dropwise (4.59 g, 30.0 mmol). The reaction was stirred at room temperature for 1 hour, and quenched by water, extracted with dichloromethane (250 mL). The organic layer was washed with brine, dried over anhydrous $Na_2SO_4$ and concentrated in vacuum.The residue was purified by flash column chromatography over silica gel to afford compound A105-2 (2.5 g). LC-MS ($ES^+$): *m/z* 464 $[M+H]^+$

Step2: Synthesis of Compound A105

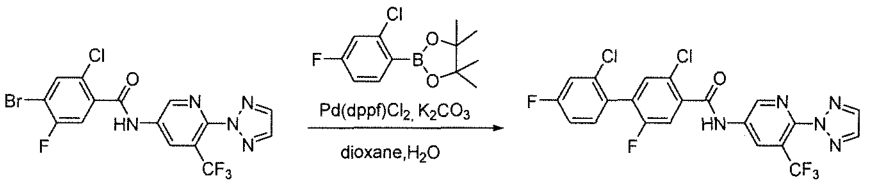

A105-2

A105

Into a 100-mL round-bottom flask, was placed A105-2 (232 mg, 0.5 mmol), 2-(2-Chloro-4-fluorophenyl)-4,4,5,5-tetramethyl-1,3,2-dioxaborane (154 mg, 0.6mmol), $Pd(dppf)Cl_2$ (40 mg, 0.05 mmol), $K_2CO_3$(414mg, 3.0 mmol), dioxane(3 mL) and $H_2O$(0.5 mL). The resulting mixture was stirred at 100°C for 2 hours under nitrogen. Removing of the solvent and purifying by column chromatography (PE / EtOAc =1 / 1) to give compound A105 (40 mg), LC-MS ($ES^+$): *m/z* 514 $[M+H]^+$

A105:

$^1H$ NMR (500 MHz, DMSO-$d_6$) δ 11.60 (s, 1H), 9.12 (d, *J* = 2.4 Hz, 1H), 8.89 (d, *J* = 2.4 Hz, 1H), 8.21 (s, 2H), 7.86 (d, *J* = 9.1 Hz, 1H), 7.74 (d, *J* = 6.3 Hz, 1H), 7.69 (dd, *J* = 8.9, 2.7 Hz, 1H), 7.60 (dd, *J* = 8.6, 6.1 Hz, 1H), 7.41 (td, *J* = 8.4, 2.6 Hz, 1H). Example67: Synthesis of Compound A67

Step 1: Synthesis of Compound A67

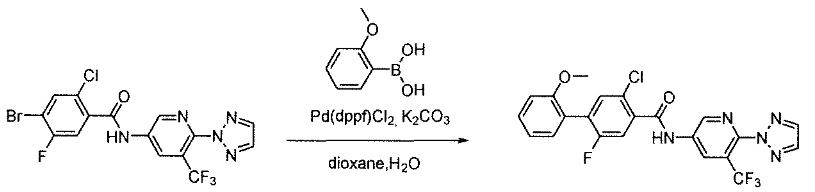

A105-2

A67

Into a 100-mL round-bottom flask, was placed A105-2 (115 mg, 0.25 mmol), 2-methoxyphenylboronic acid (45.6 mg, 0.3mmol), $Pd(dppf)Cl_2$, (18.3 mg, 0.025 mmol), $K_2CO_3$ (207mg, 1.5 mmol), dioxane (3 mL) and $H_2O$ (0.5 mL). The resulting mixture was stirred at 100°C for 2 hours under nitrogen. Removing of the solvent and purifying by column chromatography (PE/EtOAc=1/1) to give compound A67 (18 mg). LC-MS ($ES^+$): *m/z* 492 $[M+H]^+$

Example69: Synthesis of Compound A69

Step1: Synthesis of Compound A69

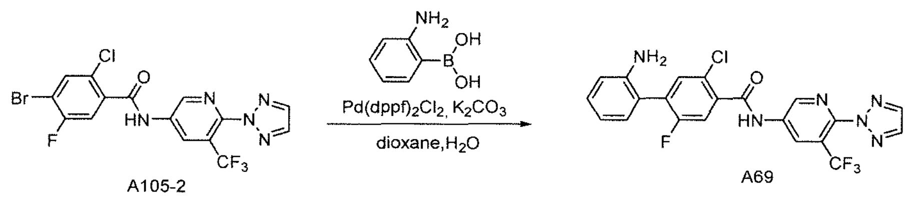


A105-2 A69

Into a 100-mL round-bottom flask, was placed A105-2 (116 mg, 0.25 mmol), 2-aminophenylboronic acid (41mg, 0.3mmol), Pd(dppf)$Cl_2$, (18.3 mg, 0.025 mmol), $K_2CO_3$ (207mg, 1.5 mmol), dioxane (3 mL) and $H_2O$ (0.5 mL). The resulting mixture was stirred at 100°C for 2 hours under nitrogen. Removing of the solvent and purifying by column chromatography(PE / EtOAc =1 / 1) to give compound A69 (24 mg), LC-MS ($ES^+$): *m/z* 477 $[M+H]^+$

Example 1 10: Synthesis of Compound A110

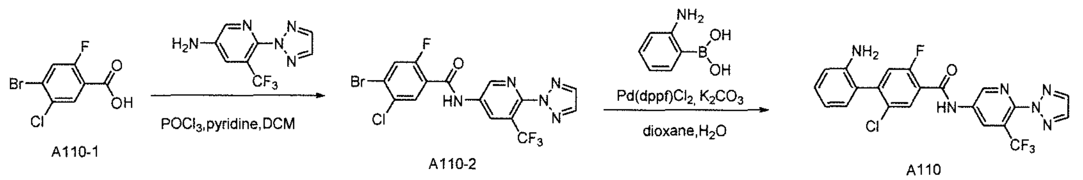


A110-1 A110-2 A110

Step1: Synthesis of Compound A 110-2

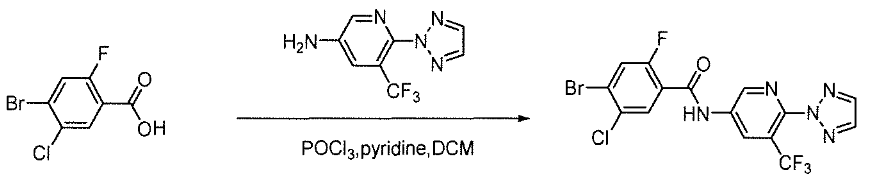


A110-1 A110-2

To a solution of A110-1 (2.53 g, 10.0 mmol) and 6-(2H-1,2,3-triazol-2-yl)-5-(trifluoromethyl)pyridin-3-amine (2.52 g, 11.0 mmol) in dichloromethane (25 mL)was added pyridine (1.19 g, 15.0 mmol) at room temperature. The mixture was cooled to 0°C in an ice bath, and then phosphorus oxychloride was added dropwise (4.59 g, 30.0 mmol). The reaction was stirred at room temperature for 1 hour, and quenched by water, extracted with dichloromethane (250 mL). The organic layer was washed with brine, dried over anhydrous $Na_2SO_4$ and concentrated in vacuum. The residue was purified by flash column chromatography over silica gel to affordcompound A110-2 (1.9 g). LC-MS ($ES^+$): *m/z* 464,466 $[M+H]^+$

Step2: Synthesis of Compound A110

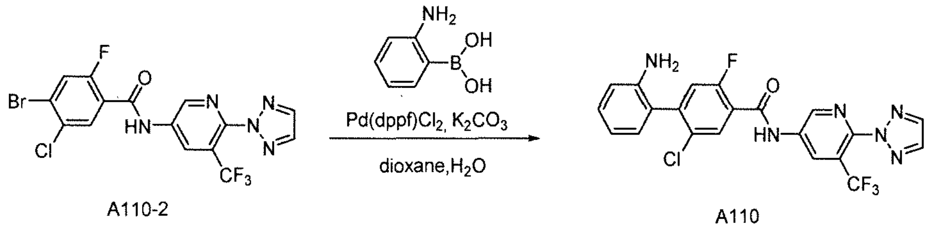


A110-2 A110

Into a 100-mL round-bottom flask, was placed A110-2 (215 mg, 0.5 mmol), 2-aminophenylboronic acid (82.2 mg, 0.6mmol), Pd(dppf)$Cl_2$ (36.6 mg, 0.05 mmol), $K_2CO_3$ (414mg, 3.0 mmol), dioxane(3 mL) and $H_2O$(0.5 mL). The resulting mixture was stirred at 100°C for 2 hours under nitrogen. Removing of the solvent and purifying by column chromatography (PE/EtOAc=1/1) to give compound A110 (55 mg). LC-MS ($ES^+$): *m/z* 477$[M+H]^+$

Example 126: Synthesis of Compound A126

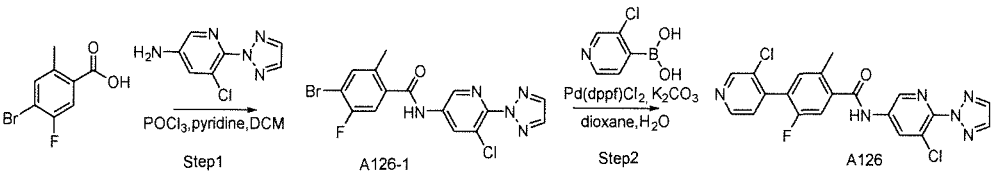


Step1: Synthesis of Compound A 126-1

To a solution of 5-chloro-6-(2H-1,2,3-triazol-2-yl)pyridin-3-amine (1.95g) and 4-bromo-5-fluoro-2-methylbenzoic acid (2.33g) in 20mL of DCM, was added pyridine (1.6g) at room temperature. The mixture was cooled to 0 °C in an ice bath, and then $POCl_3$ (3.1g) was added dropwise. The reaction was stirred at room temperature for 2 hours and quenched by water, extracted with dichloromethane (100 mL). The organic layer was washed with brine, dried over anhydrous $Na_2SO_4$ and concentrated in vacuum. The residue was purified by flash column chromatography over silica gel to afford compound A126-1 (3.7g, white solid). LC-MS ($ES^+$): *m/z* 410,412$[M+H]^+$

Step2: Synthesis of Compound A126

Into a 100-mL round-bottom flask, was placed A126-1 (41mg) , (3-Chloropyridin-4-yl)boronic acid (16mg) , dioxane (1mL), water (0.2mL), Pd(dppf)$Cl_2$ (5mg) and $K_2CO_3$ (30mg). The resulting mixture was stirred at 90°C and stirred for 16 hours under nitrogen. Removing of the solvent and purifying by column chromatography (DCM/MeOH =40/1) to give compound A126 (21mg, white solid). LC-MS ($ES^+$): *m/z* 443$[M+H]^+$

Example 113: Synthesis of Compound A113

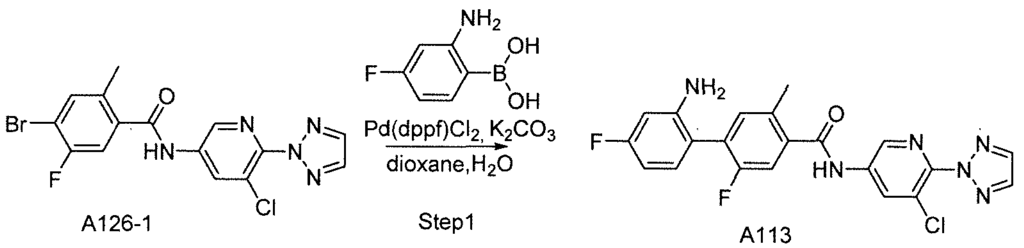


Into a 100-mL round-bottom flask, was placed A 126-1 (41mg) , (2-Amino-4-fluorophenyl)boronic acid (16mg), dioxane (1mL) , water (0.2mL), Pd(dppf)$Cl_2$ (5mg) and $K_2CO_3$ (30mg). The resulting mixture was stirred at 90 °C for 16 hours under nitrogen. Removing of the solvent and purifying by column chromatography (DCM/MeOH =40/1) to give compound A113 (29mg, white solid). LC-MS ($ES^+$): *m/z* 441$[M+H]^+$

A1 13:'H NMR (500 MHz, DMSO-$d_6$) δ 11.19 (d, *J* = 22.7 Hz, 1H), 8.86 (dd, *J*= 19.1, 2.3 Hz, 1H), 8.68 (dd, *J* = 18.5, 2.2 Hz, 1H), 8.18 (d, *J* = 1.9 Hz, 2H), 7.83 - 7.25 (m, 2H), 7.17 - 6.45 (m, 2H), 2.43 (d, *J* = 18.8 Hz, 3H).

Example 1 14: Synthesis of Compound A114

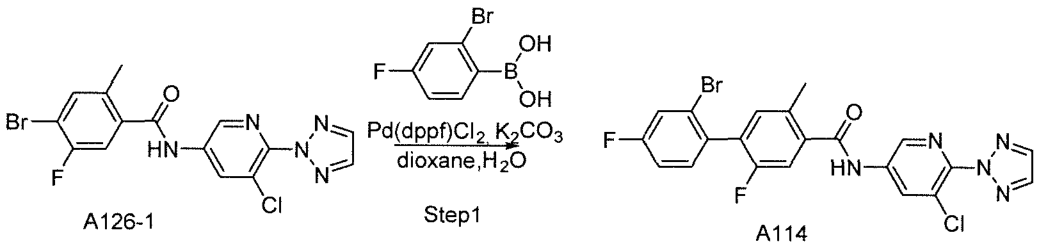


A126-1

A114

Into a 100-mL round-bottom flask, was placed A126-1 (41mg), (2-bromo-4-fluorophenyl)boronic acid (26mg), dioxane(1mL), water (0.2mL), Pd(dppf)$Cl_2$ (5mg) and $K_2CO_3$ (30mg). The resulting mixture was stirred at 90°C for 16 hours under nitrogen. Removing of the solvent and purifying by column chromatography (DCM/MeOH=40/1) to give compound A114 (19mg, white solid). LC-MS ($ES^+$): *m/z* 504,506 $[M+H]^+$A 114:
$^1H$ NMR (500 MHz, DMSO-$d_6$) δ 11.20 (s, 1H), 8.86 (d, *J* = 2.3 Hz, 1H), 8.69 (d, *J* = 2.2 Hz, 1H), 8.19 (s, 2H), 7.79 (dd, *J* = 8.6, 2.6 Hz, 1H), 7.62 (d, *J* = 9.6 Hz, 1H), 7.50 (dd, *J* = 8.6, 6.1 Hz, 1H), 7.42 (td, *J* = 8.4, 2.6 Hz, 1H), 7.37 (d, *J* = 7.1 Hz, 1H), 2.45 (s, 3H).

Example130: Synthesis of Compound A130

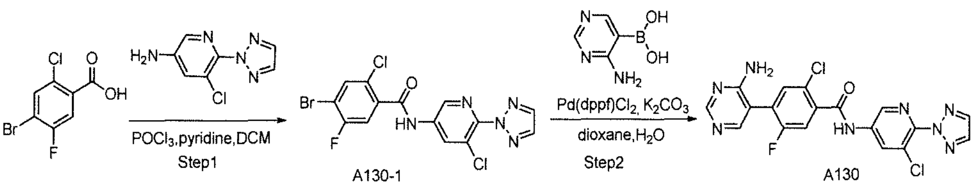


A130-1

A130

Step 1: Synthesis of Compound A130-1

To a solution of 5-chloro-6-(2H-1,2,3-triazol-2-yl)pyridin-3-amine (1.95 g) and 4-bromo-2-chloro-5-fluorobenzoic acid (2.53 g) in dichloromethane (20 mL) was added pyridine (1.6 g) at room temperature. The mixture was cooled to 0 °C in an ice bath, and then phosphorus oxychloride was added dropwise (3.1 g). The reaction was stirred at room temperature for 2 hours and quenched by water, extracted with dichloromethane (50 mL x 2). The organic layer was washed with brine, dried over anhydrous $Na_2SO_4$ and concentrated in vacuum. The residue was purified by flash column chromatography over silica gel (PE / EA = 2 / 1) to afford compound A130-1 4-bromo-2-chloro-N-(5-chloro-6-(2H-1,2,3-triazol-2-yl)pyridin-3-yl)-5-fluorobenzamide (3.9 g). LC-MS ($ES^+$): *m/z* 430 $[M+H]^+$

Step2: Synthesis of Compound A130

Into a 100-mL round-bottom flask, was placed A130-1 (43 mg), (4-aminopyrimidin-5-yl)boronic acid (14mg), Pd(dppf)$Cl_2$ (5 mg), $K_2CO_3$(30 mg), dioxane (1 mL) and $H_2O$ (0.2 mL). The resulting mixture was stirred at 90 °C for 16 hours under nitrogen. The reaction solution was diluted with EA (5 mL) washed with saturated NaCl solution, and dried over anhydrous sodium sulfate. The organic phase was concentrated and purified by column chromatography to obtain A130 (23mg) as a white solid. LC-MS ($ES^+$): *m/z* 445 $[M+H]^+$

Example 133: Synthesis of Compound A133

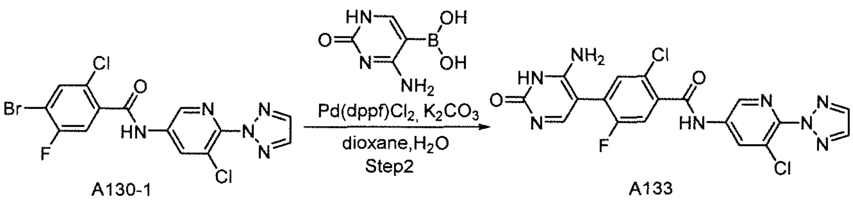


A130-1 A133

Into a 100-mL round-bottom flask, was placed A130-1 (43 mg), (4-amino-2-oxo-1,2-dihydropyrimidin-5-yl)boronic acid (16 mg), Pd(dppf)$Cl_2$ (5 mg), $K_2CO_3$ (30 mg), dioxane (1 mL) and $H_2O$ (0.2 mL). The resulting mixture was stirred at 90 °C for 16 hours under nitrogen. The reaction solution was diluted with EA (5 mL) washed with saturated NaCl solution, and dried over anhydrous sodium sulfate. The organic phase was concentrated and purified by column chromatography to obtain A133 (22 mg) as a white solid. LC-MS (ES+): *m/z* 461 [M+H]+

Example 147: Synthesis of Compound A147

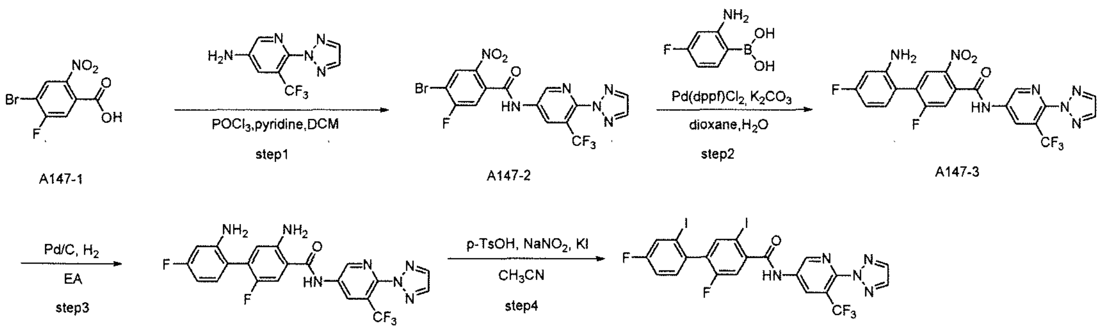


A147-1 A147-2 A147-3 A147-4 A147

Step1: Synthesis of Compound A147-2

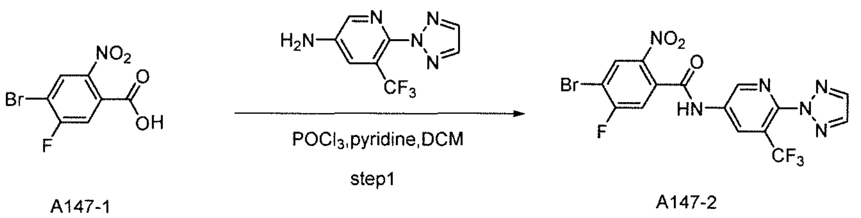


A147-1 A147-2

To a solution of A147-1 (2.64 g, 10.0 mmol) and 6-(2H-1,2,3-triazol-2-yl)-5-(trifluoromethyl)pyridin-3-amine (2.15 g, 11.0 mmol) in dichloromethane (25 mL) was added pyridine (1.19 g, 15.0 mmol) at room temperature. The mixture was cooled to 0 °C in an ice bath, and then phosphorus oxychloride was added dropwise (4.59 g, 30.0 mmol). The reaction was stirred for 1 hour and quenched by water, extracted with dichloromethane (125 mL x 2). The organic layer was washed with brine, dried over anhydrous $Na_2SO_4$ and concentrated in vacuum. The residue was purified by flash column chromatography over silica gel to afford compound A 147-2 (2.6g). LC-MS (ES+): *m/z* 475 [M+H]+

Step2: Synthesis of Compound A 147-3

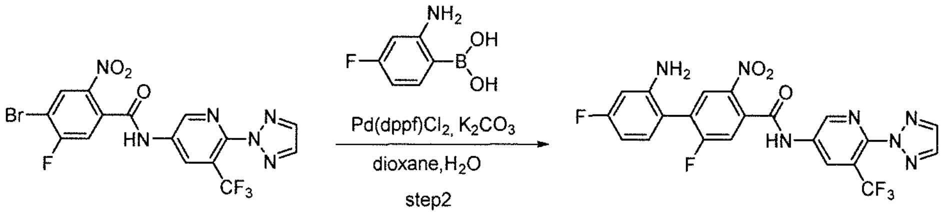


A147-2 A147-3

Into a 100-mL round-bottom flask, was placed A147-2 (950 mg, 2 mmol), (2-amino-4-fluorophenyl)boronic acid (388 mg, 2.5 mmol), Pd(dppf)$Cl_2$ (18.3 mg, 0.025 mmol), $K_2CO_3$ (207 mg, 1.5 mmol), dioxane (3 mL) and $H_2O$ (0.5 mL). The resulting mixture was stirred at 100 °C for 2 hours under nitrogen. Removing of the solvent and purifying by column chromatography to give compound A147-3 (700 mg). LC-MS ($ES^+$): *m*/*z* 506 $[M+H]^+$

Step3: Synthesis of Compound A147-4

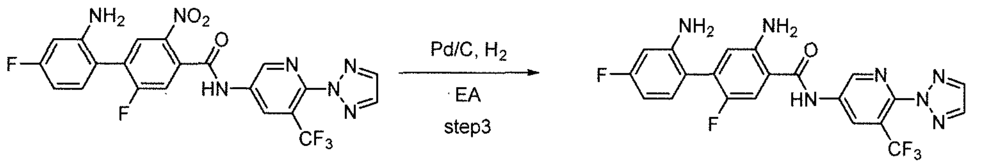


A147-3 A147-4

To a solution of A147-3 (700 mg, 2 mmol) in ethyl acetate (20 mL) was added Pd/C (10%, 100 mg) under nitrogen atmosphere in a 100 mL round bottom flask. The flask was then vacuumed and flushed with hydrogen. The reaction mixture was hydrogenated at room temperaturefor 2 hours under hydrogen atmosphere using a hydrogen balloon, then filtered through a Celite pad and concentrated under reduced pressure. The residue was used without further purification and compound A147-4 (500 mg) was obtained . LC-MS ($ES^+$): *m*/*z* 476 $[M+H]^+$

Step4: Synthesis of Compound A147

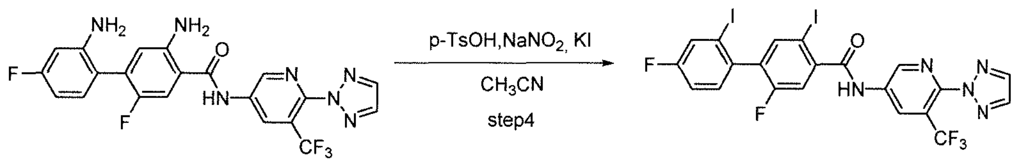


A147-4 A147

To a solution of A 147-4 (95 mg, 0.2 mmol) in acetonitrile (5 mL) was added p-toluenesulfonic acid (172 mg, 1mmol) at 0°C. An aqueous solution of sodium nitrite (69 mg, 1mmol, dissolved in 1 mL of water) was added to the resulting mixture and stirred for 0.5 hour and then potassium iodide (166 mg, 1 mmol, dissolved in 1 mL water) was added. The mixture was stirred at 25 □ for 1 hour, then raised to 100 □ and continued for 0.5 hour. The reaction was quenched by aqueous sodium thiosulfate solution and extracted by Eethyl acetate (50 mL x 2). The organic layer was concentratede and purified by column chromatography to obtain compound A147 (22 mg). LC-MS ($ES^+$): *m*/*z* 698 $[M+H]^+$

Example 157: Synthesis of Compound A157

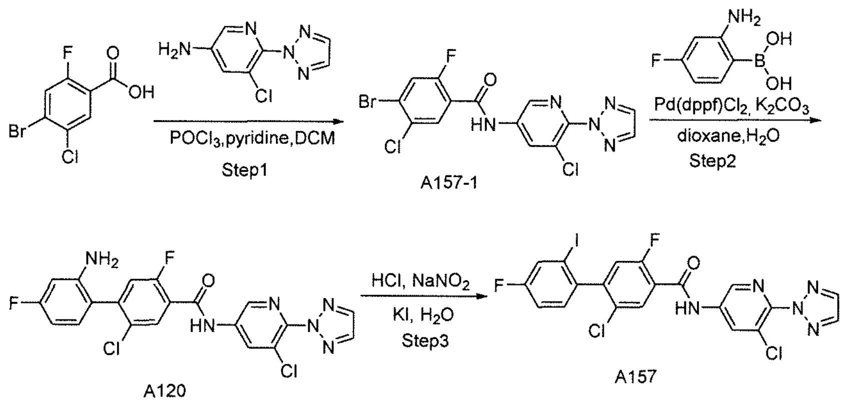


A157-1

A120

A157

Step 1: Synthesis of Compound A157-1

To a solution of 5-chloro-6-(2H-1,2,3-triazol-2-yl)pyridin-3-amine (1.95 g) and 4-bromo-5-chloro-2-fluorobenzoic acid (2.53 g) in dichloromethane (20 mL) was added pyridine (1.6 g) at room temperature. The mixture was cooled to 0 °C in an ice bath, and then phosphorus oxychloride was added dropwise (3.1 g). The reaction was stirred for 2 hour at room temperature and quenched by water, extracted with dichloromethane (50 mL x 2). The organic layer was washed with brine, dried over anhydrous $Na_2SO_4$ and concentrated in vacuum. The residue was purified by flash column chromatography over silica gel (PE / EA=2 / 1) to afford compound A157-1 4-Bromo-5-chloro-N-(5-chloro-6-(2H-1,2,3-triazol-2-yl)pyridin-3-yl)-2-fluorobenzamide (4.1 g) as a white solid. LC-MS ($ES^+$): *m/z* 430 $[M+H]^+$

Step2: Synthesis of Compound A120

Into a 50-mL round-bottom flask, was placed A157-1 (430 mg), (2-amino-4-fluorophenyl)boronic acid (155 mg), Pd(dppf)$Cl_2$ (50 mg), $K_2CO_3$(300 mg), dioxane (10 mL) and $H_2O$ (2 mL). The resulting mixture was stirred at 90 °C for 16 hours under nitrogen. Removing of the solvent and purifying by column chromatography (DCM / MeOH=40 / 1) to give compound A120 (345 mg) as a white solid. LC-MS ($ES^+$): *m/z* 461 $[M+H]^+$

A120:

$^1$H NMR (500 MHz, DMSO-$d_6$) $\delta$ 11.23 (s, 1H), 8.86 (d, $J$ = 2.3 Hz, 1H), 8.67 (d, $J$ = 2.3 Hz, 1H), 8.19 (s, 2H), 7.98 (d, $J$ = 6.5 Hz, 1H), 7.42 (d, $J$ = 10.2 Hz, 1H), 6.93 (dd, $J$ = 8.4, 6.7 Hz, 1H), 6.54 (dd, $J$ = 11.8, 2.6 Hz, 1H), 6.42 (td, $J$ = 8.5, 2.6 Hz, 1H), 5.20 (s, 2H).

Step3: Synthesis of Compound A157

A120 (92 mg) was dissolved in concentrated hydrochloric acid (5 mL). At 0°C, an aqueous solution of sodium nitrite (20 mg, dissolved in 1 mL water) was added to the resulting mixture and stirred for 1 hour and then potassium iodide (34 mg, dissolved in 1 mL water) was added. The mixture was stirred at room temperature for 2 hours. The reaction was quenched by aqueous sodium thiosulfate solution and extracted by ethyl acetate. The organic layer was concentratede and purified by column chromatography (DCM / MeOH=40 / 1) to obtain compound A157 (65 mg) as a white solid. LC-MS ($ES^+$): *m/z* 572 $[M+H]^+$

Example 124: Synthesis of Compound A124

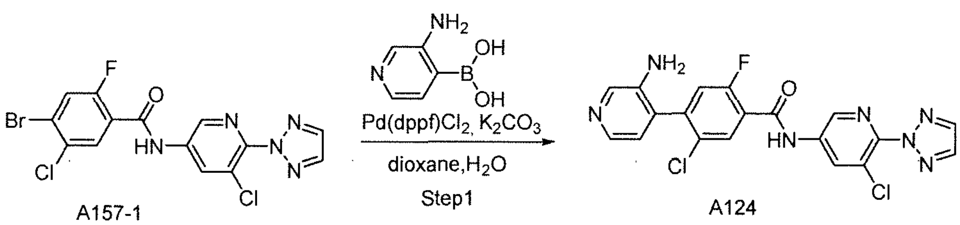


A157-1

A124

Into a 10-mL round-bottom flask, was placed A157-1 (43 mg), (3-aminopyridin-4-yl)boronic acid (15 mg), $Pd(dppf)Cl_2$ (5 mg), $K_2CO_3$(30 mg), dioxane (1 mL) and $H_2O$ (0.2 mL). The resulting mixture was stirred at 90 °C for 16 hours under nitrogen. Removing of the solvent and purifying by column chromatography (DCM / MeOH=40 / 1) to give compound A124 (26 mg) as a white solid. LC-MS ($ES^+$): *m/z* 444 $[M+H]^+$

Example220: Synthesis of Compound A220

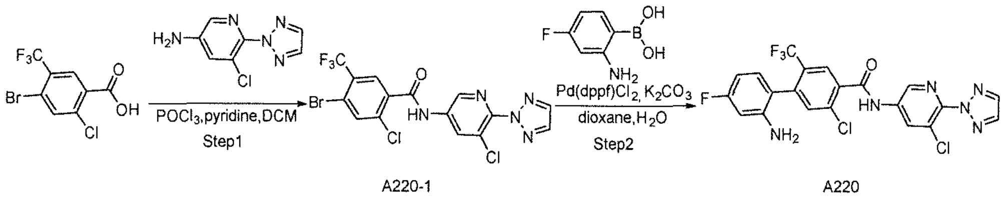


A220-1 A220

Step 1: Synthesis of Compound A220-1

To a solution of 5-chloro-6-(2H-1,2,3-triazol-2-yl)pyridin-3-amine (1.95 g) and 4-bromo-2-chloro-5-(fluorotrifluoromethyl )benzoic acid (3.03 g) in dichloromethane (20 mL) was added pyridine (1.6 g) at room temperature. The mixture was cooled to 0 °C in an ice bath, and then phosphorus oxychloride was added dropwise (3.1 g). The reaction was stirred at room temperature for 2 hours and quenched by water, extracted with dichloromethane (50 mL x 2). The organic layer was washed with brine, dried over anhydrous $Na_2SO_4$ and concentrated in vacuum. The residue was purified by flash column chromatography over silica gel to afford compound A220-1 4-bromo-2-chloro-N-(5-chloro-6-(2H-1,2,3-triazol-2-yl)pyridin-3-yl)-5-(fluorotrifluoromethyl)benzamide (4.1 g) as a white solid. LC-MS ($ES^+$): *m/z* 480 $[M+H]^+$

Step2: Synthesis of Compound A220

Into a 10-mL round-bottom flask, was placed A220-1 (48 mg), (2-amino-4-fluorophenyl)boronic acid (15 mg), $Pd(dppf)Cl_2$ (5 mg), $K_2CO_3$(30 mg), dioxane (1 mL) and $H_2O$ (0.2 mL). The resulting mixture was stirred at 90 °C for 16 hours under nitrogen. Removing of the solvent and purifying by column chromatography (DCM / MeOH=40 / 1) to give compound A220 (25 mg) as a white solid. LC-MS ($ES^+$): *m/z* 541 $[M+H]^+$

Example274: Synthesis of Compound A274

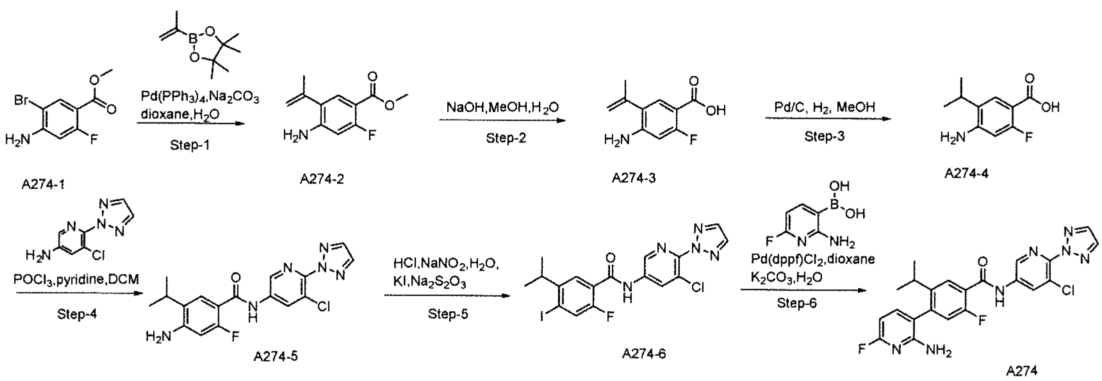


A274-1 A274-2 A274-3 A274-4 A274-5 A274-6 A274

Step1: Synthesis of Compound A274-2

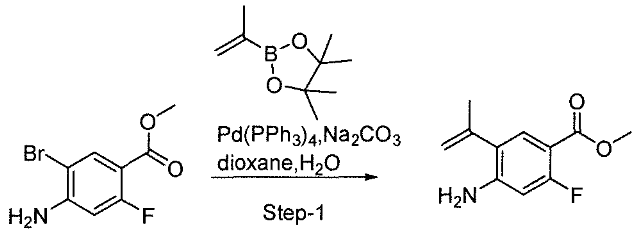


A274-1 A274-2

Into a 50-mL round-bottom flask, was placed A274-1 (1.23 g, 5 mmol), 4,4,5,5-tetramethyl-2-(prop-1-en-2-yl)-1,3,2-dioxaborolane (1.68 g, 10 mmol), $Pd(PPh_3)_4$ (578 mg, 0.5 mmol), $Na_2CO_3$ (1.59 g, 15 mmol), dioxane (12 mL) and $H_2O$ (3 mL). The resulting mixture was stirred at 85 °C for 3 hours under nitrogen. Removing of the solvent and purifying by column chromatography to give compound A274-2 (800 mg, 3.83 mmol) as a yellow solid.

Step2: Synthesis of Compound A274-3

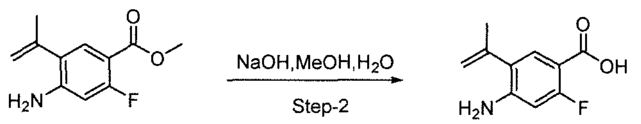


A274-2 A274-3

Into a flask, A274-2 (800 mg, 3.83 mmol) , NaOH (459 mg, 11.5 mmol), methanol (8 mL) and $H_2O$ (4 mL) were added and reacted at room temperature for 16 hours.The end of the reaction were monitored by LCMS. Adjusted the pH to 3 with concentrated hydrochloric acid under an ice bath, solid precipitated, filtered, and dried to obtain a yellow solid A274-3 (500 mg).

Step3: Synthesis of Compound A274-4

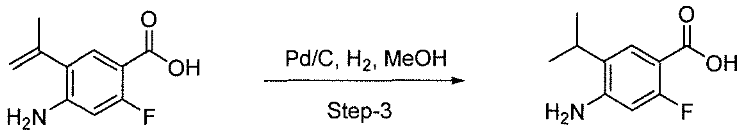


A274-3 A274-4

To a solution of A274-3(500 mg, 2.56 mmol) in methanol (8 mL) was added Pd/C (10%, 250 mg) under nitrogen atmosphere in a round bottom flask. The flask was then vacuumed and flushed with hydrogen. The reaction mixture was hydrogenated at room temperaturefor 5 hours under hydrogen atmosphere using a hydrogen balloon, then filtered through a Celite pad and concentrated under reduced pressure. The residue was used without further purification and compound A274-4 (450 mg) was obtained.

Step4: Synthesis of Compound A274-5

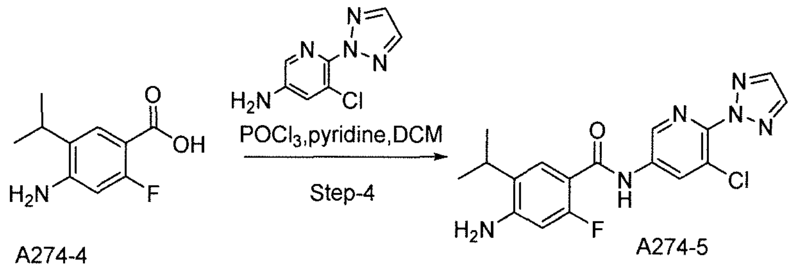


A274-4 A274-5

To a solution of A274-4 (450 mg, 2.28 mmol) and 5-chloro-6-(2H-1,2,3-triazol-2-yl)pyridin-3-amine (585 mg, 3 mmol) in dichloromethane (5 mL) was added pyridine (540 mg, 6.84 mmol) at room temperature. The mixture was cooled to 0 °C in an ice bath, and then phosphorus oxychloride was added dropwise (459 mg, 3 mmol). The reaction was stirred for 1 hour and quenched by water, extracted with dichloromethane (25 mL x 2). The organic layer was washed with brine, dried over anhydrous $Na_2SO_4$ and concentrated in vacuum. The residue was purified by flash column chromatography over silica gel to afford compound A274-5 (600 mg, 1.60 mmol) as a reddish brown solid.

StepS: Synthesis of Compound A274-6

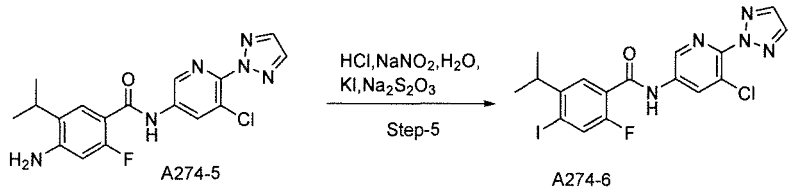


A274-5 A274-6

At 0 °C, to a solution of A274-5 (600 mg, 1.60 mmol) in 2N HCl (6 mL, 2 mol.$L^{-1}$ HCl), an aqueous solution of sodium nitrite (138 mg, 2 mmol, dissolved in 2 mL of water) was added to the resulting mixture and stirred for 15 minutes and then potassium iodide (332 mg, 2 mmol, dissolved in 2 mL water) was added. The mixture was stirred at 0 °C for 15 minutes, 4 °C for 10 minutes, 100 °C for 10 minutes, and then stirred at 0 °C for 5 minutes. The reaction was quenched by aqueous sodium thiosulfate solution and extracted by Eethyl acetate (25 mL x 2). The organic layer was concentratede and purified by column chromatography to obtain compound A274-6 (200 mg, 0.41 mmol) as a reddish brown solid.

Step6: Synthesis of Compound A274

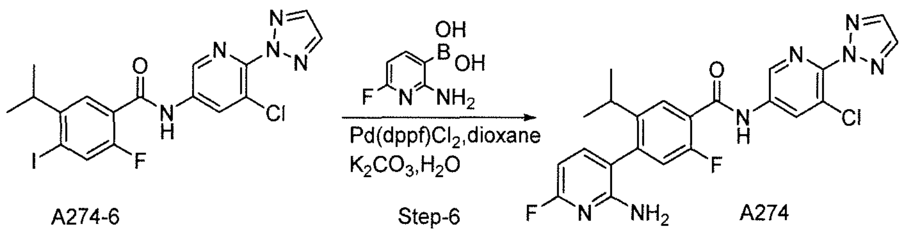


A274-6 A274

Into a round-bottom flask, was placed A274-6 (200 mg, 0.41 mmol), (2-amino-6-fluoropyridin-3-yl)boronic acid (94 mg, 0.6mmol), $Pd(dppf)Cl_2$ (29 mg, 0.04 mmol), $K_2CO_3$ (170 mg, 1.23 mmol), dioxane (2 mL) and $H_2O$ (0.5 mL). The resulting mixture was stirred at 75 °C for 2 hours under nitrogen. Removing of the solvent and purifying by column chromatography to give compound A274 (20 mg). LC-MS ($ES^+$): *m/z* 470 $[M+H]^+$

According to the synthesis method of the above example, select appropriate raw materials and/or marketed example intermediates to synthesize the example compounds in the table below. The raw materials and reagents used are all commercially available.

| Example | Structure | Chemical Name | MS [M+H]+ |
|---|---|---|---|
| Example1 (A01) | 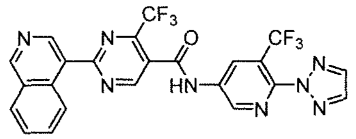 | N-(6-(2H-1,2,3-triazol-2-yl)-5-(trifluoromethyl)pyridin-3-yl)-2-(isoquinolin-4-yl)-4-(trifluoromethyl) pyrimidine -5-carboxamide | 531 |
| Example2 (A02) | 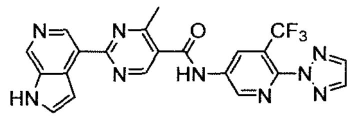 | N-(6-(2H-1,2,3-triazol-2-yl)-5-(trifluoromethyl)pyridin-3-yl)-4-methyl-2-(1H-pyrrolo[2,3-c]pyridin-4-yl) pyrimidine-5-carboxamide | 466 |
| Example3 (A03) | 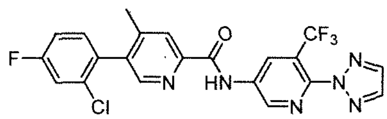 | N-(6-(2H-1,2,3-triazol-2-yl)-5-(trifluoromethyl)pyridin-3-yl)-5-(2-chloro-4-fluorophenyl)-4-methylpicolinamide | 477 |
| Example4 (A04) | 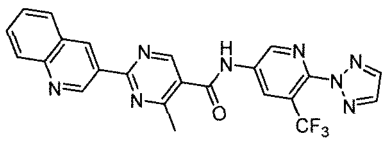 | N-(6-(2H-1,2,3-triazol-2-yl)-5-(trifluoromethyl)pyridin-3-yl)-4-methyl-2-(quinolin-3-yl)pyrimidine-5-carboxamide | 477 |
| Example6 (A06) | 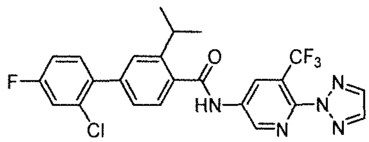 | N-(6-(2H-1,2,3-triazol-2-yl)-5-(trifluoromethyl)pyridin-3-yl)-2'-chloro-4'-fluoro-3-isopropyl-[1,1'-biphenyl]-4-carboxamide | 504 |
| Example7 (A07) | 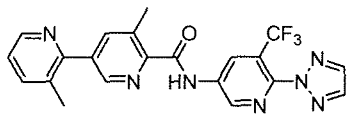 | N-(6-(2H-1,2,3-triazol-2-yl)-5-(trifluoromethyl)pyridin-3-yl)-3,5'-dimethyl-[2,3'-bipyridine]-6'-carboxamide | 440 |
| Example8 (A08) | 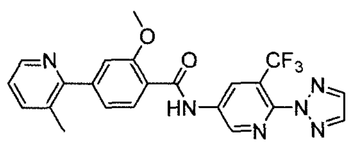 | N-(6-(2H-1,2,3 -triazol-2-yl)-5-(trifluoromethyl)pyridin-3-yl)-2-methoxy-4-(3-methylpyridin-2-yl)benzamide | 455 |
| Example9 (A09) | 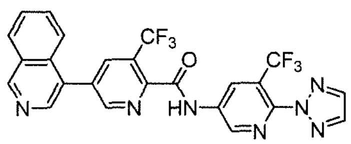 | N-(6-(2H-1,2,3-triazol-2-yl)-5-(trifluoromethyl)pyridin-3-yl)-5-(isoquinolin-4-yl)-3-(trifluoromethyl) picolinami de | 530 |
| Example10 (A10) | 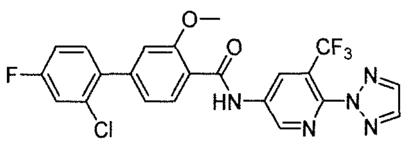 | N-(6-(2H-1,2,3-triazol-2-yl)-5-(trifluoromethyl)pyridin-3-yl)-2'-chloro-4'-fluoro-3-methoxy-[1,1'-biphenyl]-4-carboxamide | 492 |
| Example11 (Al1) | 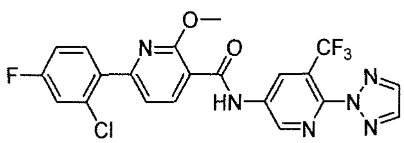 | N-(6-(2H-1,2,3-triazol-2-yl)-5-(trifluoromethyl)pyridin-3-yl)-6-(2-chloro-4-fluorophenyl)-2-methoxynicotinamide | 493 |
| Example13 (A13) | 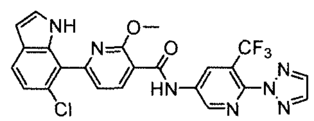 | N-(6-(2H-1,2,3-triazo)-2-yl)-5-(trifluoromethyl)pyridin-3-yl)-6-(6-chloro-1-H-indol-7-yl)-2-methoxynicotinamide | 514 |

(continued)

| Example | Structure | Chemical Name | MS [M+H]+ |
|---|---|---|---|
| Example14 (A14) | 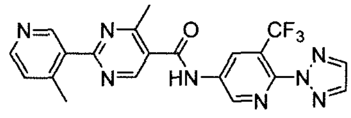 | N-(6-(2H-1,2,3-triazol-2-yl)-5-(trifluoromethyl)pyridin-3-yl)-4-methyl-2-(4-methylpyridin-3-yl)pyrimidine-5-carboxamide | 441 |
| Example15 (A15) | 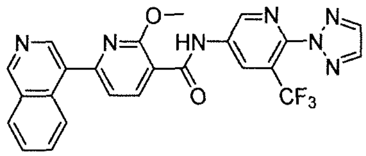 | N-(6-(2H-1,2,3 -triazol-2-yl)-5-(trifluoromethyl)pyridin-3-yl)-6-(isoquinolin-4-yl)-2-methoxynicotinamide | 492 |
| Example16 (A16) | 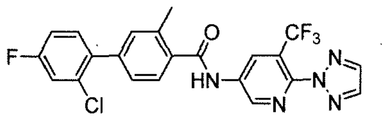 | N-(6-(2H-1,2,3-triazol-2-yl)-5-(trifluoromethyl)pyridin-3-yl)-2'-chloro-4'-fluoro-3-methyl-[1,1'-biphenyl]-4-carboxamide | 476 |
| Example17 (A17) | 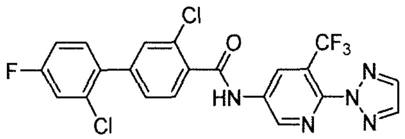 | N-(6-(2H-1,2,3-triazol-2-yl)-5-(trifluoromethyl)pyridin-3-yl)-2',3-dichloro-4'-fluoro-[1,1'-biphenyl]-4-carboxamide | 496 |
| Example18 (A18) | 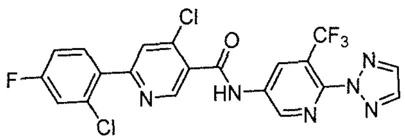 | N-(6-(2H-1,2,3-triazol-2-yl)-5-(trifluoromethyl)pyridin-3-yl)-4-chloro-6-(2-chloro-4-fluorophenyl)nicotinamide | 497 |
| Example19 (A19) | 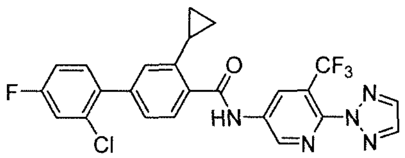 | N-(6-(2H-1,2,3-triazol-2-yl)-5-(trifluoromethyl)pyridin-3-yl)-2'-chloro-3-cyclopropyl-4'-fluoro-[1,1'-biphenyl]-4-carboxamide | 502 |
| Example20 (A20) | 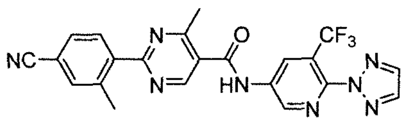 | N(6-(2H-1,2,3-triazol-2-yl)-5-(trifluoromethyl)pyridin-3-yl)-2-(4-cyano-2-methylphenyl)-4-methylpyrimidine-5-carboxamide | 465 |
| Example21 (A21) | 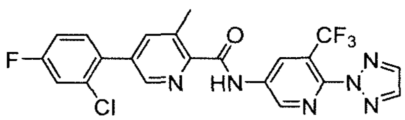 | N-(6-(2H-1,2,3-triazol-2-(trifluoromethyl) pyridin-3-yl)-5-(2-chloro-4-fluorophenyl)-3-methylpicolinamide | 477 |
| Example22 (A22) | 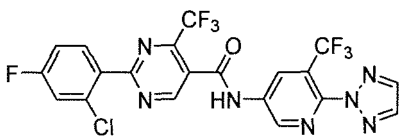 | N-(6-(2H-1,2,3-triazol-2-yl)-5-(trifluoromethyl)pyridin-3-yl)-2-(2-chloro-4-fluorophenyl)-4-(trifluoromethyl) pyrimidine -5-carboxamide | 532 |
| Example23 (A23) | 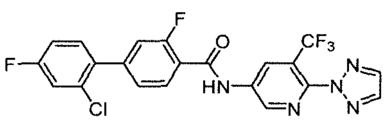 | N-(6-(2H-l,2,3-triazol-2-yl)-5-(trifluoromethyl)pyridin-3-yl)-2'-chloro-3,4'-difluoro-[1,1'-biphenyl]-4-carboxamide | 480 |

(continued)

| Example | Structure | Chemical Name | MS [M+H]+ |
|---|---|---|---|
| Example24 (A24) | 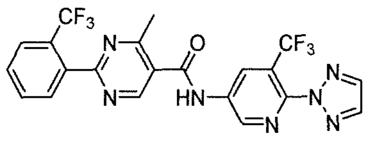 | N-(6-(2H-1,2,3-triazol-2-yl)-5-(trifluoromethyl)pyridin-3-yl)-4-methyl-2-(2-(trifluoromethyl)phenyl)pyr imidine-5-carboxamide | 494 |
| Example25 (A25) | 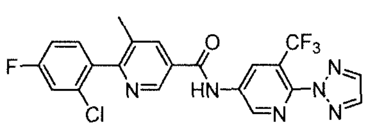 | N-(6-(2H-1,2,3-triazol-2-yl)-5-(trifluoromethyl)pyridin-3-yl)-6-(2-chloro-4-fluorophenyl)-5-methylnicotinamide | 477 |
| Example26 (A26) | 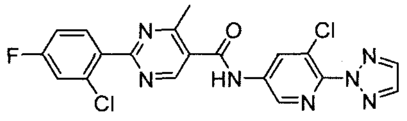 | 2-(2-chloro-4-fluorophenyl)-N-(5-chloro-6-(2H-1,2,3-triazol-2-yl)pyridin-3-yl)-4-methylpyrimidine-5-carboxamide | 444 |
| Example27 (A27) | 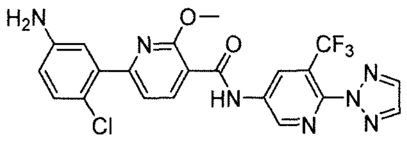 | N-(6-(2H-1,2,3-triazol-2-yl)-5-(trifluoromethyl)pyridin-3-yl)-6-(5-amino-2-chlorophenyl)-2-methoxynicotinamide | 490 |
| Example28 (A28) | 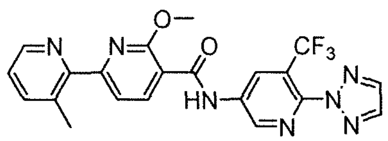 | N-(6-(2H-l,2,3-triazol-2-yl)-5-(trifluoromethyl)pyridin-3-yl)-6-methoxy-3'-methyl-[2,2'-bipyridine]-5-carboxamide | 456 |
| Example29 (A29) | 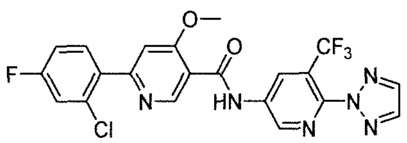 | N-(6-(2H-1,2,3-triazol-2-yl)-5-(trifluoromethyl)pyridin-3-yl)-6-(2-chloro-4-fluorophenyl)-4-methoxynicotinamide | 493 |
| Example30 (A30) | 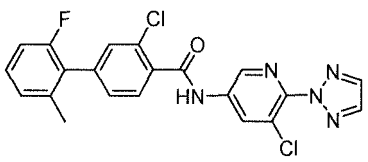 | 3-chloro-N-(5-chloro-6-(2H-1,2,3-triazol-2-yl)pyridin-3-yl)-2'-fluoro-6'-methyl-[1,1'-biphenyl]-4-carboxamide | 442 |
| Example31 (A31) | 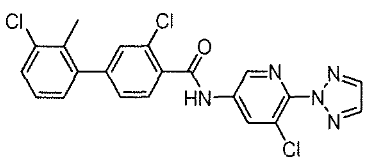 | 3,3'-dichloro-N-(5-chloro-6-(2H-1,2,3-triazol-2-yl)pyridin-3-yl)-2'-methyl-[1,1'-biphenyl]-4-carboxamide | 458 |
| Example32 (A32) | 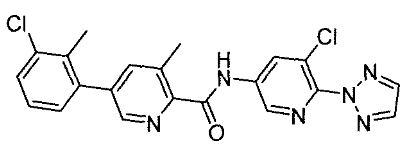 | 5-(3-chloro-2-methylphenyl)-N-(5-chloro-6-(2H-1,2,3-triazol-2-yl)pyridin-3-yl)-3-methylpicolinamide | 439 |
| Example33 (A33) | 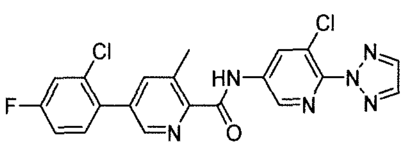 | 5-(2-chloro-4-fluorophenyl)-N-(5-chloro-6-(2H-1,2,3-triazol-2-yl)pyridin-3-yl)-3-methylpicolinamide | 443 |

(continued)

| Example | Structure | Chemical Name | MS [M+H]+ |
|---|---|---|---|
| Example34 (A34) | 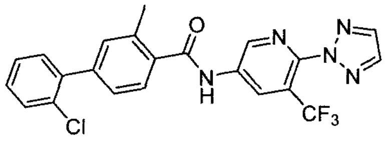 | N-(6-(2H-1,2,3-triazol-2-yl)-5-(trifluoromethyl)pyridin-3-yl)-2'-chloro-3-methyl-[1,1'-biphenyl]-4-carboxamide | 458 |
| Example36 (A36) | 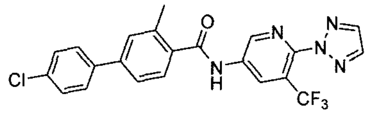 | N-(6-(2H-1,2,3-triazol-2-yl)-5-(trifluoromethyl)pyridin-3-yl)-4'-chloro-3-methyl-[1,1'-biphenyl]-4-carboxamide | 458 |
| Example37 (A37) | 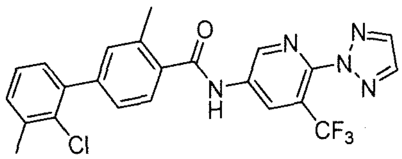 | N-(6-(2H-1,2,3-triazol-2-yl)-5-(trifluoromethyl)pyridin-3-yl)-2'-chloro-3,3'-dimethyl-[1,1'-biphenyl]-4-carboxamide | 472 |
| Example38 (A38) | 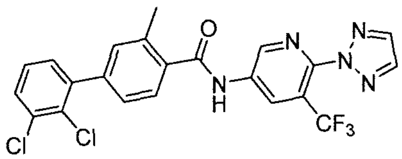 | N-(6-(2H-1,2,3-triazol-2-yl)-5-(trifluoromethyl)pyridin-3-yl)-2',3'-dichloro-3-methyl-[1,1'-biphenyl]-4-carboxamide | 492 |
| Example39 (A39) | 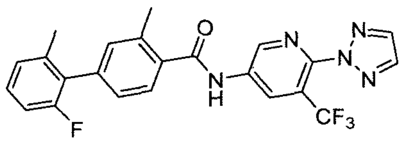 | N-(6-(2H-1,2,3-triazol-2-yl)-5-(trifluoromethyl)pyridin-3-yl)-2'-fluoro-3,6'-dimethyl-[1,1'-biphenyl]-4-carboxamide | 456 |
| Example40 (A40) | 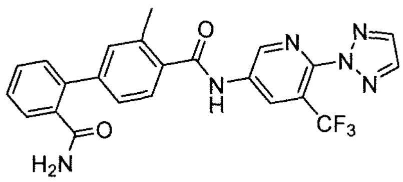 | N4'-(6-(2H-l,2,3-triazol-2-yl)-5-(trifluoromethyl)pyridin-3-yl)-3'-methyl-[1,1'-biphenyl]-2,4'-dicarboxamide | 467 |
| Example43 (A43) | 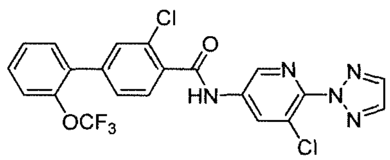 | 3-chloro-N-(5-chloro-6-(2H-1,2,3-triazol-2-yl)pyridin-3-yl)-2'-(trifluoromethoxy)-[1,1'-biphenyl]-4-carboxamide | 494 |
| Example44 (A44) | 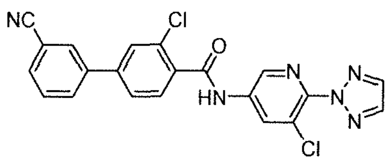 | 3-chloro-N-(5-chloro-6-(2H-1,2,3-triazol-2-yl)pyridin-3-yl)-3'-cyano-[1,1'-biphenyl]-4-carboxamide | 435 |
| Example45 (A45) | 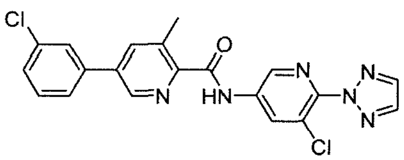 | N-(5-chloro-6-(2H-1,2,3-triazol-2-yl) pyridin-3-yl)-5-(3-chlorophenyl)-3-methylpicolinamide | 425 |

(continued)

| Example | Structure | Chemical Name | MS [M+H]+ |
|---|---|---|---|
| Example46 (A46) | 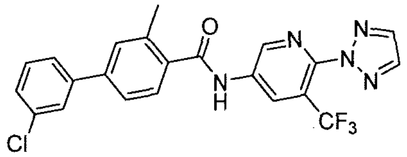 | N-(6-(2H-]1,2,3-triazol-2-yl)-5-(trifluoromethyl)pyridin-3-yl)-3'-chloro-3-methyl-[1,1'-biphenyl]-4-carboxamide | 458 |
| Example47 (A47) | 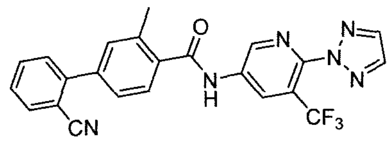 | N-(6-(2H-1,2,3-triazol-2-yl)-5-(trifluoromethyl)pyridin-3-yl)-2'-cyano-3-methyl-[1,1'-biphenyl]-4-carboxamide | 449 |
| Example48 (A48) | 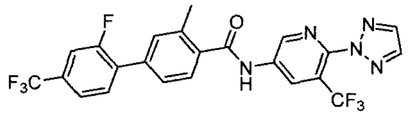 | N-(6-(2H-1,2,3-triazol-2-yl)-5-(trifluoromethyl)pyridin-3-yl)-2'-fluoro-3-methyl-4'-(trifluoromethyl)-[1,1'-biphenyl]-4-carboxamide | 510 |
| Example49 (A49) | 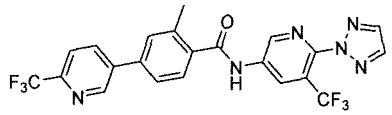 | N-(6-(2H-1,2,3 -triazol-2-yl)-5-(trifluoromethyl)pyridin-3-yl)-2-methyl-4-(6-(trifluoromethyl)pyridin-3-yl) benzamide | 493 |
| Example50 (A50) | 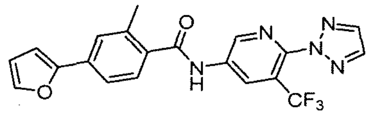 | N-(6-(2H-1,2,3-triazol-2-yl)-5-(trifluoromethyl)pyridin-3-yl)-4-(furan-2-yl)-2-methylbenzamide | 414 |
| Example51 (A51) | 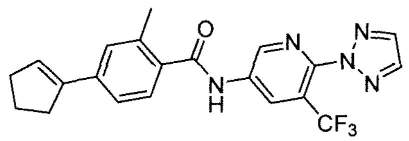 | N-(6-(2H-1,2,3-triazol-2-yl)-5-(trifluoromethyl)pyridin-3-yl)-4-(cyclopent-1-en-1-yl)-2-methylbenzamide | 414 |
| Example52 (A52) | 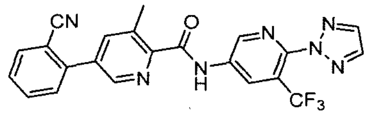 | N-(6-(2H-1,2,3-triazol-2-yl)-5-(trifluoromethyl)pyridin-3-yl)-5-(2-cyanophenyl)-3-methylpicolinamide | 450 |
| Example53 (A53) | 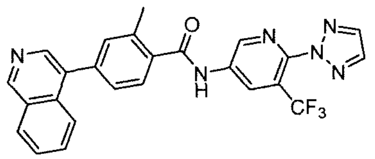 | N-(6-(2H-l,2,3-triazol-2-yl)-5-(trifluoromethyl)pyridin-3-yl)-4-(isoquinolin-4-yl)-2-methylbenzamide | 475 |
| Example54 (A54) | 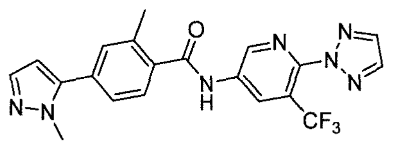 | N-(6-(2H-1,2,3-triazol-2-yl)-5-(trifluoromethyl)pyridin-3-yl)-2-methyl-4-(1-methyl-1H-pyrazol-5-yl)benzamide | 428 |
| Example55 (A55) | 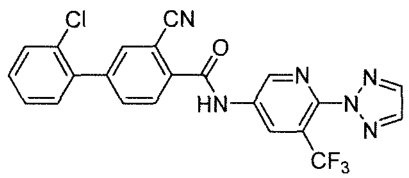 | N-(6-(2H-1,2,3-triazol-2-yl)-5-(trifluoromethyl)pyridin-3-yl)-2'-chloro-3-cyano-[1,1'-biphenyl]-4-carboxamide | 469 |

(continued)

| Example | Structure | Chemical Name | MS $[M+H]^+$ |
|---|---|---|---|
| Example56 (A56) | 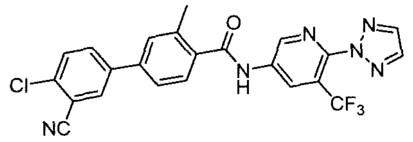 | N-(6-(2H-1,2,3-triazol-2-yl)-5-(trifluoromethyl)pyridin-3-yl)-4'-chloro-3'-cyano-3-methyl-[1,1'-biphenyl]-4-carboxamide | 483 |
| Example57 (A57) | 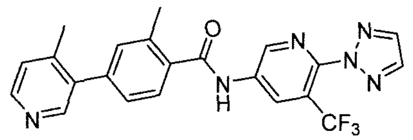 | N-(6-(2H-1,2,3-triazol-2-yl)-5-(trifluoromethyl)pyridin-3-yl)-2-methyl-4-(4-methylpyridin-3-yl)benzamide | 439 |
| Example58 (A58) | 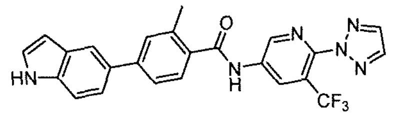 | N-(6-(2H-l,2,3-triazol-2-yl)-5-(trifluoromethyl)pyridin-3-yl)-4-(1H-indol-5-yl)-2-methylbenzamide | 463 |
| Example59 (A59) | 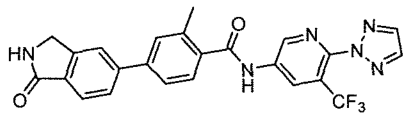 | N-(6-(2H-l,2,3-triazol-2-yl)-5-(trifluoromethyl)pyridin-3-yl)-2-methyl-4-(1-oxoisoindolin-5-yl)benzamide | 479 |
| Example60 (A60) | 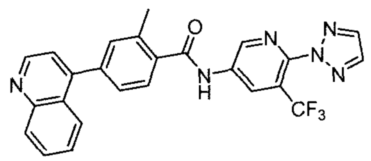 | N-(6-(2H-1,2,3-triazol-2-yl)-5-(trifluoromethyl)pyridin-3-yl)-2-methyl-4-(quinolin-4-yl)benzamide | 475 |
| Example61 (A61) | 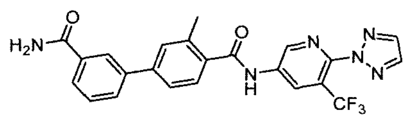 | N4'-(6-(2H-1,2,3-triazol-2-yl)-5-(trifluoromethyl)pyridin-3-yl)-3'-methyl-[1,1'-biphenyl]-3,4'-dicarboxamide | 467 |
| Example62 (A62) | 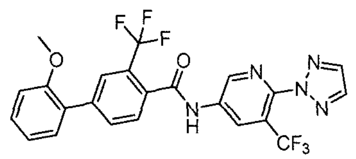 | N-(6-(2H-1,2,3-triazol-2-yl)-5-(trifluoromethyl)pyridin-3-yl)-2'-methoxy-3-(trifluoromethyl)-[1,1'-biphenyl]-4-carboxamide | 508 |
| Example63 (A63) | 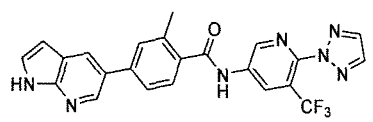 | N-(6-(2H-1,2,3-triazol-2-yl)-5-(trifluoromethyl)pyridin-3-yl)-2-methyl-4-(1H-pyrrolo[2,3-b]pyridin-5-yl) benzamide | 464 |
| Example65 (A65) | 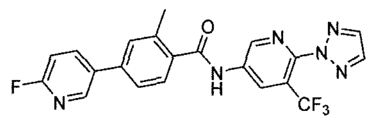 | N-(6-(2H-1,2,3-triazol-2-yl)-5-(trifluoromethyl)pyridin-3-yl)-4-(6-fluoropyridin-3-yl)-2-methylbenzamide | 443 |
| Example66 (A66) | 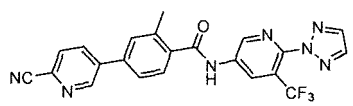 | N-(6-(2H-1,2,3-triazol-2-yl)-5-(trifluoromethyl)pyridin-3-yl)-4-(6-cyanopyridin-3-yl)-2-methylbenzamide | 450 |

(continued)

| Example | Structure | Chemical Name | MS [M+H]+ |
|---|---|---|---|
| Example68 (A68) | 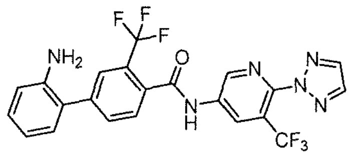 | N-(6-(2H-l,2,3-triazol-2-yl)-5-(trifluoromethyl)pyridin-3-yl)-2'-amino-3-(trifluoromethyl)-[1,1'-biphenyl]-4-carboxamide | 493 |
| Example70 (A70) | 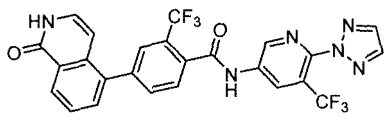 | N-(6-(2H-1,2,3-triazol-2-yl)-5-(trifluoromethyl)pyridin-3-yl)-4-(1-oxo-1,2-dihydroisoquinolin-5-yl)-2-(trifluoromethyl)benzamide | 545 |
| Example71 (A71) | 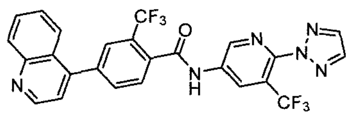 | N-(6-(2H-1,2,3-triazol-2-yl)-5-(trifluoromethyl)pyridin-3-yl)-4-(quinolin-4-yl)-2-(trifluoromethyl)benzamide | 529 |
| Example72 (A72) | 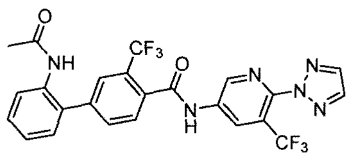 | N-(6-(2H-1,2,3-triazol-2-yl)-5-(trifluoromethyl)pyridin-3-yl)-2'-acetamido-3-(trifluoromethyl)-[1,1'-biphenyl]-4-carboxamide | 535 |
| Example73 (A73) | 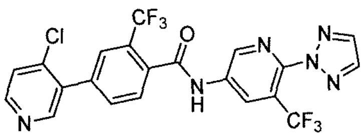 | N-(6-(2H-1,2,3-triazol-2-yl)-5-(trifluoromethyl)pyridin-3-yl)-4-(4-chloropyridin-3-yl)-2-(trifluoromethyl) benzamide | 513 |
| Example74 (A74) | 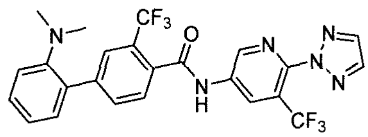 | N-(6-(2H-1,2,3-triazol-2-yl)-5-(trifluoromethyl)pyridin-3-yl)-2'-(dimethylamino)-3-(trifluoromethyl)-[1,1'-biphenyl]-4-carboxamide | 521 |
| Example75 (A75) | 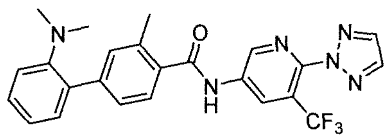 | N-(6-(2H-1,2,3-triazol-2-yl)-5-(trifluoromethyl)pyridin-3-yl)-2'-(dimethylamino)-3-methyl-[1,1'-biphenyl]-4-carboxamide | 467 |
| Example76 (A76) | 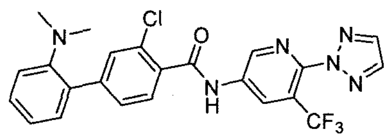 | N-(6-(2H-1,2,3-triazol-2-yl)-5-(trifluoromethyl)pyridin-3-yl)-3-chloro-2'-(dimethylamino)-[1,1'-biphenyl]-4-carboxamide | 487 |
| Example77 (A77) | 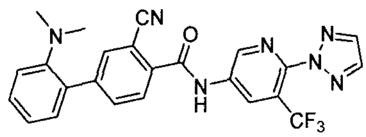 | N-(6-(2H-1,2,3-triazol-2-yl)-5-(trifluoromethyl)pyridin-3-yl)-3-cyano-2'-(dimethylamino)-[1,1'-biphenyl]-4-carboxamide | 478 |
| Example78 (A78) | 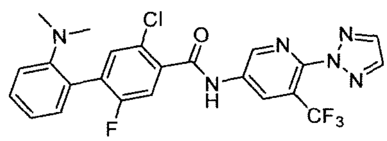 | N-(6-(2H-1,2,3-triazol-2-yl)-5-(trifluoromethyl)pyridin-3-yl)-5-chloro-2'-(dimethylamino)-2-fluoro-[1,1'-biphenyl]-4-carboxamide | 505 |

(continued)

| Example | Structure | Chemical Name | MS [M+H]+ |
|---|---|---|---|
| Example79 (A79) | 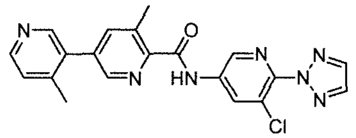 | N-(5-chloro-6-(2H-1,2,3-triazol-2-yl) pyridin-3-yl)-4',5-dimethyl-[3,3'-bipyridine]-6-carboxamide | 406 |
| Example80 (A80) | 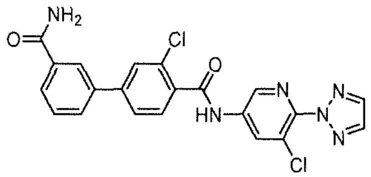 | 3'-chloro-N4'-(5-chloro-6-(2H-l,2,3-triazol-2-yl)pyridin-3-yl)-[1,1'-biphenyl]-3,4'-dicarboxamide | 453 |
| Example81 (A81) | 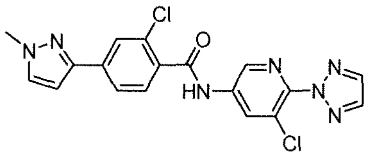 | 2-chloro-N-(5-chloro-6-(2H-1,2,3-triazol-2-yl)pyridin-3-yl)-4-(1-methyl-1H-pyrazol-3-yl)benzamide | 414 |
| Example82 (A82) | 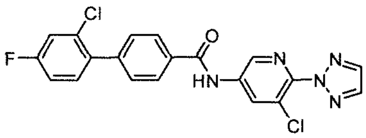 | 2'-chloro-N-(5-chloro-6-(2H-l,2,3-triazol-2-yl)pyridin-3-yl)-4'-fluoro-[1,1'-biphenyl]-4-carboxamide | 428 |
| Example83 (A83) | 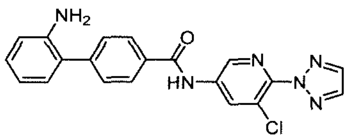 | 2'-amino-N-(5-chloro-6-(2H-1,2,3-triazol-2-yl)pyridin-3-yl)-[1,1'-biphenyl]-4-carboxamide | 391 |
| Example84 (A84) | 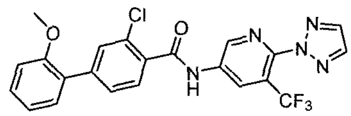 | N-(6-(2H-1,2,3-triazol-2-yl)-5-(trifluoromethyl)pyridin-3-yl)-3-chloro-2'-methoxy-[1,1'-biphenyl]-4-carboxamide | 474 |
| Example85 (A85) | 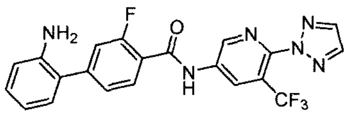 | N-(6-(2H-1,2,3-triazol-2-yl)-5-(trifluoromethyl)pyridin-3-yl)-2'-amino-3-fluoro-[1,1'-biphenyl]-4-carboxamide | 443 |
| Example86 (A86) | 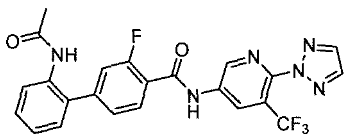 | N-(6-(2H-1,2,3-triazol-2-yl)-5-(trifluoromethyl)pyridin-3-yl)-2'-acetamido-3-fluoro-[l,l'-biphenyl]-4-carboxamide | 485 |
| Example87 (A87) | 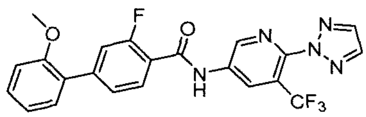 | N-(6-(2H-1,2,3-triazol-2-yl)-5-(trifluoromethyl)pyridin-3-yl)-3-fluoro-2'-methoxy-[1,1'-biphenyl]-4-carboxamide | 458 |
| Example88 (A88) | 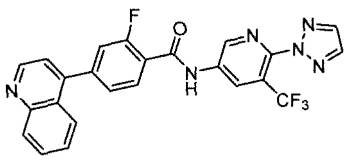 | N-(6-(2H-1,2,3-triazol-2-yl)-5-(trifluoromethyl)pyridin-3-yl)-2-fluoro-4-(quinolin-4-yl)benzamide | 479 |

(continued)

| Example | Structure | Chemical Name | MS [M+H]+ |
|---|---|---|---|
| Example89 (A89) | 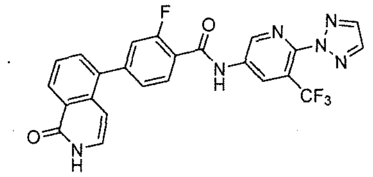 | N-(6-(2H-1,2,3-triazot-2-yl)-5-(trifluoromethyl)pyridin-3-yl)-2-fluoro-4-(1-oxo-1,2-dihydroisoquinolin-5-yl) benzamide | 495 |
| Example90 (A90) | 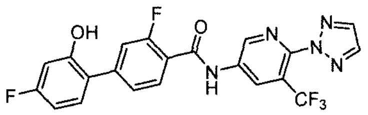 | N-(6-(2H-1,2,3-triazol-2-yl)-5-(trifluoromethyl)pyridin-3-yl)-3,4'-difluoro-2'-hydroxy-[1,1'-biphenyl]-4-carboxamide | 462 |
| Example91 (A91) | 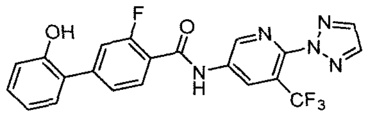 | N-(6-(2H-l,2,3-triazol-2-yl)-5-(trifluoromethyl)pyridin-3-yl)-3-fluoro-2'-hydroxy-[1,1'-biphenyl]-4-carboxamide | 444 |
| Example92 (A92) | 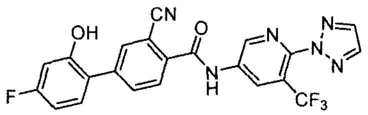 | N-(6-(2H-l,2,3-triazol-2-yl)-5-(trifluoromethyl)pyridin-3-yl)-3-cyano-4'-fluoro-2'-hydroxy-[1,1'-biphenyl)-4-carboxamide | 469 |
| Example93 (A93) | 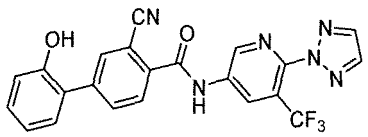 | N-(6-(2H-1,2,3-triazol-2-yl)-5-(trifluoromethyl)pyridin-3-yl)-3-cyano-2'-hydroxy-[1,1'-biphenyl]-4-carboxamide | 451 |
| Example94 (A94) | 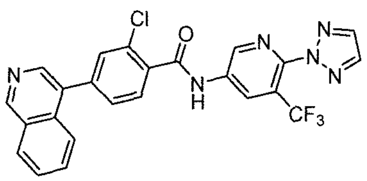 | N-(6-(2H-1,2,3-triazol-2-yl)-5-(trifluoromethyl)pyridin-3-yl)-2-chloro-4-(isoquinolin-4-yl)benzamide | 495 |
| Example95 (A95) | 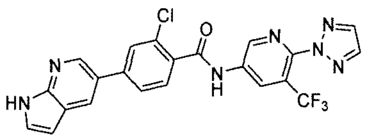 | N-(6-(2H-1,2,3-triazol-2-yl)-5-(trifluoromethyl)pyridin-3-yl)-2-chloro-4-(1H-pyrrolo[2,3-b]pyridin-5-yl) benzamide | 484 |
| Example96 (A96) | 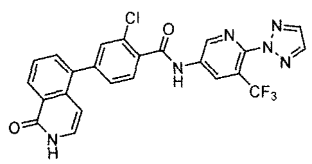 | N-(6-(2H-1,2,3-triazol-2-yl)-5-(trifluoromethyl)pyridin-3-yl)-2-chloro-4-(1-oxo-1,2-dihydroisoquinolin-5-yl) benzamide | 511 |
| Example97 (A97) | 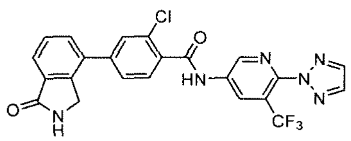 | N-(6-(2H-1,2,3-triazol-2-yl)-5-(trifluoromethyl)pyridin-3-yl)-2-chloro-4-(1-oxoisoindolin-4-yl)benzamide | 499 |
| Example99 (A99) | 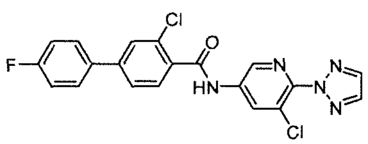 | 3-chloro-N-(5-chloro-6-(2H-1,2,3-triazol-2-yl)pyridin-3-yl)-4'-fluoro-[1,1'-biphenyl]-4-carboxamide | 428 |

(continued)

| Example | Structure | Chemical Name | MS $[M+H]^+$ |
|---|---|---|---|
| Example101 (A101) | 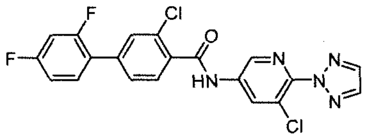 | 3-chloro-N-(5-chloro-6-(2H-l,2,3-triazol-2-yl)pyridin-3-yl)-2',4'-difluoro-[1,1'-biphenyl]-4-carboxamide | 446 |
| Example103 (A103) | 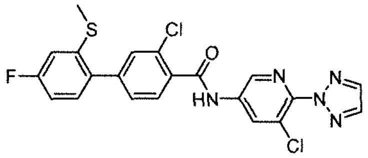 | 3-chloro-N-(5-chloro-6-(2H-1,2,3-triazol-2-yl)pyridin-3-yl)-4'-fluoro-2'-(methylthio)-[1,1'-biphenyl]-4-carboxamide | 474 |
| Example106 (A106) | 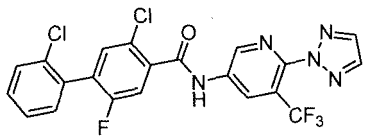 | N-(6-(2H-1,2,3-triazol-2-yl)-5-(trifluoromethyl)pyridin-3-yl)-2',5-dichloro-2-fluoro-[1,1'-biphenyl]-4-carboxamide | 496 |
| Example107 (A107) | 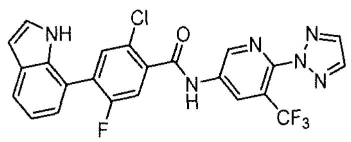 | N-(6-(2H-l,2,3-triazol-2-yl)-5-(trifluoromethyl)pyridin-3-yl)-2-chloro-5-fluoro-4-(1H-indol-7-yl)benzamide | 501 |
| Example108 (A108) | 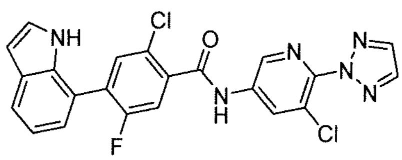 | 2-chloro-N-(5-chloro-6-(2H-1,2,3-triazol-2-yl)pyridin-3-yl)-5-fluoro-4-(1 H-indol-7-yl)benzamide | 467 |
| Example109 (A109) | 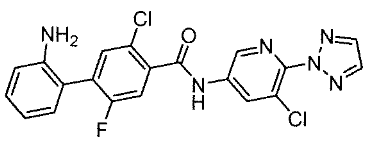 | 2'-amino-5-chloro-N-(5-chloro-6-(2H-l,2,3-triazol-2-yl)pyridin-3-yl)-2-fluoro-[1,1'-biphenyl]-4-carboxamide | 443 |
| Example111 (A111) | 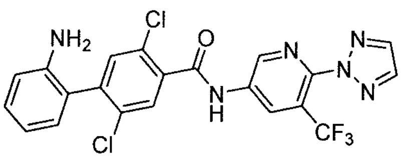 | N-(6-(2H-1,2,3-triazol-2-yl)-5-(trifluoromethyl)pyridin-3-yl)-2'-amino-2,5-dichloro-[1,1'-biphenyl]-4-carboxamide | 493 |
| Example112 (A112) | 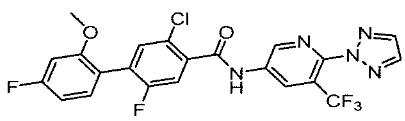 | N-(6-(2H-1,2,3-triazol-2-yl)-5-(trifluoromethyl)pyridin-3-yl)-5-chloro-2,4'-difluoro-2'-methoxy-[1,1'-biphenyl]-4-carboxamide | 510 |
| Example115 (A115) | 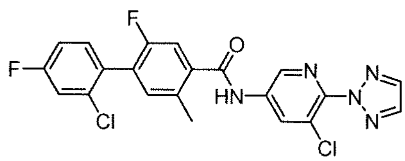 | 2'-chloro-N-(5-chloro-6-(2H-1,2,3-triazol-2-yl)pyridin-3-yl)-2,4'-difluoro-5-methyl-[1,1'-biphenyl]-4-carboxamide | 460 |
| Example116 (A116) | 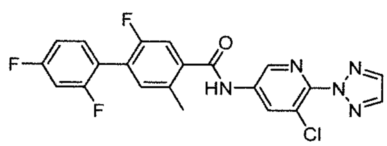 | N-(5-chloro-6-(2H-1,2,3-triazol-2-yl) pyridin-3-yl)-2,2',4'-trifluoro-5-methyl-[1,1'-biphenyl]-4-carboxamide | 444 |

(continued)

| Example | Structure | Chemical Name | MS [M+H]+ |
|---|---|---|---|
| Example117 (A117) | 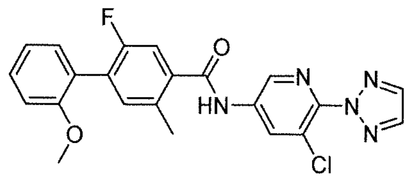 | N-(5-chloro-6-(2H-1,2,3-triazol-2-yl) pyridin-3-yl)-2-fluoro-2'-methoxy-5-methyl-[1,1'-biphenyl]-4-carboxamide | 438 |
| Example118 (A118) | 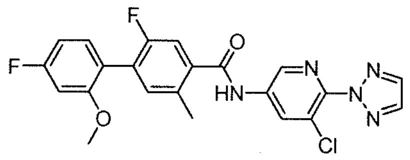 | N-(5-chloro-6-(2H-1,2,3-triazol-2-yl) pyridin-3-yl)-2,4'-difluoro-2'-methoxy-5-methyl-[1,1'-biphenyl]-4-carboxamide | 456 |
| Example119 (A119) | 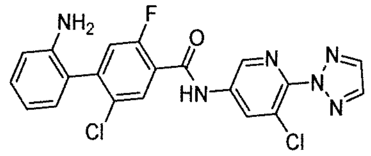 | 2'-amino-2-chloro-N-(5-chloro-6-(2H-1,2,3-triazol-2-yl)pyridin-3-yl)-5-fluoro-[1,1'-biphenyl]-4-carboxamide | 443 |
| Example120 (A120) | 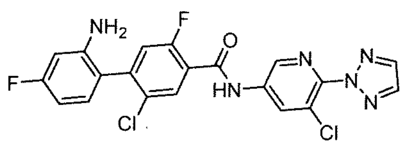 | 2'-amino-2-chloro-N-(5-chloro-6-(2H-1,2,3-triazol-2-yl)pyridin-3-yl)-4',5-difluoro-[1,1'-biphenyl]-4-carboxamide | 461 |
| Example121 (A121) | 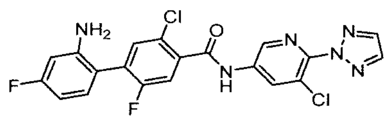 | 2'-amino-5-chloro-N-(5-chloro-6-(2H-1,2,3-triazol-2-yl)pyridin-3-yl)-2,4'-difluoro-[1,1'-biphenyl]-4-carboxamide | 461 |
| Example122 (A122) | 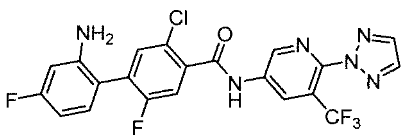 | N-(6-(2H-l,2,3-triazol-2-yl)-5-(trifluoromethyl)pyridin-3-yl)-2'-amino-5-chloro-2,4'-difluoro-[1,1'-biphenyl]-4-carboxamide | 495 |
| Example123 (A123) | 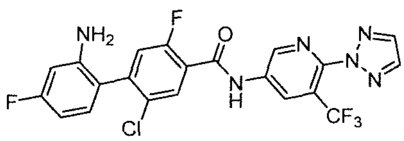 | N-(6-(2H-1,2,3-triazol-2-yl)-5-(trifluoromethyl)pyridin-3 - yl)-2'-amino-2-chloro-4',5-difluoro-[1,1'-biphenyl]-4-carboxamide | 495 |
| Example125 (A125) | 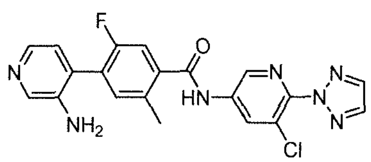 | 4-(3-aminopyridin-4-yl)-N-(5-chloro-6-(2H-1,2,3-triazol-2-yl)pyridin-3-yl)-5-fluoro-2-methylbenzamide | 424 |
| Example127 (A127) | 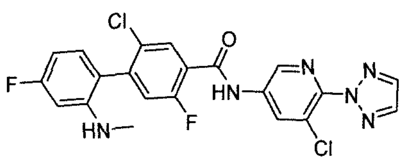 | 2-chloro-N-(5-chloro-6-(2H-l,2,3-triazol-2-yl)pyridin-3-yl)-4',5-difluoro-2'-(methylamino)-[1,1'-biphenyl]-4-carboxamide | 475 |

(continued)

| Example | Structure | Chemical Name | MS [M+H]+ |
|---|---|---|---|
| Example128 (A128) | 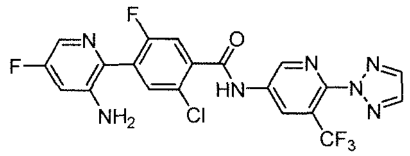 | N-(6-(2H-1,2,3-triazol-2-yl)-5-(trifluoromethyl)pyridin-3-yl)-4-(3-amino-5-fluoropyridin-2-yl)-2-chloro-5-fluorobenzamide | 496 |
| Example129 (A129) | 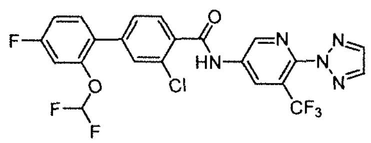 | N-(6-(2H-1,2,3-triazo)-2-yl)-5-(trifluoromethyl)pyridin-3-yl)-3-chloro-2'-(difluoromethoxy)-4'-fluoro-[1,1']-biphenyl]-4-carboxamide | 528 |
| Example131 (A131) | 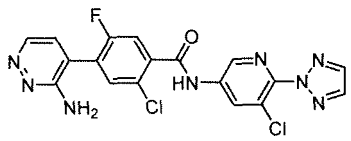 | 4-(3-aminopyridazin-4-yl)-2-chloro-N-(5-chloro-6-(2H-1,2,3-triazol-2-yl)pyridin-3-yl)-5-fluorobenzamide | 445 |
| Example132 (A132) | 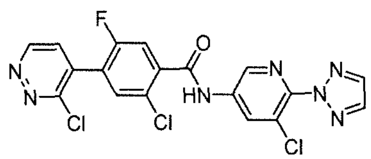 | 2-chloro-N-(5-chloro-6-(2H-1,2,3-triazol-2-yl)pyridin-3-yl)-4-(3-chloropyridazin-4-yl)-5-fluorobenzamide | 463 |
| Example134 (A134) | 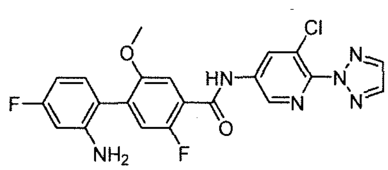 | 2'-amino-N-(5-chloro-6-(2H-l,2,3-triazol-2-yl)pyridin-3-yl)-4',5-difluoro-2-methoxy-[1,1'-biphenyl]-4-carboxamide | 457 |
| Example135 (A135) | 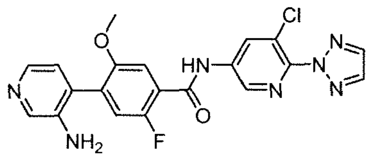 | 4-(3-aminopyridin-4-yl)-N-(5-chloro-6-(2H-1,2,3-triazol-2-yl)pyridin-3-yl)-2-fluoro-5-methoxybenzamide | 440 |
| Example136 (A136) | 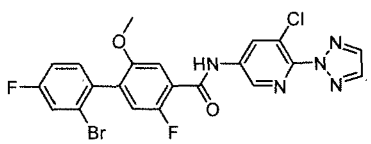 | 2'-bromo-N-(5-chloro-6-(2H-1,2,3-triazol-2-yl)pyridin-3-yl)-4',5-difluoro-2-methoxy-[1,1'-biphenyl]-4-carboxamide | 519 |
| Example137 (A137) | 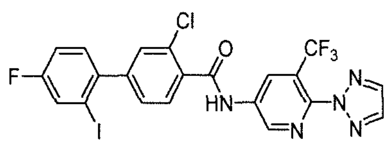 | N-(6-(2H-1,2,3-triazol-2-yl)-5-(trifluoromethyl)pyridin-3-yl)-3-chloro-4'-fluoro-2'-iodo-[1,1'-biphenyl]-4-carboxamide | 387 |
| Example138 (A138) | 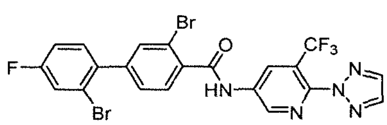 | N-(6-(2H-1,2,3-triazol-2-yl)-5-(trifluoromethyl)pyridin-3-yl)-2',3-dibromo-4'-fluoro-[1,1'-biphenyl]-4-carboxamide | 583 |
| Example139 (A139) | 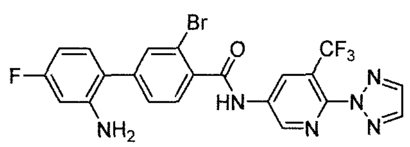 | N-(6-(2H-1,2,3-triazol-2-yl)-5-(trifluoromethyl)pyridin-3-yl)-2'-amino-3-bromo-4'-fluoro-[1,1'-biphenyl]-4-carboxamide | 522 |

(continued)

| Example | Structure | Chemical Name | MS [M+H]+ |
|---|---|---|---|
| Example140 (A140) | 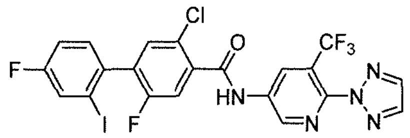 | N-(6-(2H-l,2,3-triazol-2-yl)-5-(trifluoromethyl)pyridin-3-yl)-5-chloro-2,4'-difluoro-2'-iodo-[1,1'-biphenyl]-4-carboxamide | 605 |
| Example141 (A141) | 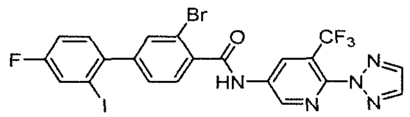 | N-(6-(2H-1,2,3-triazol-2-yl)-5-(trifluoromethyl)pyridin-3-yl)-3-bromo-4'-fluoro-2'-iodo-[1,1'-biphenyl]-4-carboxamide | 631 |
| Example142 (A142) | 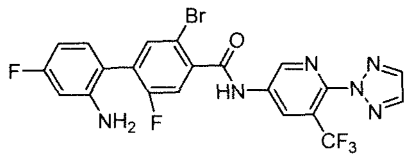 | N-(6-(2H-1,2,3-triazol-2-yl)-5-(trifluoromethyl)pyridin-3-yl)-2'-amino-5-bromo-2,4'-difluoro-[1,1'-biphenyl]-4-carboxamide | 539 |
| Example143 (A143) | 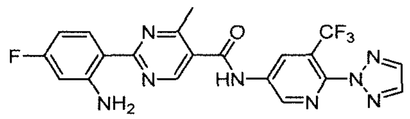 | N-(6-(2H-1,2,3-triazol-2-yl)-5-(trifluoromethyl)pyridin-3-yl)-2-(2-amino-4-fluorophenyl)-4-methylpyrimidine-5-carboxamide | 459 |
| Example144 (A144) | 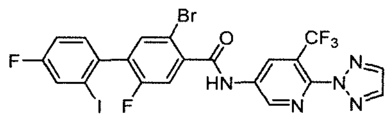 | N-(6-(2H-1,2,3-triazol-2-yl)-5-(trifluoromethyl)pyridin-3-yl)-5-bromo-2,4'-difluoro-2'-iodo-[1,1'-biphenyl]-4-carboxamide | 649 |
| Example145 (A145) | 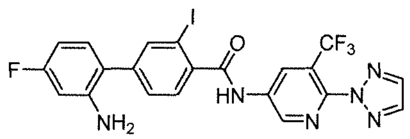 | N-(6-(2H-1,2,3-triazol-2-yl)-5-(trifluoromethyl)pyridin-3-yl)-2'-amino-4'-fluoro-3-iodo-[1,1'-biphenyl]-4-carboxamide | 569 |
| Example146 (A146) | 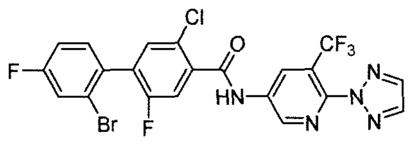 | N-(6-(2H-l,2,3-triazol-2-yl)-5-(trifluoromethyl)pyridin-3-yl)-2'-bromo-5-chloro-2,4'-difluoro-[1,1'-biphenyl]-4-carboxamide | 557 |
| Example148 (A148) | 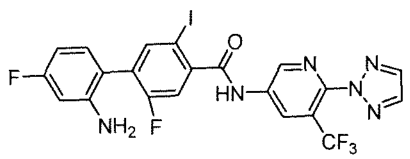 | N-(6-(2H-1,2,3-triazol-2-yl)-5-(trifluoromethyl)pyridin-3-yl)-2'-amino-2,4'-difluoro-5-iodo-[1,1'-biphenyl]-4-carboxamide | 587 |
| Example149 (A149) | 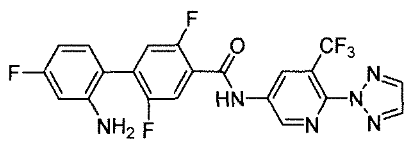 | N-(6-(2H-1,2,3-triazo)-2-yl)-5-(trifluoromethyl)pyridin-3-yl)-2'-amino-2,4',5-trifluoro-1,1'-biphenyl]-4-carboxamide | 478 |
| Example150 (A150) | 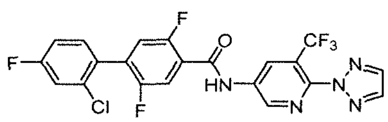 | N-(6-(2H-1,2,3-triazol-2-yl)-5-(trifluoromethyl)pyridin-3-yl)-2'-chloro-2,4',5-trifluoro-[1,1'-biphenyl]-4-carboxamide | 498 |

(continued)

| Example | Structure | Chemical Name | MS [M+H]+ |
|---|---|---|---|
| Example151 (A151) | 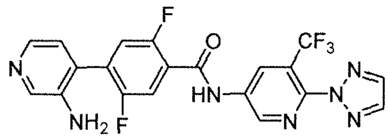 | N-(6-(2H-1,2,3-triazol-2-yl)-5-(trifluoromethyl)pyridin-3-yl)-4-(3-aminopyridin-4-yl)-2,5-difluorobenzamide | 462 |
| Example152 (A152) | 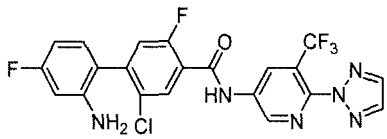 | N-(6-(2H-1,2,3-triazol-2-yl)-5-(trifluoromethyl)pyridin-3-yl)-2'-amino-2-chloro-4',5-difluoro-[1,1'-biphenyl]-4-carboxamide | 495 |
| Example153 (A153) | 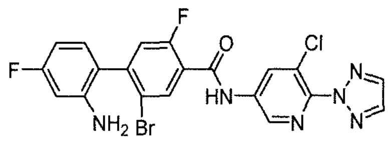 | 2'-amino-2-bromo-N-(5-chloro-6-(2H-1,2,3-triazol-2-yl)pyridin-3-yl)-4',5-difluoro-[1,1'-biphenyl]-4-carboxamide | 504 |
| Example154 (A154) | 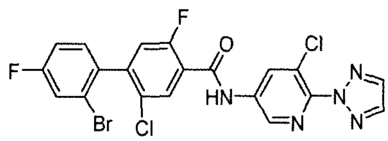 | 2'-bromo-2-chloro-N-(5-chloro-6-(2H-1,2,3-triazol-2-yl)pyridin-3-yl)-4',5-difluoro-[1,1'-biphenyl]-4-carboxamide | 523 |
| Example155 (A155) | 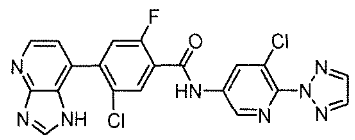 | 5-chloro-N-(5-chloro-6-(2H-1,2,3-triazol-2-yl)pyridin-3-yl)-2-fluoro-4-(1H-imidazo [4,5-b]pyridin-7-yl)benzamide | 469 |
| Example156 (A156) | 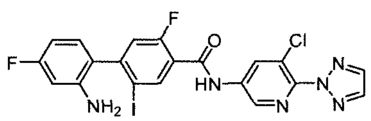 | 2'-amino-N-(5-chloro-6-(2H-1,2,3-triazol-2-yl)pyridin-3-yl)-4',5-difluoro-2-iodo-[1,1'-biphenyl]-4-carboxamide | 552 |
| Example158 (A158) | 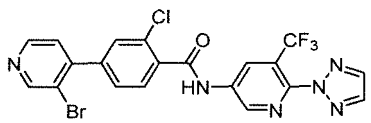 | N-(6-(2H-1,2,3-triazol-2-yl)-5-(trifluoromethyl)pyridin-3-yl)-4-(3-bromopyridin-4-yl)-2-chlorobenzamide | 522 |
| Example159 (A159) | 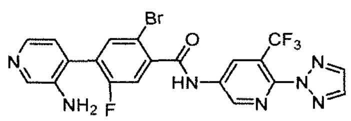 | N-(6-(2H-1,2,3-triazol-2-yl)-5-(trifluoromethyl)pyridin-3-yl)-4-(3-aminopyridin-4-yl)-2-bromo-5-fluorobenzamide | 522 |
| Example160 (A160) | 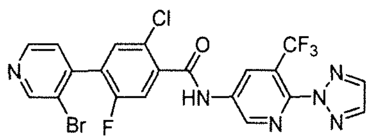 | N-(6-(2H-l,2,3-triazol-2-yl)-5-(trifluoromethyl)pyridin-3-yl)-4-(3-bromopyridin-4-yl)-2-chloro-5-fluorobenzamide | 340 |
| Example161 (A161) | 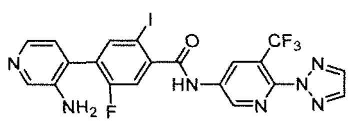 | N-(6-(2H-1,2,3-triazol-2-yl)-5-(trifluoromethyl)pyridin-3-yl)-4-(3 -aminopyridin-4-yl)-5-fluoro-2-iodobenzamide | 570 |

(continued)

| Example | Structure | Chemical Name | MS [M+H]+ |
|---|---|---|---|
| Example162 (A162) | 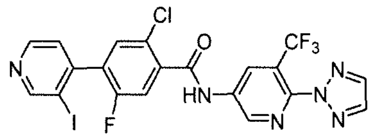 | N-(6-(2H-1,2,3-triazol-2-yl)-5-(trifluoromethyl)pyridin-3-yl)-2-chloro-5-fluoro-4-(3-iodopyridin-4-yl)benzamide | 588 |
| Example163 (A163) | 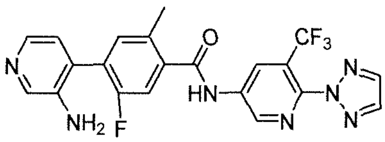 | N-(6-(2H-1,2,3-triazol-2-yl)-5-(trifluoromethyl)pyridin-3-yl)-4-(3-aminopyridin-4-yl)-5-fluoro-2-methylbenzamide | 458 |
| Example164 (A164) | 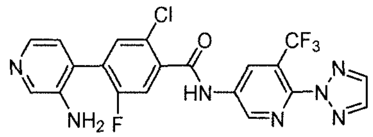 | N-(6-(2H-1,2,3-triazol-2-yl)-5-(trifluoromethyl)pyridin-3-yl)-4-(3-aminopyridin-4-yl)-2-chloro-5-fluorobenzamide | 478 |
| Example165 (A165) | 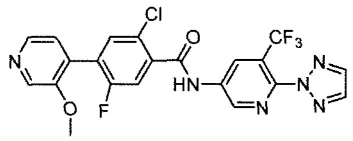 | N-(6-(2H-1,2,3-triazol-2-yl)-5-(trifluoromethyl)pyridin-3-yl)-2-chloro-5-fluoro-4-(3-methoxypyridin-4-yl) benzamide | 493 |
| Example166 (A166) | 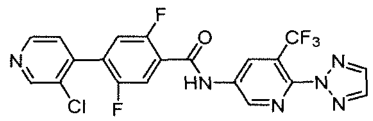 | N-(6-(2H-l,2,3-triazol-2-yl)-5-(trifluoromethyl)pyridin-3-yl)-4-(3-chloropyridin-4-yl)-2,5-difluorobenzamide | 481 |
| Example167 (A167) | 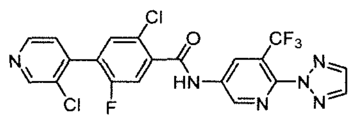 | N-(6-(2H-1,2,3-triazol-2-yl)-5-(trifluoromethyl)pyridin-3-yl)-2-chloro-4-(3-chloropyridin-4-yl)-5-fluorobenzamide | 497 |
| Example168 (A168) | 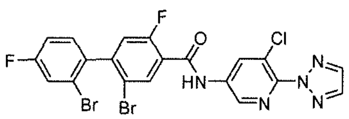 | 2,2'-dibromo-N-(5-chloro-6-(2H-1,2,3-triazol-2-yl)pyridin-3-yl)-4',5-difluoro-[1,1'-biphenyl]-4-carboxamide | 567 |
| Example169 (A169) | 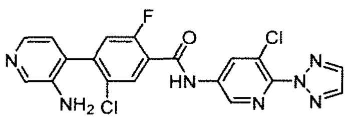 | 4-(3-aminopyridin-4-yl)-5-chloro-N-(5-chloro-6-(2H-1,2,3-triazol-2-yl)pyridin-3-yl)-2-fluorobenzamide | 444.05 |
| Example170 (A170) | 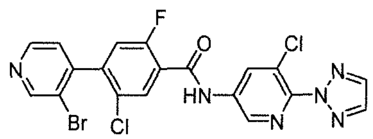 | 4-(3-bromopyridin-4-yl)-5-chloro-N-(5-chloro-6-(2H-1,2,3-triazol-2-yl)pyridin-3-yl)-2-fluorobenzamide | 506 |
| Example171 (A171) | 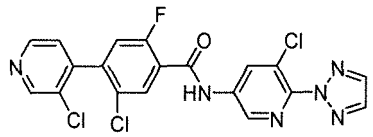 | 5-chloro-N-(5-chloro-6-(2H-1,2,3-triazol-2-yl)pyridin-3-yl)-4-(3-chloropyridin-4-yl)-2-fluorobenzamide | 463 |

(continued)

| Example | Structure | Chemical Name | MS $[M+H]^+$ |
|---|---|---|---|
| Example172 (A172) | 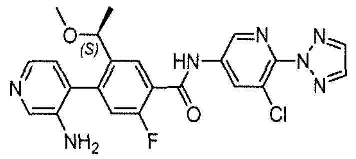 | (S)-4-(3-aminopyridin-4-yl)-N-(5-chloro-6-(2H-1,2,3-triazol-2-yl)pyridin-3-yl)-2-fluoro-5-(1-methoxyethyl)benzamide | 468 |
| Example173 (A173) | 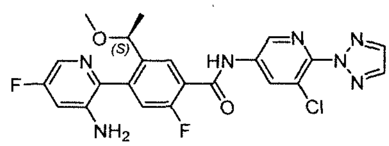 | (S)-4-(3-amino-5-fluoropyridin-2-yl)-N-(5-chloro-6-(2H-1,2,3-triazol-2-yl)pyridin-3-yl)-2-fluoro-5-(1-methoxyethyl)benzamide | 486 |
| Example174 (A174) | 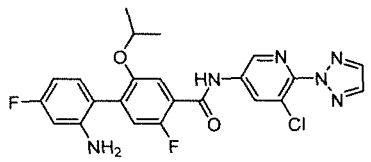 | 2'-amino-N-(5-chloro-6-(2H-l,2,3-triazol-2-yl)pyridin-3-yl)-4',5-difluoro-2-isopropoxy-[1,1'-biphenyl]-4-carboxamide | 485 |
| Examplel75 (A175) | 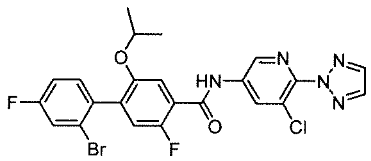 | 2'-bromo-N-(5-chloro-6-(2H-1,2,3-triazol-2-yl)pyridin-3-yl)-4',5-difluoro-2-isopropoxy-[1,1'-biphenyl]-4-carboxamide | 548 |
| Example176 (A176) | 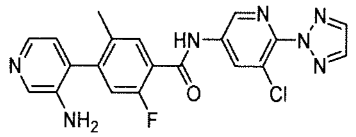 | 4-(3-aminopyridin-4-yl)-N-(5-chloro-6-(2H-1,2,3-triazol-2-yl)pyridin-3-yl)-2-fluoro-5-methylbenzamide | 424 |
| Example177 (A177) | 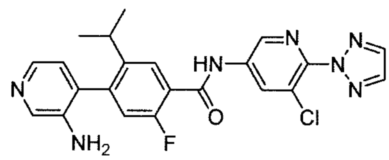 | 4-(3-aminopyridin-4-yl)-N-(5-chloro-6-(2H-1,2,3-triazol-2-yl)pyridin-3-yl)-2-fluoro-5-isopropylbenzamide | 452 |
| Example178 (A178) | 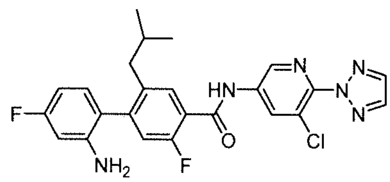 | 2'-amino-N-(5-chloro-6-(2H-l,2,3-triazol-2-yl)pyridin-3-yl)-4',5-difluoro-2-isobutyl-[1,1'-biphenyl] -4-carboxamide | 483 |
| Example179 (A179) | 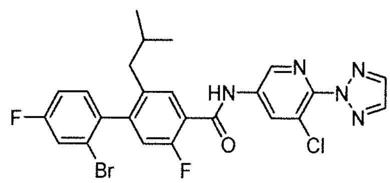 | 2'-bromo-N-(5-chloro-6-(2H-1,2,3-triazol-2-yl)pyridin-3-yl)-4',5-difluoro-2-isobutyl-[1,1'-biphenyl]-4-carboxamide | 546 |
| Example180 (A180) | 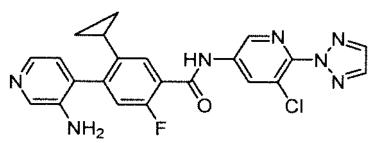 | 4-(3-aminopyridin-4-yl)-N-(5-chloro-6-(2H-1,2,3-triazol-2-yl)pyridin-3-yl)-5-cyclopropyl-2-fluorobenzamide | 450 |

(continued)

| Example | Structure | Chemical Name | MS $[M+H]^+$ |
|---|---|---|---|
| Example181 (A181) | 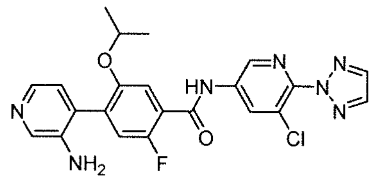 | 4-(3-aminopyridin-4-yl)-N-(5-chloro-6-(2H-1,2,3-triazol-2-yl)pyridin-3-yl)-2-fluoro-5-isopropoxybenzamide | 468 |
| Example182 (A182) | 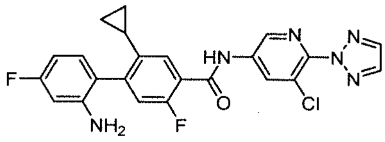 | 2'-amino-N-(5-chloro-6-(2H-1,2,3-triazol-2-yl)pyridin-3-yl)-2-cyclopropyl-4',5-difluoro-[l,l'-biphenyl]-4-carboxamide | 467 |
| Example183 (A183) | 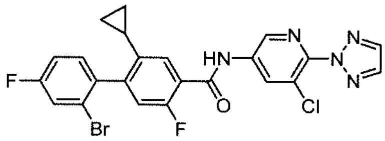 | 2'-bromo-N-(5-chloro-6-(2H-1,2,3-triazol-2-yl)pyridin-3-yl)-2-cyclopropyl-4',5-difluoro-[1,1'-biphenyl]-4-carboxamide | 330 |
| Example184 (A184) | 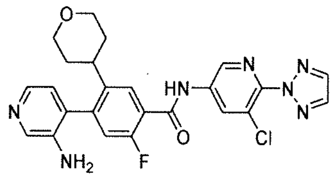 | 4-(3-aminopyridin-4-yl)-N-(5-chloro-6-(2H-l,2,3-triazol-2-yl)pyridin-3-yl)-2-fluoro-5-(tetrahydro-2H-pyran-4-yl) benzamide | 494 |
| Example185 (A185) | 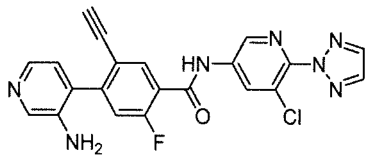 | 4-(3-aminopyridin-4-yl)-N-(5-chloro-6-(2H-1,2,3-triazol-2-yl)pyridin-3-yl)-5-ethynyl-2-fluorobenzamide | 434 |
| Example186 (A186) | 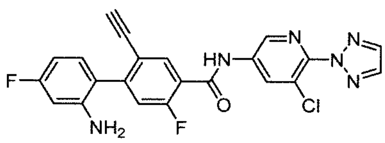 | 2'-amino-N-(5-chloro-6-(2H-1,2,3-triazol-2-yl)pyridin-3-yl)-2-ethynyl-4',5-difluoro-[1,1'-biphenyl]-4-carboxamide | 451 |
| Example187 (A187) | 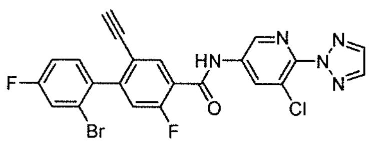 | 2'-bromo-N-(5-chloro-6-(2H-1,2,3-triazol-2-yl)pyridin-3-yl)-2-ethynyl-4',5-difluoro-[1,1'-biphenyl]-4-carboxamide | 513 |
| Example188 (A188) | 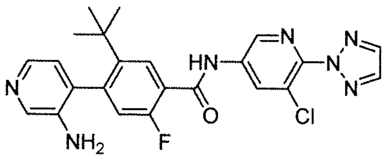 | 4-(3-aminopyridin-4-yl)-5-(tert-butyl)-N-(5-chloro-6-(2H-l,2,3-triazol-2-yl) pyridin-3-yl)-2-fluorobenzamide | 466 |
| Example189 (A189) | 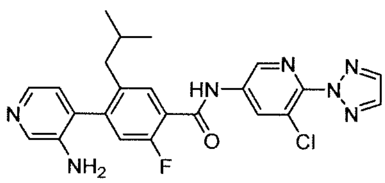 | 4-(3-aminopyridin-4-yl)-N-(5-chloro-6-(2H-1,2,3-triazol-2-yl)pyridin-3-yl)-2-fluoro-5-isobutylbenzamide | 466 |

(continued)

| Example | Structure | Chemical Name | MS [M+H]+ |
|---|---|---|---|
| Example190 (A190) | 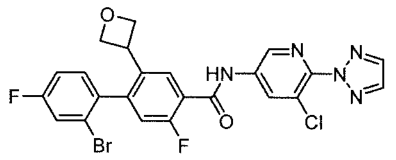 | 2'-bromo-N-(5-chloro-6-(2H-1,2,3-triazol-2-yl)pyridin-3-yl)-4',5-difluoro-2-(oxetan-3-yl)-[1,1'-biphenyl]-4-carboxamide | 546 |
| Example191 (A191) | 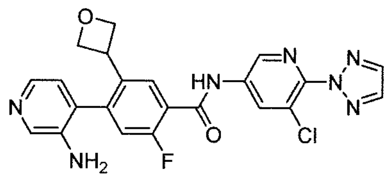 | 4-(3-aminopyridin-4-yl)-N-(5-chloro-6-(2H-1,2,3-triazol-2-yl)pyridin-3-yl)-2-fluoro-5-(oxetan-3-yl)benzamide | 466 |
| Example192 (A192) | 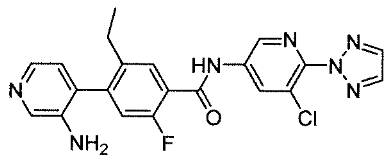 | 4-(3-aminopyridin-4-yl)-N-(5-chloro-6-(2H-1,2,3-triazol-2-yl)pyridin-3-yl)-5-ethyl-2-fluorobenzamide | 438 |
| Example193 (A193) | 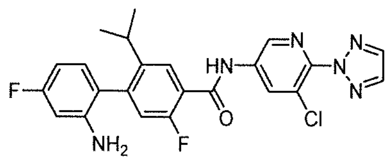 | 2'-amino-N-(5-chloro-6-(2H-l,2,3-triazol-2-yl)pyridin-3-yl)-4',5-difluoro-2-isopropyl-[1,1'-biphenyl]-4-carboxamide | 469 |
| Example194 (A194) | 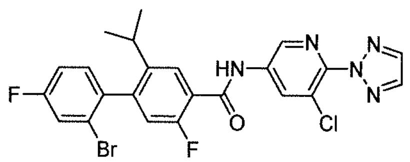 | 2'-bromo-N-(5-chloro-6-(2H-1,2,3-triazol-2-yl)pyridin-3-yl)-4',5-difluoro-2-isopropyl-[1,1'-biphenyl]-4-carboxamide | 532 |
| Example195 (A195) | 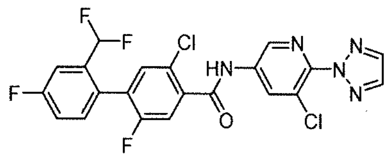 | 5-chloro-N-(5-chloro-6-(2H-1,2,3-triazol-2-yl)pyridin-3-yl)-2'-(difluoromethyl)-2,4'-difluoro-[1,1'-biphenyl]-4-carboxamide | 496 |
| Example196 (A196) | 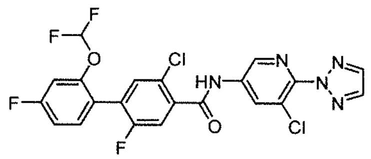 | 5-chloro-N-(5-chloro-6-(2H-1,2,3-triazol-2-yl)pyridin-3-yl)-2'-(difluoromethoxy)-2,4'-difluoro-[1,1'-biphenyl]-4-carboxamide | 512 |
| Example197 (A197) | 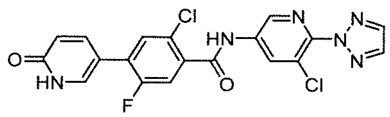 | 2-chloro-N-(5-chloro-6-(2H-1,2,3-triazol-2-yl)pyridin-3-yl)-5-fluoro-4-(6-oxo-1,6-dihydropyridin-3-yl)benzamide | 445 |
| Example198 (A198) | 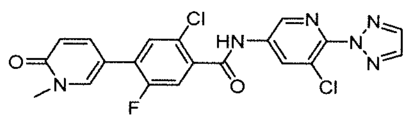 | 2-chloro-N-(5-chloro-6-(2H-1,2,3-triazol-2-yl)pyridin-3-yl)-5-fluoro-4-(1-methyl-6-oxo-1,6-dihydropyridin-3-yl)benzamide | 459 |

(continued)

| Example | Structure | Chemical Name | MS [M+H]+ |
|---|---|---|---|
| Example199 (A199) | 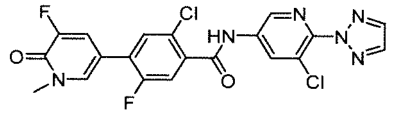 | 2-chloro-N-(5-chloro-6-(2H-1,2,3-triazol-2-yl)pyridin-3-yl)-5-fluoro-4-(5-fluoro-1-methyl-6-oxo-1,6-dihydropyridin-3-yl) benzamide | 477 |
| Example200 (A200) | 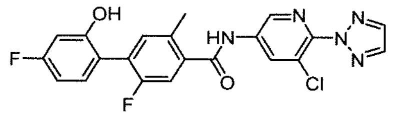 | N-(5-chloro-6-(2H-1,2,3-triazol-2-yl) pyridin-3-yl)-2,4'-difluoro-2'-hydroxy-5-methyl-[1,1'-biphenyl]-4-carboxamide | 442 |
| Example201 (A201) | 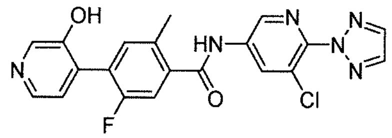 | N-(5-chloro-6-(2H-1,2,3-triazol-2-yl) pyridin-3-yl)-5-fluoro-4-(3-hydroxypyridin-4-yl)-2-methylbenzamide | 425 |
| Example202 (A202) | 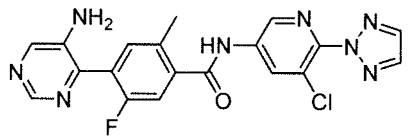 | 4-(5-aminopyrimidin-4-yl)-N-(5-chloro-6-(2H-1,2,3-triazol-2-yl)pyridin-3-yl)-5-fluoro-2-methylbenzamide | 425 |
| Example203 (A203) | 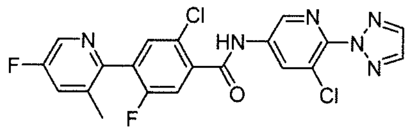 | 2-chloro-N-(5-chloro-6-(2H-1,2,3-triazol-2-yl)pyridin-3-yl)-5-fluoro-4-(5-fluoro-3-methylpyridin-2-yl)benzamide | 461 |
| Example204 (A204) | 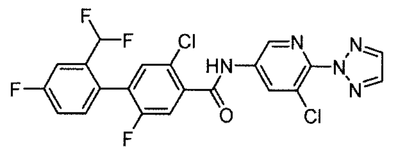 | 5-chloro-N-(5-chloro-6-(2H-l,2,3-triazol-2-yl)pyridin-3-yl)-2'-(difluoromethyl)-2,4'-difluoro-[1,1'-biphenyl]-4-carboxamide | 496 |
| Example205 (A205) | 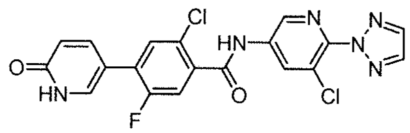 | 2-chloro-N-(5-chloro-6-(2H-l,2,3-triazol-2-yl)pyridin-3-yl)-5-fluoro-4-(6-oxo-1,6-dihydropyridin-3-yl)benzamide | 445 |
| Example206 (A206) | 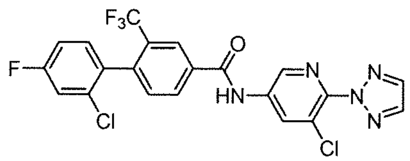 | N-(5-chloro-6-(2H-1,2,3-triazol-2-yl) pyridin-3-yl)-2,4'-difluoro-2'-hydroxy-5 -methyl-[1,1'-biphenyl]-4-carboxamide | 496 |
| Example207 (A207) | 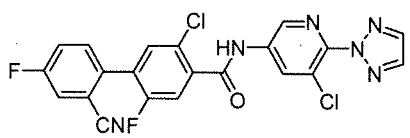 | 5-chloro-N-(5-chloro-6-(2H-1,2,3-triazol-2-yl)pyridin-3-yl)-2'-cyano-2,4'-difluoro-[1,1'-biphenyl]-4-carboxamide | 472 |
| Example208 (A208) | 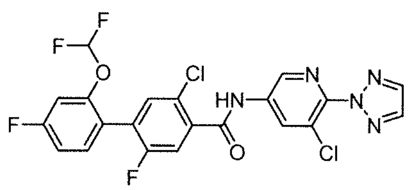 | 5-chloro-N-(5-chloro-6-(2H-1,2,3-triazol-2-yl)pyridin-3-yl)-2'-(difluoromethoxy)-2,4'-difluoro-[1,1'-biphenyl]-4-carboxamide | 512 |

(continued)

| Example | Structure | Chemical Name | MS [M+H]+ |
|---|---|---|---|
| Example209 (A209) | 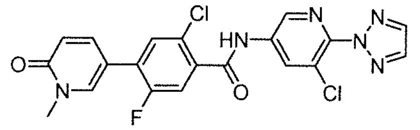 | 2-chloro-N-(5-chloro-6-(2H-1,2,3-triazol-2-yl)pyridin-3-yl)-5-fluoro-4-(1-methyl-6-oxo-1,6-dihydropyridin-3-yl)benzamide | 459 |
| Example210 (A210) | 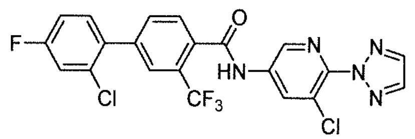 | 2'-chloro-N-(5-chloro-6-(2H-1,2,3-triazol-2-yl)pyridin-3-yl)-4'-fluoro-3-(trifluoromethyl)-[1,1'-biphenyl]-4-carboxamide | 496 |
| Example211 (A211) | 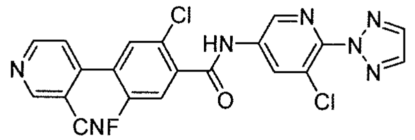 | 2-chloro-N-(5-chloro-6-(2H-1,2,3-triazol-2-yl)pyridin-3-yl)-4-(3-cyanopyridin-4-yl)-5-fluorobenzamide | 454 |
| Example212 (A212) | 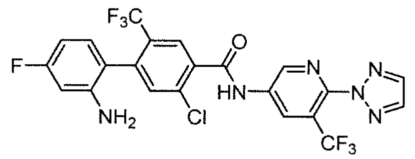 | N-(6-(2H-1,2,3-triazol-2-yl)-5-(trifluoromethyl)pyridin-3-yl)-2'-amino-5-chloro-4'-fluoro-2-(trifluoromethyl)-[1,1'-biphenyl]-4-carboxamide | 545 |
| Example213 (A213) | 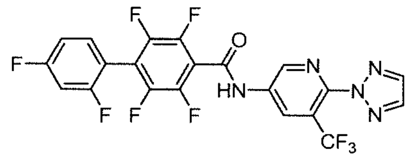 | N-(6-(2H-1,2,3-triazol-2-yl)-5-(trifluoromethyl)pyridin-3-yl)-2,2',3,4',5,6-hexafluoro-[1,1'-biphenyl]-4-carboxamide | 518 |
| Example214 (A214) | 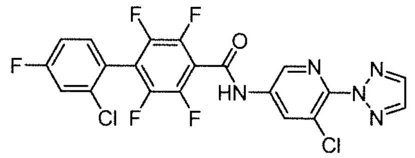 | 2'-chloro-N-(5-chloro-6-(2H-1,2,3-triazol-2-yl)pyridin-3-yl)-2,3,4',5,6-pentafluoro-[1,1'-biphenyl]-4-carboxamide | 500 |
| Example215 (A215) | 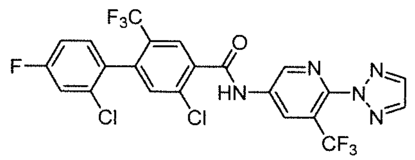 | N-(6-(2H-l,2,3-triazol-2-yl)-5-(trifluoromethyl)pyridin-3-yl)-2',5-dichloro-4'-fluoro-2-(trifluoromethyl)-[1,1'-biphenyl]-4-carboxamide | 564 |
| Example216 (A216) | 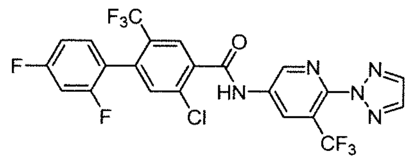 | N(6-(2H-1,2,3-triazol-2-yl)-5-(trifluoromethyl)pyridin-3-yl)-5-chloro-2',4'-difluoro-2-(trifluoromethyl)-[1,1'-1 biphenyl]-4-carboxamide | 548 |
| Example217 (A217) | 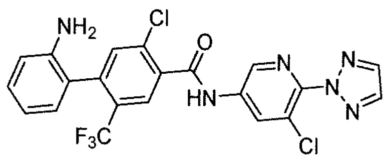 | 2'-amino-5-chloro-N-(5-chloro-6-(2H-1,2,3-triazol-2-yl)pyridin-3-yl)-2-(trifluoromethyl)-[1,1'-biphenyl]-4-carboxamide | 493 |

(continued)

| Example | Structure | Chemical Name | MS [M+H]+ |
|---|---|---|---|
| Example218 (A218) | 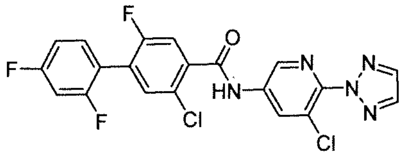 | 5-chloro-N-(5-chloro-6-(2H-l,2,3-triazol-2-yl)pyridin-3-yl)-2,2',4'-trifluoro-[1,1'-biphenyl]-4-carboxamide | 464 |
| Example219 (A219) | 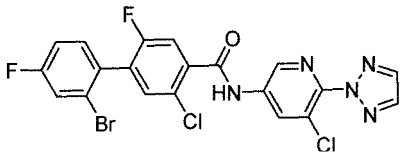 | 2'-bromo-5-chloro-N-(5-chloro-6-(2H-1,2,3-triazol-2-yl)pyridin-3-yl)-2,4'-difluoro-[1,1'-biphenyl]-4-carboxamide | 524 |
| Example221 (A221) | 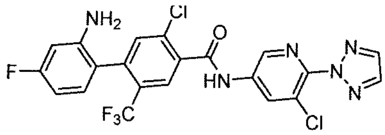 | 2'-amino-5-chloro-N-(5-chloro-6-(2H-1,2,3-triazol-2-yl)pyridin-3-yl)-2-cyclopropyl-4'-fluoro-[1,1'-biphenl-4-carboxamide | 483 |
| Example222 (A222) | 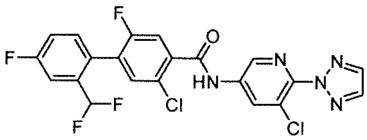 | 5-chloro-N-(5-chloro-6-(2H-1,2,3-triazol-2-yl)pyridin-3-yl)-2'-(difluoromethyl)-2,4'-difluoro-[1,1'-biphenyl]-4-carboxamide | 496 |
| Example223 (A223) | 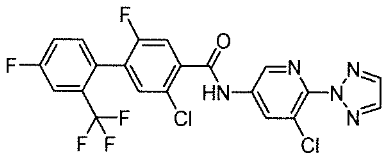 | 5-chloro-N-(5-chloro-6-(2H-1,2,3-triazol-2-yl)pyridin-3-yl)-2,4'-difluoro-2'-(trifluoromethyl)-[1,1'-biphenyl)-4-carboxamide | 514 |
| Example224 (A224) | 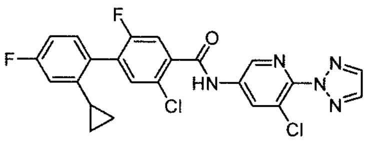 | 5-chloro-N-(5-chloro-6-(2H-1,2,3-triazol-2-yl)pyridin-3-yl)-2'-cyclopropyl-2,4'-difluoro-[1,1'-biphenyl]-4-carboxamide | 486 |
| Example225 (A225) | 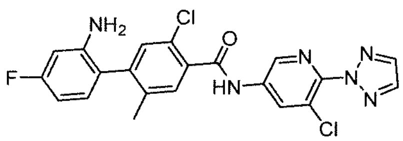 | 2'-amino-5-chloro-N-(5-chloro-6-(2H-1,2,3-triazol-2-yl)pyridin-3-yl)-4'-fluoro-2-methyl-[1,1'-biphenyl]-4-carboxamide | 457 |
| Example226 (A226) | 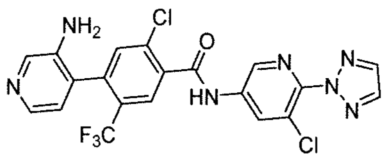 | 4-(3-aminopyridin-4-yl)-2-chloro-N-(5-chloro-6-(2H-1,2,3-triazol-2-yl)pyridin-3-yl)-5-(trifluoromethyl)benzamide | 494 |
| Example227 (A227) | 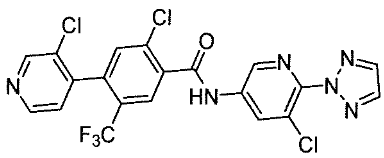 | 2-chloro-N-(5-chloro-6-(2H-1,2,3-triazol-2-yl)pyridin-3-yl)-4-(3-chloropyridin-4-yl)-5-(trifluoromethyl)benzamide | 513 |

(continued)

| Example | Structure | Chemical Name | MS [M+H]+ |
|---|---|---|---|
| Example228 (A228) | 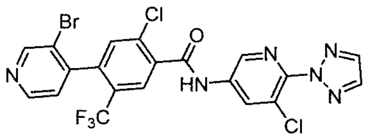 | 4-(3-bromopyridin-4-yl)-2-chloro-N-(5-chloro-6-(2H-1,2,3-triazol-2-yl)pyridin-3-yl)-5-(trifluoromethyl)benzamide | 557 |
| Example229 (A229) | 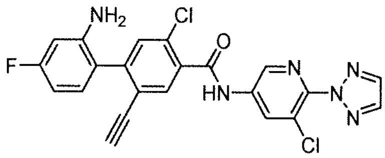 | 2'-amino-5-chloro-N-(5-chloro-6-(2H-1,2,3-triazol-2-yl)pyridin-3-yl)-2-ethynyl-4'-fluoro-[1,1'-biphenyl]-4-carboxamide | 467 |
| Example230 (A230) | 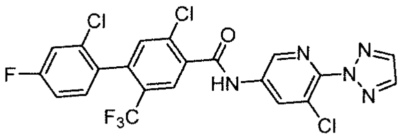 | 2',5-dichloro-N-(5-chloro-6-(2H-1,2,3-triazol-2-yl)pyridin-3-yl)-4'-fluoro-2-(trifluoromethyl)-[1,1'-biphenyl]-4-carboxamide | 530 |
| Example231 (A231) | 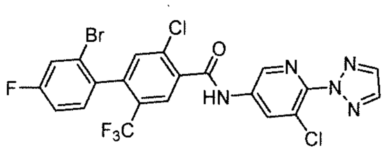 | 2'-bromo-5-chloro-N-(5-chloro-6-(2H-1,2,3-triazol-2-yl)pyridin-3-yl)-4'-fluoro-2-(trifluoromethyl)-[1,1'-biphenyl]-4-carboxamide | 574 |
| Example232 (A232) | 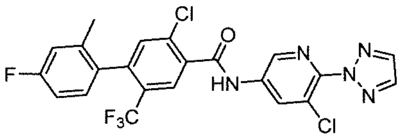 | 5-chloro-N-(5-chloro-6-(2H-1,2,3-triazol-2-yl)pyridin-3-yl)-4'-fluoro-2'-methyl-2-(trifluoromethyl)-[1,1'-biphenyl]-4-carboxamide | 510 |
| Example233 (A233) | 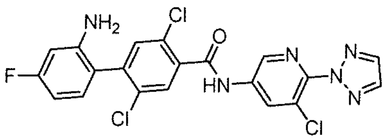 | 2'-amino-2,5-dichloro-N-(5-chloro-6-(2H-1,2,3-triazol-2-yl)pyridin-3-yl)-4'-fluoro-[1,1'-biphenyl]-4-carboxamide | 477 |
| Example234 (A234) | 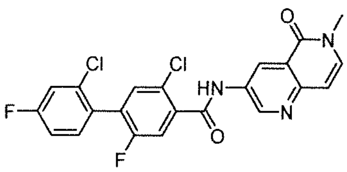 | 2',5-dichloro-2,4'-difluoro-N-(6-methyl-5-oxo-5,6-dihydro-1,6-naphthyridin-3-yl)-[l, l'-biphenyl]-4-carboxamide | 460 |
| Example235 (A235) | 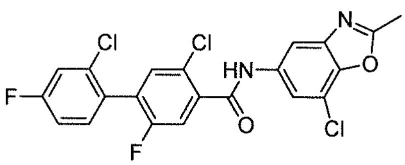 | 2',5-dichloro-N-(7-chloro-2-methylbenzo [d]oxazol-5-yl)-2,4'-difluoro-[1,1'-biphenyl]-4-carboxamide | 467 |
| Example236 (A236) | 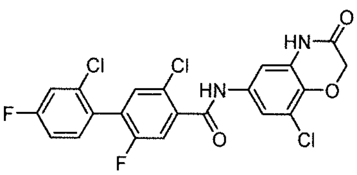 | 2',5-dichloro-N-(8-chloro-3-oxo-3,4-dihydro-2H-benzo[b][1,4]oxazin-6-yl)-2,4'-difluoro-[1,1'-biphenyl]-4-carboxamide | 483 |

(continued)

| Example | Structure | Chemical Name | MS [M+H]+ |
|---|---|---|---|
| Example237 (A237) | 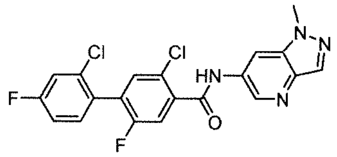 | 2',5-dichloro-2,4'-difluoro-N-(1-methyl-1H-pyrazolo[4,3-b]pyridin-6-yl)-[1,1'-biphenyl]-4-carboxamide | 433 |
| Example238 (A238) | 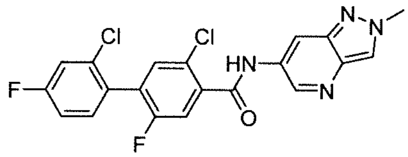 | 2',5-dichloro-2,4'-difluoro-N-(2-methyl-2H-pyrazolo[4,3-b]pyridin-6-yl)-[1,1'-biphenyl]-4-carboxamide | 433 |
| Example239 (A239) | 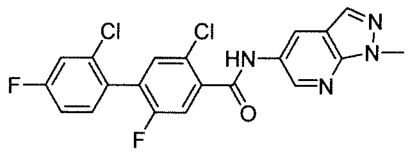 | 2',5-dichloro-2,4'-difluoro-N-(1-methyl-1H-pyrazolo[3,4-b]pyridin-5-yl)-[1,1'-biphenyl]-4-carboxamide | 433 |
| Example240 (A240) | 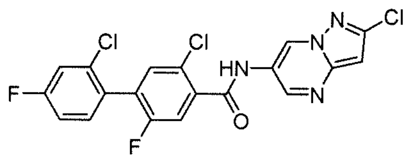 | 2',5-dichloro-N-(2-chloropyrazolo[1,5-a] pyrimidin-6-yl)-2,4'-difluoro-[1,1'-biphenyl]-4-carboxamide | 453 |
| Example241 (A241) | 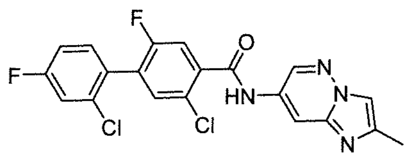 | 2',5-dichloro-2,4'-difluoro-N-(2-methylimidazo[1,2-b]pyridazin-7-yl)-[1,1'-biphenyl]-4-carboxamide | 433 |
| Example242 (A242) | 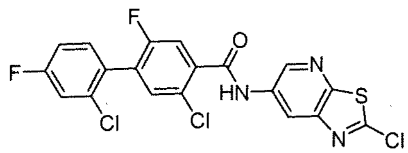 | 2',5-dichloro-N-(2-chlorothiazolo[5,4-b] pyridin-6-yl)-2,4'-difluoro-[1,1'-biphenyl]-4-carboxamide | 470 |
| Example243 (A243) | 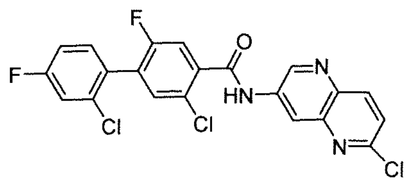 | 2',5-dichloro-N-(6-chloro-1,5-naphthyridin-3-yl)-2,4'-difluoro-[1,1'-biphenyl]-4-carboxamide | 464 |
| Example244 (A244) | 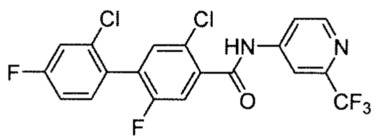 | 2',5-dichloro-2,4'-difluoro-N-(2-(trifluoromethyl)pyridin-4-yl)-[1,1'-biphenyl]-4-carboxamide | 447 |
| Example245 (A245) | 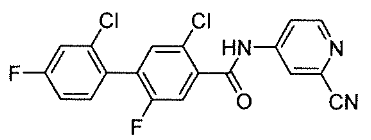 | 2',5-dichloro-N-(2-cyanopyridin-4-yl)-2,4'-difluoro-[1,1'-biphenyl]-4-carboxamide | 404 |

(continued)

| Example | Structure | Chemical Name | MS $[M+H]^+$ |
|---|---|---|---|
| Example246 (A246) | 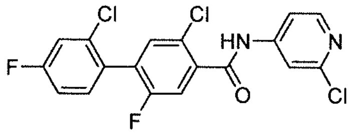 | 2',5-dichloro-N-(2-chloropyridin-4-yl)-2,4'-difluoro-[1,1'-biphenyl]-4-carboxamide | 413 |
| Example247 (A247) | 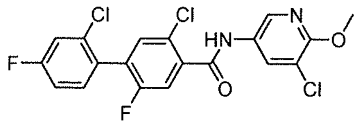 | 2',5-dichloro-N-(5-chloro-6-methoxypyridin-3-yl)-2,4'-difluoro-[1,1'-biphenyl]-4-carboxamide | 443 |
| Example248 (A248) | 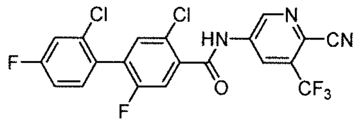 | 2',5-dichloro-N-(6-cyano-5-(trifluoromethyl)pyridin-3-yl)-2,4'-difluoro-[1,1'-biphenyl]-4-carboxamide | 472 |
| Example249 (A249) | 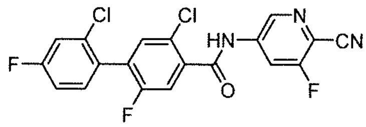 | 2',5-dichloro-N-(6-cyano-5-fluoropyridin-3-yl)-2,4'-difluoro-[1,1'-biphenyl]-4-carboxamide | 422 |
| Example250 (A250) | 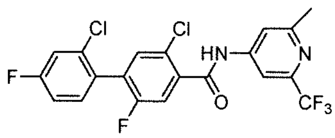 | 2',5-dichloro-2,4'-difluoro-N-(2-methyl-6-(trifluoromethyl)pyridin-4-yl)-[1,1'-biphenyl]-4-carboxamide | 461 |
| Example251 (A251) | 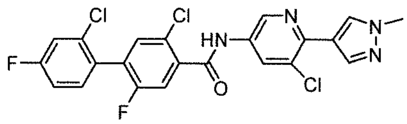 | 2',5-dichloro-N-(5-chloro-6-(1-methyl-1H-pyrazol-4-yl)pyridin-3-yl)-2,4'-difluoro-[1,1'-biphenyl]-4-carboxamide | 493 |
| Example252 (A252) | 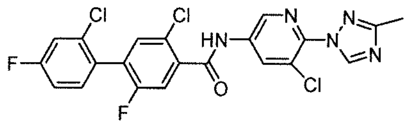 | 2',5-dichloro-N-(5-chloro-6-(3-methyl-1H-1,2,4-triazol-1-yl)pyridin-3-yl)-2,4'-difluoro-[1,1'-biphenyl]-4-carboxamide | 494 |
| Example253 (A253) | 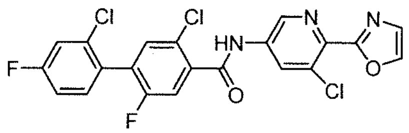 | 2',5-dichloro-N-(5-chloro-6-(oxazol-2-yl) pyridin-3-yl)-2,4'-difluoro-[1,1'-biphenyl]-4-carboxamide | 480 |
| Example254 (A254) | 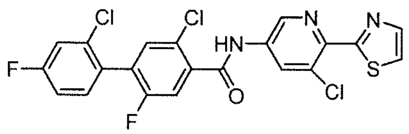 | 2',5-dichloro-N-(5-chloro-6-(thiazol-2-yl) pyridin-3-yl)-2,4'-difluoro-[1,1'-biphenyl] -4-carboxamide | 496 |
| Example255 (A255) | 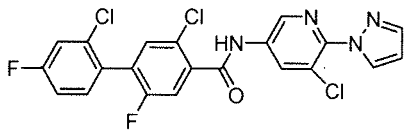 | 2',5-dichloro-N-(5-chloro-6-(1H-pyrazol-1-yl)pyridin-3-yl)-2,4'-difluoro-[1,1'-biphenyl]-4-carboxamide | 479 |
| Example256 (A256) | 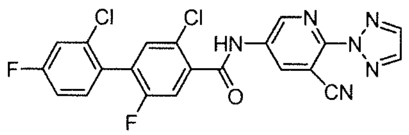 | 2',5-dichloro-N-(5-cyano-6-(2H-1,2,3-triazol-2-yl)pyridin-3-yl)-2,4'-difluoro-[1,1'-biphenyl] -4-carboxamide | 471 |

(continued)

| Example | Structure | Chemical Name | MS $[M+H]^+$ |
|---|---|---|---|
| Example257 (A257) | 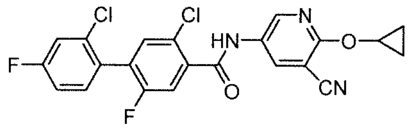 | 2',5-dichloro-N-(5-cyano-6-cyclopropoxypyridin-3-yl)-2,4'-difluoro-[1,1'-biphenyl]-4-carboxamide | 460 |
| Example258 (A258) | 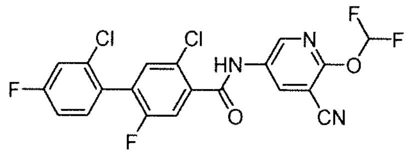 | 2',5-dichloro-N-(5-cyano-6-(difluoromethoxy)pyridin-3-yl)-2,4'-difluoro-[1,1'-biphenyl]-4-carboxamide | 470 |
| Example259 (A259) | 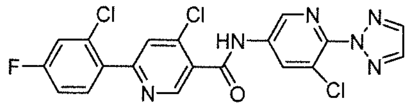 | 4-chloro-6-(2-chloro-4-fluorophenyl)-N-(5-chloro-6-(2H-l,2,3-triazol-2-yl)pyridin-3-yl) nicotinamide | 463 |
| Example260 (A260) | 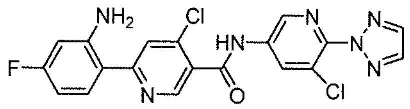 | 6-(2-amino-4-fluorophenyl)-4-chloro-N-(5-chloro-6-(2H-1,2,3-triazol-2-yl)pyridin-3-yl)nicotinamide | 444 |
| Example261 (A261) | 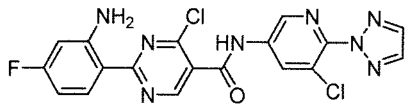 | 2-(2-amino-4-fluorophenyl)-4-chloro-N-(5-chloro-6-(2H-1,2,3-triazol-2-yl)pyridin-3-yl)pyrimidine-5-carboxamide | 445 |
| Example262 (A262) | 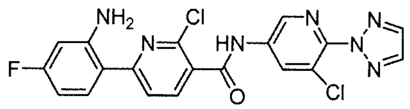 | 6-(2-amino-4-fluorophenyl)-2-chloro-N-(5-chloro-6-(2H-1,2,3-triazol-2-yl)pyridin-3-yl)nicotinamide | 444 |
| Example263 (A263) | 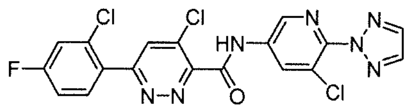 | 4-chloro-6-(2-chloro-4-fluorophenyl)-N-(5-chloro-6-(2H-1,2,3-triazol-2-yl)pyridin-3-yl)pyridazine-3-carboxamide | 464 |
| Example264 (A264) | 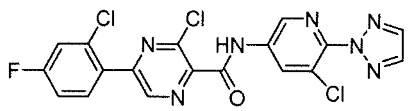 | 3-chloro-5-(2-chloro-4-fluorophenyl)-N-(5-chloro-6-(2H-1,2,3 -triazol-2-yl)pyridin-3-yl)pyrazine-2-carboxamide | 464 |
| Example265 (A265) | 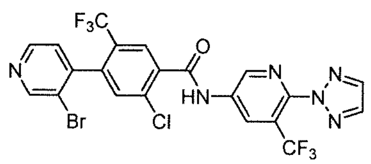 | N-(6-(2H-1,2,3-triazol-2-yl)-5-(trifluoromethyl)pyridin-3-yl)-4-(3-bromopyridin-4-yl)-2-chloro-5-(trifluoromethyl)benzamide | 591 |
| Example266 (A266) | 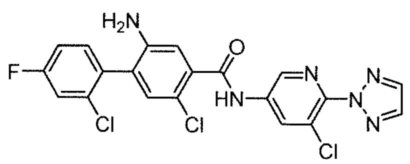 | 2-amino-2',5-dichloro-N-(5-chloro-6-(2H-1,2,3-triazol-2-yl)pyridin-3-yl)-4'-fluoro-[1,1'-biphenyl]-4-carboxamide | 477 |

(continued)

| Example | Structure | Chemical Name | MS [M+H]+ |
|---|---|---|---|
| Example267 (A267) | 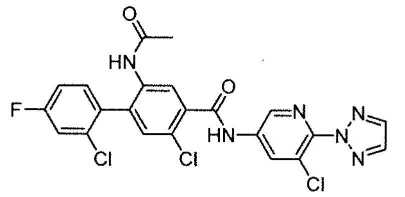 | 2-acetamido-2',5-dichloro-N-(5-chloro-6-(2H-1,2,3-triazol-2-yl)pyridin-3-yl)-4'-fluoro-[1,1'-biphenyl]-4-carboxamide | 519 |
| Example268 (A268) | 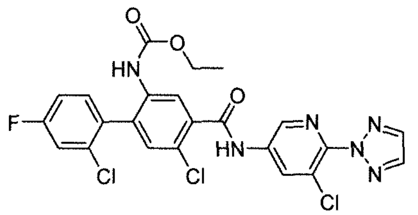 | ethyl(2',5-dichloro-4-((5-chloro-6-(2H-l, 2,3-triazol-2-yl)pyridin-3-yl)aminoformyl)-4'-fluoro-[1,1'-biphenyl]-2-yl) aminoformate | 549 |
| Example269 (A269) | 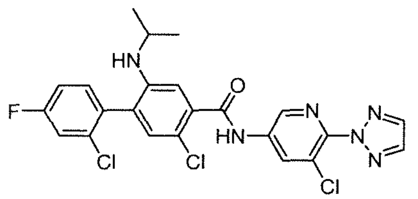 | 2',5-dichloro-N-(5-chloro-6-(2H-1,2,3-triazol-2-yl)pyridin-3-yl)-4'-fluoro-2-(isopropylamino)-[1,1'-biphenyl]-4-carboxamide | 519 |
| Example270 (A270) | 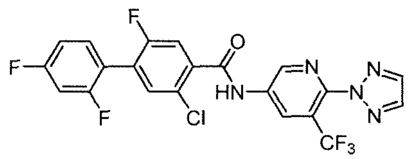 | N-(6-(2H-1,2,3-triazol-2-yl)-5-(trifluoromethyl)pyridin-3-yl)-5-chloro-2,2',4'-trifluoro-[1,1'-biphenyl]-4-carboxamide | 498 |
| Example271 (A271) | 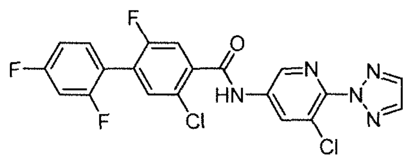 | 5-chloro-N-(5-chloro-6-(2H-l,2,3-triazol-2-yl)pyridin-3-yl)-2,2',4'-trifluoro-[1,1'-biphenyl]-4-carboxamide | 464 |
| Example272 (A272) | 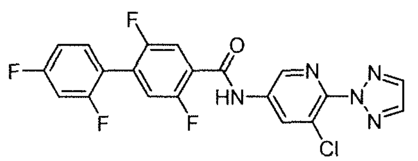 | N-(5-chloro-6-(2H-1,2,3-triazol-2-yl) pyridin-3-yl)-2,2',4',5-tetrafluoro-[1,1'-biphenyl]-4-carboxamide | 448 |
| Example273 (A273) | 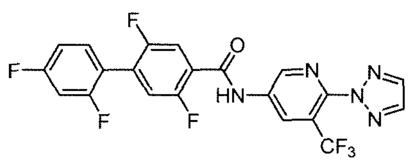 | N-(6-(2H-1,2,3-triazol-2-yl)-5-(trifluoromethyl)pyridin-3-yl)-2,2',4',5-tetrafluoro-[1,1'-biphenyl]-4-carboxamide | 482 |
| Example275 (A275) | 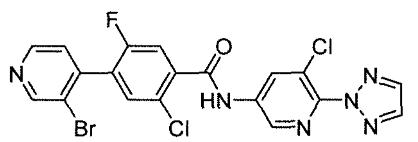 | 4-(3-bromopyridin-4-yl)-2-chloro-N-(5-chloro-6-(2H-1,2,3-triazol-2-yl)pyridin-3-yl)-5-fluorobenzamide | 506 |

A121:

$^1H$ NMR (500 MHz, DMSO-$d_6$) δ 11.44 (s, 1H), 8.85 (d, $J$ = 2.3 Hz, 1H), 8.67 (d, $J$ = 2.3 Hz, 1H), 8.19 (s, 2H), 7.77 (d, $J$ = 9.2 Hz, 1H), 7.60 (d, $J$ = 6.4 Hz, 1H), 7.05 (dd, $J$ = 8.4, 6.6 Hz, 1H), 6.66 (dd, $J$ = 11.5, 2.7 Hz, 1H), 6.59 - 6.44 (m, 1H). A122:
$^1H$ NMR (500 MHz, DMSO-$d_6$) δ 11.50 (s, 1H), 9.12 (d, $J$ = 2.4 Hz, 1H), 8.90 (d, $J$ = 2.4 Hz, 1H), 8.21 (s, 2H), 7.78 (d, $J$ = 9.2 Hz, 1H), 7.59 (d, $J$ = 6.5 Hz, 1H), 7.00 (dd, $J$ = 8.5, 6.7 Hz, 1H), 6.56 (dd, $J$ = 11.7, 2.6 Hz, 1H), 6.47 - 6.34 (m, 1H). A123:
$^1H$ NMR (500 MHz, DMSO-$d_6$) δ 11.34 (s, 1H), 9.17 (d, $J$ = 2.4 Hz, 1H), 8.90 (d, $J$ = 2.4 Hz, 1H), 8.21 (s, 2H), 8.00 (d, $J$ = 6.5 Hz, 1H), 7.43 (d, $J$ = 10.2 Hz, 1H), 6.93 (dd, $J$ = 8.4, 6.7 Hz, 1H), 6.54 (dd, $J$ = 11.7, 2.6 Hz, 1H), 6.42 (td, $J$ = 8.5, 2.6 Hz, 1H), 5.21 (s, 2H).

A217:

$^1H$ NMR (500 MHz, DMSO-$d_6$) δ 11.42 (s, 1H), 8.82 (d, $J$ = 2.3 Hz, 1H), 8.66 (d, $J$ = 2.3 Hz, 1H), 8.20 (d, $J$ = 7.0 Hz, 3H), 7.58 (s, 1H), 7.12 (td, $J$ = 8.3, 7.9, 1.6 Hz, 1H), 6.84 (d, $J$ = 7.5 Hz, 1H), 6.76 (dd, $J$ = 8.2, 1.1 Hz, 1H), 6.61 (td, $J$ = 7.4, 1.1 Hz, 1H), 4.78 (s, 2H).

A116:

$^1H$ NMR (500 MHz, DMSO-$d_6$) δ 11.23 (s, 1H), 8.85 (d, $J$ = 2.3 Hz, 1H), 8.68 (d, $J$ = 2.3 Hz, 1H), 8.50 (s, 0H), 8.18 (s, 2H), 7.74 - 7.53 (m, 2H), 7.54 - 7.37 (m, 2H), 7.27 (td, $J$ = 8.4, 2.5 Hz, 1H), 2.45 (s, 3H).

A275:

$^1H$ NMR (500 MHz, DMSO-$d_6$) δ 11.53 (s, 1H), 8.95 (s, 1H), 8.81 (s, 1H), 8.68 (d, $J$ = 28.1 Hz, 2H), 8.19 (s, 2H), 8.03 - 7.67 (m, 2H), 7.58 (s, 1H).

Example281: Synthesis of Compound A281

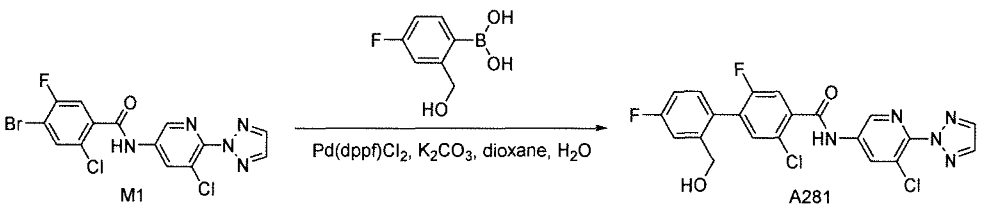


Into a 50-mL round-bottom flask, was placed 4-bromo-2-chloro-N-(5-chloro-6-(2H-1,2,3-triazol-2-yl)pyridin-3-yl)-5-fluorobenzamide (M1) (43mg), (4-Fluoro-2-(hydroxymethyl)phenyl)boronic acid (25 mg), Pd(dppf)$Cl_2$ (3mg), $K_2CO_3$(27mg), dioxane(1mL) and $H_2O$ (0.2mL) . The resulting mixture was stirred at 90 °C under nitrogen for 8 hours. The reaction was diluted with EA (5 mL), washed with brine. The organic layer was concentrated and purified by flash column chromatography(DCM/MeOH=40/1) to give compound A281(18mg) as white solid. LC-MS (ES$^+$): $m/z$ 476[M+H]$^+$

Example282: Synthesis of Compound A282

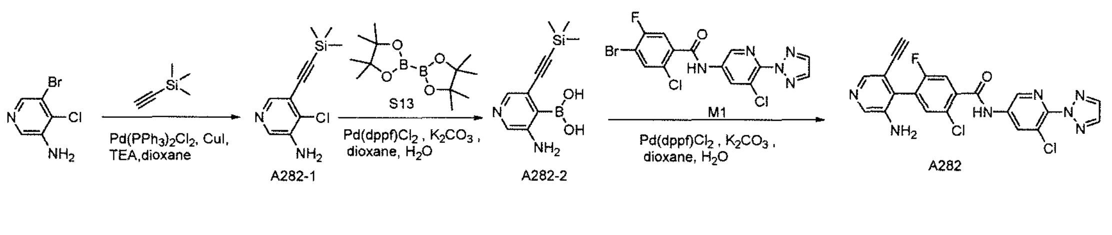

Step1: Synthesis of Compound A282-1

Into a 50-mL round-bottom flask, was placed 5-bromo-4-chloropyridin-3-amine (413mg), ethynyltrimethylsilane (300 mg), $Pd(PPh_3)_2Cl_2$ (3mg), CuI (5mg), TEA (1mL) and dioxane (10mL) .The reaction mixture was stirred at 90°C under nitrogen for 8 hours. The mixture was diluted with EA (100 mL), washed with sbrine. The organic phase was concentrated and purified by flash column chromatography (DCM/MeOH=40/1) over silica gel to afford compound A282-1(216mg) as colorless oil. LC-MS ($ES^+$): *m/z* 225$[M+H]^+$

Step2: Synthesis of Compound A282-2

The mixture of A282-1 (216mg), 4,4,4',4',5,5,5',5'-octamethyl-2,2'-bis(1,3,2-dioxaborolane alkane) (500mg), $Pd(dppf)Cl_2$ (30mg), $K_2CO_3$ (270mg), dioxane (10mL) and $H_2O$ (2mL) was stirred at 100 °C under nitrogen for 8 hours in a 50-mL round-bottom flask . Removing of the solvent and purifying by column chromatography (DCM/MeOH=40/1) to afford compound A282-2(88mg) as a red oil. LC-MS ($ES^+$): *m/z* 235$[M+H]^+$

Step3: Synthesis of Compound A282

A mixture of 4-bromo-2-chloro-N-(5-chloro-6-(2H-1,2,3-triazol-2-yl)pyridin-3-yl)-5-fluorobenzamide (M1) (43mg), A282-2 (38mg), $Pd(dppf)Cl_2$ (3mg), $K_2CO_3$ (27mg), dioxane (1mL) and $H_2O$ (0.2mL) was stirred at 90 °C for 8 hours under nitrogen. The mixture was cooled to room temperature,diluted with EA (5 mL), and washed with brine. The organic phase was concentrated and purified by flash column chromatography (DCM/MeOH=40/1) to give compound A282(12mg) as yellow solid. LC-MS ($ES^+$): *m/z* 468$[M+H]^+$

Example286: Synthesis of Compound A286

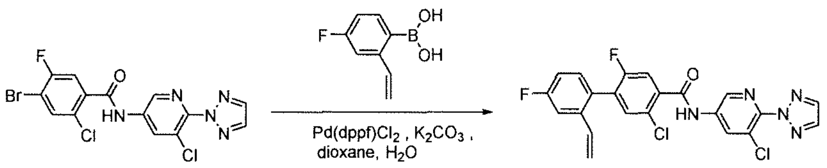


M1

A286

The mixture of 4-bromo-2-chloro-N-(5-chloro-6-(2H-1,2,3-triazol-2-yl)pyridin-3-yl)-5-fluorobenzamide (M1) (43mg), (4-fluoro-2-vinylphenyl)boronic acid (24mg), $Pd(dppf)Cl_2$ (3mg), $K_2CO_3$ (27mg), dioxane (1mL) and $H_2O$ (0.2mL) was stirred at 90 °C for 8 hoursunder nitrogen. The mixture was cooled to room temperature, diluted with EA (5 mL), and washed with brine. The organic phase was concentrated and purified by flash column chromatography (DCM/MeOH=40/1) to give compound A286(21mg) as white solid. LC-MS ($ES^+$): *m/z* 472$[M+H]^+$

Example296: Synthesis of Compound A296

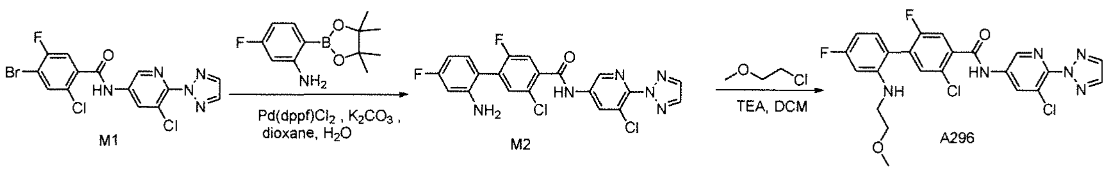


M1

M2

A296

Step1: Synthesis of Compound M2

The mixture of 4-bromo-2-chloro-N-(5-chloro-6-(2H-1,2,3-triazol-2-yl)pyridin-3-yl)-5-fluorobenzamide (M1) (43 mg), 5-fluoro-2-(4,4,5,5-tetramethyl-1,3,2-dioxaborolan-2-yl)aniline (36 mg), $Pd(dppf)Cl_2$ (3mg), $K_2CO_3$(27mg), dioxane (1mL) and $H_2O$(0.2mL) was stirred at 90°C for 8 hours under nitrogen. The mixture was cooled to room temperature, diluted with EA (5 mL) and washed with sbrine. The organic phase was concentrated and purified by flash column

chromatography (DCM/MeOH=40/1) to give compound M2(33mg) as white solid. LC-MS ($ES^+$): *m/z* 461[M+H]$^+$

Step2: Synthesis of Compound A296

To a solution of M2 (23mg), in DCM (1mL)was added TEA (5mg) and 1-chloro-2-methoxyethane (6mg). The reaction mixture was was stirred at room temperature for 2 hours and diluted with EA (5 mL), washed with brine. The organic phase was concentrated and purified by flash column chromatography (DCM/MeOH=40/1) to give compound A296(13mg) as white solid. LC-MS ($ES^+$): *m/z* 519 [M+H]$^+$

Example298: Synthesis of Compound A298

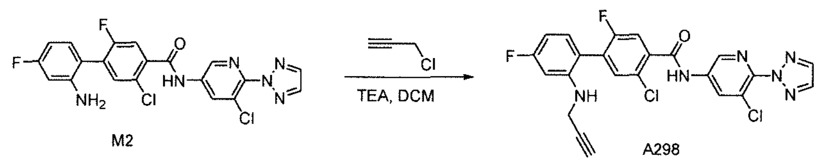


To a solution of M2 (23mg) in DCM (1mL) was added TEA (5mg) and 3-Chloropropyl-1-yne (5mg). The reaction mixture was was stirred at room temperature for 2 hours and diluted with EA (5 mL), washed with brine. The organic phase was concentrated and purified by flash column chromatography (DCM/MeOH=40/1) to give compound A298 (18mg), as yellow solid. LC-MS ($ES^+$): *m/z* 499 [M+H]$^+$ Example300: Synthesis of Compound A300

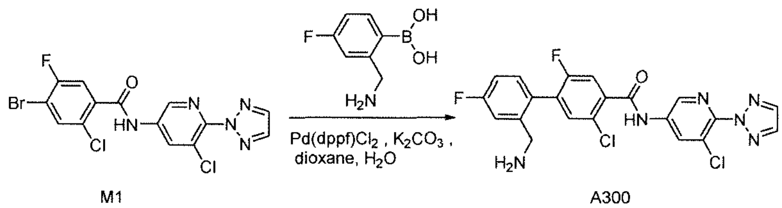


The mixture of M1 (43mg), (2-(aminomethyl)-4-fluorophenyl)boronic acid (26mg), Pd(dppf)$Cl_2$ (3mg), $K_2CO_3$ (27mg), dioxane (1mL) and $H_2O$ (0.2mL) was stirred 90°C for 8 hours under nitrogen. The mixture was cooled to room temperature, diluted with EA (5 mL) and washed with brine. The organic phase was concentrated and purified by flash column chromatography (DCM/MeOH=40/1) to give compound A300 (12mg) as yellow solid. LC-MS ($ES^+$): *m/z* 475[M+H]$^+$

Example302: Synthesis of Compound A302

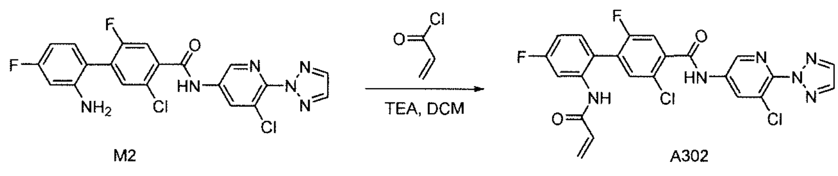


To a solution of M2 (23mg) in DCM (1mL) was addedTEA (5mg) and acryloyl chloride (5mg) at 0 °C. The reaction mixture was stirred at room temperature for 2 hours and diluted with EA (5 mL), washed with sbrine. The organic phase was concentrated and purified by flash column chromatography (DCM/MeOH=40/1) to give compound A298(18mg) as white solid. LC-MS ($ES^+$): *m/z* 515[M+H]$^+$

Example308: Synthesis of Compound A308

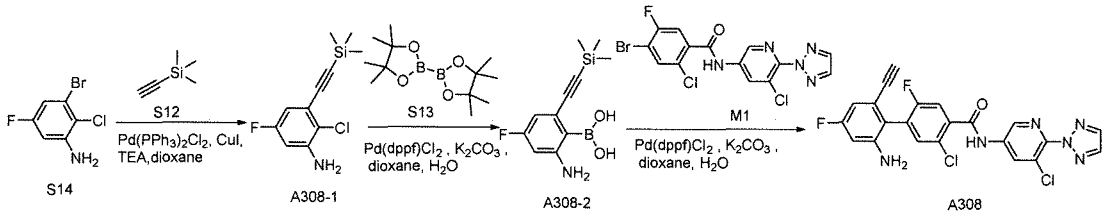


S14 A308-1 A308-2 A308

Step 1: Synthesis of Compound A308-1

The mixture of S14 (443mg), S12 (300mg), $Pd(PPh_3)_2Cl_2$ (3mg), CuI (5mg), TEA (1mL) and dioxane (10mL) was stirred at 90°C for 8 hours under nitrogen. The mixture was cooled to room temperature, and diluted with EA (100 mL) and washed with brine. The organic phase was concentrated and purified by flash column chromatography (DCM/MeOH=40/1) to give compound A308-1(240mg) as colorless oil. LC-MS ($ES^+$): *m/z* 242$[M+H]^+$

Step2: Synthesis of Compound A308-2

The mixture of A308-1 (240mg), S13 (500mg), $Pd(dppf)Cl_2$ (30mg), $K_2CO_3$ (270mg), dioxane (10mL) and $H_2O$ (2mL) was stirred at 110°C for 8 hours under nitrogen. The mixture was cooled to room temperature, diluted with EA (100 mL) and washed withbrine. The organic phase was concentrated and purified by flash column chromatography(DCM/MeOH=40/1) to give compound A308-2 (101mg) as yellow oil. LC-MS ($ES^+$): *m/z* 252$[M+H]^+$

Step3: Synthesis of Compound A308

The mixture of M1 (43mg), A308-2 (37mg), $Pd(dppf)Cl_2$ (3mg), $K_2CO_3$ (27mg), dioxane (1mL) and $H_2O$ (0.2mL) was stirred at 90°C for 8 hours under nitrogen . The mixture was cooled to room temperature,diluted with EA (5 mL) and washed with brine. The organic phase was concentrated and purified by flash column chromatography (DCM/MeOH=40/1) to give compound A308(19mg) as yellow solid. LC-MS ($ES^+$): *m/z* 485 $[M+H]^+$

Example310: Synthesis of Compound A310

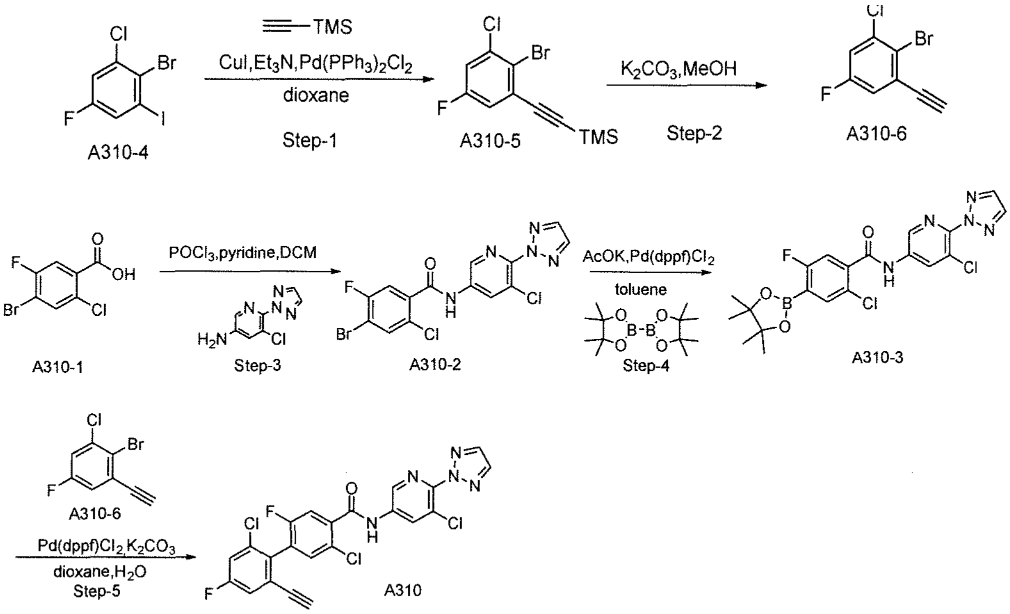


A310-4 A310-5 A310-6 A310-1 A310-2 A310-3 A310-6 A310

Step1: Synthesis of Compound A310-5

To a solution of A310-4 (3.33 g, 10.0 mmol)in dioxane (35 mL) was added CuI (0.38 g, 2.0 mmol), EtsN (3.03 g, 30.0 mmol), $Pd(PPh_3)_2Cl_2$ (0.73 g, 1 mmol)and ethyltrimethylsilane (2.04 g, 20.0 mmol). The reaction mixture was

stirred at room temperature under nitrogen for 5 hours and quenched by water, extracted with dichloromethane (250 mLxa). The organic layer was washed by brine, dried over sodium sulfate, filtrated and evaporated in vacuo. The residue was purified by column chromatography to give compound A310-5 (2.5 g) as yellow solid. LC-MS ($ES^+$): *m/z* 305 $[M+H]^+$

Step2: Synthesis of Compound A310-6

To a solution of A310-5 (2.5 g, 8.3 mmol)in methanol (30 mL) was added $K_2CO_3$ (3.45 g, 24.9 mmol). The reaction mixture was stirred at room temperature under nitrogen for 2 hours. Removing the solvent and purifying by column chromatography to give compound A310-6 (1.7 g) as Off-white solid. LC-MS ($ES^+$): *m/z* 233$[M+H]^+$

Step3: Synthesis of Compound A310-2

To a solution of A310-1 (2.52 g, 10 mmol)in DCM (30 mL) was added pyridine (2.37 g, 30 mmol) and 5-chloro-6-(2H-1,2,3-triazol-2-yl)pyridin-3-amine (1.95 g, 10 mmol). Then $POCl_3$ (1.68 g, 11 mmol) was added dropwise. The reaction was stirred at room temperature under nitrogen for 2 hours. The reaction was quenched by water and extracted by DCM (100 mLx2). The organic layer was concentrated and purified by column chromatography to give compound A310-2 (3.9 g ) as off-white solid. LC-MS ($ES^+$): *m/z* 430$[M+H]^+$

Step4: Synthesis of Compound A310-3

The mixture of A310-2(3.9 g, 9.0 mmol) , potassium acetate (2.65 g, 27.0 mmol) Pd(dppf)$Cl_2$ (658 mg, 0.9 mmol), and pinacol diborate (3.71 g, 14.5 mmol) in toluene (50 mL), was stirred at 75°C under nitrogen for 2 hours. Removing the solvent and purifying by column chromatography to give compound A310-3 (3.7 g) as off-white solid LC-MS ($ES^+$): *m/z* 478$[M+H]^+$

StepS: Synthesis of Compound A310

The mixture of A310-3 (95 mg, 0.2 mmol) $K_2CO_3$ (83 mg, 0.6 mmol), Pd(dppf)$Cl_2$(15 mg, 0.02 mmol), A310-6(46 mg, 0.2 mmol), dioxane (1.5mL) and $H_2O$(0.3 mL) was stirred at 75 °C under nitrogen for 2 hours. The reaction was concentrated and purified by column chromatography to give compound A310 (35 mg) as off-white solid. LC-MS ($ES^+$): *m/z* 504$[M+H]^+$

Example315: Synthesis of Compound A315

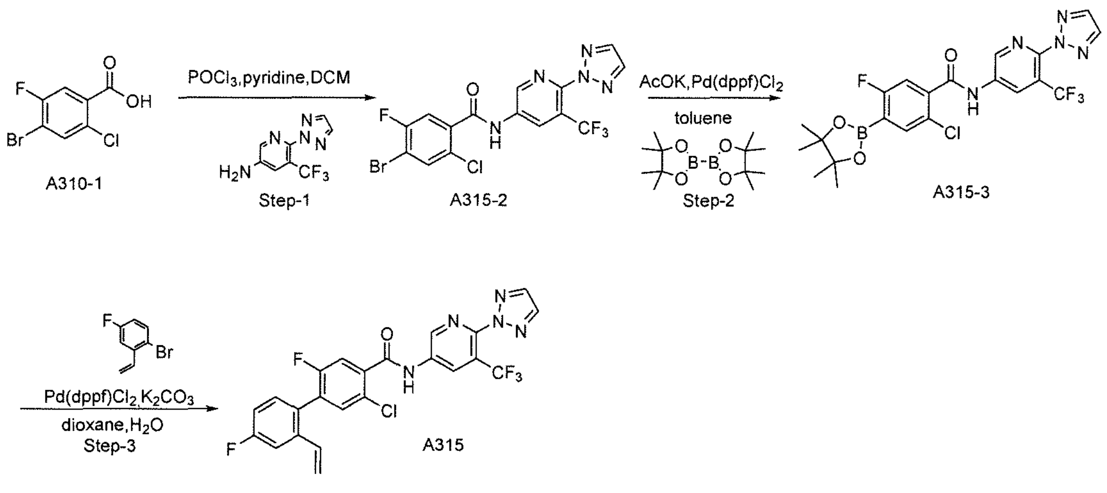


Step1: Synthesis of Compound A315-2

To a solution of A315-2 (2.52 g, 10 mmol) in DCM (30 mL), was added pyridine (2.37 g, 30 mmol) and 6-(2H-1,2,3-triazol-2-yl)-5-(trifluoromethyl)pyridin-3-amine (2.29 g, 10 mmol). Then $POCl_3$ (1.68 g, 11 mmol) was added dropwise. The reaction was stirred at room temperature for 2 hours. The reaction was quenched by water and extracted by

DCM (200 mLx2), the organic layer was concentrated in vacuum and purified by column chromatography to afford compound A315-2 (3.9 g) as off-white solid. LC-MS ($ES^+$): *m/z* 464$[M+H]^+$

Step2: Synthesis of Compound A315-3

The mixture of A310-2 (3.9 g, 8.4 mmol) potassium acetate (2.48 g, 25.2 mmol), Pd(dppf)$Cl_2$ (585 mg, 0.8 mmol) and pinacol diborate (3.28 g, 12.8 mmol) in toluene (50 mL) was stirred at 75°C under nitrogen for 2 hours. Removing the solvent and purifying by column chromatography to affored compound A315-3 (3.7 g) as off-white solid. LC-MS ($ES^+$): *m/z* 512$[M+H]^+$

Step3: Synthesis of Compound A315

The mixture of A310-3 (102 mg, 0.2 mmol) $K_2CO_3$ (83 mg, 0.6 mmol), Pd(dppf)$Cl_2$ (15 mg, 0.02 mmol), 1-bromo-4-fluoro-2-vinylbenzene (40 mg, 0.2 mmol), dioxane (1.5mL) and $H_2O$ (0.3 mL) was stirred at 75°C under nitrogen for 2 hours. The reaction was concentrated and purified by column chromatography to give compound A315 (30 mg) as off-white solid. LC-MS ($ES^+$): *m/z* 504$[M+H]^+$

Example316: Synthesis of Compound A316

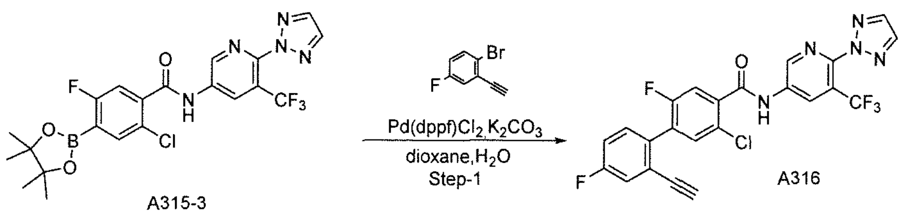


A315-3

A316

The mixture of A315-3 (102 mg, 0.2 mmol) , $K_2CO_3$ (83 mg, 0.6 mmol), Pd(dppf)$Cl_2$ (15 mg, 0.02 mmol), 1-bromo-2-ethynyl-4-fluorobenzene (40 mg, 0.2 mmol), dioxane (1.5mL) and $H_2O$ (0.3 mL) was stirred at 75°C under nitrogen for 2 hours. The reaction was concentrated and purified by column chromatography to give compound A316 (30 mg) as off-white solid. LC-MS ($ES^+$): *m/z* 504$[M+H]^+$

Example318: Synthesis of Compound A318

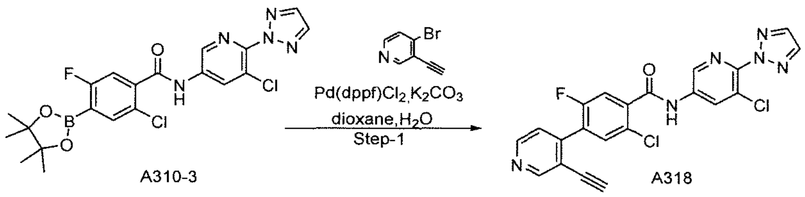


A310-3

A318

The mixture of A310-3 (95 mg, 0.2 mmol), $K_2CO_3$ (83 mg, 0.6 mmol), Pd(dppf)$Cl_2$ (15 mg, 0.02 mmol), 4-bromo-3-ethynylpyridine (36 mg, 0.2 mmol), dioxane (1.5 mL) and $H_2O$(0.3 mL) was stirred at 75°C under nitrogen for 2 hours. The reaction was concentrated and purified by column chromatography to give Compound A318 (36 mg) as off-white solid. LC-MS ($ES^+$): *m/z* 453$[M+H]^+$

Example319: Synthesis of Compound A319

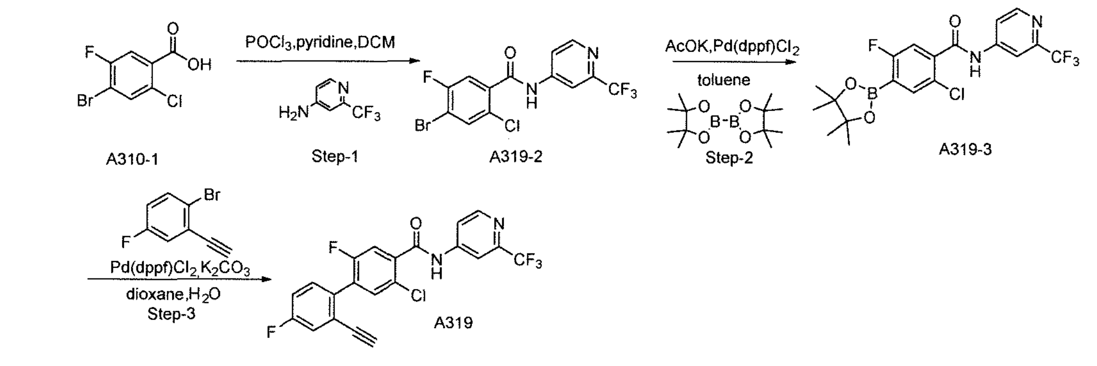


Step1: Synthesis of Compound A319-2

To a solution of A310-1 (2.52 g, 10 mmol) in DCM (30 mL), was added pyridine (2.37 g, 30 mmol) and 5-(trifluoromethyl)pyridin-3-amine (1.62 g, 10 mmol). Then $POCl_3$ (1.68 g, 11 mmol) was added dropwise. The reaction wasstirred at room temperature for 2 hours. The reaction was quenched by water and extacted by DCM (100 mLx2), the organic layer was concentrated and purified by column chromatography to give compound A319-2 (3.1 g) as off-white solid. LC-MS ($ES^+$): *m/z* 397$[M+H]^+$

Step2: Synthesis of Compound A319-3

The mixture of A319-2 (3.1 g, 7.8 mmol) , potassium acetate (2.30 g, 23.5 mmol), $Pd(dppf)Cl_2$ (585 mg, 0.8 mmol), and pinacol diborate (3.00 g, 11.7 mmol) in toluene (35 mL), was stirred at 75 °C under nitrogen for 2 hours. The reaction was concentrated and purified by column chromatography to give compound A319-3 (3.7 g) as off-white solid. LC-MS ($ES^+$): *m/z* 445$[M+H]^+$

Step3: Synthesis of Compound A319

The mixture of A319-3 (90 mg, 0.2 mmol), $K_2CO_3$ (83 mg, 0.6 mmol), $Pd(dppf)Cl_2$ (15 mg, 0.02 mmol), 1-bromo-2-ethynyl-4-fluorobenzene (40 mg, 0.2 mmol), dioxane (1.5mL) and $H_2O$ (0.3 mL) was stirred at 75°C under nitrogen for 2 hours. The reaction was concentrated in vacuum and purified by column chromatography to give compound A319 (33 mg) as off-white solid. LC-MS ($ES^+$): *m/z* 437$[M+H]^+$

Example323: Synthesis of Compound A323

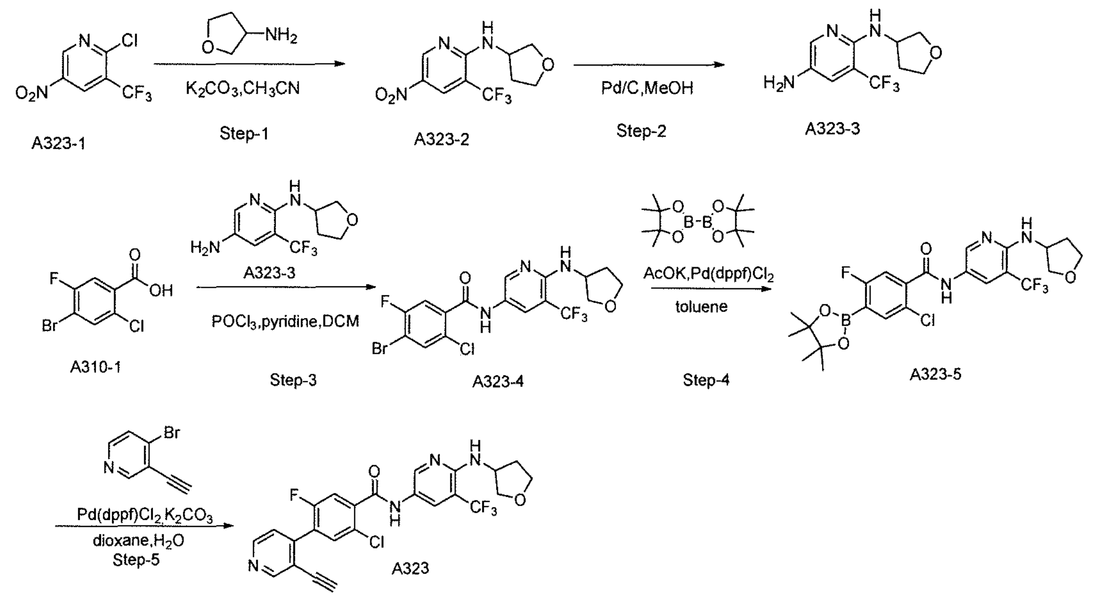

Step1: Synthesis of Compound A323-2

To a solution of A323-1 (2.26 g, 10 mmol) in acetonitrile (30 mL) was added potassium carbonate (4.14 g, 30 mmol) and tetrahydrofuran-3-amine (1.75 g, 20 mmol). The reaction was stirred at 75°C under nitrogen for 2 hours. The reaction was concentrated and purified by column chromatography to give compound A323-2(2.3 g) as off-white solid. LC-MS ($ES^+$): *m/z* 278$[M+H]^+$

Step2: Synthesis of Compound A323-3

To a solution of A323-2 (2.26 g, 8.2 mmol) in methanol (30 mL) was added Pd/C (10%, 435 mg) under nitrogen atmosphere in a 100 mL round bottom flask. The flask was then vacuumed and flushed with hydrogen. The reaction mixture was hydrogenated at 75°C for 2 hours under hydrogen atmosphere using a hydrogen balloon, then filtered through a Celite pad and concentrated under reduced pressure. The residue was purified by column chromatography to afford compound A323-3 (1.8 g) as off-white solid. LC-MS ($ES^+$): *m/z* 248$[M+H]^+$

Step3: Synthesis of Compound A323-4

To a solution of A310-1 (1.8 g, 7.2 mmol) in DCM (30 mL) was added pyridine (1.71 g, 21.6 mmol), $N^2$-(tetrahydrofuran-3-yl)-3-(trifluoromethyl)pyridine-2,5-diamine (1.41 g, 7.2 mmol) and $POCl_3$ (1.21 g, 7.2 mmol). The reaction was stirred at room temperature for 2 hours. The reaction was quenched by water and extracted by DCM (100 mL x2), the organic layer was concentrated and purified by column chromatography to give compound A323-4(3.1 g) as off-white solid. LC-MS ($ES^+$): *m/z* 482$[M+H]^+$

Step4: Synthesis of Compound A323-5

The mixture of A323-4 (3.1 g, 6.4 mmol) potassium acetate (2.47 g, 15.2 mmol), $Pd(dppf)Cl_2$ (439 mg, 0.6 mmol) and pinacol diborate (2.45 g, 9.6 mmol) in toluene (35 mL) was stirred at 75 °C under nitrogen for 2 hours. The reaction was concentrated and purified by column chromatography to give A323-5 (3.1 g) as off-white solid. LC-MS ($ES^+$): *m/z* 530$[M+H]^+$

StepS: Synthesis of Compound A323

The mixture of A323-5 (106 mg, 0.2 mmol) ,$K_2CO_3$ (83 mg, 0.6 mmol), $Pd(dppf)Cl_2$ (15 mg, 0.02 mmol), 4-bromo-3-ethynylpyridine (36 mg, 0.2 mmol), dioxane (1.5mL) and $H_2O$ (0.3 mL) was stirred at 75°C under nitrogen for 2 hours. The reaction solution was concentrated and purified by column chromatography to give compound A323 (37 mg) as off-white solid. LC-MS ($ES^+$): *m/z* 505$[M+H]^+$

Example327: Synthesis of Compound A327

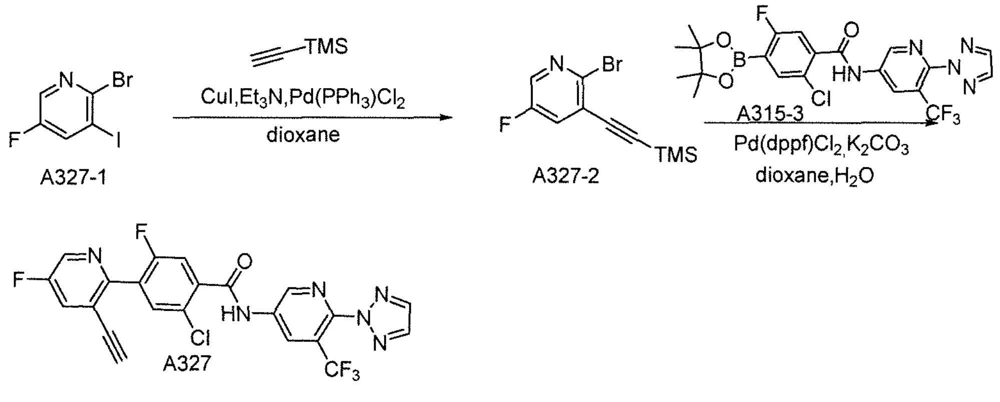

A327-1
A327-2
A327

Step1: Synthesis of Compound A327-2

The mixture of A327-1 (3.01 g, 10.0 mmol) ,CuI (0.38 g, 2.0 mmol), $Et_3N$ (3.03 g, 30.0 mmol), $Pd(PPh_3)_2Cl_2$

(0.73 g, 1 mmol), dioxane (35 mL) and ethyltrimethylsilane (2.04 g, 20.0 mmol) was stirred at room temperature under nitrogen for 5 hours. The reaction wasquenched by water, extracted with dichloromethane (250 mLx2). The organic phase was dried over sodium sulfate, concentrated in vacuum and purified by column chromatography to give compound A327-2 (2.5 g) as yellow solid. LC-MS ($ES^+$): $m/z$ 272$[M+H]^+$

Step2: Synthesis of Compound A327

The mixture of A315-3 (102 mg, 0.2 mmol), $K_2CO_3$ (83 mg, 0.6 mmol), $Pd(dppf)Cl_2$ (15 mg, 0.02 mmol), A327-2 (54 mg, 0.2 mmol), dioxane (1.5mL) and $H_2O$ (0.3 mL) was stirred at 75°C under nitrogen for 2 hours. The reaction solution was concentrated in vacuum and purified by column chromatography to give compound A327(38 mg) as off-white solid. LC-MS ($ES^+$): $m/z$ 505$[M+H]^+$

Example331: Synthesis of Compound A331

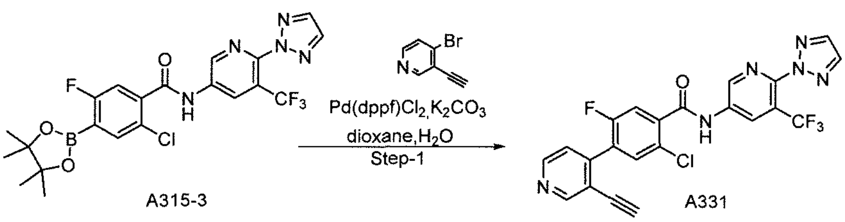


The mixture of A315-3 (95 mg, 0.2 mmol) $K_2CO_3$ (83 mg, 0.6 mmol), $Pd(dppf)Cl_2$ (15 mg, 0.02 mmol), 4-bromo-3-ethynylpyridine (36 mg, 0.2 mmol), dioxane (1.5mL) and $H_2O$ (0.3 mL) was stirred at 75°C under nitrogen for 2 hours. The reaction solution was concentrated in vacuum and purified by column chromatography to give compound A331 (37 mg) as off-white solid. LC-MS ($ES^+$): $m/z$ 487$[M+H]^+$

Example332: Synthesis of Compound A332

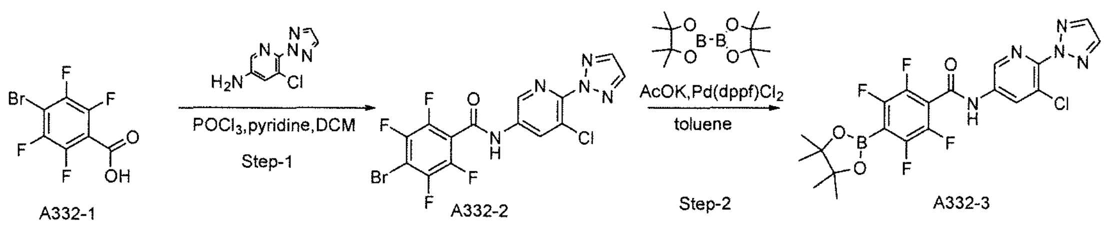


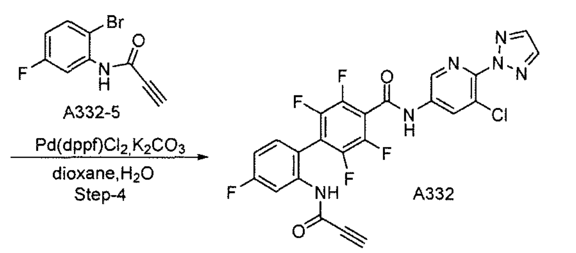


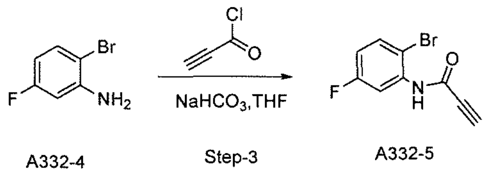

Step1: Synthesis of Compound A332-2

To a solution of A332-1 (2.72 g, 10 mmol) in DCM (30 mL) was added pyridine (2.37 g, 30 mmol), 5-chloro-6-(2H-1,2,3-triazol-2-yl)pyridin-3-amine (1.95 g, 10 mmol) and $POCl_3$ (1.68 g, 11 mmol). The reaction mixture was stirred at room temperature for 2 hours , quenched by water and extracted by DCM (100 mL x2). The organic layer was concentrated and purified by column chromatography to give compound A332-2 (3.9 g) as off-white solid. LC-MS ($ES^+$): *m/z* 450$[M+H]^+$

Step2: Synthesis of Compound A332-3

The mixture of A310-2 (3.9 g, 8.6 mmol) potassium acetate (2.53 g, 25.8 mmol), $Pd(dppf)Cl_2$ (658 mg, 0.9 mmol), pinacol diborate (3.31 g, 12.9 mmol) and toluene (50 mL) was stirred at 75°C under nitrogen for 2 hours. The reaction solution was concentrated and purified by column chromatography to give Compound A332-3( 3.7 g) as off-white solid. LC-MS ($ES^+$): *m/z* 501$[M+H]^+$

Step3: Synthesis of Compound A332-5

To a solution of A332-4 (1.89 g,10.0 mmol) , in THF (30 mL) was added sodium bicarbonate (2.52 g, 30 mmol) and propargyl chloride (0.89g,10.0 mmol). The reaction was stirred at room temperature for 2 hours. The reaction was quenched by water and extracted by EA (100 mLx2), the organic layer was concentrated and purified by column chromatography to obtain compound A332-5(1.7 g) as off-white solid. LC-MS ($ES^+$): *m/z* 242$[M+H]^+$

Step4: Synthesis of Compound A332

The mixture of A332-3 (100 mg, 0.2 mmol) , $K_2CO_3$ (83 mg, 0.6 mmol), $Pd(dppf)Cl_2$ (15 mg, 0.02 mmol), A332-5 (48 mg, 0.2 mmol), dioxane (1.5mL) and $H_2O$ (0.3 mL) was stirred at 75 °C under nitrogen for 2 hours. The reaction was concentrated in vacuum and purified by column chromatography to give compound A332 (35 mg) as off-white solid. LC-MS ($ES^+$): *m/z* 513$[M+H]^+$

Example343: Synthesis of Compound A343

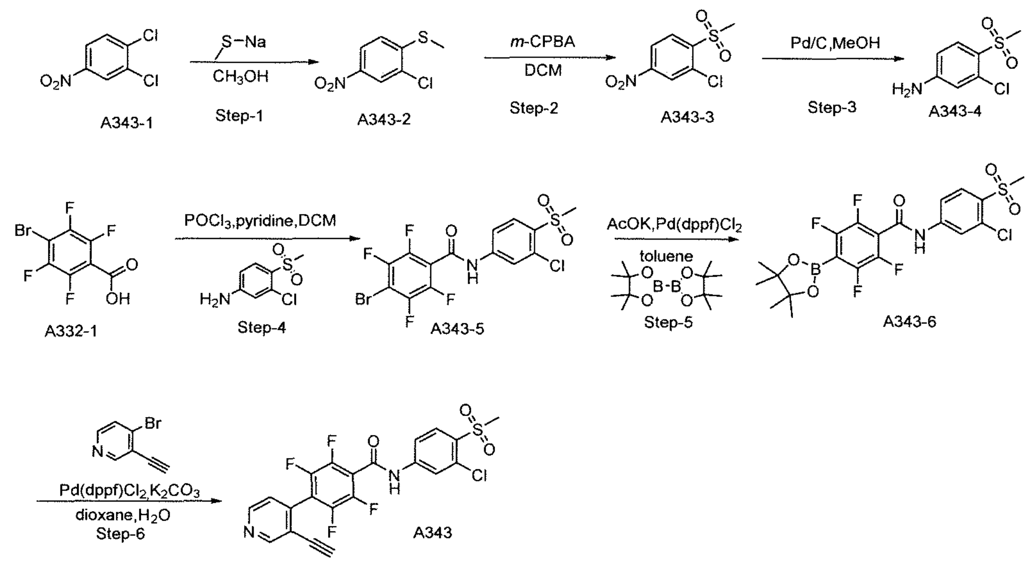


Step1: Synthesis of Compound A343-2

To a solution of A343-1 (1.91 g,10.0 mmol) in methanol (25 mL) was added sodium methylmercaptide (1.40 g, 20 mmol). The reaction was stirred at 75°C for 2 hours. The reaction solution was concentrated and purified by column chromatography to give off-white solid A343-2 (1.5 g). LC-MS ($ES^+$): *m/z* 204$[M+H]^+$

Step2: Synthesis of Compound A343-3

To a solution of A343-2 (1.50 g, 7.3 mmol) in dichloromethane (20 mL)was added *m*-CPBA (1.73 g, 10 mmol). The reaction was stirred at room temperature for 2 hours. Monitored by LCMS,then concentrated and purified by column chromatography to give off-white solid A343-3(1.1 g). LC-MS ($ES^+$): *m/z* 236$[M+H]^+$

Step3: Synthesis of Compound A343-4

A343-3 (1.10 g, 4.7 mmol) was added to the reaction flask, and then palladium on carbon (212 mg, 2 mmol) and methanol (15 mL) were added. The reaction was stirred at room temperature under hydrogen for 2 hours. The end of the reaction were monitored by LCMS. The reaction solution was filtered, evaporated to obtain off-white solid 900 mg. LC-MS ($ES^+$): *m/z* 206$[M+H]^+$.

Step4: Synthesis of Compound A343-5

A332-1 (2.72 g, 10 mmol) was added to the reaction flask, and then DCM (30 mL), pyridine (2.37 g, 30 mmol) and 3-chloro-4-methylsulfonylanilide (2.06 g, 10 mmol) were added. Then $POCl_3$ (1.68 g, 11 mmol) was added dropwise. The reaction was carried out at room temperature for 2 hours. The end of the reaction were monitored by LCMS. The reaction solution was purified by column chromatography to obtain off-white solid product 3.9 g. LC-MS ($ES^+$): *m/z* 460$[M+H]^+$

StepS: Synthesis of Compound A343-6

A343-5 (3.9 g, 8.5 mmol) was added to the reaction flask, and then potassium acetate (2.50 g, 25.5 mmol), $Pd(dppf)Cl_2$ (658 mg, 0.9 mmol), toluene (50 mL), pinacol diborate (3.31 g, 12.9 mmol) were added. The reaction was carried out at 75°C under nitrogen for 2 hours. The end of the reaction were monitored by LCMS. The reaction solution was purified by column chromatography to obtain off-white solid product 3.7 g. LC-MS ($ES^+$): *m/z* 508$[M+H]^+$

Step6: Synthesis of Compound A343

A343-6 (114 mg, 0.2 mmol) was added to the reaction flask, and then $K_2CO_3$(83 mg, 0.6 mmol), $Pd(dppf)Cl_2$ (15 mg, 0.02 mmol), 4-bromo-3-ethynylpyridine (36 mg, 0.2 mmol), dioxane (1.5mL) and $H_2O$ (0.3 mL) were added. The reaction was carried out at 75°C under nitrogen for 2 hours. The end of the reaction were monitored by LCMS. The reaction solution was purified by column chromatography to obtain off-white solid product 39 mg. LC-MS ($ES^+$): *m/z* 483$[M+H]^+$

Example344: Synthesis of Compound A344

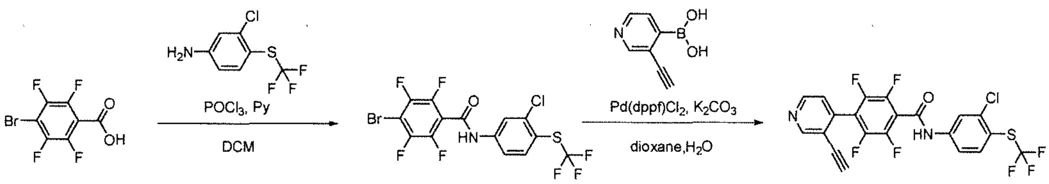


A344-1

A344

Step1: Synthesis of Compound A344-1

4-bromo-2,3,5,6-tetrafluorobenzoic acid (92 mg), 3-chloro-4-((trifluoromethyl)thio)aniline (72 mg), pyridine (180 mg) and DCM(10 mL) were added to the 50mL reaction flask, the solution was cooled to 0°C. then $POCl_3$ (200 mg) was added dropwise. the reaction was carried out at room temperature for 2 hour The reaction solution was poured into ice water, and dichloromethane (50 mL) was added to the above soluton. The reaction solution was washed with saturated NaCl solution. The organic phase was purified by silica gel column (PE/EA=1/1) to obtain product A344-1 (55 mg), LC-MS ($ES^+$): *m/z* 481$[M+H]^+$

Step2: Synthesis of Compound A344

A344-1 (55 mg), (3-ethynylpyridin-4-yl)boronic acid (40 mg), Pd(dppf)Ch (5 mg), $K_2CO_3$(80 mg), dioxane (10mL) and $H_2O$(1mL) were added to a 25mL round flask and replaced with nitrogen. The reaction mixture was heated to 90°C and reacted for 4 hours. The end of the reaction were monitored by LCMS. The reaction solution was diluted with EA (5 mL), washed with saturated NaCl solution. The organic phase was concentrated and purified by silica gel column (PE/EA=1/1) to obtain product A344 (16 mg), LC-MS ($ES^+$): *m/z* 505$[M+H]^+$.

Example361: Synthesis of Compound A361

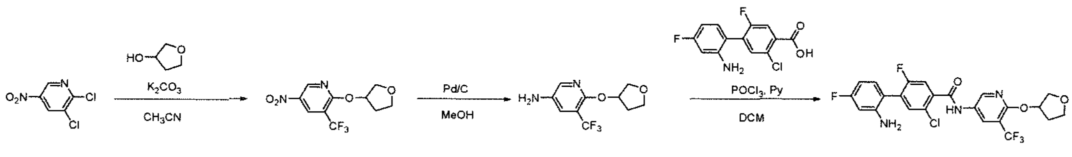


A361-1 A361-2 A361

Step1: Synthesis of Compound A361-1

2,3-dichloro-5-nitropyridine (400 mg), tetrahydrofuran-3-ol (160 mg), potassium carbonate (552mg) and ethipyridine (20 mL) were added to a 25mLround flask and heated to 80°C and reacted for 3 hours. The reaction solution was cooled to room temperature and filtered. The reaction solution was diluted with EA (200 mL), washed with saturated NaCl solution. The organic phase was concentrated and purified by silica gel column (PE/EA=1/1) to obtain product A361-1 (110 mg), LC-MS ($ES^+$): *m/z* 279 $[M+H]^+$

Step2: Synthesis of Compound A361-2

A361-1(110 mg), ethanol (10 mL) and Pd/C(40 mg) were added to a 50mLround flask and replaced with hydrogen three times. The reaction was stirred at room temperature under hydrogen for 1 hours.. The reaction solution was filtered and concentrated to obtain product A361-2 (60 mg), LC-MS ($ES^+$): *m/z* 249$[M+H]^+$

Step3: Synthesis of Compound A361

A361-2 (50 mg), 2'-amino-5-chloro-2,4'-difluoro-[1,1'-biphenyl]-4-carboxylic acid (48 mg), pyridine (90 mg) and DCM(10 mL) were added to a 50mL reaction flask, and cooled to 0°C. And $POCl_3$ (100 mg) was added dropwise. the reaction was carried out at room temperature for 2 hour. The reaction solution was poured into ice water. The reaction solution was diluted with DCM (50 mL), washed with saturated NaCl solution. The organic phase was concentrated and purified by silica gel column (PE/EA=1/1) to obtain product A361 (13 mg), LC-MS ($ES^+$): *m/z* 514$[M+H]^+$

Example371: Synthesis of Compound A371

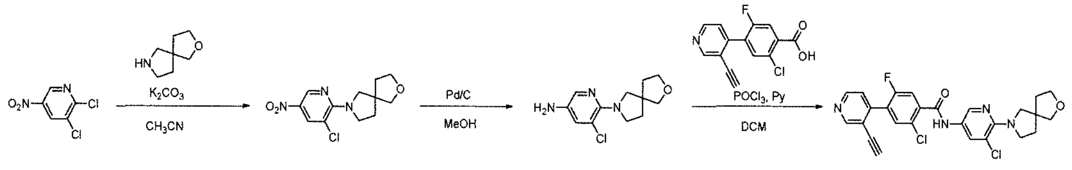


A371-1 A371-2 A371

Step1: Synthesis of Compound A371-1

2,3-dichloro-5-nitropyridine (400 mg), 2-oxo-7-azaspiro[4.4]nonane (250 mg), potassium carbonate (552 mg) and acetonitrile (20 mL) were added to a 50mL reaction flask, and heated to 80°C and reacted for 3 hours. The reaction solution was cooled to room temperature and filtered. The reaction solution was diluted with EA (200 mL), washed with

saturated NaCl solution twice. The organic phase was concentrated and purified by silica gel column (PE/EA=1/1) to obtain product A371-1 (300 mg), LC-MS ($ES^+$): $m/z$ 284 $[M+H]^+$

Step2: Synthesis of Compound A371-2

A371-1 (160 mg), ethanol (10 mL) and Pd/C(50 mg) were added to a 50mL reaction flask and replaced with hydrogen three times. The reaction was stirred at room temperature under hydrogen for 1 hours.. The reaction solution was filtered and concentrated to obtain product A371-2 (60 mg), LC-MS ($ES^+$): $m/z$ 254$[M+H]^+$

Step3: Synthesis of Compound A371

A371-2(50 mg), 2-chloro-4-(3-ethynylpyridin-4-yl)-5-fluorobenzoic acid (76 mg), pyridine (100 mg) and DCM (10 mL) were added to a 50mL reaction flask, and stirred and cooled to 0°C. And $POCl_3$ (105 mg) was added dropwise. the reaction was carried out at room temperature for 2 hour. The reaction solution was poured into ice water to quench the reaction. The reaction solution was diluted with DCM (50 mL), washed with saturated NaCl solution. The organic phase was concentrated and purified by silica gel column (PE/EA=1/1) to obtain product A371 (23 mg), LC-MS ($ES^+$): $m/z$ 511$[M+H]^+$

Example373: Synthesis of Compound A373

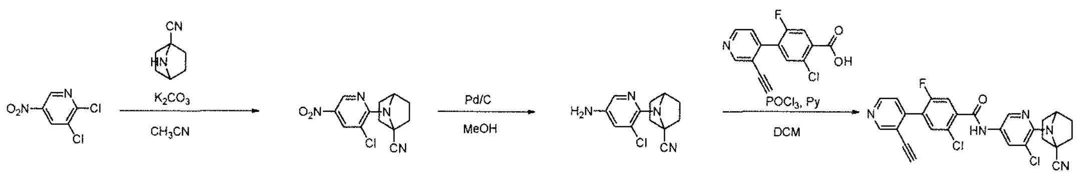


A373-1 A373-2 A373

Step1: Synthesis of Compound A373-1

2,3-dichloro-5-nitropyridine (400 mg), 7-azabicyclo[2.2.1]heptane-1-carbonitrile (212 mg), potassium carbonate (552 mg) and acetonitrile (20 mL) were added to a 50mL reaction flask, and heated to 80°C and reacted for 3 hours. The reaction solution was cooled to room temperature and filtered. The reaction solution was diluted with EA (200 mL), washed with saturated NaCl solution twice. The organic phase was concentrated and purified by silica gel column (PE/EA=1/1) to obtain product A373-1 (120 mg), LC-MS ($ES^+$): $m/z$ 279 $[M+H]^+$

Step2: Synthesis of Compound A373-2

A373-1(120 mg), ethanol (10 mL) and Pd/C(40 mg) were added to a 50mL reaction flask and replaced with hydrogen three times. The reaction was stirred at room temperature under hydrogen for 1 hours.. The reaction solution was filtered and concentrated to obtain product A373-2 (40 mg), LC-MS ($ES^+$): $m/z$ 249$[M+H]^+$

Step3: Synthesis of Compound A373

A373-2(40 mg), 2-chloro-4-(3-ethynylpyridin-4-yl)-5-fluorobenzoic acid (45 mg), pyridine (70 mg), and DCM(10 mL) were added to a 50mL reaction flask, and cooled to 0°C. And $POCl_3$ (75 mg) was added dropwise. the reaction was carried out at room temperature for 2 hour. The reaction solution was poured into ice water The reaction solution was diluted with DCM (50 mL), washed with saturated NaCl solution twice. The organic phase was concentrated and purified by silica gel column (PE/EA=1/1) to obtain product A373 (7 mg), LC-MS ($ES^+$): $m/z$ 506$[M+H]^+$

Example374: Synthesis of Compound A374

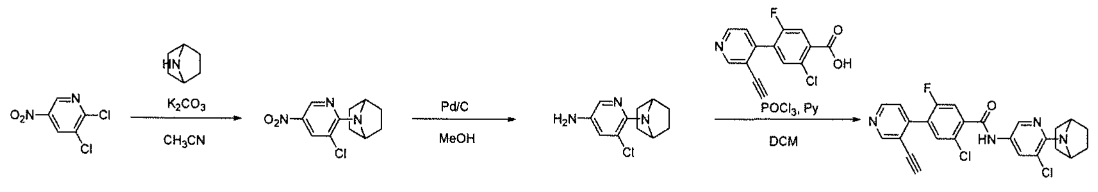


A374-1 A374-2 A374

Step1: Synthesis of Compound A374-1

2,3-Dichloro-5-nitropyridine (400 mg), (7-azabicyclo[2.2.1]heptane (196 mg), potassium carbonate (552mg) and acetonitrile (20 mL) were added to a 50mL reaction flask, and heated to 80°C and reacted for 3 hours. The reaction solution was cooled to room temperature and filtered. The filtrate was diluted with EA (200 mL), washed with saturated NaCl solution twice. The organic phase was concentrated and purified by silica gel column (PE/EA=1/1) to obtain product A374-1 (150 mg), LC-MS (ES$^+$): *m/z* 254 [M+H]$^+$

Step2: Synthesis of Compound A374-2

A374-1 (150 mg), ethanol (10 mL) and Pd/C(50 mg) were added to a 50mL reaction flask and replaced with hydrogen three times. The reaction was stirred at room temperature under hydrogen for 1 hours. The reaction solution was filtered and concentrated to obtain product A374-2 (60 mg), LC-MS (ES$^+$): *m/z* 224[M+H]$^+$

Step3: Synthesis of Compound A374

A374-2 (40 mg), 2-chloro-4-(3-ethynylpyridin-4-yl)-5-fluorobenzoic acid (55 mg), pyridine (70mg) and DCM(10 mL) were added to a 50mL reaction flask, and stirred and cooled to 0°C. And $POCl_3$ (90 mg) was added dropwise. the reaction was carried out at room temperature for 2 hour. The reaction solution was poured into ice water The reaction solution was diluted with DCM (50 mL), washed twice with saturated NaCl solution. The organic phase was concentrated and purified by silica gel column (PE/EA=1/1) to obtain product A374 (6 mg), LC-MS (ES$^+$): *m/z* 481[M+H]$^+$

Example377: Synthesis of Compound A377

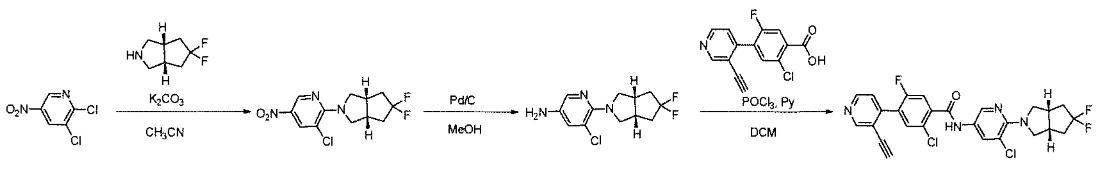


A377-1 A377-2 A377

Step1: Synthesis of Compound A377-1

2,3-dichloro-5-nitropyridine (400 mg), (3aR,6aS)-5,5-difluorooctahydropenta[c]pyrrole (294 mg), potassium carbonate (552mg) and acetonitrile (20 mL) were added to a 50mL reaction flask, and heated to 80°C and reacted for 3 hours. The reaction solution was cooled to room temperature and filtered. The reaction solution was diluted with EA (200 mL), washed with saturated NaCl solution twice. The organic phase was concentrated and purified by silica gel column (PE/EA=1/1) to obtain product A377-1 (180 mg), LC-MS (ES$^+$): *m/z* 304 [M+H]$^+$

Step2: Synthesis of Compound A377-2

A377-1 (150 mg), ethanol (10 mL) and Pd/C(50 mg) were added to a 50mL reaction flask and replaced with hydrogen three times. The reaction was stirred at room temperature under hydrogen for 1 hours. The reaction solution was filtered and concentrated to obtain product A377-2 (70 mg), LC-MS (ES$^+$): *m/z* 274[M+H]$^+$

Step3: Synthesis of Compound A377

A377-2 (50 mg), 2-chloro-4-(3-ethynylpyridin-4-yl)-5-fluorobenzoic acid (55 mg), pyridine (70mg) and DCM(10 mL) were added to a 50mL reaction flask, and stirred and cooled to 0°C. And $POCl_3$ (90 mg) was added dropwise. the reaction was carried out at room temperature for 2 hour. The reaction solution was poured into ice water The reaction solution was diluted with DCM (50 mL), washed twice with saturated NaCl solution. The organic phase was concentrated and purified by silica gel column (PE/EA=1/1) to obtain product A377 (11 mg), LC-MS ($ES^+$): *m/z* 531$[M+H]^+$

Example381: Synthesis of Compound A381

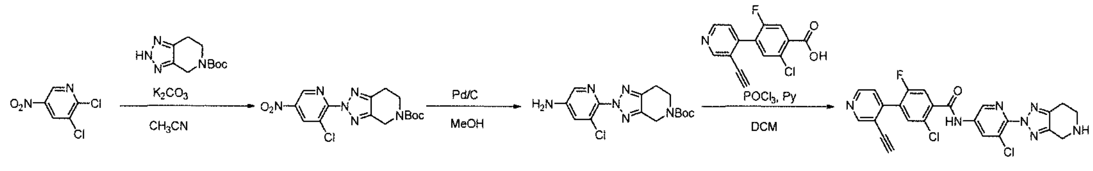


A383-1 A381-2 A381

Step1: Synthesis of Compound A381-1

2,3-dichloro-5-nitropyridine (400 mg), 2,4,6,7-tetrahydro-5H-[1,2,3]triazolyl[4,5-c]pyridine-5 -tert-butyl carboxylate (450 mg), potassium carbonate (552 mg) and acetonitrile (20 mL) were added to a 50mL reaction flask, and heated to 80°C and reacted for 3 hours. The reaction solution was cooled to room temperature and filtered. The reaction solution was diluted with EA (200 mL), washed with saturated NaCl solution twice. The organic phase was concentrated and purified by silica gel column (PE/EA=1/1) to obtain product A381-1 (406 mg), LC-MS ($ES^+$): *mlz 381* $[M+H]^+$

Step2: Synthesis of Compound A381-2

A381-1 (200 mg), ethanol (10 mL) and Pd/C(100 mg) were added to a 50mL reaction flask and replaced with hydrogen three times. The reaction was stirred at room temperature under hydrogen for 1 hour. The reaction solution was filtered and concentrated to obtain product A381-2 (110 mg), LC-MS ($ES^+$): *m/z* 351$[M+H]^+$

Step3: Synthesis of Compound A381

A381-2 (45 mg), 2-chloro-4-(3-ethynylpyridin-4-yl)-5-fluorobenzoic acid (50 mg), pyridine (70mg) and DCM(10 mL) were added to a 50mL reaction flask, and stirred and cooled to 0°C. And $POCl_3$ (80 mg) was added dropwise. the reaction was carried out at room temperature for 2 hour. The reaction solution was poured into ice water The reaction solution was diluted with DCM (50 mL), washed twice with saturated NaCl solution. The organic phase was concentrated and purified by silica gel column (PE/EA=1/1) to obtain product A381 (7 mg), LC-MS ($ES^+$): *m/z* 508 $[M+H]^+$

Example383: Synthesis of Compound A383

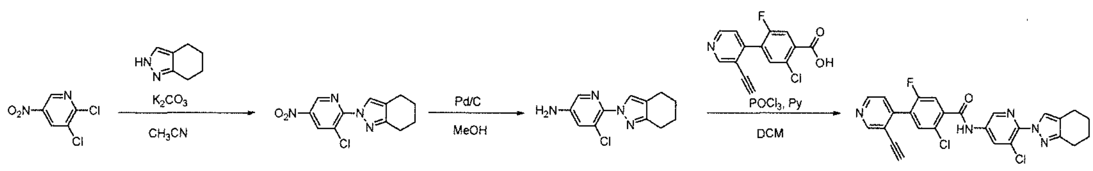


A383-1 A383-2 A383

Step1: Synthesis of Compound A383-1

2,3-dichloro-5-nitropyridine (400 mg), 4,5,6,7-tetrahydro-2H-indazole (245mg), potassium carbonate (552mg) and acetonitrile (20 mL) were added to a 50mL reaction flask, and heated to 80°C and reacted for 3 hours. The reaction

solution was cooled to room temperature and filtered. The reaction solution was diluted with EA (200 mL), washed with saturated NaCl solution twice. The organic phase was concentrated and purified by silica gel column (PE/EA=1/1) to obtain product A383-1 (310 mg), LC-MS ($ES^+$): *m/z* 279 $[M+H]^+$

Step2: Synthesis of Compound A383-2

A383-1 (310 mg), ethanol (10 mL) and Pd/C(100 mg) were added to a 50mL reaction bottle and replaced with hydrogen three times. The reaction was stirred at room temperature under hydrogen for 1 hour. The reaction solution was filtered and concentrated to obtain product A383-2 (160 mg), LC-MS ($ES^+$): *m/z* 249$[M+H]^+$

Step3: Synthesis of Compound A383

A383-2 (45 mg, 0.18 mmol), 2-chloro-4-(3-ethynylpyridin-4-yl)-5-fluorobenzoic acid (50 mg, 0.18 mmol), pyridine (70 mg, 0.89 mmol) and DCM(10 mL) were added to a 50 mL reaction flask, and stirred and cooled to 0°C. And $POCl_3$ (80 mg, 0.52 mmol) was added dropwise. After the dripping, the reaction was stirred at room temperature for 2 hours. The reaction solution was poured into ice water to quench the reaction. The reaction solution was diluted with DCM (50 mL), washed twice with saturated NaCl solution. The organic phase was concentrated and separated by silica gel column (PE/EA=1/1) to obtain product A383 (11 mg, 0.022 mmol), LC-MS ($ES^+$): *m/z* 506 $[M+H]^+$

Example388: Synthesis of Compound A388

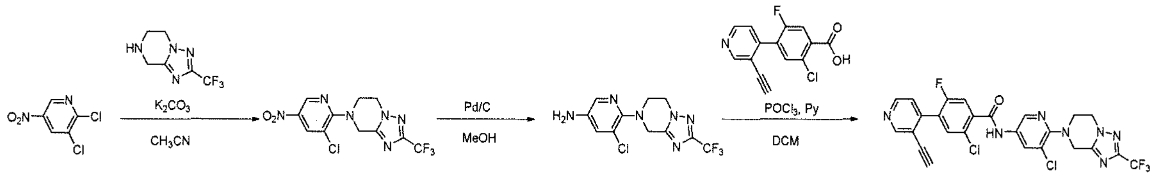


A388-1 A388-2 A388

Step1: Synthesis of Compound A388-1

2,3-dichloro-5-nitropyridine (400 mg, 2.08 mmol), 2-(trifluoromethyl)-5,6,7,8-tetrahydro-[1,2,4]triazolo[1, 5-a]pyrazine (400 mg, 2.08 mmol), potassium carbonate (552 mg, 4 mmol) and acetonitrile (20 mL) were added to a 50 mL reaction flask, and heated to 80°C and reacted for 3 hours. The reaction solution was cooled to room temperature and filtered. The reaction solution was diluted with EA (200 mL), washed twice with saturated NaCl solution. The organic phase was concentrated and separated by silica gel column (PE/EA=1/1) to obtain product A388-1 (506 mg, 1.45 mmol), LC-MS ($ES^+$): *m/z* 349 $[M+H]^+$

Step2: Synthesis of Compound A388-2

A388-1(480 mg, 1.38 mmol), MeOH(10 mL) and Pd/C(100 mg) were added to a 50 mL reaction bottle and replaced with hydrogen three times. Hydrogen was added and stirred for 1 hour at room temperature. The reaction solution was filtered and concentrated to obtain product A388-2 (300 mg, 0.94 mmol), LC-MS ($ES^+$): *m/z* 319$[M+H]^+$

Step3: Synthesis of Compound A388

A388-2 (50 mg, 0.16 mmol), 2-chloro-4-(3-ethynylpyridin-4-yl)-5-fluorobenzoic acid (43 mg, 0.16 mmol), pyridine (62 mg, 0.78 mmol) and DCM(10 mL) were added to a 50 mL reaction flask, and stirred and cooled to 0°C. And $POCl_3$ (80 mg, 0.52 mmol) was added dropwise. After the dripping, the reaction was stirred at room temperature for 2 h. The reaction solution was poured into ice water to quench the reaction. The reaction solution was diluted with DCM (50 mL), washed twice with saturated NaCl solution. The organic phase was concentrated and separated by silica gel column (PE/EA=1/1) to obtain product A388 (20 mg, 0.035 mmol), LC-MS ($ES^+$): *m/z* 576 $[M+H]^+$

Example392: Synthesis of Compound A392

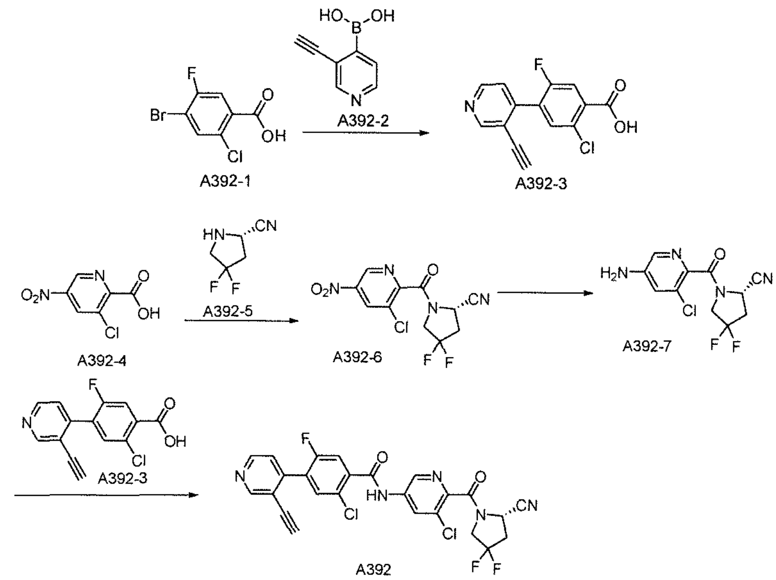
A392-1 A392-2 A392-3
A392-4 A392-5 A392-6 A392-7
A392-3 A392

Step1: Synthesis of Compound A392-3

A392-1 (253 mg, 1 mmol), A392-2 (161mg, 1.1 mmol), $PdCl_2(dppf)CH_2Cl_2$(10 mg, 0.012 mol), $K_2CO_3$(276 mg, 2 mmol), dioxane (5 mL) and $H_2O$(1 mL) were added to a 50 mL reaction bottle and replaced with nitrogen. The reaction mixture was heated to 90°C and reacted for 4 hours. The end of the reaction were monitored by LCMS and stopped heating. The reaction solution was diluted with EA (50 mL), washed twice with saturated NaCl solution. The organic phase was concentrated and separated by silica gel column (PE/EA=1/1) to obtain product A392-3(210 mg, 0.76 mmol), LC-MS ($ES^+$): *m/z* 276$[M+H]^+$.

Step2: Synthesis of Compound A392-6

A392-4 (202 mg, 1 mmol), A392-5 (158 mg, 1.2 mmol), HATU (570 mg, 1.5 mmol), DIEA(258 mg, 2 mmol), DMF(5 mL) were added to a 50 mL reaction flask, and heated to 80°C and reacted for 3 hours. Then the reaction solution was stopped heating and cooled to room temperature. The reaction solution was diluted with EA (50 mL), washed twice with saturated NaCl solution. The organic phase was concentrated and separated by silica gel column (PE/EA=1/1) to obtain product A392-6 (187 mg, 0.59 mmol), LC-MS ($ES^+$): *m/z* 317$[M+H]^+$

Step3: Synthesis of Compound A392-7

A392-6 (158 mg, 2 mmol) and ethanol (5 mL), $H_2O$(3 ml)were added to a 50 mL reaction flask and stirred. Then Fe powder (280 mg, 5 mmol) and $NH_4Cl$ (265 mg, 5 mmol) was added in batches. After the addition was completed, heating to 70°C and reacting for 2 hours. Then the reaction solution was stopped heating and cooled to room temperature. The reaction solution was filtered and concentrated. The leftover was separated by silica gel column (DCM/MeOH=20/1) to obtain product A392-7 (103 mg, 0.36 mmol), LC-MS ($ES^+$): *m/z* 287$[M+H]^+$

Step4: Synthesis of Compound A392

A392-7 (50 mg, 0.17 mmol), A392-3 (48 mg, 0.17 mmol), pyridine (67 mg, 0.85 mmol) and DCM(5mL) were added to a 50 mL reaction flask, and stirred at 0°C. And $POCl_3$ (80 mg, 0.52 mmol) was added dropwise. After the dripping, the reaction was stirred at room temperature for 2 hours. The reaction solution was diluted with DCM (50 mL), washed twice with saturated NaCl solution. The organic phase was concentrated and separated by silica gel column (PE/EA=1/1) to obtain product A392 (38 mg, 0.15 mmol), LC-MS ($ES^+$): *m/z* 544$[M+H]^+$

Example393: Synthesis of Compound A393

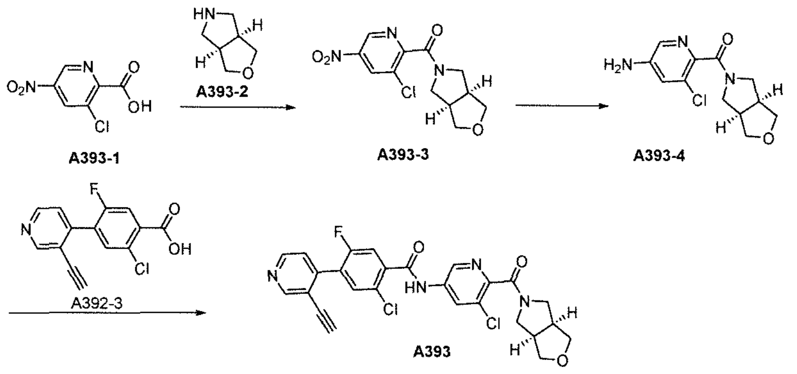


Step1: Synthesis of Compound A393-3

A393-1 (202 mg, 1 mmol), A393-2 (124 mg, 1.1 mmol), HATU (570 mg, 1.5 mmol), DIEA(258 mg, 2 mmol) and DMF (5 mL) were added to a 50 mL reaction flask, and heated to 80°C for 3 hours. Then the reaction solution was stopped heating and cooled to room temperature. The reaction solution was diluted with EA (50 mL), washed twice with saturated NaCl solution. The organic phase was concentrated and separated by silica gel column (PE/EA=1/1) to obtain product A393-3 (170 mg, 0.57 mmol), LC-MS ($ES^+$): *m/z* 298$[M+H]^+$

Step2: Synthesis of Compound A393-4

A393-3 (150 mg, 0.51 mmol) and ethanol (5 mL), $H_2O$(3 ml)were added to a 50mL reaction flask and stirred. Then Fe powder (280 mg, 5 mmol) and $NH_4Cl$ (265 mg, 5 mmol) was added in batches. After the addition was completed, heating to 70°C and reacting for for 2 hours. Then the reaction solution was stopped heating and cooled to room temperature. The reaction solution was filtered-and concentrated. The leftover was separated by silica gel column (DCM/MeOH=20/1) to obtain product A393-4 (105 mg, 0.39 mmol), LC-MS ($ES^+$): *m/z* 268$[M+H]^+$

Step3: Synthesis of Compound A393

A393-4 (47 mg, 0.18 mmol), A392-3 (48 mg, 0.16 mmol), pyridine (67 mg, 0.85 mmol) and DCM(5 mL) were added to a 50 mL reaction flask, and stirred and cooled to 0°C. Then $POCl_3$ (78 mg, 0.52 mmol) was added dropwise. After the dripping, the reaction was stirred at room temperature for 2 hours. The reaction solution was diluted with DCM (50 mL), washed twice with saturated NaCl solution. The organic phase was concentrated and separated by silica gel column (PE/EA=1/1) to obtain product A393 (30 mg, 0.057 mmol), LC-MS ($ES^+$): *m/z* 525$[M+H]^+$

Example395: Synthesis of CompoundA395

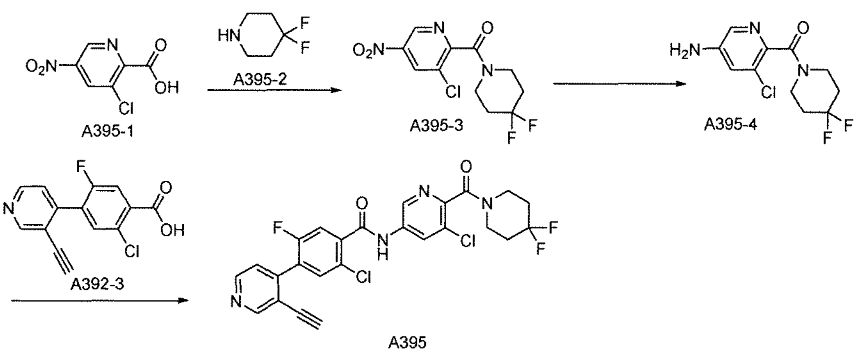


Step1: Synthesis of Compound A395-3

A395-1 (202 mg, 1 mmol), A395-2 (133 mg, 1.1 mmol), HATU (570 mg, 1.5 mmol), DIEA (258 mg, 2 mmol) and DMF(5 mL) were added to a 50 mL reaction flask, and heated to 80°C for 3 hours. Then the reaction solution was

stopped heating and cooled to room temperature. The reaction solution was diluted with EA (50 mL), washed twice with saturated NaCl solution. The organic phase was concentrated and separated by silica gel column (PE/EA=1/1) to obtain product A395-3 (150 mg, 0.49 mmol), LC-MS ($ES^+$): *m/z* 306$[M+H]^+$

Step2: Synthesis of Compound A395-4

A395-3 (150 mg, 0.49 mmol) and ethanol (5 mL), $H_2O$(3 ml)were added to a 50 mL reaction flask and stirred. Then Fe powder (270 mg, 5 mmol), $H_2O$(3 ml) was added in batches. After the addition was completed, heating to 70°C for 2 hours. Then the reaction solution was stopped heating and cooled to room temperature. The reaction solution was filtered and concentrated. The leftover was separated by silica gel column (DCM/MeOH=20/1) to obtain product A395-4 (100 mg, 0.36 mmol), LC-MS ($ES^+$): *m/z* 276$[M+H]^+$

Step3: Synthesis of Compound A395

A395-4 (48 mg, 0.17 mmol), A392-3 (48 mg, 0.16 mmol), pyridine (67 mg, 0.85 mmol) and DCM(5 mL) were added to a 50 mL reaction flask, and stirred at 0°C. And $POCl_3$ (78 mg, 0.51 mmol) was added dropwise. After the dripping, the reaction was stirred at room temperature for 2 hours. The reaction solution was diluted with DCM (50 mL), washed twice with saturated NaCl solution. The organic phase was concentrated and separated by silica gel column (PE/EA=1/1) to obtain product A395 (22 mg, 0.041 mmol), LC-MS ($ES^+$): *m/z* 533 $[M+H]^+$

Example398: Synthesis of Compound A398

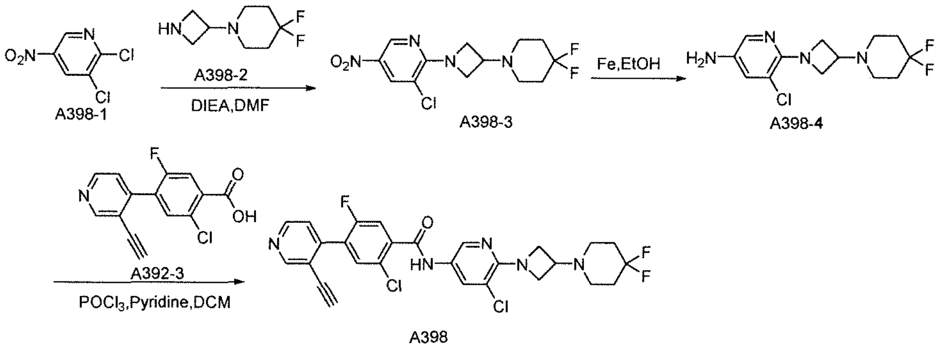


Step1: Synthesis of Compound A398-3

Into a 100-mL round-bottom flask, was placed A398-1 (192mg), A398-2 (193mg), DIEA (258mg) and DMF (5mL), and The resulting mixture was stirred at 100°C for 3 hours. Then the reaction solution was cooled to room temperature, diluted with EA (50 mL), washed twice with saturated NaCl solution. The organic phase was concentrated and purified by silica gel column (PE/EA=1/1) to give compound A398-3 (220mg), LC-MS ($ES^+$): *m/z*333$[M+H]^+$

Step2: Synthesis of Compound A398-4

To a solution of A398-3(165mg) in ethanol (5mL) was added Fe (270mg) in batches. The reaction was stirred at 70°C for 2 hours. The reaction solution was filtered, removing of the solvent and purified by silica gel column (DCM/MeOH=20/1) to give compound A398-4 (120mg), LC-MS ($ES^+$): *m/z*303$[M+H]^+$

Step3: Synthesis of Compound A398

To a solution of A398-4 (52mg), A392-3 (48mg) in DCM(5mL) was added pyridine (67mg) at room temperature. The mixture was cooled to 0°C in an ice bath. Then $POCl_3$ (78 mg) was added dropwise. The reaction was stirred for 1 hour at room temperature and quenched by water, extracted with dichloromethane. The organic layer was washed with brine, dried over anhydrous $Na_2SO_4$ and concentrated in vacuum. The residue was purified by silica gel column (PE/EA=1/1) to give compound A398 (40mg), LC-MS ($ES^+$): *m/z* 560 $[M+H]^+$

Example399: Synthesis of Compound A399

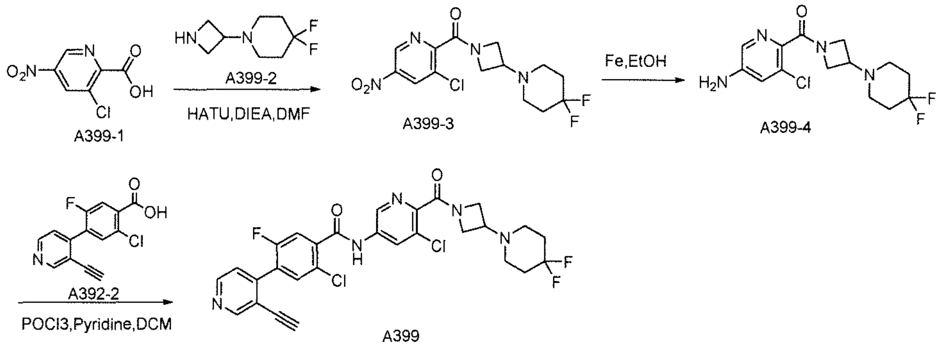


Step1: Synthesis of Compound A399-3

To a solution of A399-1 (202mg) in DMF (5mL) was added A399-2 (193mg), HATU (570mg), DIEA(258mg). The reaction was stirred at 80°C for 3 hours. The reaction solution was diluted with EA (50 mL), washed twice with saturated NaCl solution. The organic phase was concentrated and purified by silica gel column (PE/EA=1/1) to give compound A399-3 (136mg), LC-MS ($ES^+$): *m/z* 361$[M+H]^+$

Step2: Synthesis of Compound A399-4

To a solution of A399-3 (180mg) in ethanol (5mL) was added Fe (270mg) in batches. The reaction was stirred at 70°C 2 hours. The reaction solution was filtered, removing of the solvent and purified by silica gel column (DCM/MeOH=20/1) to give compound A399-4 (122mg), LC-MS ($ES^+$): *m/z* 331$[M+H]^+$

Step3: Synthesis of Compound A399

To a solution of A399-4 (57mg) and A392-2 (48mg) in DCM(5mL) was added pyridine (67mg) at room temperature. The mixture was cooled to 0°C in an ice bath. Then $POCl_3$ (78 mg) was added dropwise. The reaction was stirred for 2 hour at room temperature and quenched by water, extracted with dichloromethane. The organic layer was washed with brine, dried over anhydrous $Na_2SO_4$ and concentrated in vacuum. The residue was purified by silica gel column (PE/EA=1/1) to give compound A399 (34mg), LC-MS ($ES^+$): *m/z* 588 $[M+H]^+$

Example402: Synthesis of Compound A402

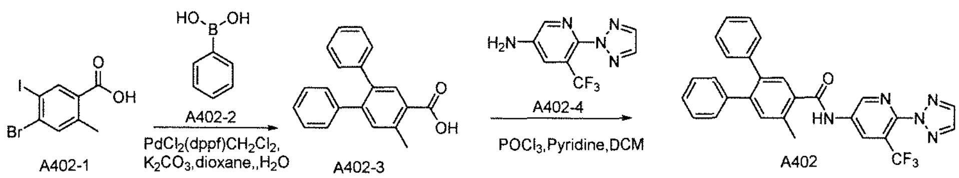


Step1: Synthesis of Compound A402-3

Into a 100-mL round-bottom flask, was placed A402-1 (340mg), A402-2 (268mg), $PdCl_2(dppf)CH_2Cl_2$(10mg), $K_2CO_3$(552mg), dioxane (10mL) and $H_2O$(1mL). The resulting mixture was stirred at 100°C for 2 hours under nitrogen. Removing of the solvent and purified by silica gel column (PE/EA=3/1) to give compound A402-3 (230mg), LC-MS ($ES^+$): *m/z* 289$[M+H]^+$.

Step2: Synthesis of Compound A402

To a solution of A402-3 (50mg), A402-4(39mg), pyridine (67mg) in DCM(5mL) was added pyridine (67mg) at room temperature. The mixture was cooled to 0°C in an ice bath. Then $POCl_3$ (78 mg) was added dropwise. The reaction was stirred for 2 hour at room temperature and quenched by water, extracted with dichloromethane. The organic layer was washed with brine, dried over anhydrous $Na_2SO_4$ and concentrated in vacuum. The residue was purified by silica gel column (PE/EA=1/1) to give compound A402 (28mg), LC-MS ($ES^+$): *m/z* 500 $[M+H]^+$

Example404: Synthesis of Compound A404

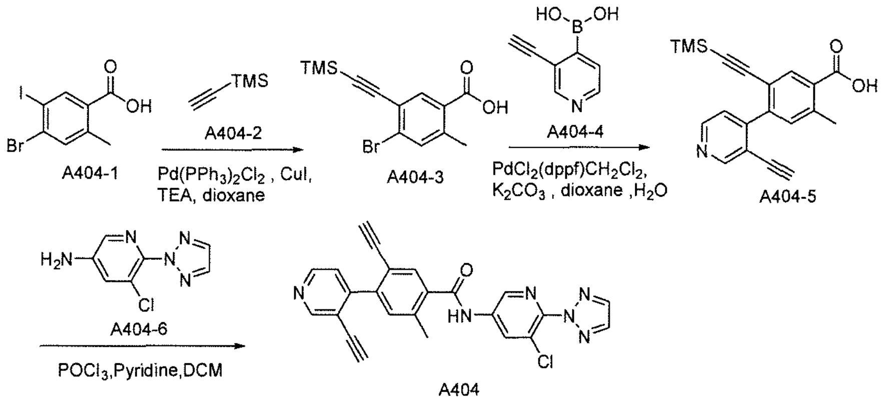


Step1: Synthesis of Compound A404-3

Into a 100-mL round-bottom flask, was placed A404-1 (340mg), A404-2 (98mg), $Pd(PPh_3)_2Cl_2$ (10mg), CuI(10mg), TEA(552mg), dioxane (10mL). The resulting mixture was stirred at 90°C for 4 hours under nitrogen. The reaction solution was diluted with EA (50 mL), washed twice with saturated NaCl solution. The organic phase was concentrated and purified by silica gel column (PE/EA=1/1) to give compound A404-3 (160mg), LC-MS ($ES^+$): *m/z* 311$[M+H]^+$.

Step2: Synthesis of Compound A404-5

Into a 100-mL round-bottom flask , was placed A404-3 (155mg), A404-4 (110mg), $PdCl_2(dppf)CH_2Cl_2$(10mg), $K_2CO_3$ (138mg), dioxane (5mL) and $H_2O$(1mL).The resulting mixture was stirred at 90°C for 4 hours under nitrogen. The reaction solution was diluted with EA (50 mL), washed twice with saturated NaCl solution. The organic phase was concentrated and purified by silica gel column (PE/EA=3/1) to give compound A404-5 (1 10mg), LC-MS ($ES^+$): *m/z* 334$[M+H]^+$.

Step3: Synthesis of Compound A404

To a solution of A404-5 (50mg), A404-6 (29mg) in DCM(5mL) was added pyridine (67mg) at room temperature. The mixture was cooled to 0°C in an ice bath. Then $POCl_3$ (78 mg) was added dropwise. The reaction was stirred for 2 hour at room temperature and quenched by water, extracted with dichloromethane. The organic layer was washed with brine, dried over anhydrous $Na_2SO_4$ and concentrated in vacuum. The residue was purified by silica gel column (PE/EA=1/1) to give compound A404 (17mg), LC-MS ($ES^+$): *m/z* 439 $[M+H]^+$

Example405: Synthesis of Compound A405

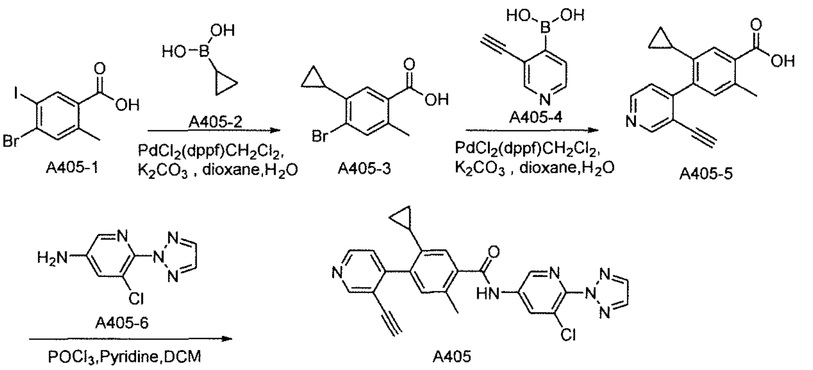


Step1: Synthesis of Compound A405-3

Into a 100-mL round-bottom flask, was placed A405-1 (340mg), A405-2 (86mg), $PdCl_2(dppf)CH_2Cl_2$ (10mg), $K_2CO_3$ (276mg), dioxane (5mL) and $H_2O$ (1mL). The resulting mixture was stirred at 90°C for 4 hours under nitrogen. The reaction solution was diluted with EA (50 mL), washed twice with saturated NaCl solution. The organic phase was concentrated and purified by silica gel column (PE/EA=5/1) to give compound A405-3 (110mg), LC-MS ($ES^+$): *m/z* 254 ,256$[M+H]^+$.

Step2: Synthesis of Compound A405-5

Into a 100-mL round-bottom flask, was placed A405-3 (110mg), A405-4 (95mg), $PdCl_2(dppf)CH_2Cl_2$ (10mg), $K_2CO_3$ (130mg), dioxane (5mL) and $H_2O$ (1mL). The resulting mixture was stirred at 90°C for 4 hours under nitrogen. The reaction solution was diluted with EA (50 mL), washed twice with saturated NaCl solution. The organic phase was concentrated and purified by silica gel column (PE/EA=3/1) to give compound A405-5 (60mg), LC-MS ($ES^+$): *m/z*278 $[M+H]^+$.

Step3: Synthesis of Compound A405

To a solution of A405-5 (50mg), A405-6 (35mg), pyridine (70mg) in DCM(5mL) was added pyridine (70mg) at room temperature. The mixture was cooled to 0°C in an ice bath. Then $POCl_3$ (82 mg) was added dropwise. The reaction was stirred for 2 hour at room temperature and quenched by water, extracted with dichloromethane. The organic layer was washed with brine, dried over anhydrous $Na_2SO_4$ and concentrated in vacuum. The residue was purified by silica gel column (PE/EA=1/1) to give compound A405 (33mg), LC-MS ($ES^+$): *m/z* 455 $[M+H]^+$.

According to the synthesis method of the above example, select appropriate raw materials and/or marketed example intermediates to synthesize the example compounds in the table below. The raw materials and reagents used are all commercially available.

| Example | Structure | Chemical Name | MS $[M+H]^+$ |
|---|---|---|---|
| Example276 (A276) | 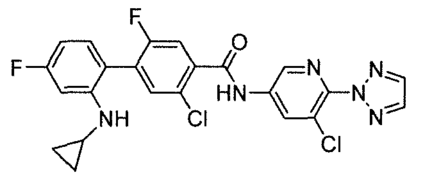 | 5-chloro-N-(5-chloro-6-(2H-1,2,3-triazol-2-yl)pyridin-3-yl)-2'-(cyclopropylamino)-2,4'-difluoro-[1,1'-biphenyl]-4-carboxamide | 501 |
| Example277 (A277) | 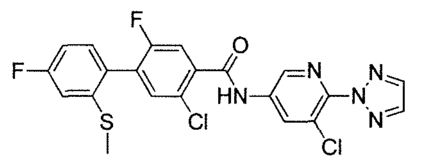 | 5-chloro-N-(5-chloro-6-(2H-1,2,3-triazol-2-yl)pyridin-3-yl)-2,4'-difluoro-2'-(methylthio)-[1,1'-biphenyl]-4-carboxamide | 492 |

(continued)

| Example | Structure | Chemical Name | MS $[M+H]^+$ |
|---|---|---|---|
| Example278 (A278) | 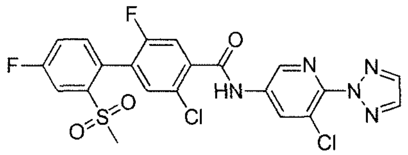 | 5-chloro-N-(5-chloro-6-(2H-1,2,3-triazol-2-yl)pyridin-3-yl)-2,4'-difluoro-2'-(methylsulfonyl)-[1,1'-biphenyl]-4-carboxamide | 524 |
| Example279 (A279) | 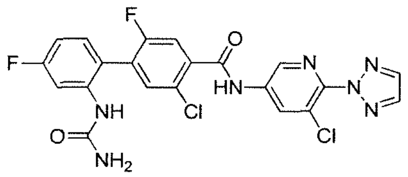 | 5-chloro-N-(5-chloro-6-(2H-1,2,3-triazol-2-yl)pyridin-3-yl)-2,4'-difluoro-2'-ureido-[1,1'-biphenyl]-4-carboxamide | 504 |
| Example280 (A280) | 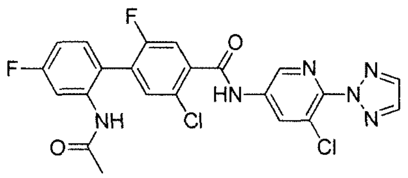 | 2'-acetamido-5-chloro-N-(5-chloro-6-(2H-1,2,3-triazol-2-yl)pyridin-3-yl)-2,4'-difluoro-[1,1'-biphenyl]-4-carboxamide | 503 |
| Example283 (A283) | 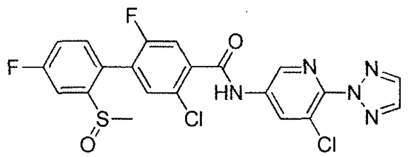 | 5-chloro-N-(5-chloro-6-(2H-1,2,3-triazol-2-yl)pyridin-3-yl)-2,4'-difluoro-2'-(methylsulfinyl)-[1,1'-biphenyl]-4-carboxamide | 508 |
| Example284 (A284) | 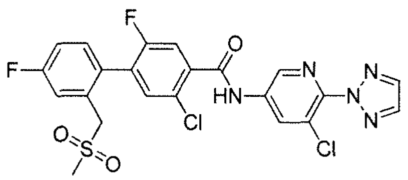 | 5-chloro-N-(5-chloro-6-(2H-1,2,3-triazol-2-yl)pyridin-3-yl)-2,4'-difluoro-2'-((methylsulfonyl)methyl)-[1,1'-biphenyl]-4-carboxamide | 538 |
| Example285 (A285) | 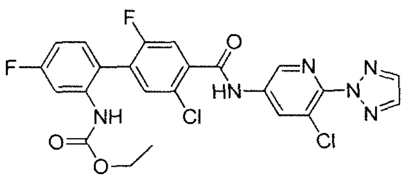 | ethyl (5'-chloro-4'-((5-chloro-6-(2H-1,2,3-triazol-2-yl)pyridin-3-yl)carbamoyl)-2',4-difluoro-[1,1'-biphenyl]-2-yl)carbamate | 533 |
| Example287 (A287) | 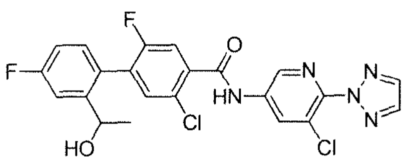 | 5-chloro-N-(5-chloro-6-(2H-1,2,3-triazol-2-yl)pyridin-3-yl)-2,4'-difluoro-2'-vinyl-[1,1'-biphenyl]-4-carboxamide | 490 |
| Example288 (A288) | 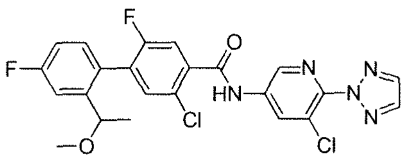 | 5-chloro-N-(5-chloro-6-(2H-1,2,3-triazol-2-yl)pyridin-3-yl)-2,4'-difluoro-2'-(1-hydroxyethyl)-[1,1'-biphenyl]-4-carboxamide | 504 |

(continued)

| Example | Structure | Chemical Name | MS [M+H]+ |
|---|---|---|---|
| Example289 (A289) | 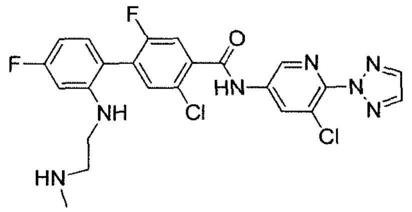 | 5-chloro-N-(5-chloro-6-(2H-1,2,3-triazol-2-yl)pyridin-3-yl)-2,4'-difluoro-2'-(1-methoxyethyl)-[1,1'-biphenyl]-4-carboxamide | 518 |
| Example290 (A290) | 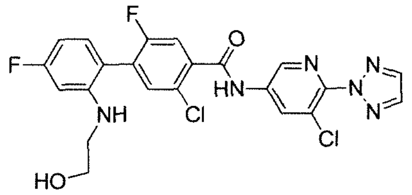 | 5-chloro-N-(5-chloro-6-(2H-1,2,3-triazol-2-yl)pyridin-3-yl)-2,4'-difluoro-2'-((2-(methylamino)ethyl)amino)-[1,1'-biphenyl]-4-carboxamide | 505 |
| Example291 (A291) | 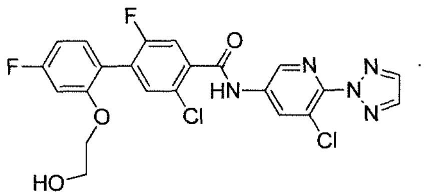 | 5-chloro-N-(5-chloro-6-(2H-1,2,3-triazol-2-yl)pyridin-3-yl)-2,4'-difluoro-2'-(2-hydroxyethoxy)-[1,1'-biphenyl]-4-carboxamide | 506 |
| Example292 (A292) | 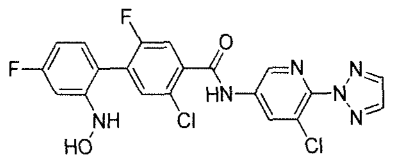 | 5-chloro-N-(5-chloro-6-(2H-1,2,3-triazol-2-yl)pyridin-3-yl)-2,4'-difluoro-2'-(hydroxyamino)-[1,1'-biphenyl]-4-carboxamide | 477 |
| Example293 (A293) | 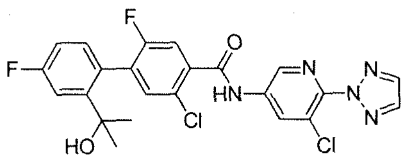 | 5-chloro-N-(5-chloro-6-(2H-1,2,3-triazol-2-yl)pyridin-3-yl)-2,4'-difluoro-2'-(2-hydroxypropan-2-yl)-[1,1'-biphenyl]-4-carboxamide | 504 |
| Example294 (A294) | 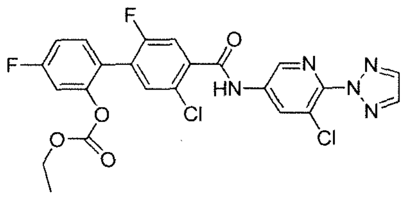 | 5'-chloro-4'-((5-chloro-6-(2H-1,2,3-triazol-2-yl)pyridin-3-yl)carbamoyl)-2',4-difluoro-[1,1'-biphenyl]-2-yl ethyl carbonate | 534 |
| Example295 (A295) | 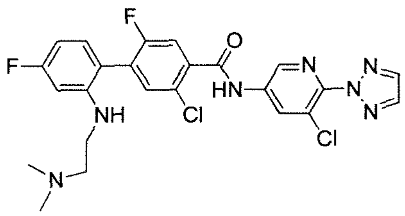 | 5-chloro-N-(5-chloro-6-(2H-1,2,3-triazol-2-yl)pyridin-3-yl)-2'-((2-(dimethylamino) ethyl)amino) -2,4'-difluoro-[1,1'-biphenyl]-4-carboxamide | 532 |

(continued)

| Example | Structure | Chemical Name | MS $[M+H]^+$ |
|---|---|---|---|
| Example297 (A297) | 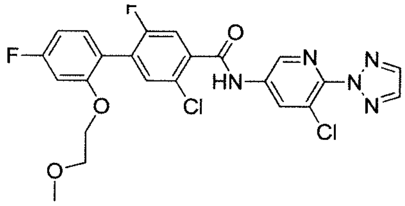 | 5-chloro-N-(5-chloro-6-(2H-1,2,3-triazol-2-yl)pyridin-3-yl)-2,4'-difluoro-2'-(2-methoxyethoxy)-1,1'-biphenyl]-4-carboxamide | 520 |
| Example299 (A299) | 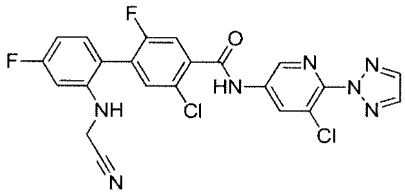 | 5-chloro-N-(5-chloro-6-(2H-1,2,3-triazol-2-yl)pyridin-3-yl)-2'-((cyanomethyl) amino)-2,4'-difluoro-[1,1'-biphenyl]-4-carboxamide | 500 |
| Example301 (A301) | 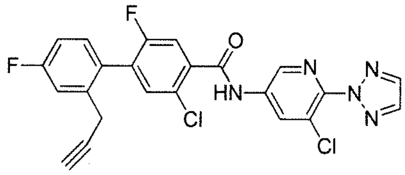 | 5-chloro-N-(5-chloro-6-(2H-1,2,3-triazol-2-yl)pyridin-3-yl)-2,4'-difluoro-2'-(prop-2-yn-1-yl)-[1,1'-biphenyl]-4-carboxamide | 484 |
| Example303 (A303) | 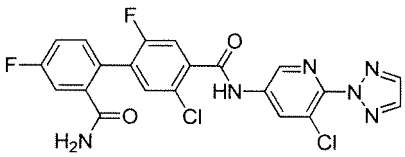 | 5'-chloro-N4'-(5-chloro-6-(2H-1,2,3-triazol-2-yl)pyridin-3-yl)-2',4-difluoro-[1,1'-biphenyl]-2,4'-dicarboxamide | 489 |
| Example304 (A304) | 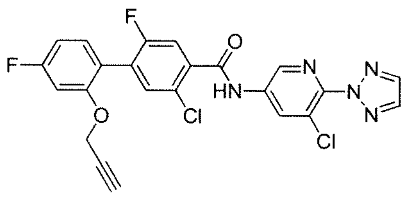 | 5-chloro-N-(5-chloro-6-(2H-1,2,3-triazol-2-yl)pyridin-3-yl)-2,4'-difluoro-2'-(prop-2-yn-1-yloxy)-[1,1'-biphenyl]-4-carboxamide | 500 |
| Example305 (A305) | 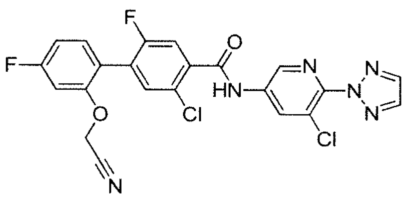 | 5-chloro-N-(5-chloro-6-(2H-1,2,3-triazol-2-yl)pyridin-3- yl)-2'-(cyanomethoxy)-2,4'-difluoro-[1,1'-biphenyl]-4-carboxamide | 501 |
| Example306 (A306) | 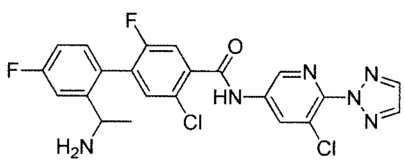 | 2'-(1-aminoethyl)-5-chloro-N-(5-chloro-6-(2H-1,2,3-triazol-2-yl)pyridin-3-yl)-2,4'-difluoro-[1,1'-biphenyl]-4-carboxamide | 489 |
| Example307 (A307) | 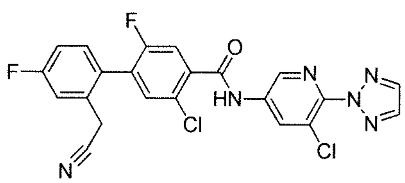 | 5-chloro-N-(5-chloro-6-(2H-1,2,3-triazol-2-yl)pyridin-3-yl)-2'-(cyanomethyl)-2,4'-difluoro-[1,1'-biphenyl]-4-carboxamide | 485 |

(continued)

| Example | Structure | Chemical Name | MS [M+H]+ |
|---|---|---|---|
| Example309 (A309) | 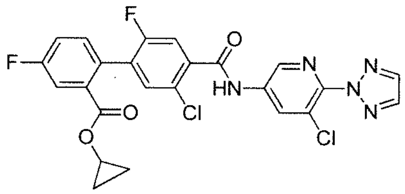 | cyclopropyl 5'-chloro-4'-((5-chloro-6-(2H-1,2,3-triazol-2-yl)pyridin-3-yl)carbamoyl)-2',4-difluoro-[1,1'-biphenyl]-2-carboxylate | 530 |
| Example311 (A311) | 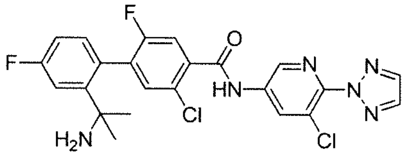 | 2'-(2-aminopropan-2-yl)-5-chloro-N-(5-chloro-6-(2H-1,2,3-triazol-2-yl)pyridin-3-yl)-2,4'-difluoro-[1,1'-biphenyl]-4-carboxamide | 503 |
| Example312 (A312) | 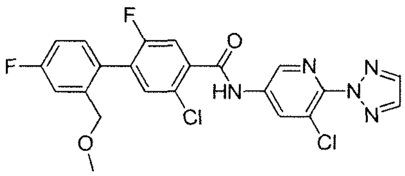 | 5-chloro-N-(5-chloro-6-(2H-1,2,3-triazol-2-yl)pyridin-3-yl)-2,4'-difluoro-2'-(methoxymethyl)-[1,1'-biphenyl]-4-carboxamide | 490 |
| Example313 (A313) | 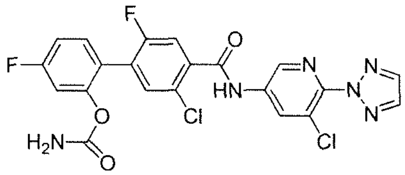 | 5'-chloro-4'-((5-chloro-6-(2H-1,2,3-triazol-2-yl)pyridin-3-yl)carbamoyl)-2',4-difluoro-[1,1'-biphenyl]-2-yl carbamate | 505 |
| Example314 (A314) | 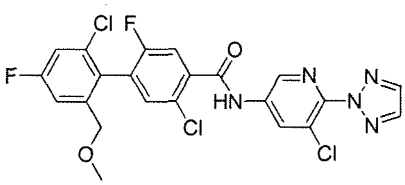 | 2',5-dichloro-N-(5-chloro-6-(2H-1,2,3-triazol-2-yl)pyridin-3-yl)-2,4'-difluoro-6'-(methoxymethyl)-[1,1'-biphenyl]-4-carboxamide | 525 |
| Example317 (A317) | 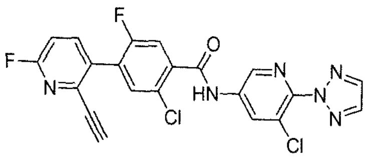 | 2-chloro-N-(5-chloro-6-(2H-1,2,3-triazol-2-yl)pyridin-3-yl)-4-(2-ethynyl-6-fluoropyridin-3-yl)-5-fluorobenzamide | 471 |
| Example320 (A320) | 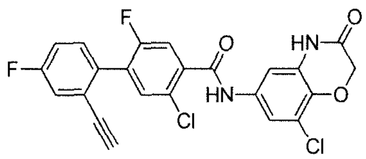 | 5-chloro-N-(8-chloro-3-oxo-3,4-dihydro-2H-benzo[b][1,4]oxazin-6-yl)-2'-ethynyl-2,4'-difluoro-[1,1'-biphenyl]-4-carboxamide | 473 |
| Example321 (A321) | 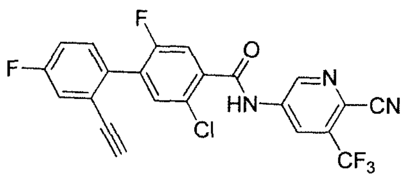 | 5-chloro-N-(6-cyano-5-(trifluoromethyl) pyridin-3-yl)-2'-ethynyl-2,4'-difluoro-[1,1'-biphenyl]-4-carboxamide | 462 |

(continued)

| Example | Structure | Chemical Name | MS [M+H]+ |
|---|---|---|---|
| Example322 (A322) | 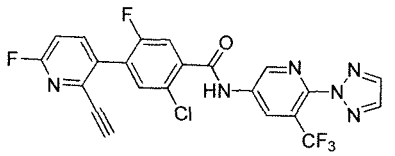 | N-(6-(2H-1,2,3-triazol-2-yl)-5-(trifluoromethyl)pyridin-3-yl)-2-chloro-4-(2-ethynyl-6-fluoropyridin-3-yl)-5-fluorobenzamide | 505 |
| Example324 (A324) | 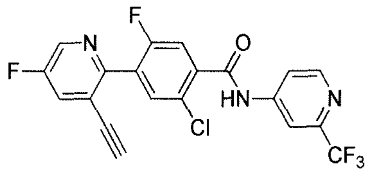 | 2-chloro-4-(3-ethynyl-5-fluoropyridin-2-yl)-5-fluoro-N-(2-(trifluoromethyl)pyridin-4-yl)benzamide | 438 |
| Example325 (A325) | 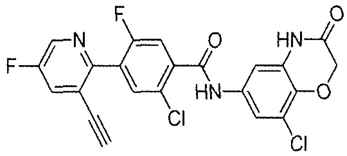 | 2-chloro-N-(8-chloro-3-oxo-3,4-dihydro-2H-benzo[b][1,4]oxazin-6-yl)-4-(3-ethynyl-5-fluoropyridin-2-yl)-5-fluorobenzamide | 474 |
| Example326 (A326) | 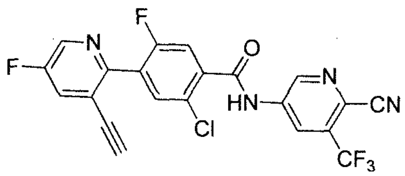 | 2-chloro-N-(6-cyano-5-(trifluoromethyl) pyridin-3-yl)-4-(3-ethynyl-5-fluoropyridin-2-yl)-5-fluorobenzamide | 463 |
| Example328 (A328) | 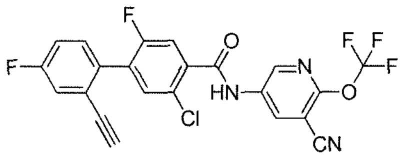 | 5-chloro-N-(5-cyano-6-(trifluoromethoxy) pyridin-3-yl)-2'-ethynyl-2,4'-difluoro-[1,1'-biphenyl]-4-carboxamide | 478 |
| Example329 (A329) | 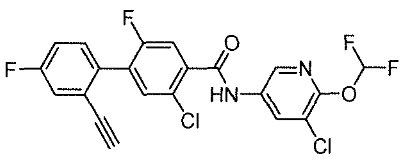 | 5-chloro-N-(5-chloro-6-(difluoromethoxy) pyridin-3-yl)-2'-ethynyl-2,4'-difluoro-[1,1'-biphenyl]-4-carboxamide | 469 |
| Example330 (A330) | 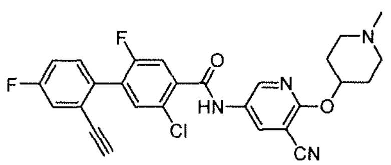 | 5-chloro-N-(5-cyano-6-((1-methylpiperidin-4-yl)oxy)pyridin-3-yl)-2'-ethynyl-2,4'-difluoro-[1,1'-biphenyl]-4-carboxamide | 507 |
| Example333 (A333) | 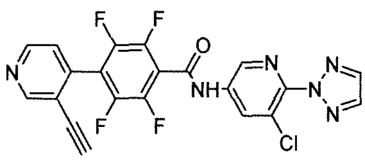 | N-(5-chloro-6-(2H-1,2,3-triazol-2-yl) pyridin-3-yl)-4-(3-ethynylpyridin-4-yl)-2,3,5,6-tetrafluorobenzamide | 473 |
| Example334 (A334) | 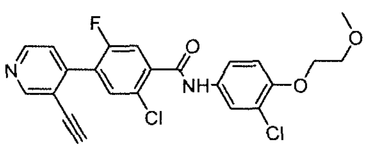 | 2-chloro-N-(3-chloro-4-(2-methoxyethoxy) phenyl)-4-(3-ethynylpyridin-4-yl)-5-fluorobenzamide | 459 |

(continued)

| Example | Structure | Chemical Name | MS [M+H]+ |
|---|---|---|---|
| Example335 (A335) | 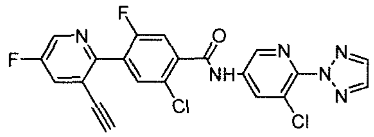 | 2-chloro-N-(5-chloro-6-(2H-1,2,3-triazol-2-yl)pyridin-3-yl)-4-(3-ethynyl-5-fluoropyridin-2-yl)-5-fluorobenzamide | 471 |
| Example336 (A336) | 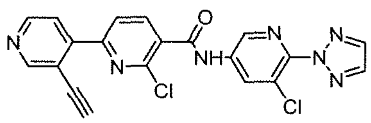 | 6-chloro-N-(5-chloro-6-(2H-1,2,3-triazol-2-yl)pyridin-3-yl)-3'-ethynyl-[2,4'-bipyridine]-5-carboxamide | 436 |
| Example337 (A337) | 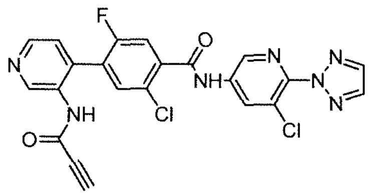 | 2-chloro-N-(5-chloro-6-(2H-1,2,3-triazol-2-yl)pyridin-3-yl)-5-fluoro-4-(3-propiolamidopyridin-4-yl)benzamide | 496 |
| Example338 (A338) | 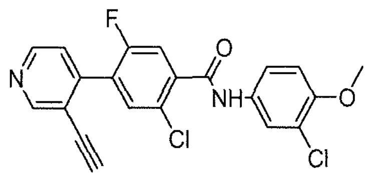 | 2-chloro-N-(3-chloro-4-methoxyphenyl)-4-(3-ethynylpyridin-4-yl)-5-fluorobenzamide | 415 |
| Example339 (A339) | 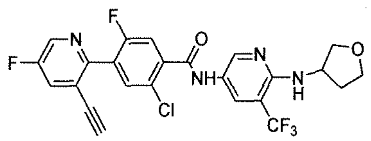 | 2-chloro-4-(3-ethynyl-5-fluoropyridin-2-yl)-5-fluoro-N-(6-((tetrahydrofuran-3-yl) amino)-5-(trifluoromethyl)pyridin-3-yl) benzamide | 523 |
| Example340 (A340) | 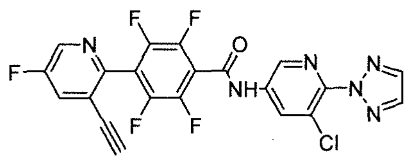 | N-(5-chloro-6-(2H-1,2,3-triazol-2-yl) pyridin-3-yl)-4-(3-ethynyl-5-fluoropyridin-2-yl)-2,3,5,6-tetrafluorobenzamide | 491 |
| Example341 (A341) | 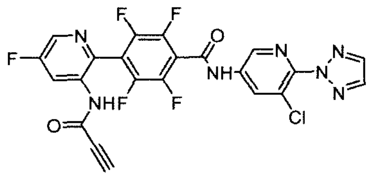 | N-(5-chloro-6-(2H-1,2,3-triazol-2-yl) pyridin-3-yl)-2,3,5,6-tetrafluoro-4-(5-fluoro-3-propiolamidopyridin-2-yl) benzamide | 534 |
| Example342 (A342) | 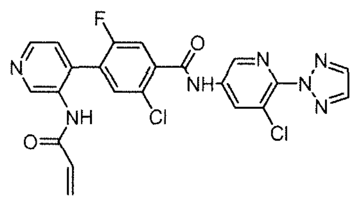 | 4-(3-acrylamidopyridin-4-yl)-2-chloro-N-(5-chloro-6-(2H-1,2,3-triazol-2-yl) pyridin-3-yl)-5-fluorobenzamide | 498 |
| Example345 (A345) | 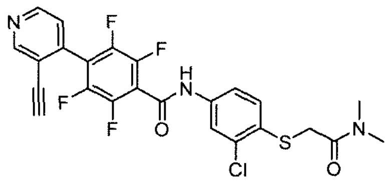 | N-(3-chloro-4-((2-(dimethylamino)-2-oxoethyl)thio)phenyl)-4-(3-ethynylpyridin-4-yl)-2,3,5,6-tetrafluorobenzamide | 522 |

(continued)

| Example | Structure | Chemical Name | MS [M+H]+ |
|---|---|---|---|
| Example346 (A346) | 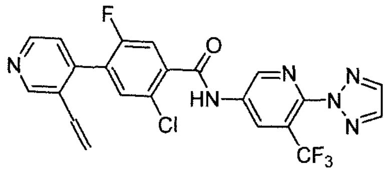 | N-(6-(2H-1,2,3-triazol-2-yl)-5-(trifluoromethyl)pyridin-3-yl)-2-chloro-5-fluoro-4-(3-vinylpyridin-4-yl)benzamide | 489 |
| Example347 (A347) | 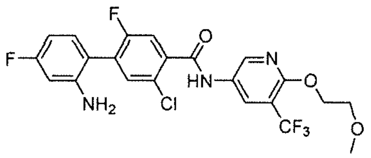 | 2'-amino-5-chloro-2,4'-difluoro-N-(6-(2-methoxyethoxy)-5-(trifluoromethyl) pyridin-3-yl)-[1,1'-biphenyl]-4-carboxamide | 502 |
| Example348 (A348) | 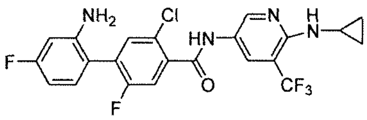 | 2'-amino-5-chloro-N-(6-(cyclopropylamino)-5-(trifluoromethyl)pyridin-3-yl)-2,4'-difluoro-[1,1'-biphenyl]-4-carboxamide | 483 |
| Example349 (A349) | 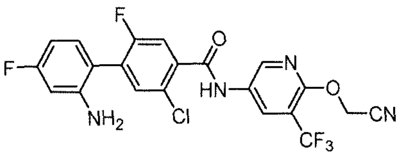 | 2'-amino-5-chloro-N-(6-(cyanomethoxy)-5-(trifluoromethyl)pyridin-3-yl)-2,4'-difluoro-[1,1'-biphenyl]-4-carboxamide | 483 |
| Example350 (A350) | 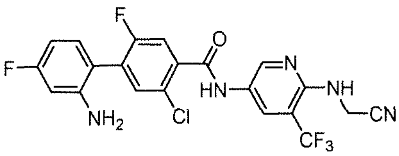 | 2'-amino-5-chloro-N-(6-((cyanomethyl) amino)-5-(trifluoromethyl)pyridin-3-yl)-2,4'-difluoro-[1,1'-biphenyl]-4-carboxamide | 482 |
| Example351 (A351) | 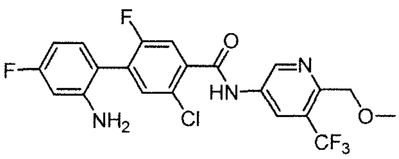 | 2'-amino-5-chloro-2,4'-difluoro-N-(6-(methoxymethyl)-5-(trifluoromethyl) pyridin-3-yl)-[1,1'-biphenyl]-4-carboxamide | 472 |
| Example352 (A352) | 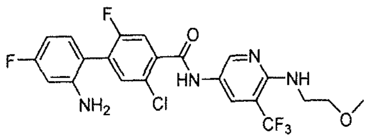 | 2'-amino-5-chloro-2,4'-difluoro-N-(6-((2-methoxyethyl)amino)-5-(trifluoromethyl) pyridin-3-yl)-[1,1'-biphenyl]-4-carboxamide | 501 |
| Example353 (A353) | 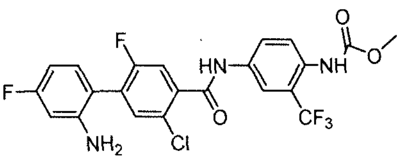 | methyl (4-(2'-amino-5-chloro-2,4'-difluoro-[1,1'-biphenyl]-4-carboxamido)-2-(trifluoromethyl)phenyl)carba mate | 500 |
| Example354 (A354) | 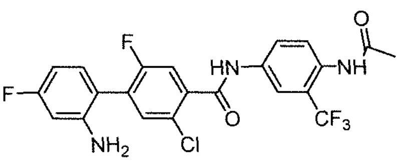 | N-(4-acetamido-3-(trifluoromethyl) phenyl)-2'-amino-5-chloro-2,4'-difluoro-[1,1'-biphenyl]-4-carboxamide | 484 |

(continued)

| Example | Structure | Chemical Name | MS [M+H]+ |
|---|---|---|---|
| Example355 (A355) | 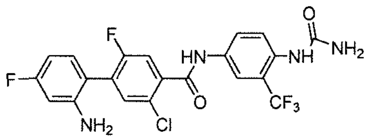 | 2’-amino-5-chloro-2,4’-difluoro-N-(3-(trifluoromethyl)-4-ureidophenyl)-[1,1’-biphenyl]-4-carboxamide | 485 |
| Example3 56 (A356) | 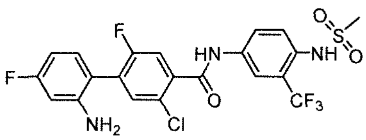 | 2’-amino-5-chloro-2,4’-difluoro-N-(4-(methylsulfonamido)-3-(trifluoromethyl)phenyl)-[1,1’-biphenyl]-4-carboxamide | 520 |
| Example3 57 (A357) | 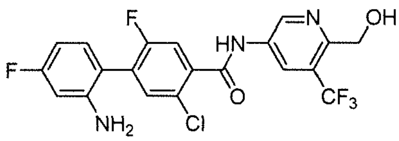 | 2’-amino-5-chloro-2,4’-difluoro-N-(6-(hydroxymethyl)-5-(trifluoromethyl) pyridin-3-yl)-[1,1’-biphenyl]-4-carboxamide | 458 |
| Example358 (A358) | 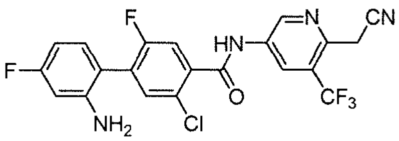 | 2’-amino-5-chloro-N-(6-(cyanomethyl)-5-(trifluoromethyl)pyridin-3-yl)-2,4’-difluoro-[1,1’-biphenyl]-4-carboxamide | 467 |
| Example359 (A359) | 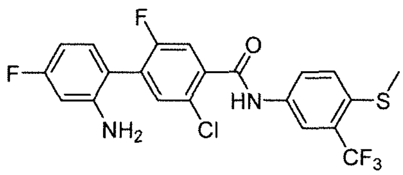 | 2’-amino-5-chloro-2,4’-difluoro-N-(4-(methylthio)-3-(trifluoromethyl) phenyl)-[1,1’-biphenyl]-4-carboxamide | 473 |
| Example360 (A360) | 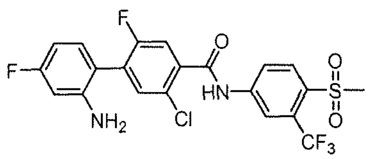 | 2’-amino-5-chloro-2,4’-difluoro-N-(4-(methylsulfonyl)-3-(trifluoromethyl) phenyl)-[1,1’-biphenyl]-4-carboxamide | 505 |
| Example362 (A362) | 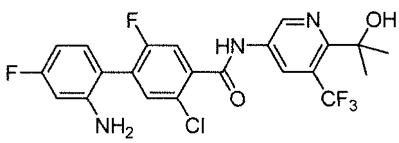 | 2’-amino-5-chloro-2,4’-difluoro-N-(6-(2-hydroxypropan-2-yl)-5-(trifluoromethyl) pyridin-3-yl)-[1,1’-biphenyl]-4-carboxamide | 486 |
| Example363 (A363) | 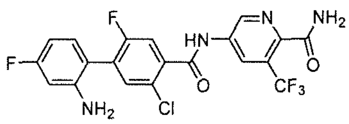 | 5-(2’-amino-5-chloro-2,4’-difluoro-[1,1’-biphenyl]-4-carboxamido)-3-(trifluoromethyl)picolinamide | 471 |
| Example364 (A364) | 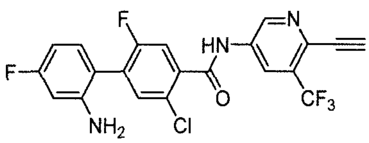 | 2’-amino-5-chloro-N-(6-ethynyl-5-(trifluoromethyl)pyridin-3-yl)-2,4’-difluoro-[1,1’-biphenyl]-4-carboxamide | 452 |
| Example365 (A365) | 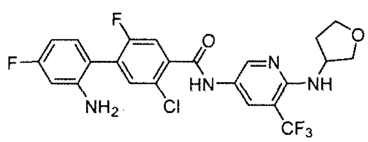 | 2’-amino-5-chloro-2,4’-difluoro-N-(6-((tetrahydrofuran-3-yl)amino)-5-(trifluoromethyl)pyridin-3-yl)-[1,1’-biphenyl]-4-carboxamide | 513 |

(continued)

| Example | Structure | Chemical Name | MS [M+H]+ |
|---|---|---|---|
| Example366 (A366) | 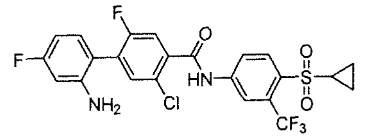 | 2'-amino-5-chloro-N-(4-(cyclopropylsulfonyl)-3-(trifluoromethyl)phenyl)-2,4'-difluoro-[1,1'-biphenyl]-4-carboxamide | 531 |
| Example367 (A367) | 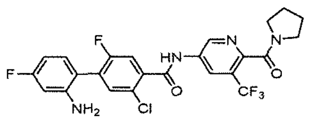 | 2'-amino-5-chloro-2,4'-difluoro-N-(6-(pyrrolidine-1-carbonyl)-5-(trifluoromethyl)pyridin-3-yl)-[1,1'-biphenyl]-4-carboxamide | 525 |
| Example368 (A368) | 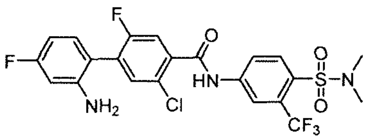 | 2'-amino-5-chloro-N-(4-(N,N-dimethylsulfamoyl)-3-(trifluoromethyl) phenyl)-2,4'-difluoro-[1,1'-biphenyl]-4-carboxamide | 534 |
| Example369 (A369) | 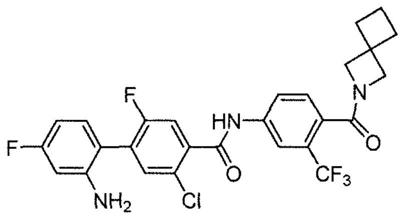 | N-(4-(2-azaspiro[3.3]heptane-2-carbonyl)-3-(trifluoromethyl)phenyl)-2'-amino-5-chloro-2,4'-difluoro-[1,1'-biphenyl]-4-carboxamide | 550 |
| Example370 (A370) | 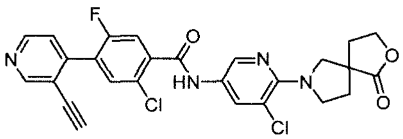 | 2-chloro-N-(5-chloro-6-(1-oxo-2-oxa-7-azaspiro[4.4]nonan-7-yl)pyridin-3-yl)-4-(3-ethynylpyridin-4-yl)-5-fluorobenzamide | 525 |
| Example372 (A372) | 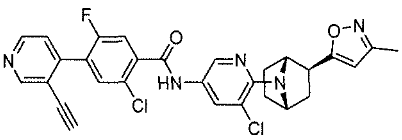 | 2-chloro-N-(5-chloro-6-((1R,2S,4S)-2-(3-methylisoxazol-5-yl)-7-azabicyclo[2.2.1] heptan-7-yl)pyridin-3-yl)-4-(3-ethynylpyridin-4-yl)-5-fluorobenzamide | 562 |
| Example375 (A375) | 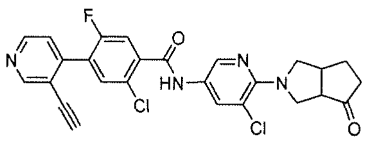 | 2-chloro-N-(5-chloro-6-(4-oxohexahydrocyclopenta[c]py rrol-2 (1H)-yl)pyridin-3-yl)-4-(3-ethynylpyridin-4-yl)-5-fluorobenzamide | 509 |
| Example376 (A376) | 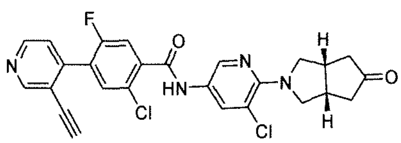 | 2-chloro-N-(5-chloro-6-((3aR,6aS)-5-oxohexahydrocyclopenta[c]py rrol-2 (1H)-yl)pyridin-3-yl)-4-(3-ethynylpyridin-4-yl)-5-fluorobenzamide | 509 |
| Example378 (A378) | 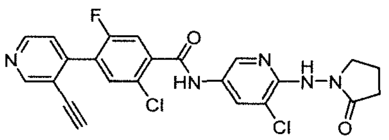 | 2-chloro-N-(5-chloro-6-((2-oxopyrrolidin-1-yl)amino)pyridin-3-yl)-4-(3-ethynylpyridin-4-yl)-5-fluorobenzamide | 484 |

(continued)

| Example | Structure | Chemical Name | MS $[M+H]^+$ |
|---|---|---|---|
| Example379 (A379) | 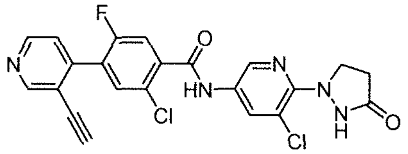 | 2-chloro-N-(5-chloro-6-(3-oxopyrazolidin-1-yl)pyridin-3-yl)-4-(3-ethynylpyridin-4-yl)-5-fluorobenzamide | 470 |
| Example380 (A380) | 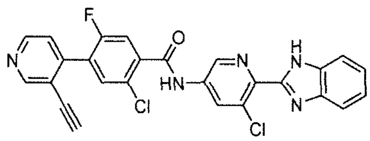 | N-(6-(1H-benzo[d]imidazol-2-yl)-5-chloropyridin-3-yl)-2-chloro-4-(3-ethynylpyridin-4-yl)-5-fluorobenzamide | 502 |
| Example382 (A382) | 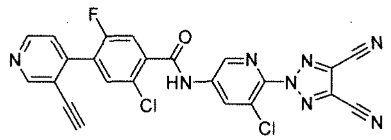 | 2-chloro-N-(5-chloro-6-(4,5-dicyano-2H-1,2,3-triazol-2-yl)pyridin-3-yl)-4-(3-ethynylpyridin-4-yl)-5-fluorobenzamide | 503 |
| Example384 (A384) | 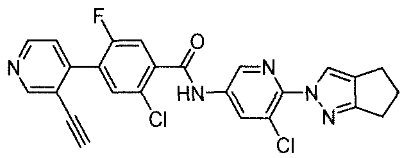 | 2-chloro-N-(5-chloro-6-(5,6-dihydrocyclopenta[c]pyrazol-2(4H)-yl) pyridin-3-yl)-4-(3-ethynylpyridin-4-yl)-5-fluorobenzamide | 492 |
| Example385 (A385) | 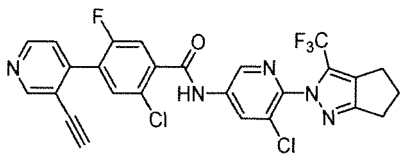 | 2-chloro-N-(5-chloro-6-(3-(trifluoromethyl)-5,6-dihydrocyclopenta[c]pyrazol-2(4H)-yl) pyridin-3-yl)-4-(3-ethynylpyridin-4-yl)-5-fluorobenzamide | 560 |
| Example386 (A386) | 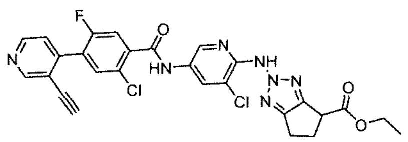 | ethyl 2-((3-chloro-5-(2-chloro-4-(3-ethynylpyridin-4-yl)-5-fluorobenzamido) pyridin-2-yl)amino)-2,4,5,6-tetrahydrocyclopenta[d][1,2,3 ]triazole-4-carboxylate | 580 |
| Example387 (A387) | 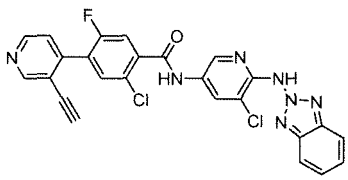 | N-(6-((2H-benzo[d][1,2,3]triazol-2-yl) amino)-5-chloropyridin-3-yl)-2-chloro-4-(3-ethynylpyridin-4-yl)-5-fluorobenzamide | 518 |
| Example389 (A389) | 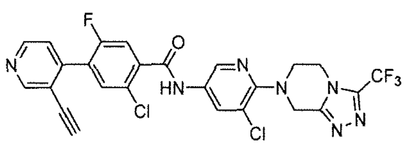 | 2-chloro-N-(5-chloro-6-(3-(trifluoromethyl)-5,6-dihydro-[1,2,4] triazolo[4,3-a]pyrazin-7(8H)-yl)pyridin-3-yl)-4-(3-ethynylpyridin-4-yl)-5-fluorobenzamide | 576 |
| Example390 (A390) | 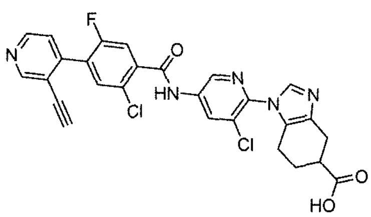 | 1-(3-chloro-5-(2-chloro-4-(3-ethynylpyridin-4-yl)-5-fluorobenzamido) pyridin-2-yl)-4,5,6,7-tetrahydro-1H-benzo [d]imidazole-5-carboxylic acid | 550 |

(continued)

| Example | Structure | Chemical Name | MS [M+H]+ |
|---|---|---|---|
| Examples391 (A391) | 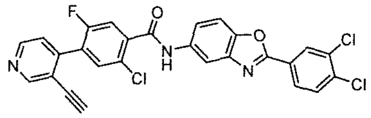 | 2-chloro-N-(2-(3,4-dichlorophenyl)benzo [d]oxaz ol-5-yl)-4-(3-ethynylpyridin-4-yl)-5-fluorobenzamide | 536 |
| Example394 (A394) | 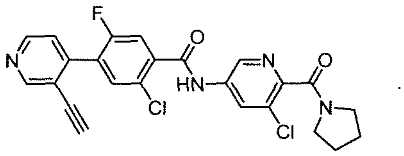 | 2-chloro-N-(5-chloro-6-(pyrrolidine-1-carbonyl)pyridin-3-yl)-4-(3-ethynylpyridin-4-yl)-5-fluorobenzamide | 483 |
| Example396 (A396) | 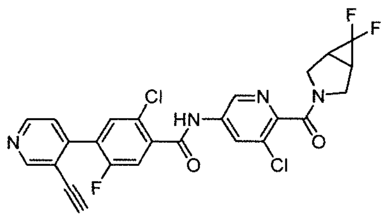 | 2-chloro-N-(5-chloro-6-(6,6-difluoro-3-azabicyclo[3.1.0]hexane-3-carbonyl) pyridin-3-yl)-4-(3-ethynylpyridin-4-yl)-5-fluorobenzamide | 531 |
| Example397 (A397) | 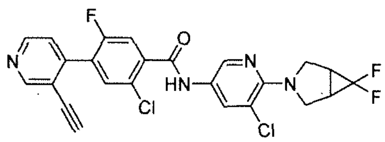 | 2-chloro-N-(5-chloro-6-(6,6-difluoro-3-azabicyclo[3.1.0]hexan-3-yl)pyridin-3-yl)-4-(3-ethynylpyridin-4-yl)-5-fluorobenzamide | 503 |
| Example400 (A400) | 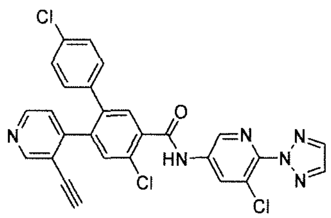 | 4,4'-dichloro-N-(5-chloro-6-(2H-1,2,3-triazol-2-yl)pyridin-3-yl)-6-(3-ethynylpyridin-4-yl)-[1,1'-biphenyl]-3-carboxamide | 545 |
| Example401 (A401) | 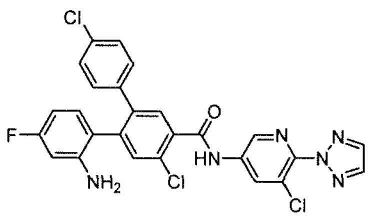 | 2-amino-4",5'-dichloro-N-(5-chloro-6-(2H-1,2,3-triazol-2-yl)pyridin-3-yl)-4-fluoro-[1,1':2',1"-terphenyl]-4'-carboxamide | 553 |
| Example403 (A403) | 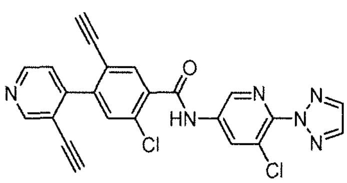 | 2-chloro-N-(5-chloro-6-(2H-1,2,3-triazol-2-yl)pyridin-3-yl)-5-ethynyl-4-(3-ethynylpyridin-4-yl)benzamide | 459 |
| Example406 (A406) | 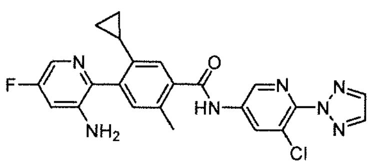 | 4-(3-amino-5-fluoropyridin-2-yl)-N-(5-chloro-6-(2H-1,2,3-triazol-2-yl)pyridin-3-yl)-5-cyclopropyl-2-methylbenzamide | 464 |

(continued)

| Example | Structure | Chemical Name | MS $[M+H]^+$ |
|---|---|---|---|
| Example407 (A407) | 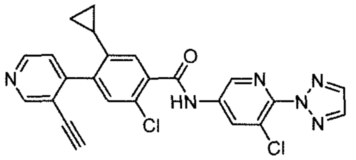 | 2-chloro-N-(5-chloro-6-(2H-1,2,3-triazol-2-yl)pyridin-3-yl)-5-cyclopropyl-4-(3-ethynylpyridin-4-yl)benzamide | 475 |
| Example408 (A408) | 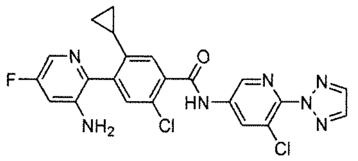 | 4-(3-amino-5-fluoropyridin-2-yl)-2-chloro-N-(5-chloro-6-(2H-1,2,3-triazol-2-yl) pyridin-3-yl)-5-cyclopropylbenzamide | 484 |

Example409: Synthesis of Compound A409

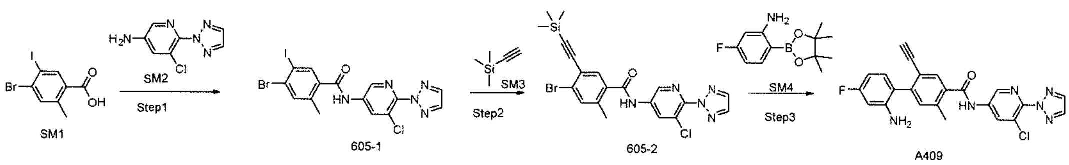


Step1: Synthesis of Compound A605-1

Under an ice-water bath, $POCl_3$ (0.31g,2.0mmoL) was added dropwise to a DCM (10mL) solution containing SM1 (0.34g,1.0mmoL), SM2 (0.20g,1.0mmoL) and pyridine (0.84g,10mmoL). The reaction mixture was stirred at room temperature for 2 hours. After the reaction was completed, the reaction solution was diluted with DCM (50mL), washed with saturated brine (3*50mL). The organic phase was dried over anhydrous sodium sulfate, concentrated and separated using a Flash silica gel column ( PE/EA=2/1) to obtain product 605-1 (0.46g, 0.89mmoL) as yellow solid. LC-MS ($ES^+$): *m/z* 517$[M+H]^+$

Step2: Synthesis of Compound A605-2

SM3 (0.1g,1.0mmoL), 605-1 (0.26g,0.5mmoL), CuI(2.0mg,0.01mmoL), $Pd(PPh_3)_2Cl_2$(3.5mg,0.005mmoL) and TEA(0.3g,3.0mmoL) were added in sequence to a single-neck bottle containing THF (10mL) and a stirrer. The reaction liquid container were replaced with nitrogen. The reaction mixture was stirred at room temperature for 5 hours. After the reaction was completed, the reaction solution was diluted with EA (50mL), washed with saturated brine (3*50mL). The organic phase was dried over anhydrous sodium sulfate, concentrated and separated using a Flash silica gel column ( PE/EA=2/1) to obtain product 605-2 (0.21g,0.4 mmoL) as white solid. LC-MS ($ES^+$): *m/z* 488 $[M+H]^+$

Step3: Synthesis of Compound A409

SM4 (0.14g,0.6mmoL), 605-2 (0.21g,0.4mmoL), $Pd(dppf)Cl_2$ (14.6mg,0.02mmoL) and $K_2CO_3$(0.12g,0.8mmoL) were added in sequence to a single-neck bottle containing dioxane(4mL), $H_2O$ (1mL) and a stirrer. The reaction liquid container were replaced with nitrogen. The reaction mixture was stirred in a 90°C oil bath for 8 hours. After the reaction was completed, the reaction solution was diluted with EA (50mL), washed with saturated brine (3*50mL). The organic phase was dried over anhydrous sodium sulfate, concentrated and separated using a Flash silica gel column (PE/EA=2/1) to obtain product A409 (0.11g,0.24 mmoL) as white solid. LC-MS ($ES^+$): *m/z* 447 $[M+H]^+$

Example412: Synthesis of Compound A412

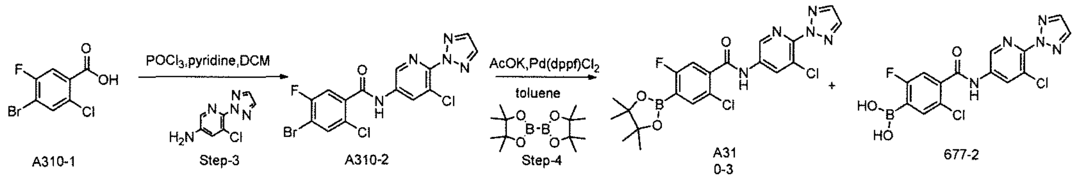


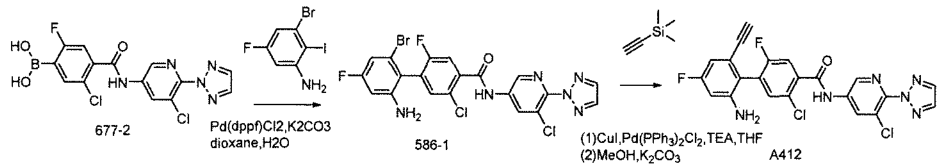


For the synthesis method of 677-2, please refer to the synthesis of A310-3.

Step1: Synthesis of Compound A586-1

3-bromo-5-fluoro-2-iodoaniline (0.16g,0.5mmoL), 677-2 (0.12g,0.3mmoL), Pd(dppf)$Cl_2$ (14.6mg,0.02mmoL) and $K_2CO_3$ (0.12g,0.8mmoL) were added in sequence to a single-neck bottle containing dioxane(4mL), $H_2O$ (1mL) and a stirrer. The reaction liquid container were replaced with nitrogen. The reaction mixture was stirred in a 90°C oil bath for 8 hours. After the reaction was completed, the reaction solution was diluted with EA (50mL), washed with saturated brine (3*50mL). The organic phase was dried over anhydrous sodium sulfate, concentrated and separated using a Flash silica gel column ( PE/EA=2/1) to obtain product 586-1 (0.065g,0.12 mmoL) as white solid. LC-MS ($ES^+$): *m/z* 538/540 $[M+H]^+$

Step 2: Synthesis of Compound A412

ethynyltrimethylsilane (0.06g,0.6mmoL), 586-1 (0.065g,0.12mmoL), CuI (2.0mg,0.01mmoL), $Pd(PPh_3)_2Cl_2$ (3.5mg,0.005mmoL) and TEA (0.3g,3.0mmoL) were added in sequence to a single-neck bottle containing THF (4mL) and a stirrer. The reaction liquid container were replaced with nitrogen. The reaction mixture was stirred at room temperature for 5 hours, and then $K_2CO_3$ (0.42g, 3mmoL) and MeOH (10mL) were added to the reaction mixture and stirring was continued for 2 hours. After the reaction was completed, the reaction solution was diluted with EA (50mL), washed with saturated brine (3*50mL). The organic phase was dried over anhydrous sodium sulfate, concentrated and separated using a Flash silica gel column ( PE/EA=2/1) to obtain product A412 (0.05g,0.1 mmoL) as white solid. LC-MS ($ES^+$): *m/z* 485$[M+H]^+$

Example413: Synthesis of Compound A413

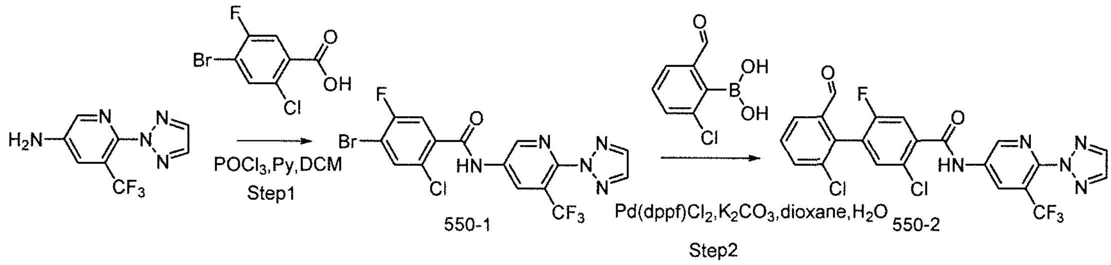


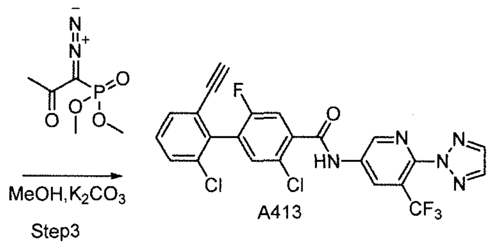


Step 1: Synthesis of Compound A550-1

Under an ice-water bath, $POCl_3$ (0.31g,2.0mmoL) was added dropwise to a DCM (10mL) solution containing 4-bromo-2-chloro-5-fluorobenzoic acid (0.25g, 1.0mmoL), 6-(2H-1,2,3-triazol-2-yl)-5-(trifluoromethyl)pyridine- 3-amine (0.20g, 1.0mmoL) and pyridine (0.84g, 10mmoL). The reaction mixture was stirred at room temperature for 2 hours. After the reaction was completed, the reaction solution was diluted with DCM (50mL), washed with saturated brine (3*50mL). The organic phase was dried over anhydrous sodium sulfate, concentrated and separated using a Flash silica gel column ( PE/EA=2/1) to obtain product 550-1 (0.42g,0.9mmoL) as white solid. LC-MS ($ES^+$): *m/z* 463/465 $[M+H]^+$

Step 2: Synthesis of Compound A550-2

(2-chloro-6-formylphenyl)boronic acid (0.092g,0.5mmoL), 550-1 (0.092g,0.3mmoL), $Pd(dppf)Cl_2$ (14.6mg,0.02mmoL) and $K_2CO_3$ (0.12g,0.8mmoL) were added in sequence to a single-neck bottle containing dioxane(4mL), $H_2O$ (1mL) and a stirrer. The reaction liquid container were replaced with nitrogen. The reaction mixture was stirred in a 90°C oil bath for 8 hours. After the reaction was completed, the reaction solution was diluted with EA (50mL), washed with saturated brine (3*50mL). The organic phase was dried over anhydrous sodium sulfate, concentrated and separated using a Flash silica gel column ( PE/EA=2/1) to obtain product 550-2 (0.11g, 0.21 mmoL) as white solid. LC-MS ($ES^+$): *m/z* 524$[M+H)^+$

Step 3: Synthesis of Compound A413

(1-dizo-2-oxopropyl)phosphonate dimethyl ester (0.11g,0.5mmoL), 550-2 (0.11g,0.21mmoL) and $K_2CO_3$ (0.12g,0.8mmoL) were added in sequence to a single-neck bottle containing MeOH (4mL) and a stirrer. The reaction mixture was stirred at room temperature for 4 hours. After the reaction was completed, the reaction solution was diluted with EA (50mL), washed with saturated brine (3*50mL). The organic phase was dried over anhydrous sodium sulfate, concentrated and separated using a Flash silica gel column ( PE/EA=2/1) to obtain product A413 (0.08g,0.15 mmoL) as white solid. LC-MS ($ES^+$): *m/z* 520$[M+H]^+$

Example415: Synthesis of Compound A415

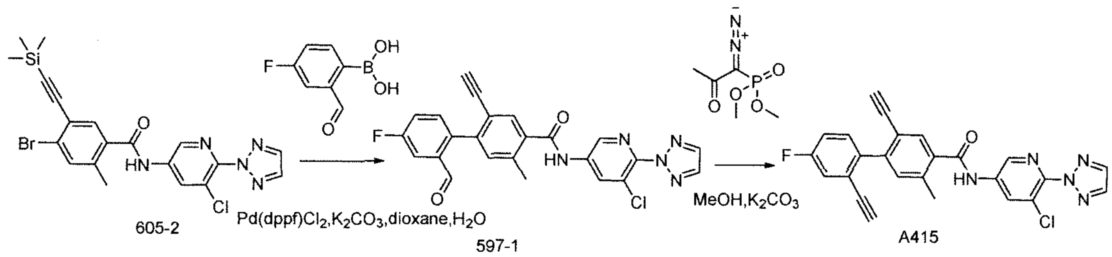


Step 1: Synthesis of Compound A587-1

(4-fluoro-2-formylphenyl)boronic acid (0.09g,0.5mmoL), 605-2 (0.21g,0.4mmoL), $Pd(dppf)Cl_2$ (14.6mg,0.02mmoL) and $K_2CO_3$ (0.12g,0.8mmoL) were added in sequence to a single-neck bottle containing dioxane(4mL), $H_2O$ (1mL) and a stirrer. The reaction liquid container were replaced with nitrogen. The reaction mixture was stirred in a 90°C oil bath for 8 hours. After the reaction was completed, the reaction solution was diluted with EA (50mL), washed with saturated brine (3*50mL). The organic phase was dried over anhydrous sodium sulfate, concentrated and separated using a Flash silica gel column ( PE/EA=2/1) to obtain product 597-1 (0.09g,0.2 mmoL) as white solid. LC-MS ($ES^+$): *m/z* 460$[M+H]^+$

Step 2: Synthesis of Compound A415

(1-dizo-2-oxopropyl)phosphonate dimethyl ester (0.11g,0.5mmoL), 597-1 (0.09g,0.2mmoL) and $K_2CO_3$ (0.12g,0.8mmoL) were added in sequence to a single-neck bottle containing MeOH (4mL) and a stirrer. The reaction mixture was stirred at room temperature for 4 hours. After the reaction was completed, the reaction solution was diluted with EA (50mL), washed with saturated brine (3*50mL). The organic phase was dried over anhydrous sodium sulfate, concentrated and separated using a Flash silica gel column ( PE/EA=2/1) to obtain product A415 (0.06g,0.15 mmoL) as white solid. LC-MS ($ES^+$): *m/z* 456$[M+H]^+$

Example423: Synthesis of Compound A423

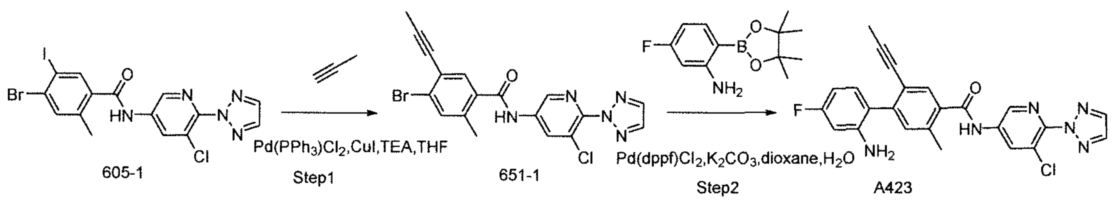


Step 1: Synthesis of Compound A651-1

propyne (0.1g,2.5mmoL), 605-1 (0.26g,0.5mmoL), CuI (2.0mg,0.01mmoL), $Pd(PPh_3)_2Cl_2$ (3.5mg,0.005mmoL) and TEA(0.3g,3.0mmoL) were added in sequence to a single-neck bottle containing THF(10mL) and a stirrer. The reaction liquid container were replaced with nitrogen. The reaction mixture was stirred at room temperature for 5 hours. After the reaction was completed, the reaction solution was diluted with EA (50mL), washed with saturated brine (3*50mL). The organic phase was dried over anhydrous sodium sulfate, concentrated and separated using a Flash silica gel column ( PE/EA=2/1) to obtain product 651-1(0.13g,0.3 mmoL) as white solid. LC-MS ($ES^+$): *m/z* 430/432$[M+H]^+$

Step 2: Synthesis of Compound A423

5-fluoro-2-(4,4,5,5-tetramethyl-1,3,2-dioxaborolan-2-yl)aniline (0.12g,0.5mmoL), 651-1(0.13g,0.3mmoL), $Pd(dppf)Cl_2$ (14.6mg,0.02mmoL) and $K_2CO_3$ (0.12g,0.8mmoL) were added in sequence to a single-neck bottle containing dioxane(4mL), $H_2O$ (1mL) and a stirrer. The reaction liquid container were replaced with nitrogen. The reaction mixture was stirred in a 90°C oil bath for 8 hours. After the reaction was completed, the reaction solution was diluted with EA (50mL), washed with saturated brine (3*50mL). The organic phase was dried over anhydrous sodium sulfate, concentrated and separated using a Flash silica gel column ( PE/EA=2/1) to obtain product A423 (0.09g,0.2 mmoL) as white

solid. LC-MS ($ES^+$): *m/z* 461 $[M+H]^+$

Example425: Synthesis of Compound A425

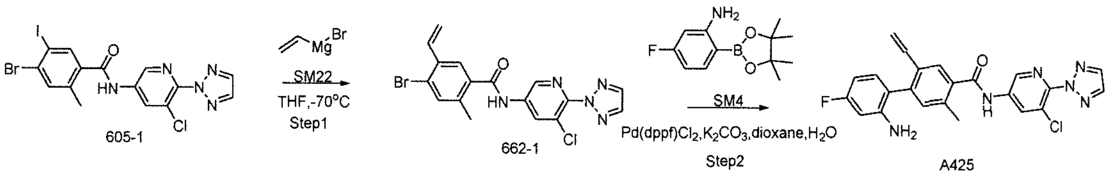

605-1
662-1
A425

Step 1: Synthesis of Compound A662-1

Vinyl magnesium bromide (2.0 mL, 2.0 mmoL) was added dropwise to a THF (10mL) solution containing 605-1 (0.52g,1.0mmoL) at -70°C. The reaction mixture was stirred at -70°C for 2 hours. After the reaction was completed, the reaction solution was quenched saturated ammonium chloride solution (30mL) and extracted with DCM (50mL). The organic phase was washed with saturated brine (3*50mL) and dried over anhydrous sodium sulfate, concentrated and separated using a Flash silica gel column ( PE/EA=2/1) to obtain product 662-1 (0.28g,0.7mmoL) as white solid. LC-MS ($ES^+$): *m/z* 418/420$[M+H]^+$

Step 2: Synthesis of Compound A425

5-fluoro-2-(4,4,5,5-tetramethyl-1,3,2-dioxaborolan-2-yl)aniline (0.14g,0.6mmoL), 662-1 (0.17g,0.4mmoL), $Pd(dppf)Cl_2$ (14.6mg,0.02mmoL) and $K_2CO_3$ (0.12g,0.8mmoL) were added in sequence to a single-neck bottle containing dioxane(4mL), $H_2O$ (1mL) and a stirrer. The reaction liquid container were replaced with nitrogen. The reaction mixture was stirred in a 90°C oil bath for 8 hours. After the reaction was completed, the reaction solution was diluted with EA (50mL), washed with saturated brine (3*50mL). The organic phase was dried over anhydrous sodium sulfate, concentrated and separated using a Flash silica gel column ( PE/EA=2/1) to obtain product A425 (0.12g,0.3 mmoL) as white solid. LC-MS ($ES^+$): *m/z* 449 $[M+H]^+$.

Example510 and 511: Synthesis of Compound A510 and A511

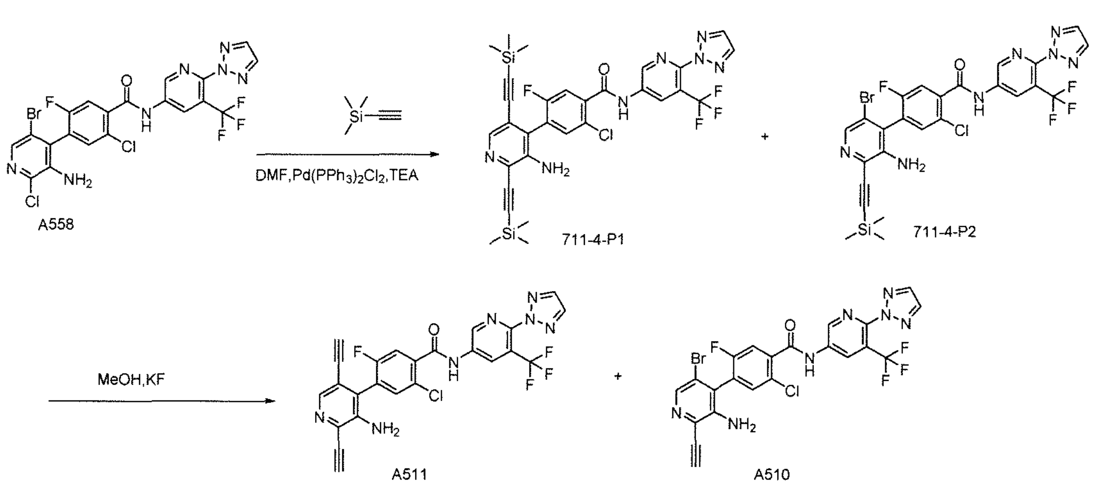

A558
711-4-P1
+
711-4-P2
A511
+
A510

Step 1: Synthesis of Compound A711-4-P1 and A711-4-P2

Into a 25-mL round-bottom flask, was placed A558 (100 mg, 0.17 mmol), ethynyltrimethylsilane (16.62 mg, 0.17 mmol), $Pd(PPh_3)_2Cl_2$ (11.86 mg, 0.017 mmol), TEA (34.24 mg, 0.05mmol), DMF (2 mL). The resulting mixture was stirred at 80°C for 16 hours under nitrogen. Removing of the solvent and purifying by column chromatography (PE/EA=2/1) to give compound 711-4-P1 (LC-MS ($ES^+$): *m/z* 470$[M+H]^+$) and 711-4-P2(LC-MS ($ES^+$): *m/z* 652/654 $[M+H]^+$) mixture

(40 mg) as white solid.

Step 2: Synthesis of Compound A510 and A511

To a solution of 711-4-P1 and 711-4-P2 (40 mg) in MeOH(5.0mL) was added KF (3.56 mg, 0.06 mmol). The resulting mixture was stirred at 50 °C for 2 hours, solvent was removed in vacuo and the mixture was purified by pre-HPLC to give compound A511 (1.1 mg) (LC-MS ($ES^+$): *m/z* 526 $[M+H]^+$)and A510 (1.3 mg) (LC-MS ($ES^+$): *m/z* 579/581 $[M+H]^+$)as white solids.

Example520: Synthesis of Compound A520

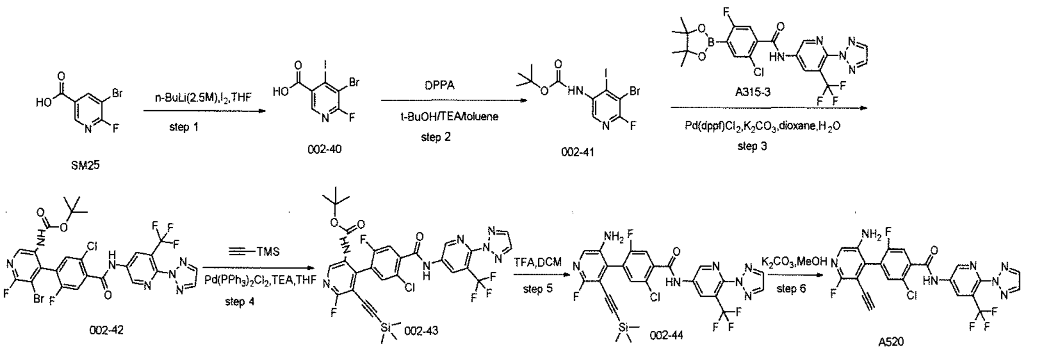


Step 1: Synthesis of Compound A002-40

Into a 250-mL three-neck flask, 2,2,6,6-tetramethylpiperidine (3.98g, 28.18mmol) was dissolved in THF (20mL) under nitrogen, and n-butyl lithium (2.5mol/L in THF, 10.9ml 27.27mmol) was dropwised at -50□. Keeping -50□ for half an hour after dropwise, a solution of 5-bromo-6-fluoronicotinic acid (2.00g, 9.09mmol) in THF (20mL) was dropwised, slowly raising the temperature to -20□, keeping at -20□ for 1h ,cooling the reaction to -60□, $I_2$ (3.46g, 13.63mmol) dissolved in THF(20.0mL) was dropwised. After the reaction completed, it is diluted with 100mL of water and extracted with EA (3*30mL). Keep the water phase,adjust the pH to 3 with 3N HCl,then extract with EA (3*50mL),The organic phase wash with saturated brine (3*30mL), dry with anhydrous sodium sulfate and concentrate to obtain the product 002-40 (1.5 g, 4.35mmol) as a red solid. LC-MS ($ES^+$): *m/z* 345/347 $[M+H]^+$

Step 2: Synthesis of Compound A002-41

To a solution of 002-40 (1.5g, 4.35mmol) in toluene(15.0mL) and tert-butanol (15mL)was added DPPA (1.794g, 6.52mmol) and TEA (1.318g, 13.05mmol). The resulting mixture was stirred at 110 °C for 2 hours under nitrogen, solvent was removed in vacuo and the mixture was separated on a silica gel column with PE:EA=96:4 to give compound 002-41 (1.5 g, 3.59mmol) as a brown solid. LC-MS ($ES^+$): *m/z* 416/418 $[M+H]^+$

Step 3: Synthesis of Compound A002-42

To 002-41 (0.6 g,1.44 mmol) in 1,4-dioxane(6.0mL) and $H_2O$ (1mL)was added A315-3 (1.104g,2.16mmoL), $Pd(dppf)Cl_2$ (105.29mg,0.15mmoL) and $K_2CO_3$ (0.39g,2.88 mmoL). The resulting mixture was stirred at 80 °C for 16 hours under nitrogen. Water was added, and the organic layer was extracted with EtOAc(3*20mL).The organic layers were combined,washed with brine (3*50mL), dried over $Na_2SO_4$, and filtered.The filter was under reduced pressure to give compound 002-42 (0.13g,0.19 mmoL) as white solid. LC-MS ($ES^+$): *m/z* 674/676 $[M+H]^+$

Step 4: Synthesis of Compound A002-43

To 002-42 (70 mg,0.1 mmol) in THF(2.0mL) was added ethynyltrimethylsilane (12.25 mg, 0.12 mmol), $Pd(PPh_3)_2Cl_2$ (7.3 mg, 0.01 mmol) and TEA (20.99 mg, 0.21mmol). The resulting mixture was stirred at 80 °C for 3 hours under nitrogen. Water was added, and the organic layer was extracted with EtOAc(3*20mL).The organic layers were combined,washed with brine (3*50mL), dried over $Na_2SO_4$, and filtered. The filter was under reduced pressure

and the mixture was separated on a silica gel column with PE:EA=2/1 to give compound 002-43 (85 mg, 0.12 mmol) as yellow oil. LC-MS ($ES^+$): *m/z* 692 $[M+H]^+$

Step 5: Synthesis of Compound A002-44

To 002-43 (85 mg, 0.12 mmol) in DCM(2.0mL) was added TFA (0.14mL). The resulting mixture was stirred at room temperature for 2 hours, after the reaction completed, it is diluted with EA (20mL).The mixture was adjusted the pH to 9 with saturated $NaHCO_3$ solution,then extract with EA (2*30mL),The organic phase wash with saturated brine (3*30mL), dry with anhydrous sodium sulfate and and filtered.The filter was under reduced pressure to give compound 002-44 (70mg, 0.12mmol) as yellow solid. LC-MS ($ES^+$): *m/z* 592 $[M+H]^+$

Step 6: Synthesis of Compound A520

To 002-44 (60 mg, 0.1 mmol) in MeOH(2.0mL) was added $K_2CO_3$ (19.60 mg, 0.14 mmol). The resulting mixture was stirred at 50 °C for 10 minutes, solvent was removed in vacuo and the mixture was separated on pre-HPLC to give compound A520 (12 mg, 0.023 mmol) as white solid. LC-MS ($ES^+$): *m/z* 520 $[M+H]^+$

Example553: Synthesis of Compound A553

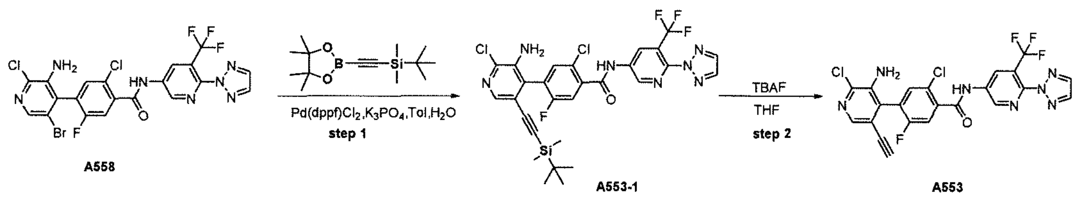

A558 A553-1 A553

Step 1: Synthesis of Compound A553-1

To A558 (60.0mg, 0.102mmol) in toluene(10.0mL) and $H_2O$ (1.0mL)was added tert-butyldimethyl ((4,4,5,5-tetramethyl-1,3 ,2-dioxobenzaldehyde-2-yl)ethynyl)silane (50.0mg, 0.36mmol), $[PdCl_2(dppf)]CH_2Cl_2$ (30mg, 0.037mmol)and $K_2CO_3$ (0.39g,2.88 mmol). The resulting mixture was stirred at 95 °C for 16 hours under nitrogen. Water was added, and the organic layer was extracted with EtOAc(3*20mL).The organic layers were combined,washed with brine (3*50mL), dried over $Na_2SO_4$, and filtered.The filter was under reduced pressure to give compound A553-1 (35mg, 0.054mmol) as white solid. LC-MS ($ES^+$): *m/z* 650.25 $[M+H]^+$

Step 2: Synthesis of Compound A553

To A553-1 (200 mg, 0.308 mmol) in THF(10.0mL) was added TBAF (0.1M in THF,1.0 ml ). The resulting mixture was stirred at room temperature for 30 minutes, solvent was removed in vacuo and the mixture was separated on pre-HPLC to give compound A553 (92 mg, 0.0.172mmol) as white solid. LC-MS ($ES^+$): *m/z* 536.10 $[M+H]^+$ .

$^1H$ NMR (500 MHz, DMSO-$d_6$) $\delta$ 11.57 (s, 1H), 9.16 (d, *J* = 2.4 Hz, 1H), 8.91 (d, *J* = 2.4 Hz, 1H), 8.22 (s, 2H), 7.90 - 7.84 (m, 2H), 7.70 (d, *J* = 6.1 Hz, 1H), 5.59 (s, 2H), 4.33 (s, 1H).

Example555: Synthesis of Compound A555

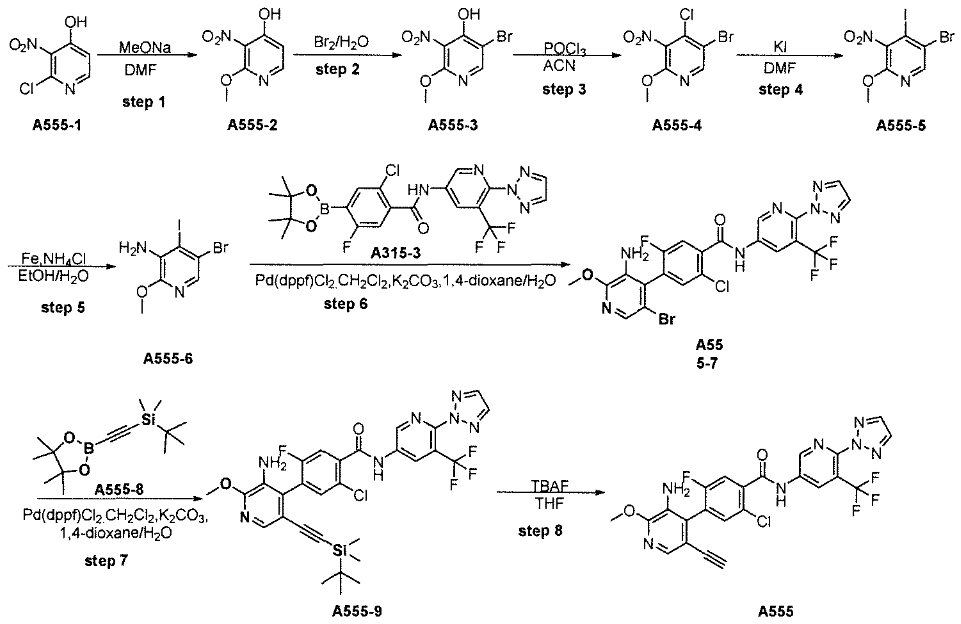


Step 1 : Synthesis of Compound A555-2

To A555-1(1.00 g, 5.73 mmol) in DMF(10.0mL) was added sodium methoxide (5.16 g, 30%wt, 28.65 mmol). The resulting mixture was stirred at 100 °C for 2.5 hours. Water was added, and the organic layer was extracted with EtOAc(3*20mL).The organic layers were combined,washed with brine (3*50mL), dried over $Na_2SO_4$, and filtered.The filter was under reduced pressure to give compound A555-2 (0.90 g, 92.34% yield) as a yellow powder. LC-MS ($ES^+$): *m/z* 171.0 $[M+H]^+$ .

Step2: Synthesis of Compound A555-3

To A555-2 (0.500 g, 2.94 mmol) in glacial acetic acid ( 10.0mL) was added a solution of bromine (0.17 mL, 3.23 mmol) in glacial acetic acid (5 mL) slowly at room temperature. The resulting mixture was stirred at room temperature for 16 hours. Water was added,the mixture is filtered, collect the filter cake and dry to give compound A555-3 (465 mg, 63.53% yield) as a yellow solid. LC-MS ($ES^+$): *m/z* 249/251 $[M+H]^+$ .

Step3: Synthesis of Compound A555-4

To A555-3(115 mg, 0.46 mmol) in acetonitrile (2.0mL) was added phosphorus oxychloride (0.22 mL, 2.31 mmol) at room temperature. The resulting mixture was stirred at 85 °C for 2 hours. Water was added,the mixture is filtered, collect the filter cake and dry to give compound A555-4 (55 mg, 44.53% yield) as a yellow solid. LC-MS ($ES^+$): *m/z* 249/251 $[M+H]^+$ .

Step4: Synthesis of Compound A555-5

To A555-4 (200 mg, 0.75 mmol) in DMF(5.0mL) was added potassium iodide (1.24 g, 7.48 mmol). The resulting mixture was stirred at 100 °C for 5 hours. Water was added, and the organic layer was extracted with EtOAc(3*20mL).The organic layers were combined,washed with brine (3*50mL), dried over $Na_2SO_4$, and filtered. The filter was under reduced pressure the mixture was separated on a silica gel column with PE:EA=1/1 to give compound A555-5 (78 mg, 29.06% yield) as a yellow solid. LC-MS ($ES^+$): *m/z* 359/361 $[M+H]^+$ .

StepS: Synthesis of Compound A555-6

To A555-5 (240 mg, 0.67 mmol) in EtOH/H2O (5 mL/1 mL) was added Fe(187 mg, 3.34 mmol) and $NH_4Cl$ (179 mg, 3.34 mmol). The resulting mixture was stirred at 60°C for 4 hours..The mixture was filtered. The filter was under reduced pressure the mixture was separated on a silica gel column with PE:EA=3/2 to give compound A555-6 (185 mg, 71.49% yield) as a yellow solid. LC-MS ($ES^+$): *m/z* 329/331 $[M+H]^+$ .

Step6: Synthesis of Compound A555-7

To A558 (60.0mg, 0.102mmol) in 1,4-dioxane/water (SmL/1mL) was added A315-3 (418 mg, 0.97 mmoL), $Pd(dppf)Cl_2$ (60 mg, 0.07mmoL) and $K_2CO_3$ (134 mg , 0.97 mmoL). The resulting mixture was stirred at 80 °C for 2 hours under nitrogen. Water was added, and the organic layer was extracted with EtOAc(3*20mL).The organic layers were combined,washed with brine (3*50mL), dried over $Na_2SO_4$, and filtered. The filter was under reduced pressure and the mixture separated on a silica gel column with PE:EA=0/1 to give compound A555-7 (120 mg, 42.05% yield) as a yellow solid. LC-MS ($ES^+$): *m/z* 586/588 $[M+H]^+$

Step7: Synthesis of Compound A555-9

To A555-7 (100 mg, 0.17 mmol) in 1,4-dioxane/water (2mL/0.2mL) was added A555-8 (68 mg, 0.26 mmoL), $Pd(dppf)Cl_2.CH_2Cl_2$ (28 mg, 0.03mmoL) and $K_2CO_3$ ( 71 mg, 0.51 mmoL). The resulting mixture was stirred at 80 °C for 6 hours under nitrogen. Water was added, and the organic layer was extracted with EtOAc(3*20mL).The organic layers were combined,washed with brine (3*50mL), dried over $Na_2SO_4$, and filtered. The filter was under reduced pressure and the mixture separated on a silica gel column with DCM/MeOH=20/1 to give compound A555-9 (25 mg, 22.71% yield) as a yellow solid. LC-MS ($ES^+$): *m/z* 646 $[M+H]^+$

Step8: Synthesis of Compound A555

To A555-9 (25 mg, 0.04 mmol) in THF(1.0mL) was added TBAF (0.1M in THF,1.0 ml ). The resulting mixture was stirred at room temperature for 60 minutes, solvent was removed in vacuo and the mixture was separated on pre-HPLC to give compound A555 (6.6 mg, 32.07%) as a yellow solid. LC-MS ($ES^+$): *m/z* 532$[M+H]^+$ . $^1$H NMR (500 MHz, DMSO-$d_6$) $\delta$ 11.50 (s, 1H), 9.17 (d, *J* = 2.4 Hz, 1H), 8.89 (d, *J* = 2.4 Hz, 1H), 8.22 (s, 2H), 7.89 (d, *J* = 9.0 Hz, 2H), 7.74 (d, *J* = 6.0 Hz, 1H), 5.82 (s, 2H), 4.08 (s, 1H), 3.83 (s, 3H).

Example558: Synthesis of Compound A558

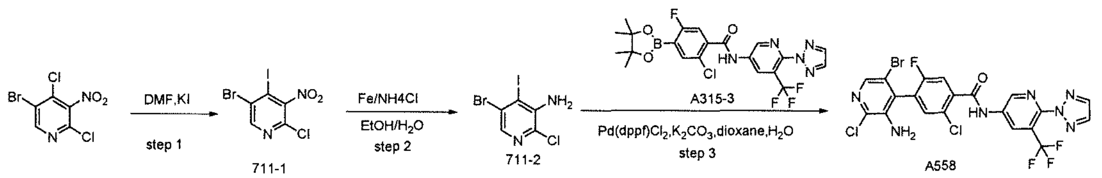


Step 1: Synthesis of Compound A711-1

To 5-bromo-2,4-dichloro-3-nitropyridine (2 g, 7.35 mmol) in DMF(20.0mL) was added KI (3.67 g, 22.05 mmol). The resulting mixture was stirred at 100 °C for 16 hours. Water was added, the mixture is filtered, the filter cake wash with ice water (3*10 mL),collect the filter cake and dry to give compound 771-1 (2.7 g, 7.43 mmol) as a gray solid. LC-MS ($ES^+$): *m/z* 362/364 $[M+H]^+$ .

Step 2: Synthesis of Compound A711-2

To 711-1 (2.7 g, 7.43 mmoL) in EtOH(20.0mL) and H2O(20.0mL) was added Fe2.08 g, 37.2mmoL) and $NH_4Cl$ (2.0 g, 37.2 mmol). The resulting mixture was stirred at 40°C for 3 hours.The mixture was filtered. The filter was extracted with EtOAc(3*50mL).The organic layers were combined,washed with saturated sodium bicarbonate solution(100ml) and brine (100mL), dried over $Na_2SO_4$, and filtered.The filter was under reduced pressure and the mixture separated on a silica gel column with PE/EA=90/10 to give compound 711-2 (1.2 g, 3.6 mmoL) as a white solid. LC-MS ($ES^+$): *m/z* 332/334 $[M+H]^+$ .

Step 3: Synthesis of Compound A558

To A315-3 (2.76g,5.4mmoL) in 1,4-dioxane/water (30mL/3.0mL) was added 711-2 (1.2 g,3.6 mmoL), $Pd(dppf)Cl_2$ (26.35mg,0.036mmoL) and $K_2CO_3$ (0.99 g,7.2 mmoL). The resulting mixture was stirred at 80 °C for 16 hours under nitrogen. Water was added, and the organic layer was extracted with EtOAc(3*20mL).The organic layers

were combined,washed with brine (3*50mL), dried over $Na_2SO_4$, and filtered.The filter was under reduced pressure and the mixture separated on a silica gel column with DCM/EA=3/1 to give compound A558 (0.48g, 0.8 mmoL) as white solid. LC-MS ($ES^+$): *m*/*z* 589/591 $[M+H]^+$ .

Example559 and 560: Synthesis of Compound A559 and A560

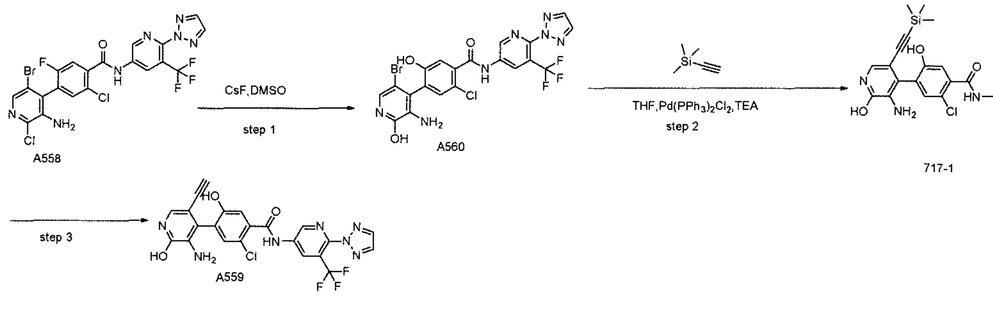


Step 1: Synthesis of Compound A560

To A558 (100 mg, 0.17mmol) in DMSO (2.0mL) was added CSF (51.40 mg, 0.34mmol). The resulting mixture was stirred at 130 °C for 16 hours. Water was added, and the organic layer was extracted with EtOAc(3*20mL).The organic layers were combined,washed with brine (3*50mL), dried over $Na_2SO_4$, and filtered. The filter was under reduced pressure to give compound A560 (15 mg,0.026mmol) as white solid. LC-MS ($ES^+$): *m*/*z* 569/571 $[M+H]^+$.

Step 2: Synthesis of Compound A717-1

To A560 (15 mg,0.17 mmol) in THF(2.0mL) was added ethynyltrimethylsilane (2.6 mg, 0.026 mmol), $Pd(PPh_3)_2Cl_2$ (1.8 mg, 0.0026 mmol) and TEA (5.3mg, 0.05mmol). The resulting mixture was stirred at 80 °C for 16 hours under nitrogen. Water was added, and the organic layer was extracted with EtOAc(3*10mL).The organic layers were combined,washed with brine (3*10mL), dried over $Na_2SO_4$, and filtered.The filter was under reduced pressure to give compound 717-1 (20 mg,0.03mmol) as white solid. LC-MS ($ES^+$): *m*/*z* 588 $[M+H]^+$ .

Step 3: Synthesis of Compound A559

To 717-1 (20 mg,0.03) in MeOH(1.0mL) was added $K_2CO_3$ (9.40 mg, 0.07 mmol). The resulting mixture was stirred at 50 °C for 2 hours, solvent was removed in vacuo and the mixture was separated on pre-HPLC to give compound A559 (2.0 mg)as a white solid. LC-MS ($ES^+$): *m*/*z* 516 $[M+H]^+$ .

According to the synthesis method of the above example, select appropriate raw materials and/or marketed example intermediates to synthesize the example compounds in the table below. The raw materials and reagents used are all commercially available.

| Example | Structure | Chemical Name | MS [M+H]+ |
|---|---|---|---|
| Example4 10 (A410) | 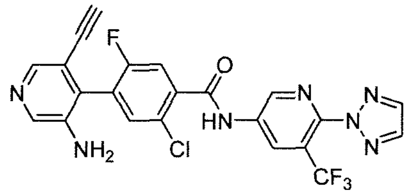 | N-(6-(2H-1,2,3-triazol-2-yl)-5-(trifluoromethyl)pyridin-3-yl)-4-(3-amino-5-ethynylpyridin-4-yl)-2-chloro-5-fluorobenzamide | 502 |
| Example411 (A411) | 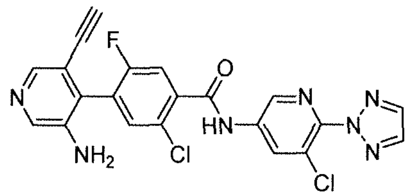 | 4-(3-amino-5-ethynylpyridin-4-yl)-2-chloro-N-(5-chloro-6-(2H-1,2,3-triazol-2-yl)pyridin-3-yl)-5-fluorobenzamide | 468 |
| Example414 (A414) | 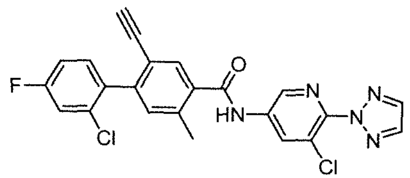 | 2'-chloro-N-(5-chloro-6-(2H-1,2,3-triazol-2-yl)pyridin-3-yl)-2-ethynyl-4'-fluoro-5-methyl-[1,1'-biphenyl]-4-carboxamide | 466 |
| Example416 (A416) | 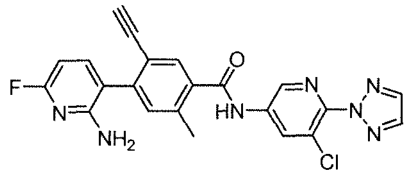 | 4-(2-amino-6-fluoropyridin-3-yl)-N-(5-chloro-6-(2H-1,2,3-triazol-2-yl)pyridin-3-yl)-5-ethynyl-2-methylbenzamide | 448 |
| Example417 (A417) | 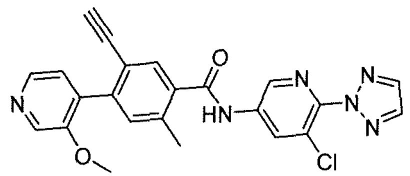 | N-(5-chloro-6-(2H-1,2,3-triazol-2-yl)pyridin-3-yl)-5-ethynyl-4-(3-methoxypyridin-4-yl)-2-methylbenzamide | 445 |

(continued)

| Example | Structure | Chemical Name | MS $[M+H]^+$ |
|---|---|---|---|
| Example418 (A418) | 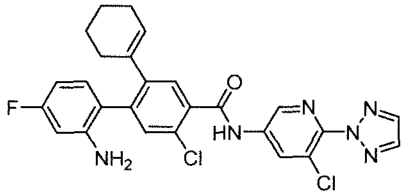 | 2-amino-5'-chloro-N-(5-chloro-6-(2H-1,2,3-triazol-2-yl)pyridin-3-yl)-4-fluoro-2",3",4",5"-tetrahydro-[1,1':2',1"-terphenyl]-4'-carboxamide | 523 |
| Example419 (A419) | 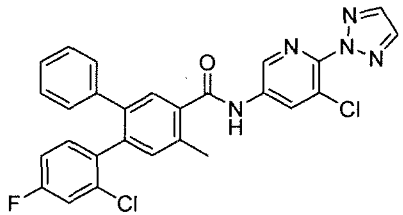 | 2-chloro-N-(5-chloro-6-(2H-1,2,3-triazol-2-yl)pyridin-3-yl)-4-fluoro-5'-methyl-[1,1':2',1"-terphenyl]-4'-carboxamide | 518 |
| Example420 (A420) | 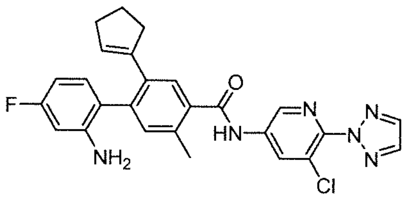 | 2'-amino-N-(5-chloro-6-(2H-1,2,3-triazol-2-yl)pyridin-3-yl)-2-(cyclopent-1-en-1-yl)-4'-fluoro-5-methyl-[1,1'-biphenyl]-4-carboxamide | 489 |
| Example421 (A421) | 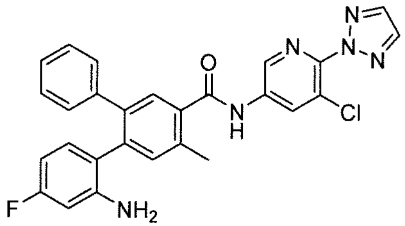 | 2-amino-N-(5-chloro-6-(2H-1,2,3-triazol-2-yl)pyridin-3-yl)-4-fluoro-5'-methyl-[1,1':2',1"-terphenyl]-4'-carboxamide | 499 |

(continued)

| Example | Structure | Chemical Name | MS $[M+H]^+$ |
|---|---|---|---|
| Example422 (A422) | 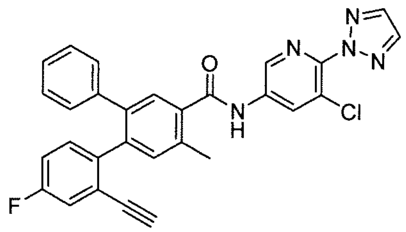 | N-(5-chloro-6-(2H-1,2,3-triazol-2-yl)pyridin-3-yl)-2-ethynyl-4-fluoro-5'-methyl-[1,1':2',1"-terphenyl]-4'-carboxamide | 508 |
| Example424 (A424) | 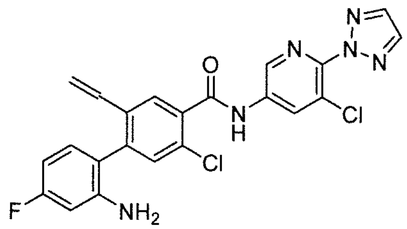 | 2'-amino-5-chloro-N-(5-chloro-6-(2H-1,2,3-triazol-2-yl)pyridin-3-yl)-4'-fluoro-2-vinyl-[1,1'-biphenyl]-4-carboxamide | 469 |
| Example426 (A426) | 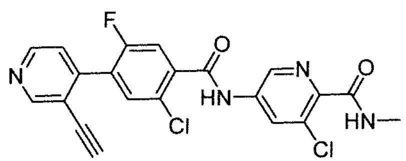 | 3-chloro-5-(2-chloro-4-(3-ethynylpyridin-4-yl)-5-fluorobenzamido)-N-methylpicolinamide | 443 |
| Example427 (A427) | 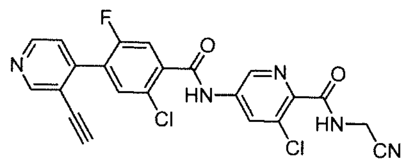 | 3-chloro-5-(2-chloro-4-(3-ethynylpyridin-4-yl)-5-fluorobenzamido)-N-(cyanomethyl) picolinamide | 468 |
| Example428 (A428) | 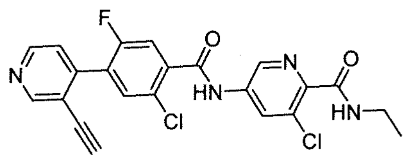 | 3-chloro-5-(2-chloro-4-(3-ethynylpyridin-4-yl)-5-fluorobenzamido)-N-ethylpicolinamide | 457 |

(continued)

| Example | Structure | Chemical Name | MS [M+H]+ |
|---|---|---|---|
| Example429 (A429) | 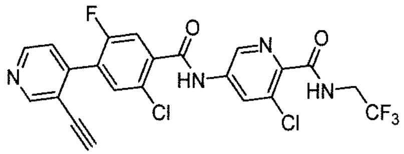 | 3-chloro-5-(2-chloro-4-(3-ethynylpyridin-4-yl)-5-fluorobenzamido)-N-(2,2,2-trifluoroethyl) picolinamide | 511 |
| Example430 (A430) | 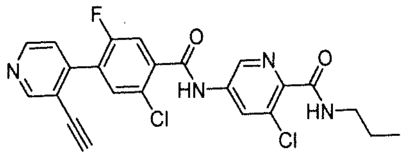 | 3-chloro-5-(2-chloro-4-(3-ethynylpyridin-4-yl)-5-fluorobenzamido)-N-propylpicolinamide | 471 |
| Example431 (A431) | 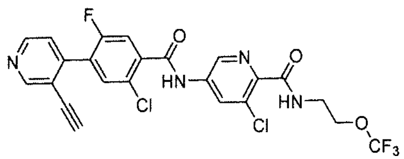 | 3-chloro-5-(2-chloro-4-(3-ethynylpyridin-4-yl)-5-fluorobenzamido)-N-(2-(trifluoromethoxy) ethyl)pico linamide | 541 |
| Example432 (A432) | 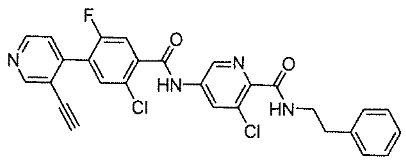 | 3-chloro-5-(2-chloro-4-(3-ethynylpyridin-4-yl)-5-fluorobenzamido)-N-phenethylpicolinamide | 533 |
| Example433 (A433) | 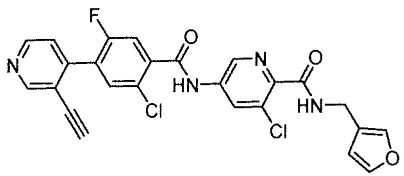 | 3-chloro-5-(2-chloro-4-(3-ethynylpyridin-4-yl)-5-fluorobenzamido)-N-(furan-3-ylmethyl) picolinamide | 509 |
| Example434 (A434) | 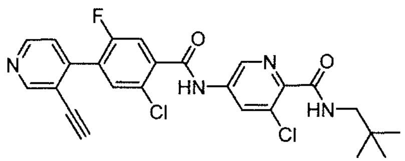 | 3-chloro-5-(2-chloro-4-(3-ethynylpyridin-4-yl)-5-fluorobenzamido)-N-neopentylpicolinamide | 499 |

(continued)

| Example | Structure | Chemical Name | MS [M+H]+ |
|---|---|---|---|
| Example435 (A435) | 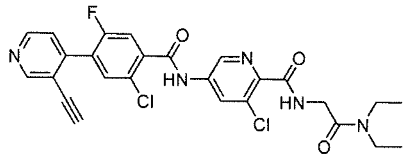 | 3-chloro-5-(2-chloro-4-(3-ethynylpyridin-4-yl)-5-fluorobenzamido)-N-(2-(diethylamino)-2-oxoethyl)picolinamide | 542 |
| Example436 (A436) | 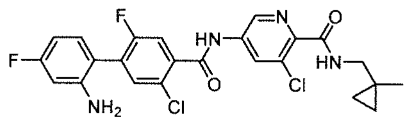 | 5-(2'-amino-5-chloro-2,4'-difluoro-[1,1'-biphenyl]-4-carboxamido)-3-chloro-N-((1-methylcyclopropyl)methyl)p icolinamide | 505 |
| Example437 (A437) | 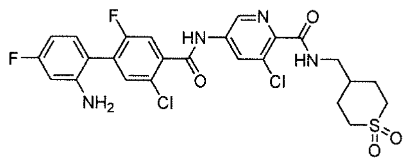 | 5-(2'-amino-5-chloro-2,4'-difluoro-[1,1'-biphenyl]-4-carboxamido)-3-chloro-N-((1,1-dioxidotetrahydro-2H-thiopyran-4-yl)methyl)picolinamide | 583 |
| Example438 (A438) | 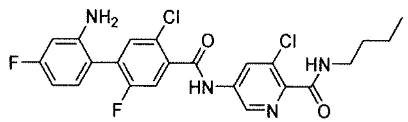 | 5-(2'-amino-5-chloro-2,4'-difluoro-[1,1'-biphenyl]-4-carboxamido)-N-butyl-3-chloropicolinamide | 493 |
| Example439 (A439) | 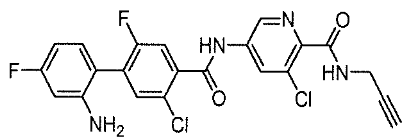 | 5-(2'-amino-5-chloro-2,4'-difluoro-[1,1'-biphenyl]-4-carboxamido)-3-chloro-N-(prop-2-yn-1-yl) picolinamide | 475 |
| Example440 (A440) | 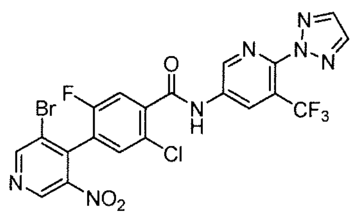 | N-(6-(2H-1,2,3-triazol-2-yl)-5-(trifluoromethyl)pyridin-3-yl)-4-(3-bromo-5-nitropyridin-4-yl)-2-chloro-5-fluorobenzamide | 586 |

(continued)

| Example | Structure | Chemical Name | MS $[M+H]^+$ |
|---|---|---|---|
| Example441 (A441) | 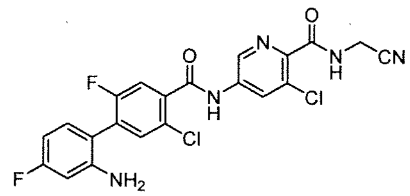 | 5-(2'-amino-5-chloro-2,4'-difluoro-[1,1'-biphenyl]-4-carboxamido)-3-chloro-N-(cyanomethyl) picolinamide | 476 |
| Example442 (A442) | 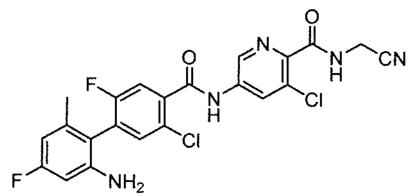 | 5-(2'-amino-5-chloro-2,4'-difluoro-6'-methyl-[1,1'-biphenyl]-4-carboxamido)-3-chloro-N-(cyanomethyl)picolinamide | 490 |
| Example443 (A443) | 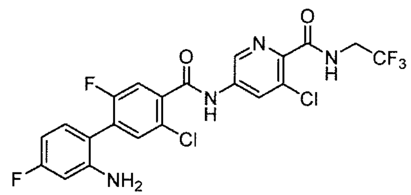 | 5-(2'-amino-5-chloro-2,4'-difluoro-[1,1'-biphenyl]-4-carboxamido)-3-chloro-N-(2,2,2-trifluoroethyl)picolinamide | 519 |
| Example444 (A444) | 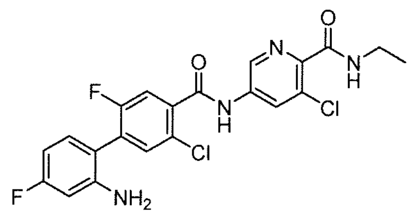 | 5-(2'-amino-5-chloro-2,4'-difluoro-[1,1'-biphenyl]-4-carboxamido)-3-chloro-N-ethylpicolinamide | 465 |

(continued)

| Example | Structure | Chemical Name | MS [M+H]$^+$ |
|---|---|---|---|
| Example445 (A445) | 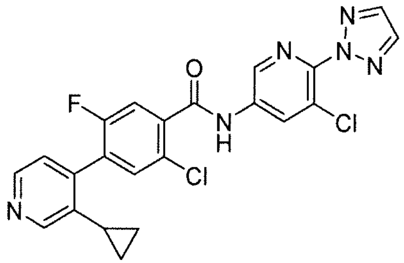 | 2-chloro-N-(5-chloro-6-(2H-1,2,3-triazol-2-yl)pyridin-3-yl)-4-(3-cyclopropylpyridin-4-yl)-5-fluorobenzamide | 469 |
| Example446 (A446) | 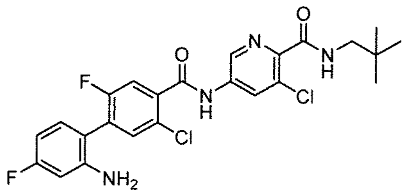 | 5-(2'-amino-5-chloro-2,4'-difluoro-[1,1'-biphenyl]-4-carboxamido)-3-chloro-N-neopentylpicolinamide | 507 |
| Example447 (A447) | 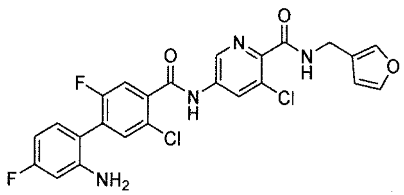 | 5-(2'-amino-5-chloro-2,4'-difluoro-[1,1'-biphenyl]-4-carboxamido)-3-chloro-N-(furan-3-ylmethyl)picolinamide | 517 |
| Example448 (A448) | 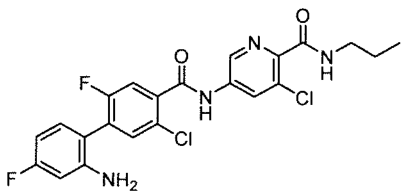 | 5-(2'-amino-5-chloro-2,4'-difluoro-[1,1'-biphenyl]-4-carboxamido)-3-chloro-N-propylpicolinamide | 479 |

(continued)

| Example | Structure | Chemical Name | MS [M+H]$^+$ |
|---|---|---|---|
| Example449 (A449) | 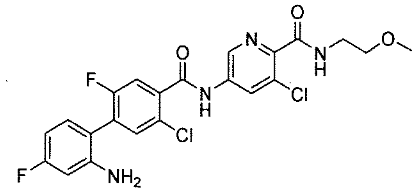 | 5-(2'-amino-5-chloro-2,4'-difluoro-[1,1'-biphenyl]-4-carboxamido)-3-chloro-N-(2-methoxyethyl) picolinamide | 495 |
| Example450 (A450) | 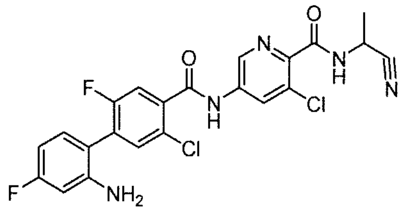 | 5-(2'-amino-5-chloro-2,4'-difluoro-[1,1'-biphenyl]-4-carboxamido)-3-chloro-N-(1-cyanoethyl) picolinamide | 490 |
| Example451 (A451) | 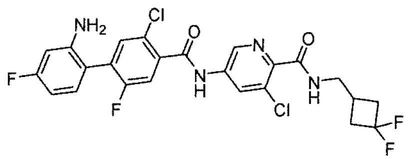 | 5-(2'-amino-5-chloro-2,4'-difluoro-[1,1'-biphenyl]-4-carboxamido)-3-chloro-N-((3,3-difluorocyclobutyl)methyl)pi colinamide | 541 |
| Example452 (A452) | 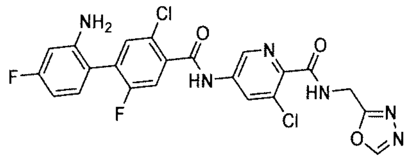 | N-((1,3,4-oxadiazol-2-yl)methyl)-5-(2'-amino-5-chloro-2,4'-difluoro-[1,1'-biphenyl]-4-carboxamido)-3-chloropicolinamide | 519 |
| Example453 (A453) | 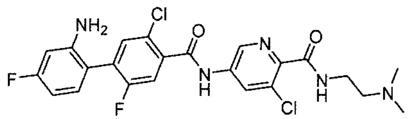 | 5-(2'-amino-5-chloro-2,4'-difluoro-[1,1'-biphenyl]-4-carboxamido)-3-chloro-N-(2-(dimethylamino)ethyl)picoli namide | 508 |

(continued)

| Example | Structure | Chemical Name | MS [M+H]+ |
|---|---|---|---|
| Example454 (A454) | 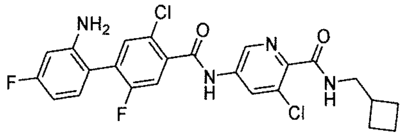 | 5-(2'-amino-5-chloro-2,4'-difluoro-[1,1'-biphenyl]-4-carboxamido)-3-chloro-N-(cyclobutylmethyl)picolinam ide | 505 |
| Example455 (A455) | 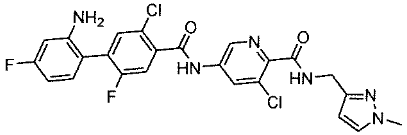 | 5-(2'-amino-5-chloro-2,4'-difluoro-[1,1'-biphenyl]-4-carboxamido)-3-chloro-N-((1-methyl-1H-pyrazol-3-yl)methyl)picolinamide | 531 |
| Example456 (A456) | 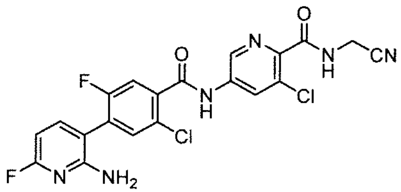 | 5-(4-(2-amino-6-fluoropyridin-3-yl)-2-chloro-5-fluorobenzamido)-3-chloro-N-(cyanomethyl) picolinamide | 523 |
| Example457 (A457) | 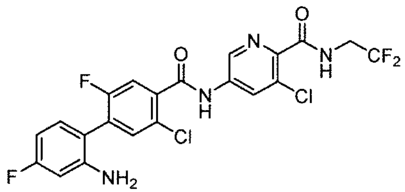 | 5-(2'-amino-5-chloro-2,4'-difluoro-[1,1'-biphenyl]-4-carboxamido)-3-chloro-N-(2,2-difluoro-2λ3-ethyl)picolinamide | 500 |
| Example458 (A458) | 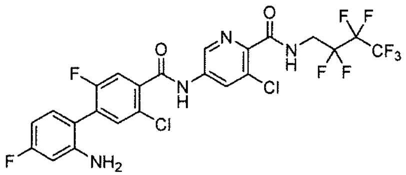 | 5-(2'-amino-5-chloro-2,4'-difluoro-[1,1'-biphenyl]-4-carboxamido)-3-chloro-N-(2,2,3,3,4,4,4-heptafluorobutyl)picolinami de | 619 |

(continued)

| Example | Structure | Chemical Name | MS [M+H]+ |
|---|---|---|---|
| Example459 (A459) | 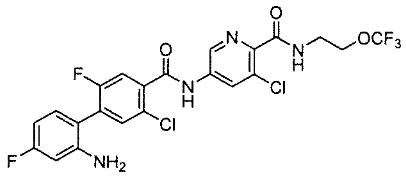 | 5-(2'-amino-5-chloro-2,4'-difluoro-[1,1'-biphenyl]-4-carboxamido)-3-chloro-N-(2-(trifluoromethoxy)ethyl)pico linamide | 549 |
| Example460 (A460) | 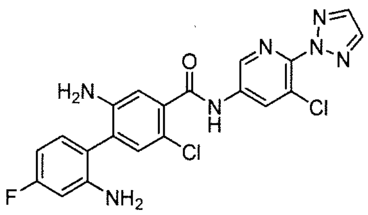 | 2,2'-diamino-5-chloro-N-(5-chloro-6-(2H-1,2,3-triazol-2-yl)pyridin-3-yl)-4'-fluoro-[1,1'-biphenyl]-4-carboxamide | 458 |
| Example461 (A461) | 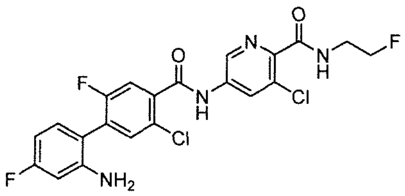 | 5-(2'-amino-5-chloro-2,4'-difluoro-[1,1'-biphenyl]-4-carboxamido)-3-chloro-N-(2-fluoroethyl) picolinamide | 483 |
| Example462 (A462) | 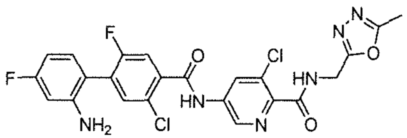 | 5-(2'-amino-5-chloro-2,4'-difluoro-[1,1'-biphenyl]-4-carboxamido)-3-chloro-N-((5-methyl-1,3,4-oxadiazol-2-yl)methyl)picolinamide | 533 |
| Example463 (A463) | 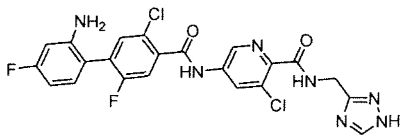 | N-((1H-1,2,4-triazol-3-yl)methyl)-5-(2'-amino-5-chloro-2,4'-difluoro-[1,1'-biphenyl]-4-carboxamido)-3-chloropicolinamide | 518 |

(continued)

| Example | Structure | Chemical Name | MS [M+H]+ |
|---|---|---|---|
| Example464 (A464) | 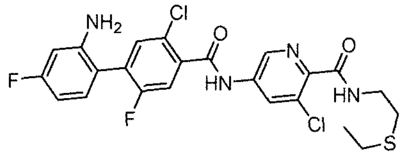 | 5-(2'-amino-5-chloro-2,4'-difluoro-[1,1'-biphenyl]-4-carboxamido)-3-chloro-N-(2-(ethylthio) ethyl)picolinamid e | 525 |
| Example465 (A465) | 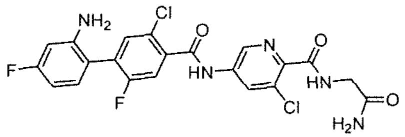 | N-(2-amino-2-oxoethyl)-5-(2'-amino-5-chloro-2,4'-difluoro-[1,1'-biphenyl]-4-carboxamido)-3-chloropicolinamide | 494 |
| Example466 (A466) | 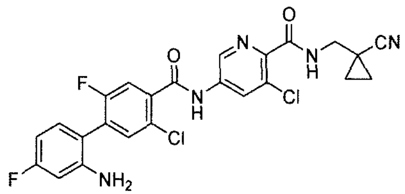 | 5-(2'-amino-5-chloro-2,4'-difluoro-[1,1'-biphenyl]-4-carboxamido)-3-chloro-N-((1-cyanocyclopropyl)methyl)pi colinamide | 516 |
| Example467 (A467) | 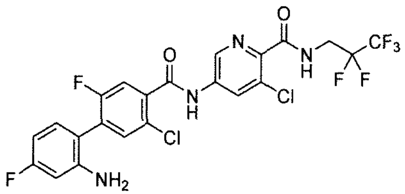 | 5-(2'-amino-5-chloro-2,4'-difluoro-[1,1'-biphenyl]-4-carboxamido)-3-chloro-N-(2,2,3,3,3-pentafluoropropyl)picolinam ide | 569 |
| Example468 (A468) | 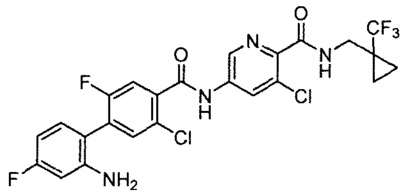 | 5-(2'-amino-5-chloro-2,4'-difluoro-[1,1'-biphenyl]-4-carboxamido)-3-chloro-N-((1-(trifluoromethyl)cyclopropyl )methyl)picolinamide | 559 |

(continued)

| Example | Structure | Chemical Name | MS [M+H]+ |
|---|---|---|---|
| Example469 (A469) | 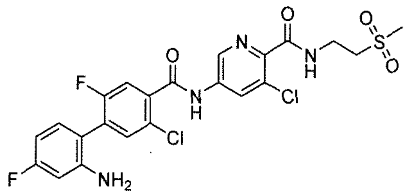 | 5-(2'-amino-5-chloro-2,4'-difluoro-[1,1'-biphenyl]-4-carboxamido)-3-chloro-N-(2-(methylsulfonyl)ethyl)picoli namide | 543 |
| Example470 (A470) | 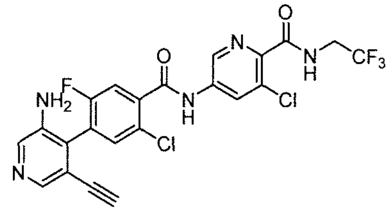 | 5-(4-(3-amino-5-ethynylpyridin-4-yl)-2-chloro-5-fluorobenzamido)-3-chloro-N-(2,2,2-trifluoroethyl)picolinamide | 526 |
| Example471 (A471) | 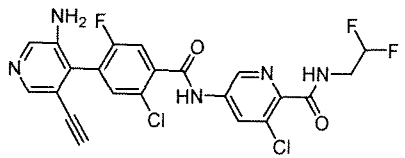 | 5-(4-(3-amino-5-ethynylpyridin-4-yl)-2-chloro-5-fluorobenzamido)-3-chloro-N-(2,2-difluoroethyl)picolinamide | 508 |
| Example472 (A472) | 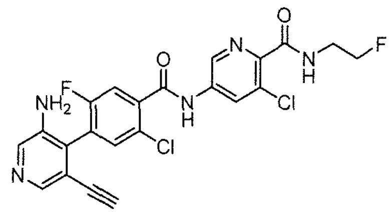 | 5-(4-(3-amino-5-ethynylpyridin-4-yl)-2-chloro-5-fluorobenzamido)-3-chloro-N-(2-fluoroethyl) picolinamide | 490 |
| Example473 (A473) | 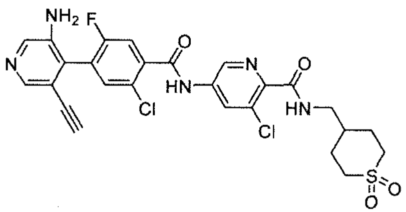 | 5-(4-(3-amino-5-ethynylpyridin-4-yl)-2-chloro-5-fluorobenzamido)-3-chloro-N-((1,1-dioxidotetrahydro-2H-thiopyran-4-yl)methyl)picolinamide | 590 |

(continued)

| Example | Structure | Chemical Name | MS [M+H]+ |
|---|---|---|---|
| Example474 (A474) | 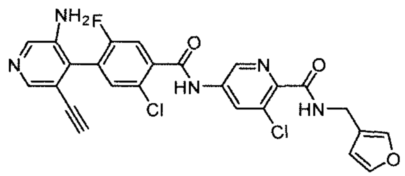 | 5-(4-(3-amino-5-ethynylpyridin-4-yl)-2-chloro-5-fluorobenzamido)-3-chloro-N-(furan-3-ylmethyl)picolinamide | 524 |
| Example475 (A475) | 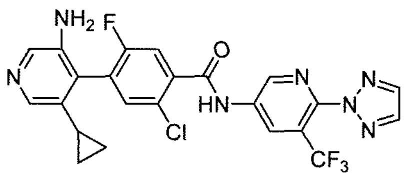 | N-(6-(2H-1,2,3-triazol-2-yl)-5-(trifluoromethyl)pyridin-3-yl)-4-(3-amino-5-cyclopropylpyridin-4-yl)-2-chloro-5-fluorobenzamide | 518 |
| Example476 (A476) | 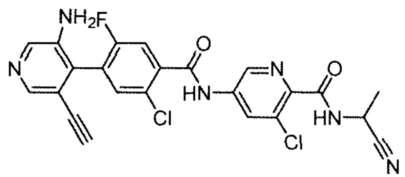 | 5-(4-(3-amino-5-ethynylpyridin-4-yl)-2-chloro-5-fluorobenzamido)-3-chloro-N-(1-cyanoethyl) picolinamide | 497 |
| Example477 (A477) | 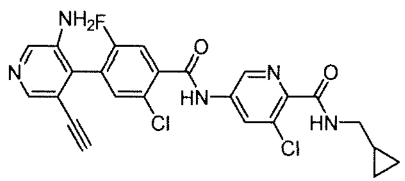 | 5-(4-(3-amino-5-ethynylpyridin-4-yl)-2-chloro-5-fluorobenzamido)-3-chloro-N-(cyclopropylmethyl)picolina mide | 498 |
| Example478 (A478) | 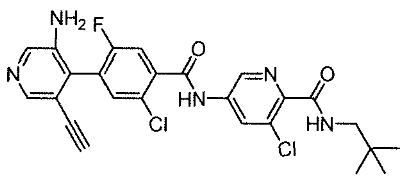 | 5-(4-(3-amino-5-ethynylpyridin-4-yl)-2-chloro-5-fluorobenzamido)-3-chloro-N-neopentylpicolinamide | 514 |

(continued)

| Example | Structure | Chemical Name | MS [M+H]+ |
|---|---|---|---|
| Example479 (A479) | 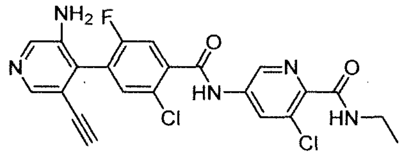 | 5-(4-(3-amino-5-ethynylpyridin-4-yl)-2-chloro-5-fluorobenzamido)-3-chloro-N-ethylpicolinamide | 472 |
| Example480 (A480) | 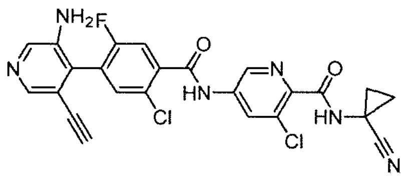 | 5-(4-(3-amino-5-ethynylpyridin-4-yl)-2-chloro-5-fluorobenzamido)-3-chloro-N-(1-cyanocyclopropyl)picolinam ide | 509 |
| Example481 (A481) | 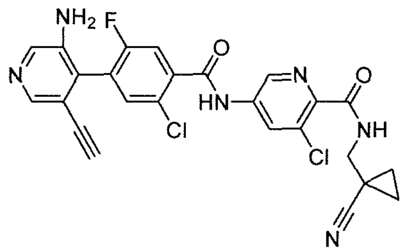 | 5-(4-(3-amino-5-ethynylpyridin-4-yl)-2-chloro-5-fluorobenzamido)-3-chloro-N-((1-cyanocyclopropyl)methyl)pi colinamide | 523 |
| Example482 (A482) | 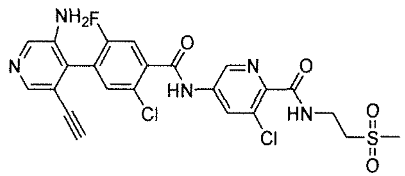 | 5-(4-(3-amino-5-ethynylpyridin-4-yl)-2-chloro-5-fluorobenzamido)-3-chloro-N-(2-(methylsulfonyl)ethyl)picoli namide | 550 |
| Example483 (A483) | 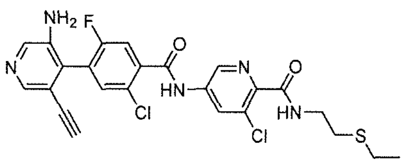 | 5-(4-(3-amino-5-ethynylpyridin-4-yl)-2-chloro-5-fluorobenzamido)-3-chloro-N-(2-(ethylthio) ethyl)picolinamid e | 532 |

(continued)

| Example | Structure | Chemical Name | MS $[M+H]^+$ |
|---|---|---|---|
| Example484 (A484) | 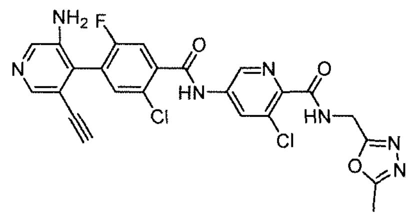 | 5-(4-(3-amino-5-ethynylpyridin-4-yl)-2-chloro-5-fluorobenzamido)-3-chloro-N-((5-methyl-1,3,4-oxadiazol-2-yl)methyl)picolinamide | 540 |
| Example485 (A485) | 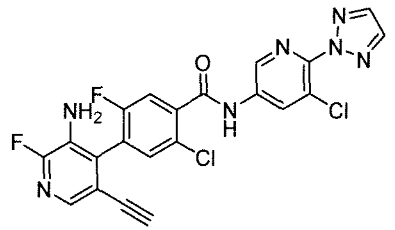 | 4-(3-amino-5-ethynyl-2-fluoropyridin-4-yl)-2-chloro-N-(5-chloro-6-(2H-1,2,3-triazol-2-yl) pyridin-3-yl)-5-fluorobenzamide | 486 |
| Example486 (A486) | 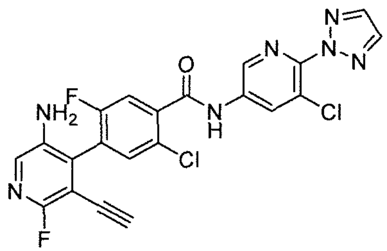 | 4-(5-amino-3-ethynyl-2-fluoropyridin-4-yl)-2-chloro-N-(5-chloro-6-(2H-1,2,3-triazol-2-yl) pyridin-3-yl)-5-fluorobenzamide | 486 |
| Example487 (A487) | 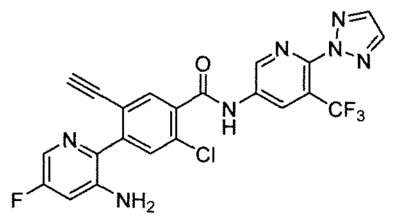 | N-(6-(2H-1,2,3-triazol-2-yl)-5-(trifluoromethyl)pyridin-3-yl)-4-(3-amino-5-fluoropyridin-2-yl)-2-chloro-5-ethynylbenzamide | 502 |

(continued)

| Example | Structure | Chemical Name | MS $[M+H]^+$ |
|---|---|---|---|
| Example488 (A488) | 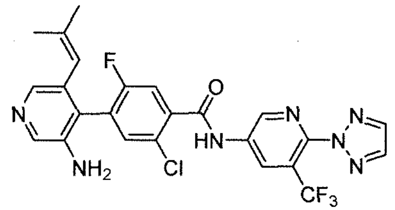 | N-(6-(2H-1,2,3-triazol-2-yl)-5-(trifluoromethyl)pyridin-3-yl)-4-(3-amino-5-(2-methylprop-1-en-1-yl)pyridin-4-yl)-2-chloro-5-fluorobenzamide | 532 |
| Example489 (A489) | 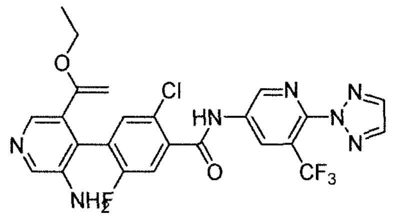 | N-(6-(2H-1,2,3-triazol-2-yl)-5-(trifluoromethyl)pyridin-3-yl)-4-(3-amino-5-(1-ethoxyvinyl) pyridin-4-yl)-2-chloro-5-fluorobenzamide | 548 |
| Example490 (A490) | 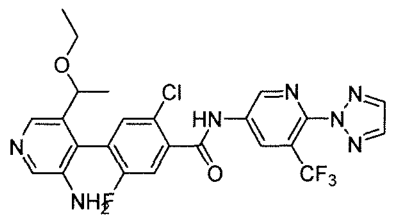 | N-(6-(2H-1,2,3-triazol-2-yl)-5-(trifluoromethyl)pyridin-3-yl)-4-(3-amino-5-(1-ethoxyethyl) pyridin-4-yl)-2-chloro-5-fluorobenzamide | 550 |
| Example491 (A491) | 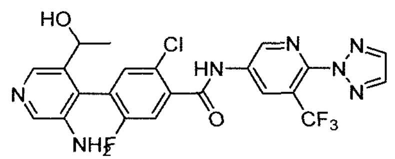 | N-(6-(2H-1,2,3-triazol-2-yl)-5-(trifluoromethyl)pyridin-3-yl)-4-(3-amino-5-(1-hydroxyethyl) pyridin-4-yl)-2-chloro-5-fluorobenzamide | 522 |

(continued)

| Example | Structure | Chemical Name | MS $[M+H]^+$ |
|---|---|---|---|
| Example492 (A492) | 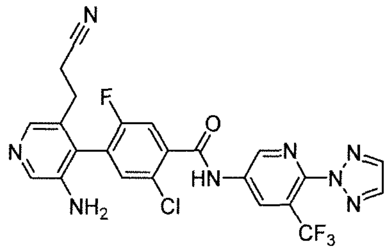 | N-(6-(2H-1,2,3-triazol-2-yl)-5-(trifluoromethyl)pyridin-3-yl)-4-(3-amino-5-(2-cyanoethyl)pyridin-4-yl)-2-chloro-5-fluorobenzamide | 531 |
| Example493 (A493) | 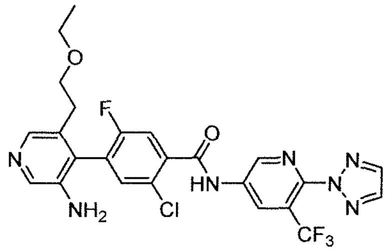 | N-(6-(2H-1,2,3-triazol-2-yl)-5-(trifluoromethyl)pyridin-3-yl)-4-(3-amino-5-(2-ethoxyethyl) pyridin-4-yl)-2-chloro-5-fluorobenzamide | 550 |
| Example494 (A494) | 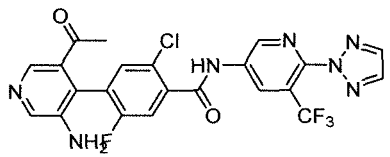 | N-(6-(2H-1,2,3-triazol-2-yl)-5-(trifluoromethyl)pyridin-3-yl)-4-(3-acetyl-5-aminopyridin-4-yl)-2-chloro-5-fluorobenzamide | 520 |
| Example495 (A495) | 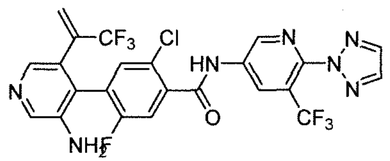 | N-(6-(2H-1,2,3-triazol-2-yl)-5-(trifluoromethyl)pyridin-3-yl)-4-(3-amino-5-(3,3,3-trifluoroprop-1-en-2-yl)pyridin-4-yl)-2-chloro-5-fluorobenzamide | 572 |

(continued)

| Example | Structure | Chemical Name | MS $[M+H]^+$ |
|---|---|---|---|
| Example496 (A496) | 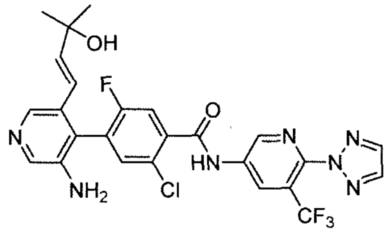 | (E)-N-(6-(2H-1,2,3-triazol-2-yl)-5-(trifluoromethyl)pyridin-3-yl)-4-(3-amino-5-(3-hydroxy-3-methylbut-1-en-1-yl)pyridin-4-yl)-2-chloro-5-fluorobenzamide | 562 |
| Example497 (A497) | 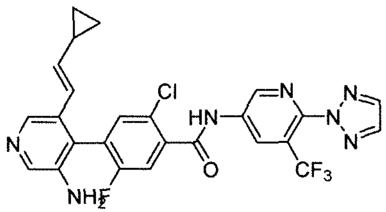 | (E)-N-(6-(2H-1,2,3-triazol-2-yl)-5-(trifluoromethyl)pyridin-3-yl)-4-(3-amino-5-(2-cyclopropylvinyl)pyridin-4-yl)-2-chloro-5-fluorobenzamide | 544 |
| Example498 (A498) | 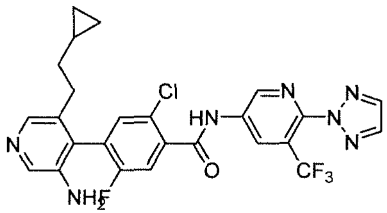 | N-(6-(2H-1,2,3-triazol-2-yl)-5-(trifluoromethyl)pyridin-3-yl)-4-(3-amino-5-(2-cyclopropylethyl) pyridin-4-yl)-2-chloro-5-fluorobenzamide | 546 |
| Example499 (A499) | 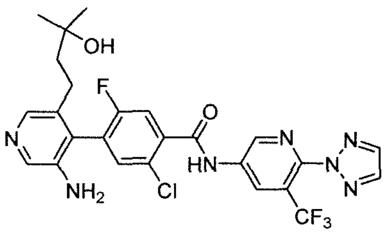 | N-(6-(2H-1,2,3-triazol-2-yl)-5-(trifluoromethyl)pyridin-3-yl)-4-(3-amino-5-(3-hydroxy-3-methylbutyl)pyridin-4-yl)-2-chloro-5-fluorobenzamide | 564 |

(continued)

| Example | Structure | Chemical Name | MS $[M+H]^+$ |
|---|---|---|---|
| Example500 (A500) | 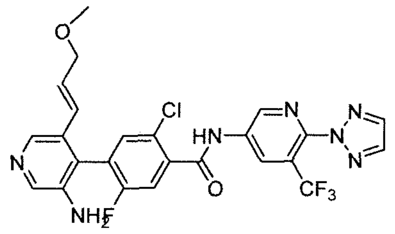 | (E)-N-(6-(2H-1,2,3-triazol-2-yl)-5-(trifluoromethyl)pyridin-3-yl)-4-(3-amino-5-(3-methoxyprop-1-en-1- yl)pyridin-4-yl)-2-chloro-5-fluorobenzamide | 548 |
| Example501 (A501) | 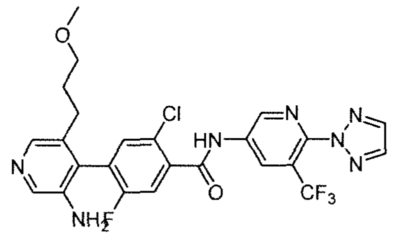 | N-(6-(2H-1,2,3-triazol-2-yl)-5-(trifluoromethyl)pyridin-3-yl)-4-(3-amino-5-(3-methoxypropyl) pyridin-4-yl)-2-chloro-5-fluorobenzamide | 550 |
| Example502 (A502) | 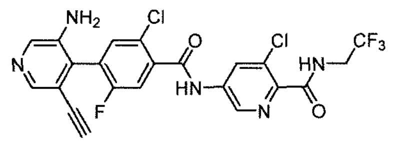 | 5-(4-(3-amino-5-ethynylpyridin-4-yl)-2-chloro-5-fluorobenzamido)-3-chloro-N-(2,2,2-trifluoroethyl)picolinamide | 526 |
| Example503 (A503) | 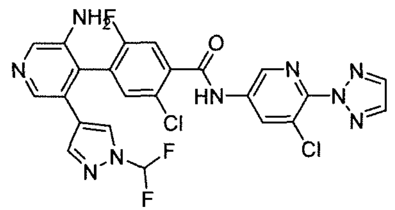 | 4-(3-amino-5-(1-(difluoromethyl)-1H-pyrazol-4-yl)pyridin-4-yl)-2-chloro-N-(5-chloro-6-(2H-1,2,3-triazol-2-yl)pyridin-3-yl)-5-fluorobenzamide | 560 |
| Example504 (A504) | 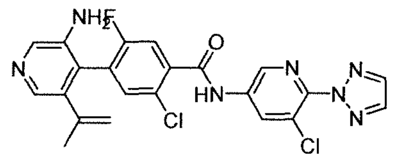 | 4-(3-amino-5-(prop-1-en-2-yl)pyridin-4-yl)-2-chloro-N-(5-chloro-6-(2H-1,2,3-triazol-2-yl) pyridin-3-yl)-5-fluorobenzamide | 484 |

(continued)

| Example | Structure | Chemical Name | MS [M+H]+ |
|---|---|---|---|
| Example505 (A505) | 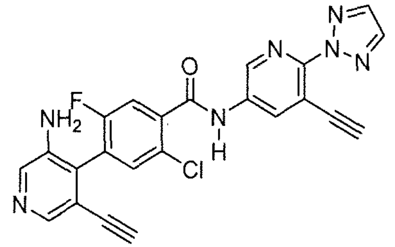 | 4-(3-amino-5-ethynylpyridin-4-yl)-2-chloro-N-(5-ethynyl-6-(2H-1,2,3-triazol-2-yl)pyridin-3-yl)-5-fluorobenzamide | 458 |
| Example506 (A506) | 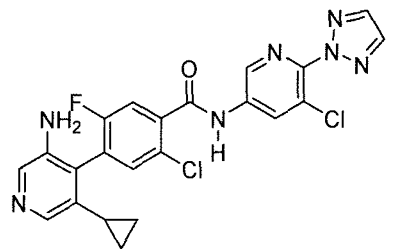 | 4-(3-amino-5-cyclopropylpyridin-4-yl)-2-chloro-N-(5-chloro-6-(2H-1,2,3-triazol-2-yl)pyridin-3-yl)-5-fluorobenzamide | 484 |
| Example507 (A507) | 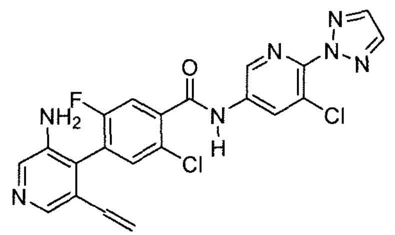 | 4-(3-amino-5-vinylpyridin-4-yl)-2-chloro-N-(5-chloro-6-(2H-1,2,3-triazol-2-yl)pyridin-3-yl)-5-fluorobenzamide | 470 |
| Example508 (A508) | 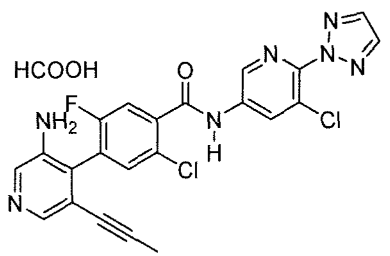 | 4-(3-amino-5-(prop-1-yn-1-yl)pyridin-4-yl)-2-chloro-N-(5-chloro-6-(2H-1,2,3-triazol-2-yl) pyridin-3-yl)-5-fluorobenzamide formate | 528 |

(continued)

| Example | Structure | Chemical Name | MS [M+H]+ |
|---|---|---|---|
| Example509 (A509) | 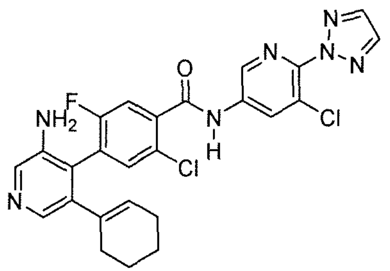 | 4-(3-amino-5-(cyclohex-1-en-1-yl)pyridin-4-yl)-2-chloro-N-(5-chloro-6-(2H-1,2,3-triazol-2-yl) pyridin-3-yl)-5-fluorobenzamide | 524 |
| Examples 12 (A512) | 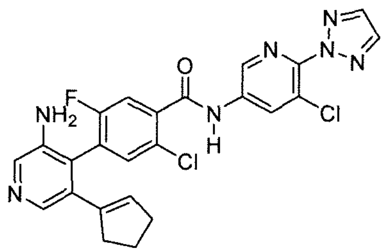 | 4-(3-amino-5-(cyclopent-1-en-1-yl)pyridin-4-yl)-2-chloro-N-(5-chloro-6-(2H-1,2,3-triazol-2-yl) pyridin-3-yl)-5-fluorobenzamide | 556 |
| Example513 (A513) | 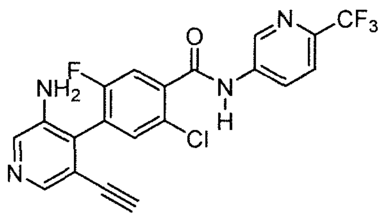 | 4-(3-amino-5-ethynylpyridin-4-yl)-2-chloro-5-fluoro-N-(6-(trifluoromethyl)pyridin-3-yl) benzamide | 435 |
| Example514 (A514) | 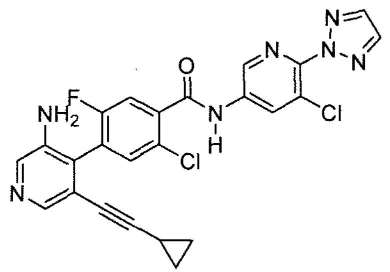 | 4-(3-amino-5-(cyclopropylethynyl)pyridin-4-yl)-2-chloro-N-(5-chloro-6-(2H-1,2,3-triazol-2-yl) pyridin-3-yl)-5-fluorobenzamide | 508 |

(continued)

| Example | Structure | Chemical Name | MS [M+H]+ |
|---|---|---|---|
| Example515 (A515) | 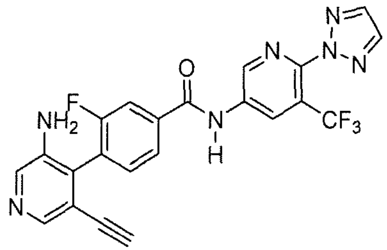 | N-(6-(2H-1,2,3-triazol-2-yl)-5-(trifluoromethyl)pyridin-3-yl)-4-(3-amino-5-ethynylpyridin-4-yl)-3-fluorobenzamide | 467 |
| Example516 (A516) | 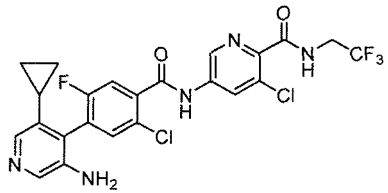 | 5-(4-(3-amino-5-cyclopropylpyridin-4-yl)-2-chloro-5-fluorobenzamido)-3-chloro-N-(2,2,2-trifluoroethyl)picolinamide | 542 |
| Example517 (A517) | 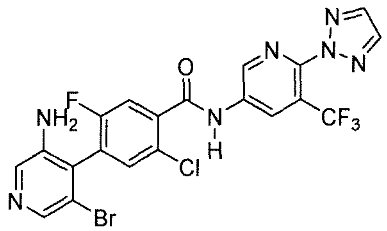 | N-(6-(2H-1,2,3-triazol-2-yl)-5-(trifluoromethyl)pyridin-3-yl)-4-(3-amino-5-bromopyridin-4-yl)-2-chloro-5-fluorobenzamide | 603 |
| Example518 (A518) | 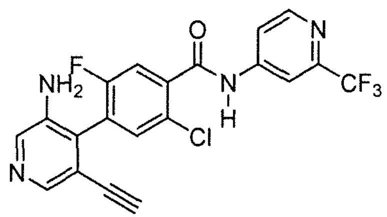 | 4-(3-amino-5-ethynylpyridin-4-yl)-2-chloro-5-fluoro-N-(2-(trifluoromethyl)pyridin-4-yl) benzamide | 481 |

(continued)

| Example | Structure | Chemical Name | MS $[M+H]^+$ |
|---|---|---|---|
| Example519 (A519) | 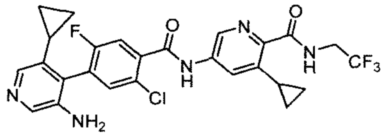 | 5-(4-(3-amino-5-cyclopropylpyridin-4-yl)-2-chloro-5-fluorobenzamido)-3-cyclopropyl-N-(2,2,2-trifluoroethyl)picolinamide | 548 |
| Example521 (A521) | 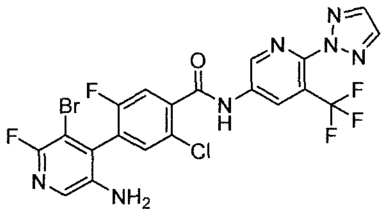 | N-(6-(2H-1,2,3-triazol-2-yl)-5-(trifluoromethyl)pyridin-3- yl)-4-(5-amino-3-bromo-2-fluoropyridin-4-yl)-2-chloro-5-fluorobenzamide | 575 |
| Example522 (A522) | 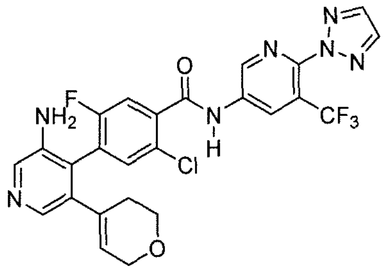 | N-(6-(2H-1,2,3-triazol-2-yl)-5-(trifluoromethyl)pyridin-3-yl)-4-(3-amino-5-(3,6-dihydro-2H-pyran-4-yl)pyridin-4-yl)-2-chloro-5-fluorobenzamide | 596 |
| Example523 (A523) | 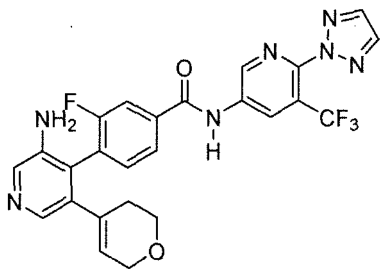 | N-(6-(2H-1,2,3-triazol-2-yl)-5-(trifluoromethyl)pyridin-3-yl)-4-(3-amino-5-(3,6-dihydro-2H-pyran-4-yl)pyridin-4-yl)-3-fluorobenzamide | 562 |

(continued)

| Example | Structure | Chemical Name | MS [M+H]+ |
|---|---|---|---|
| Example524 (A524) | 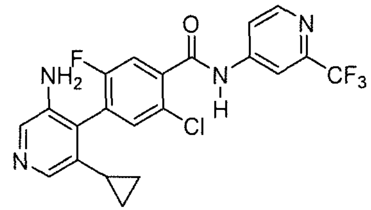 | 4-(3-amino-5-cyclopropylpyridin-4-yl)-2-chloro-5-fluoro-N-(2-(trifluoromethyl)pyridin-4-yl) benzamide | 451 |
| Example525 (A525) | 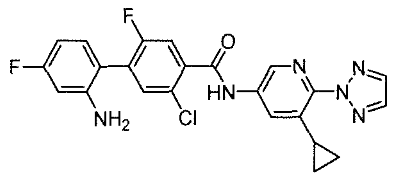 | 2'-amino-5-chloro-N-(5-cyclopropyl-6-(2H-1,2,3-triazol-2-yl)pyridin-3-yl)-2,4'-difluoro-[1,1'-biphenyl]-4-carboxamide | 467 |
| Example526 (A526) | 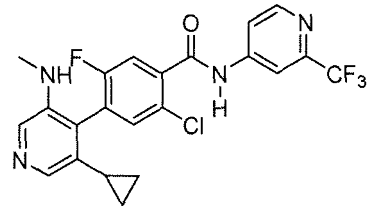 | 2-chloro-4-(3-cyclopropyl-5-(methylamino)pyridin-4-yl)-5-fluoro-N-(2-(trifluoromethyl)pyridin-4-yl)benzamide | 465 |
| Example527 (A527) | 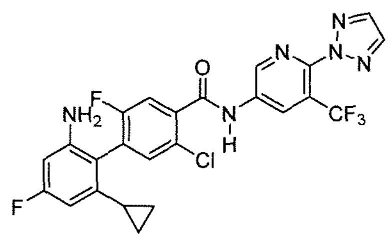 | N-(6-(2H-1,2,3-triazol-2-yl)-5-(trifluoromethyl)pyridin-3-yl)-2'-amino-5-chloro-6'-cyclopropyl-2,4'-difluoro-[1,1'-biphenyl]-4-carboxamide | 535 |

(continued)

| Example | Structure | Chemical Name | MS [M+H]+ |
|---|---|---|---|
| Example528 (A528) | 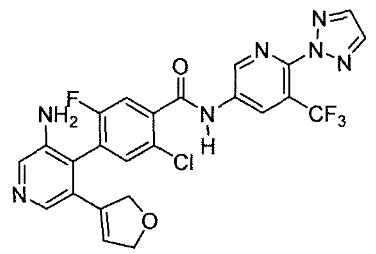 | N-(6-(2H-1,2,3-triazol-2-yl)-5-(trifluoromethyl)pyridin-3-yl)-4-(3-amino-5-(2,5-dihydrofuran-3-yl)pyridin-4-yl)-2-chloro-5-fluorobenzamide | 546 |
| Example529 (A529) | 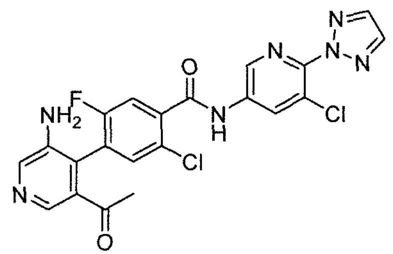 | 4-(3-acetyl-5-aminopyridin-4-yl)-2-chloro-N-(5-chloro-6-(2H-1,2,3-triazol-2-yl)pyridin-3-yl)-5-fluorobenzamide | 486 |
| Example530 (A530) | 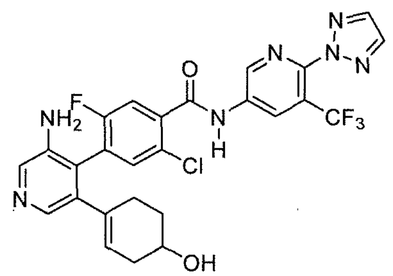 | N-(6-(2H-1,2,3-triazol-2-yl)-5-(trifluoromethyl)pyridin-3-yl)-4-(3-amino-5-(4-hydroxycyclohex-1-en-1-yl)pyridin-4-yl)-2-chloro-5-fluorobenzamide | 574 |

(continued)

| Example | Structure | Chemical Name | MS $[M+H]^+$ |
|---|---|---|---|
| Example531 (A531) | 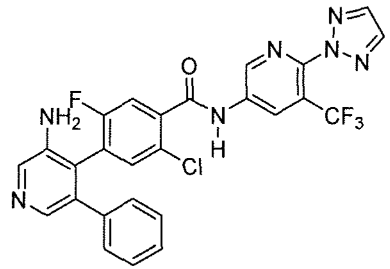 | N-(6-(2H-1,2,3-triazol-2-yl)-5-(trifluoromethyl)pyridin-3-yl)-4-(3-amino-5-phenylpyridin-4-yl)-2-chloro-5-fluorobenzamide | 554 |
| Example532 (A532) | 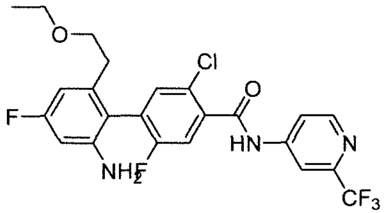 | 2'-amino-5-chloro-6'-(2-ethoxyethyl)-2,4'-difluoro-N-(2-(trifluoromethyl)pyridin-4-yl)-[1,1'-biphenyl]-4-carboxamide | 500 |
| Example533 (A533) | 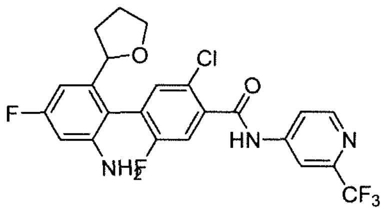 | 2'-amino-5-chloro-2,4'-difluoro-6'-(tetrahydrofuran-2-yl)-N-(2-(trifluoromethyl)pyridin-4-yl)-[1,1'-biphenyl]-4-carboxamide | 498 |
| Example534 (A534) | 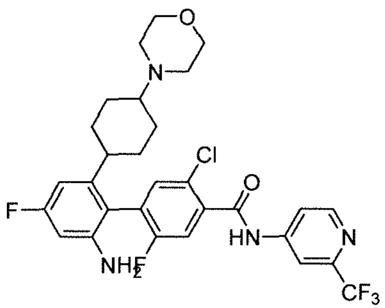 | 2'-amino-5-chloro-2,4'-difluoro-6'-(4-morpholinocyclohexyl)-N-(2-(trifluoromethyl)pyridin-4-yl)-[1,1'-biphenyl]-4-carboxamide | 595 |

(continued)

| Example | Structure | Chemical Name | MS $[M+H]^+$ |
|---|---|---|---|
| Example535 (A535) | 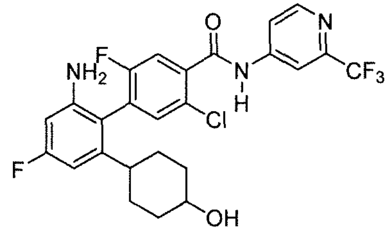 | 2'-amino-5-chloro-2,4'-difluoro-6'-(4-hydroxycyclohexyl)-N-(2-(trifluoromethyl)pyridin-4-yl)-[1,1'-biphenyl]-4-carboxamide | 526 |
| Example536 (A536) | 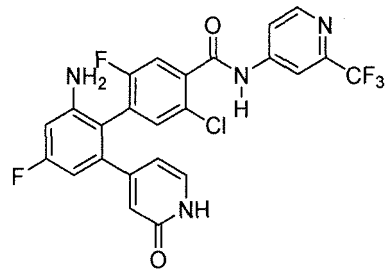 | 2'-amino-5-chloro-2,4'-difluoro-6'-(2-oxo-1,2-dihydropyridin-4-yl)-N-(2-(trifluoromethyl)pyridin-4-yl)-[1,1'-biphenyl]-4-carboxamide | 521 |
| Example537 (A537) | 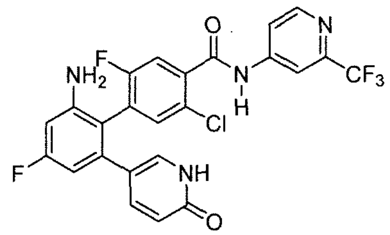 | 2'-amino-5-chloro-2,4'-difluoro-6'-(6-oxo-1,6-dihydropyridin-3-yl)-N-(2-(trifluoromethyl)pyridin-4-yl)-[1,1'-biphenyl]-4-carboxamide | 521 |
| Example538 (A538) | 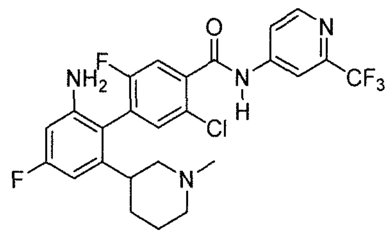 | 2'-amino-5-chloro-2,4'-difluoro-6'-(1-methylpiperidin-3-yl)-N-(2-(trifluoromethyl)pyridin-4-yl)-[1,1'-biphenyl]-4-carboxamide | 525 |

(continued)

| Example | Structure | Chemical Name | MS $[M+H]^+$ |
|---|---|---|---|
| Example539 (A539) | 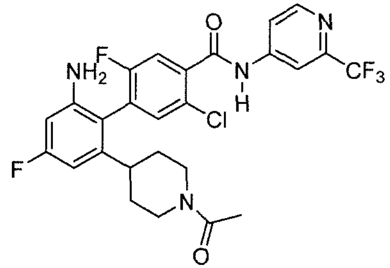 | 2'-(1-acetylpiperidin-4-yl)-6'-amino-5-chloro-2,4'-difluoro-N-(2-(trifluoromethyl)pyridin-4-yl)-[1,1'-biphenyl]-4-carboxamide | 553 |
| Example540 (A540) | 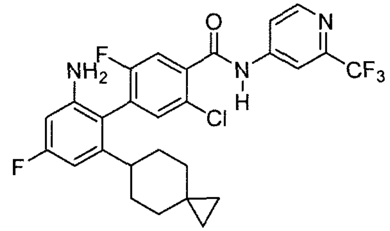 | 2'-amino-5-chloro-2,4'-difluoro-6'-(spiro[2.5]octan-6-yl)-N-(2-(trifluoromethyl)pyridin-4-yl)-[1,1'-biphenyl]-4-carboxamide | 536 |
| Example541 (A541) | 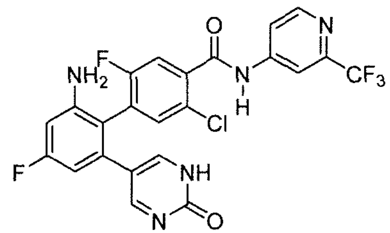 | 2'-amino-5-chloro-2,4'-difluoro-6'-(2-oxo-1,2-dihydropyrimidin-5-yl)-N-(2-(trifluoromethyl) pyridin-4-yl)-[1,1'-biphenyl]-4-carboxamide | 522 |

(continued)

| Example | Structure | Chemical Name | MS [M+H]+ |
|---|---|---|---|
| Example542 (A542) | 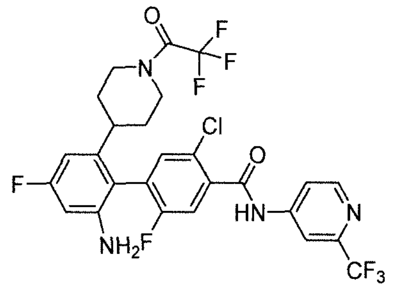 | 2'-amino-5-chloro-2,4'-difluoro-6'-(1-(2,2,2-trifluoroacetyl)piperidin-4-yl)-N-(2-(trifluoromethyl) pyridin-4-yl)-[1,1'-biphenyl]-4-carboxamide | 607 |
| Example543 (A543) | 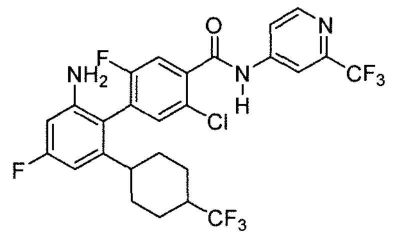 | 2'-amino-5-chloro-2,4'-difluoro-6'-(4-(trifluoromethyl)cyclohexyl) -N-(2-(trifluoromethyl)pyridin-4-yl)-[1,1'-biphenyl]-4-carboxamide | 578 |
| Example544 (A544) | 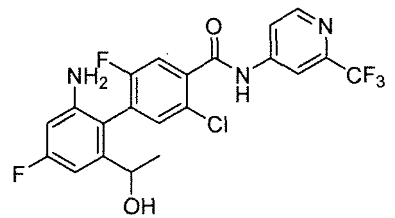 | 2'-amino-5-chloro-2,4'-difluoro-6'-(1-hydroxyethyl)-N-(2-(trifluoromethyl)pyridin-4-yl)-[1,1'-biphenyl]-4-carboxamide | 472 |
| Example545 (A545) | 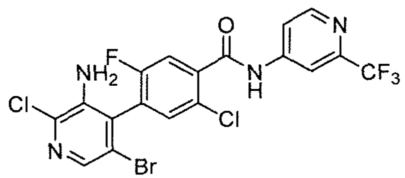 | 4-(3-amino-5-bromo-2-chloropyridin-4-yl)-2-chloro-5-fluoro-N-(2-(trifluoromethyl)pyridin-4-yl) benzamide | 524 |

(continued)

| Example | Structure | Chemical Name | MS [M+H]+ |
|---|---|---|---|
| Example546 (A546) | 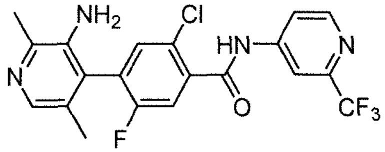 | 4-(3-amino-2,5-dimethylpyridin-4-yl)-2-chloro-5-fluoro-N-(2-(trifluoromethyl)pyridin-4-yl) benzamide | 439 |
| Example547 (A547) | 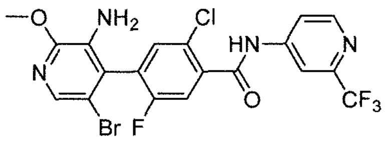 | 4-(3-amino-5-bromo-2-methoxypyridin-4-yl)-2-chloro-5-fluoro-N-(2-(trifluoromethyl)pyridin-4-yl)benzamide | 520 |
| Example548 (A548) | 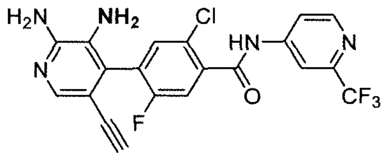 | 2-chloro-4-(2,3-diamino-5-ethynylpyridin-4-yl)-5-fluoro-N-(2-(trifluoromethyl)pyridin-4-yl) benzamide | 450 |
| Example549 (A549) | 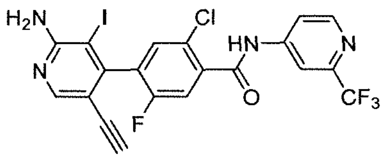 | 4-(2-amino-5-ethynyl-3-iodopyridin-4-yl)-2-chloro-5-fluoro-N-(2-(trifluoromethyl)pyridin-4-yl) benzamide | 561 |
| Example550 (A550) | 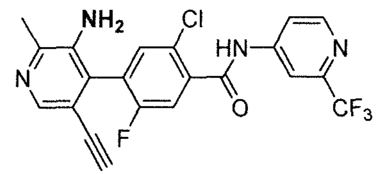 | 4-(3-amino-5-ethynyl-2-methylpyridin-4-yl)-2-chloro-5-fluoro-N-(2-(trifluoromethyl)pyridin-4-yl) benzamide | 449 |
| Example551 (A551) | 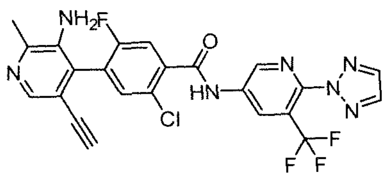 | N-(6-(2H-1,2,3-triazol-2-yl)-5-(trifluoromethyl)pyridin-3-yl)-4-(3-amino-5-ethynyl-2-methylpyridin-4-yl)-2-chloro-5-fluorobenzamide | 516 |

(continued)

| Example | Structure | Chemical Name | MS [M+H]+ |
|---|---|---|---|
| Example552 (A552) | 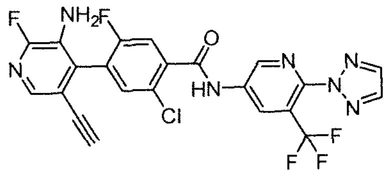 | N-(6-(2H-1,2,3-triazol-2-yl)-5-(trifluoromethyl)pyridin-3-yl)-4-(3-amino-5-ethynyl-2-fluoropyridin-4-yl)-2-chloro-5-fluorobenzamide | 520 |
| Example554 (A554) | 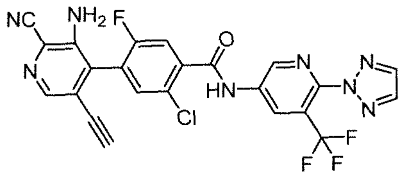 | N-(6-(2H-1,2,3-triazol-2-yl)-5-(trifluoromethyl)pyridin-3-yl)-4-(3-amino-2-cyano-5-ethynylpyridin-4-yl)-2-chloro-5-fluorobenzamide | 527 |
| Example556 (A556) | 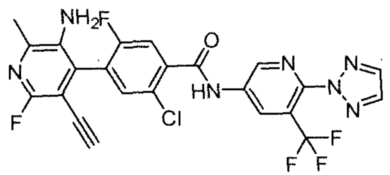 | N-(6-(2H-1,2,3-triazol-2-yl)-5-(trifluoromethyl)pyridin-3-yl)-4-(3-amino-5-ethynyl-6-fluoro-2-methylpyridin-4-yl)-2-chloro-5-fluorobenzamide | 534 |
| Example557 (A557) | 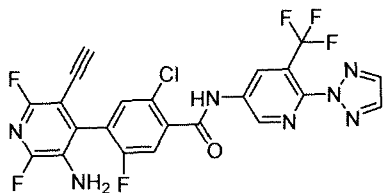 | N-(6-(2H-1,2,3-triazol-2-yl)-5-(trifluoromethyl)pyridin-3-yl)-4-(3-amino-5-ethynyl-2,6-difluoropyridin-4-yl)-2-chloro-5-fluorobenzamide | 538 |
| Example561 (A561) | 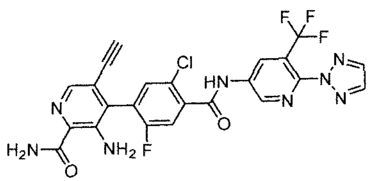 | 4-(4-((6-(2H-1,2,3-triazol-2-yl)-5-(trifluoromethyl)pyridin-3-yl)carbamoyl)-5-chloro-2-fluorophenyl)-3-amino-5-ethynylpicolinamide | 545 |

(continued)

| Example | Structure | Chemical Name | MS $[M+H]^+$ |
|---|---|---|---|
| Example562 (A562) | 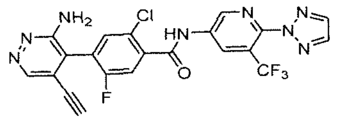 | N-(6-(2H-1,2,3-triazol-2-yl)-5-(trifluoromethyl)pyridin-3-yl)-4-(3-amino-5-ethynylpyridazin-4-yl)-2-chloro-5-fluorobenzamide | 503 |
| Example563 (A563) | 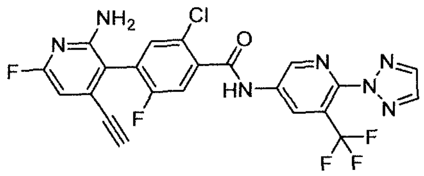 | N-(6-(2H-1,2,3-triazol-2-yl)-5-(trifluoromethyl)pyridin-3-yl)-4-(2-amino-4-ethynyl-6-fluoropyridin-3-yl)-2-chloro-5-fluorobenzamide | 520 |
| Example564 (A564) | 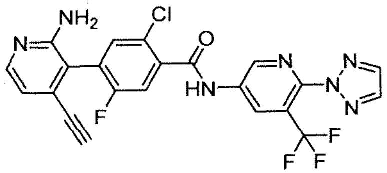 | N-(6-(2H-1,2,3-triazol-2-yl)-5-(trifluoromethyl)pyridin-3-yl)-4-(2-amino-4-ethynylpyridin-3-yl)-2-chloro-5-fluorobenzamide | 502 |
| Example565 (A565) | 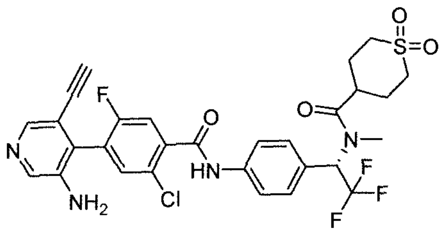 | N-((1S)-1-(4-(4-(3-amino-5-ethynylpyridin-4-yl)-2-chloro-5-fluorobenzamide)phenyl)-2,2,2-trifluoroethyl)-N-methyltetrahydro2H-thiopyran-4-carboxamide 1,1-dioxide | 637 |
| Example566 (A566) | 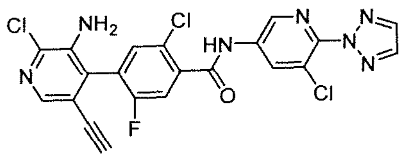 | 4-(3-amino-2-chloro-5-ethynylpyridin-4-yl)-2-chloro-N-(5-chloro-6-(2H-1,2,3-triazol-2-yl) pyridin-3-yl)-5-fluorobenzamide | 502 |

(continued)

| Example | Structure | Chemical Name | MS $[M+H]^+$ |
|---|---|---|---|
| Example567 (A567) | 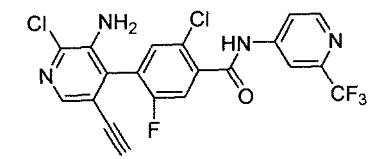 | 4-(3-amino-2-chloro-5-ethynylpyridin-4-yl)-2-chloro-5-fluoro-N-(2-(trifluoromethyl)pyridin-4-yl) benzamido | 469 |
| Example568 (A568) | 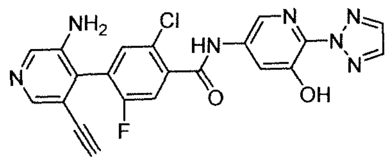 | 4-(3-amino-5-ethynylpyridin-4-yl)-2-chloro-5-fluoro-N-(5-hydroxy -6-(2H-1,2,3-triazol-2-yl) pyridin-3-yl)benzamido | 450 |
| Example569 (A569) | 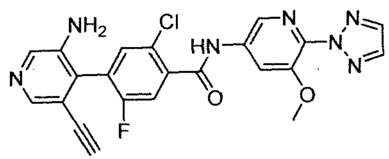 | 4-(3-amino-5-ethynylpyridin-4-yl)-2-chloro-5-fluoro-N-(5-methoxy-6-(2H-1,2,3-triazol-2-yl) pyridin-3-yl)benzamido | 464 |
| Example570 (A570) | 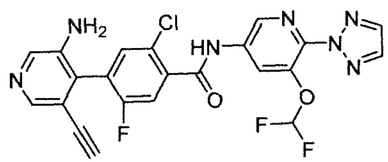 | 4-(3-amino-5-ethynylpyridin-4-yl)-2-chloro-N-(5-(difluoromethoxy)-6-(2H-1,2,3-triazol-2-yl) pyridin-3-yl)-5-fluorobenzamide | 500 |
| Example571 ( A571 ) | 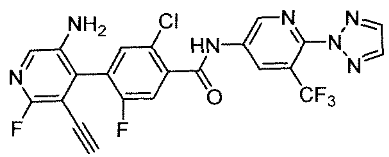 | N-(6-(2H-1,2,3-triazol-2-yl)-5-(trifluoromethyl)pyridin-3-yl)-4-(5-amino-3-ethynyl-2-fluoropyridin-4-yl)-2-chloro-5-fluorobenzamide | 520 |

A410: ' ' '

$^1H$ NMR (500 MHz, DMSO-$d_6$) δ 11.65 (s, 1H), 9.17 (d, *J* = 2.4 Hz, 1H), 8.93 (d, *J* = 2.4 Hz, 1H), 8.25 - 8.15 (m, 4H), 7.91 (d, *J* = 9.0 Hz, 1H), 7.71 (d, *J* = 6.0 Hz, 1H), 5.76 (s, 2H), 4.50 (s, 1H).

A545:

$^1H$ NMR (500 MHz, DMSO-$d_6$) δ 11.57 (s, 1H), 9.15 (d, *J* = 2.4 Hz, 1H), 8.91 (d, *J* = 2.4 Hz, 1H), 8.21 (d, *J* = 1.7 Hz, 2H), 7.95 - 7.83 (m, 2H), 7.69 (d, *J* = 6.0 Hz, 1H), 5.73 (s, 2H).

A571:

$^1H$ NMR (500 MHz, DMSO-$d_6$) δ 11.60 (s, 1H), 9.14 (d, *J* = 2.4 Hz, 1H), 8.91 (d, *J* = 2.4 Hz, 1H), 8.21 (s, 2H), 7.88 (d, *J* = 9.0 Hz, 1H), 7.75 - 7.66 (m, 2H), 5.29 (s, 2H), 4.50 (s, 1H).

**Pharmacological experiments**

**Example A: MALT1**

Test compounds of the present invention (in 100% DMSO) were spotted into a 384-well dilution plate and serially diluted to the required concentrations with DMSO. Then, 0.2 μL of the test compounds were transfered into a new 384-well reaction plate by Echo following centrifugation. The MALT enzyme and its substrate, AMC-labeled peptide, were prepared in 1× protease buffer to yield 2x enzyme solution and 2x peptide solution. Subsequently, 10 μL of 2x enzyme solution was added into the 384-well reaction plate, incubating with the test compounds for 15 minutes at 25°C. After that, 10 μL of 2x peptide solution was added into the plate, continuing to incubate for 90 minutes at 25°C. The plate was then placed on an Envision multifunctional microplate reader to collect raw data, which would be converted into inhibition rate:

$$\text{Inhibition rate \%} = (\text{max-sample})/(\text{max-min}) * 100\%.$$

"max" values were measured using the reaction with enzyme but no compound treated, while "min" values were generated from control wells containing assay buffer without enzyme.

Curve fitting was done with Graphpad Prism software to determine the $IC_{50}$ values. The specific experimental operations are as follows:

1. Protease buffer preparation (200 mL):

| Stock solution | Volume | Assay buffer final concentration |
|---|---|---|
| 1 M HEPES solution | 10 mL | 50 mM |
| 5 M NaCl solution | 6 mL | 150 mM |
| 1% CHAPS solution | 10 mL | 0.05% |
| Na Citrate • $2H_2O$ powder (MW=294.10 g/mol) | 58.82 g | 1 M |
| 1 M DTT | Added before use | 1 mM |

58.82 g Na Citrate • $2H_2O$ powder was added into 140 mL dd$H_2O$ and mixed in a 37°C water bath. Once there were no visible particles, the remaining solutions were added successively with continuous stirring for 10 minutes. The final volume was adjusted to 200 mL. The buffer was stored at room temperature. DTT was added before use.

2. Preparation of reaction plate:

1) All test compounds were diluted from 10 mM stock solution into 1 mM in a 384 well dilution plate (labcyte, PP-0200);
2) Ten various concentrations of the diluted compound solution above were obtained by serial dilution at an equal ratio of 1:3;
3) 0.2 μL DMSO was transferred to two empty wells of the 384 reaction plate as blank controls without compound and enzyme respectively;

4) 0.2 μL of the test compound at different concentrations was also transfered into the 384 reaction plate by Echo, with two duplicate wells for each concentration, then the plate was centrifuged at 1000 rpm for 1 min.

3. Enzymatic reaction:

1) The MALT1 protease and AMC-labeled peptide was prepared in 1× protease buffer to obtain 2x enzyme solution and 2x peptide solution. 10 μL of 2x enzyme solution was added into the 384-well reaction plate. The plate was centrifuged at 1000 rpm for 1 min and incubated for 15 min at 25°C.
2) 10 μL of 2x peptide solution was then transfered to the 384 reaction plate, and the plate was centrifuged at 1000 rpm for 1 min and incubate for 90 min at 25°C.
The final concentration of the compounds were 10.00, 3.33, 1.11, 0.37, 0.12, 0.04, 0.014, 0.0046, 0.0015, 0.0005, 0 μM.
3) The 360/460 signal value of the reaction plate was collected using the Envision multifunctional microplate reader to calculated the inhibition rate, which was fitted to determine the $IC_{50}$.

The results are expressed with $IC_{50}$, wherein "A" stands for "$IC_{50}$≤10nM", and "B" stands for "10nM<$IC_{50}$≤100nM", and "C" stands for "100nM<$IC_{50}$≤500nM", and "D" stands for "$IC_{50}$>500nM". The $IC_{50}$ data for MALT1 inhibition activity of representative compounds are shown in Table 1.

Table 1

| Compound | Ac-LRSR-AMC ($IC_{50}$, nM) |
|---|---|
| A109 | A |
| A110 | A |
| A113 | A |
| A114 | A |
| A115 | A |
| A116 | A |
| A119 | A |
| A120 | A |
| A121 | A |
| A122 | A |
| A123 | A |
| A124 | A |
| A126 | A |
| A127 | A |
| A281 | A |
| A324 | A |
| A335 | A |
| A338 | A |
| A346 | A |
| A342 | A |
| A333 | A |
| A409 | A |
| A410 | A |
| A411 | A |

(continued)

| Compound | Ac-LRSR-AMC ($IC_{50}$, nM) |
|---|---|
| A412 | A |
| A413 | A |
| A414 | B |
| A415 | B |
| A416 | C |
| A417 | B |
| A418 | D |
| A419 | D |
| A420 | D |
| A421 | D |
| A422 | D |
| A423 | B |
| A424 | B |
| A425 | B |
| A426 | A |
| A427 | A |
| A428 | A |
| A429 | A |
| A430 | A |
| A431 | B |
| A432 | B |
| A433 | B |
| A434 | B |
| A435 | B |
| A436 | C |
| A437 | B |
| A438 | B |
| A439 | D |
| A440 | B |
| A441 | B |
| A442 | B |
| A443 | A |
| A444 | A |
| A445 | B |
| A446 | A |
| A447 | B |
| A448 | B |
| A449 | B |

(continued)

| Compound | Ac-LRSR-AMC ($IC_{50}$, nM) |
|---|---|
| A450 | B |
| A451 | B |
| A452 | C |
| A453 | C |
| A454 | C |
| A455 | B |
| A456 | B |
| A457 | B |
| A458 | D |
| A459 | C |
| A460 | B |
| A461 | B |
| A462 | B |
| A463 | B |
| A464 | B |
| A465 | B |
| A466 | B |
| A467 | C |
| A468 | C |
| A469 | B |
| A470 | A |
| A471 | A |
| A472 | A |
| A475 | A |
| A478 | A |
| A479 | A |
| A485 | A |
| A486 | A |
| A504 | A |
| A505 | A |
| A506 | A |
| A507 | A |
| A517 | A |
| A518 | A |
| A521 | A |
| A551 | A |
| A552 | A |
| A553 | A |

(continued)

| Compound | Ac-LRSR-AMC ($IC_{50}$, nM) |
|---|---|
| A554 | A |
| A556 | A |
| A557 | A |
| A558 | A |

According to Table 1, the compounds of the present invention exhibit strong inhibitory activities on MALT1.

**Example B: ELISA method to detect IL-2 secretion induced by PMA/Ionomycin**

12.5*$10^3$ Jurkat cells were seeded on a 96-well plate and incubated with various concentrations of compound solution for 30 min. Then, 500x PMA/Ionomycin stimulating factor was diluted with culture medium and added to the cell plate. The final concentrations of PMA/Ionomycin were 81nM and 1.34μM respectively. The final concentrations of the compounds were 10000, 3333.3, 1111.1, 370.4, 123.5, 41.2, 13.7, 4.6, 1.5, 0 nM (The final concentration of DMSO is 0.5%). After incubation for 20 h, the supernatant was collected via centrifugation. The final IL-2 concentration was detected by ELISA method (IL-2 Duoset, R&D Systems, DY202). The signal value of each sample at a wavelength of 450 nm was collected by EnVision multifunctional microplate reader and converted to the IL-2 concentration via the ELISACalc.exe software. The IL-2 secretion inhibitory activity $IC_{50}$ was fitted by GraphPad Prism software. The cell viability was tested simultaneously using the Cell-Titer Glo kit.

The compounds of the present invention show strong inhibitory effect on IL-2 secretion of Jurkat cells (PMA/Ionomycin inducing). The $IC_{50}$ data of representative compounds for inhibiting IL-2 secretion of Jurkat cells are shown in Table 2.

Table 2

| Compound | IL-2 secretion ($IC_{50}$, nM) |
|---|---|
| A12 | 285 |
| A16 | 194 |
| A17 | 191 |
| A18 | 598 |
| A19 | 632 |
| A42 | 471 |
| A64 | 463 |
| A67 | 325 |
| A69 | 195 |
| A100 | 369 |
| A102 | 154 |
| A104 | 294 |
| A105 | 134 |
| A106 | 265 |
| A109 | 94 |
| A110 | 186 |
| A111 | 283 |
| A112 | 234 |
| A113 | 35 |

(continued)

| Compound | IL-2 secretion ($IC_{50}$, nM) |
|---|---|
| A114 | 34 |
| A115 | 40 |
| A116 | 54 |
| A117 | 184 |
| A118 | 82 |
| A119 | 466 |
| A120 | 57 |
| A121 | 43 |
| A122 | 121 |
| A123 | 58 |
| A124 | 252 |
| A126 | 136 |
| A127 | 444 |
| A281 | 77 |
| A410 | 66 |
| A411 | 17 |
| A412 | 32 |
| A426 | 77 |
| A427 | 77 |
| A428 | 164 |
| A429 | 259 |
| A470 | 113 |
| A505 | 36 |
| A506 | 76 |
| A529 | 208 |

**Example C: Liver microsome metabolism stability assay**

1. Test buffer preparation:

1900 mg $MgCl_2$ was dissolved into a final volume of 400 mL ultrapure water.

17.42 g $K_2HPO_4$ and 13.65 g $KH_2PO_4$ were dissolved respectively into ultrapure water with a final volume of 1000 mL.

The $K_2HPO_4$ and $KH_2PO_4$ stock solutions above were mixed with pH adjusted to 7.30 + 0.10 to obtain 100 mM potassium phosphate (K-PBS) buffer.

2. Reaction stop solution preparation:

The stop solution was acetonitrile containing 1 ng/mL labetalol and 1 ng/mL glyburide, and stored at 4°C.

3. Working solution preparation:

The verapamil (positive control) and test sample stock solution were diluted to 50 μM and 200 μM respectively

with MeOH/ACN/$H_2O$ solution (1:1:2, volume ratio).

4. Experiment procedure:

1) 40 μL $MgCl_2$ and 306 μL K-buffer were mixed in 96 plate wells containing blank control wells and test compound wells (the compound test wells without NADPH);
2) 4 μL compound working solution was added to each well (blank wells correspond to 4 μL K-buffer) (Note: The final concentration of DMSO volume in the system was ≤0.5%);
3) 10 μL liver microsomes (concentration: 20 mg/mL) was then added to each well, and the mixture was incubated at 37°C for 10 min;
4) 40 μL NADPH working solution was added to each well to start the reaction, and the final total reaction volume reached 400 μL;
5) 50 μL samples from the reaction solution were aspirated out at 0, 5, 15, and 45 minutes, and added into 400 μL stop solution to terminate the reaction respectively;
6) The sample plate was placed in a shaker at 600 rpm for about 5 minutes at room temperature, then centrifuged at 4000 rpm for 10 minutes at 4°C;
7) An aliquot of 50 μL of the supernatant after centrifugation was transfered to the pre-added 20% acetonitrile water, and shaked on a shaker at 600 rpm for 2 minutes at room temperature to mix well, and then analyzed using LC-MS/MS method.

5. Data analysis:

The $T_{1/2}$ and CL was calculated using the first-order kinetic equation:
the first-order kinetic equation:

$$C_t = C_0 * e\ \text{-}kt$$

$$C_t = (1/2) * C_0$$

$$T_{1/2} = \ln 2/k = 0.693/k$$

Slope k was determined from natural logarithmic linear regression of percent parent drug remaining versus incubation time.
The *in vitro* half-life (in vitro $T_{1/2}$) was calculated from the slope value:

$$in\ vitro\ T_{1/2} = -(0.693/k)$$

The *in vitro* intrinsic clearance rate (*in vitro* $CL_{int}$, in μL/min/mg proteins) was calculated according to the following formula (repeated average):

$$in\ vitro\ CL_{int} = \frac{0.693}{T_{1/2}} * \frac{\text{volume of incubation (μL)}}{\text{amount of proteins (mg)}}$$

Test positive control: verapamil.
Any compound value not within the specified range would be excluded and repeat experiment.
The compounds of the present invention had moderate or slow metabolism in three species: humans, rats, and mice, and the compounds of the present invention had good stability in liver microsomes in vitro of humans, rats.

Example D: Mice pharmacokinetic test

The female BALB/c mice (20-30 g) was purchased from Beijing Weitonglihua Experimental Animal Technology

Co., Ltd. to study the pharmacokinetic characteristics in mice (intravenous administration of 1 mg/kg and intragastric administration of 5 mg/kg).

1. Compound solution preparation:

| Route of administratio n | Dosag e (mg/kg) | Dosing volume (mL/kg) | Compo und concentratio n (mg/mL) | Solvent |
|---|---|---|---|---|
| IV | 1 | 5 | 0.2 | 5% DMSO/5% |
| tail vein administratio n | | | | Solutol /90% saline |
| PO Oral gavage | 5 | 10 | 0.5 | 5%DMSO/5%So lutol /90% water |
| Note: All solutions need to be prepared one hour before administration. | | | | |

2. Sample collection and storage:

The blood was collected at 5 min (intravenous administration only), 15 min, 30 min, 1 h, 2 h, 4 h, 7 h and 24 h after administration through the orbital venous plexus into a centrifuge tube containing EDTA anticoagulant, and 100 μL of whole blood was collected at each time point. After the centrifugation, the plasma was separated and stored in a -80°C refrigerator until analysis.

3. Sample processing and testing:

The above samples were processed by the protein precipitation method. The standard curve and other samples were also preperaed in the same way. The precipitated samples were centrifuged at 3200 rpm for 10 minutes at 4°C. The supernatant was aspirated and mixed with water 1:1, and then analyzed by LC-MS/MS. The quantitative limit of the standard curve of this compound in BALB/c mouse plasma was 1.00-1000 ng/mL.

4. Data analysis:

The non-compartmental model of Phoenix WinNonlin software was used to calculate the pharmacokinetic parameters of animals after drug administration.
The compound of the present invention had higher exposure and higher bioavailability after oral administration in female BALB/c mice.

## Claims

1. A compound of Formula I, or a stereoisomer, tautomer, deuterated compound, pharmaceutically acceptable salt, prodrug, chelate, non-covalent complex or solvate thereof,

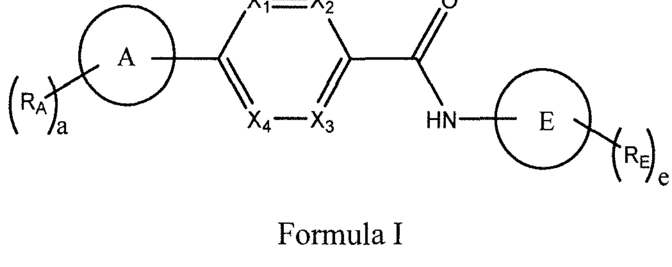

Formula I

wherein,

$X_1$ is selected from $CR_1$ or N;
$X_2$ is selected from $CR_2$ or N;
$X_3$ is selected from $CR_3$ or N;
$X_4$ is selected from $CR_4$ or N;
$R_1$, $R_2$, $R_3$, $R_4$ are the same or different, and are each independently selected from the group consisting of H, CN, $NO_2$, halogen, $-C_{1-6}$ alkyl, $-C_{2-6}$ alkenyl, $-C_{2-6}$ alkynyl, - $OR_5$, $-NR_5R_6$, $SR_5$, $-C_{0-6}$ alkyl-$C_{3-8}$ carbocyclyl, $-C_{0-6}$ alkyl-3-8 membered heterocyclyl, $-C_{0-6}$ alkyl-$C_{6-10}$ aryl, $-C_{0-6}$ alkyl-5-10 membered heteroaryl, $-C(O)R_5$, - $N(R_5)C(O)R_6$, $-C(O)NR_5R_6$, $-N(R_5)C(O)OR_6$, $-OC(O)NR_5R_6$, $-N(R_5)C(O)NR_6R_7$, - $S(O)R_5$, $-S(O)_2R_5$, $-S(O)NR_5R_6$, $-S(O)_2NR_5R_6$, $-N(R_5)S(O)R_6$, $-N(R_5)S(O)_2R_6$, - $C(O)OR_5$, $-OC(O)R_5$ and $-OC(O)OR_5$; wherein the $-C_{1-6}$ alkyl, $-C_{2-6}$ alkenyl, $-C_{2-6}$ alkynyl, $-C_{0-6}$ alkyl-$C_{3-8}$ carbocyclyl, $-C_{0-6}$ alkyl-3-8 membered heterocyclyl, $-C_{0-6}$ alkyl-$C_{6-10}$ aryl, $-C_{0-6}$ alkyl-5-10 membered heteroaryl, $R_5$, $R_6$ and $R_7$ are optionally substituted with one or more substituents selected from hydrogen, halogen, CN, $-OR_8$, $-NR_8R_9$, $-SR_8$, $-C(O)R_8$, $-N(R_8)C(O)R_9$, $-C(O)NR_8R_9$, $-N(R_8)C(O)OR_9$, $-OC(O)NR_8R_9$, $-N(R_8)C(O)NR_9R_{10}$, $-S(O)R_8$, $-S(O)_2R_8$, $-S(O)NR_8R_9$, $-S(O)_2NR_8R_9$, $-N(R_8)S(O)R_9$, - $N(R_8)S(O)_2R_9$, $-C(O)OR_8$, $-OC(O)R_8$, $-OC(O)OR_8$, oxo, $-C_{1-6}$ alkyl, $-C_{2-6}$ alkenyl, $-C_{2-6}$ alkynyl, $-C_{0-6}$ alkyl-$C_{3-8}$ carbocyclyl or $-C_{0-6}$ alkyl-3-8 membered heterocyclyl;
ring A is selected from $-C_{5-6}$ carbocyclyl, 5-6 membered heterocyclyl, $-C_6$ aryl or 5-6 membered heteroaryl;
each $R_A$ is the same or different, and is independently selected from the group consisting of hydrogen, halogen, oxo, CN, $NO_2$, $-C_{1-6}$ alkyl, $-C_{2-6}$ alkenyl, $-C_{2-6}$ alkynyl, $-OR_5$, $-NR_5R_6$, $-SR_5$, $-C_{0-6}$ alkyl-$C_{3-8}$ carbocyclyl, $-C_{0-6}$ alkyl-3-8 membered heterocyclyl, $-C_{0-6}$ alkyl-$C_{6-10}$ aryl, $-C_{0-6}$ alkyl-5-10 membered heteroaryl, $-C(O)R_5$, - $N(R_5)C(O)R_6$, $-C(O)NR_5R_6$, $-N(R_5)C(O)OR_6$, $-OC(O)NR_5R_6$, $-N(R_5)C(O)NR_6R_7$, - $S(O)R_5$, $-S(O)_2R_5$, $-S(O)NR_5R_6$, $-S(O)_2NR_5R_6$, $-N(R_5)S(O)R_6$, $-N(R_5)S(O)_2R_6$, - $C(O)OR_5$, $-OC(O)R_5$ and $-OC(O)OR_5$; wherein the $-C_{1-6}$ alkyl, $-C_{2-6}$ alkenyl, $-C_{2-6}$ alkynyl, $-C_{0-6}$ alkyl-$C_{3-8}$ carbocyclyl, $-C_{0-6}$ alkyl-3-8 membered heterocyclyl, $-C_{0-6}$ alkyl-$C_{6-10}$ aryl, $-C_{0-6}$ alkyl-5-10 membered heteroaryl, $R_5$, $R_6$ and $R_7$ are optionally substituted with one or more substituents selected from hydrogen, halogen, CN, oxo, - $C_{1-6}$ alkyl, $-C_{2-6}$ alkenyl, $-C_{2-6}$ alkynyl, $-C_{0-6}$ alkyl-$C_{3-8}$ carbocyclyl, $-C_{0-6}$ alkyl-3-8 membered heterocyclyl, $-OR_8$, $-NR_8R_9$, $-SR_8$, $-S(O)R_8$, $-S(O)_2R_8$, $-C(O)R_8$, $-C(O)OR_8$, $-OC(O)R_8$, $-N(R_8)C(O)R_9$ or $-C(O)NR_8R_9$;
ring E is selected from 5-10 membered heterocyclyl, $C_{6-10}$ aryl or 5-10 membered heteroaryl;
each $R_E$ is the same or different, and is independently selected from the group consisting of hydrogen, halogen, oxo, CN, $NO_2$, $-C_{1-6}$ alkyl, $-C_{2-6}$ alkenyl, $-C_{2-6}$ alkynyl, $-C_{0-6}$ alkyl-5-10 membered heteroaryl, $-C_{0-6}$ alkyl-$C_{3-8}$ carbocyclyl, $-C_{0-6}$ alkyl-3-8 membered heterocyclyl, $-OR_5$, $-NR_5R_6$, $-SR_5$, $-C(O)R_5$, $-N(R_5)C(O)R_6$, - $C(O)NR_5R_6$, $-N(R_5)C(O)OR_6$, $-OC(O)NR_5R_6$, $-N(R_5)C(O)NR_6R_7$, $-S(O)R_5$, $-S(O)_2R_5$, $-S(O)NR_5R_6$, $-S(O)_2NR_5R_6$, $-N(R_5)S(O)R_6$, $-N(R_5)S(O)_2R_6$, $-C(O)OR_5$, $-OC(O)R_6$ and $-OC(O)OR_7$; wherein the $-C_{1-6}$ alkyl, $-C_{2-6}$ alkenyl, $-C_{2-6}$ alkynyl, $-C_{0-6}$ alkyl-5-10 membered heteroaryl, $-C_{0-6}$ alkyl-$C_{3-8}$ carbocyclyl, $-C_{0-6}$ alkyl-3-8 membered heterocyclyl, $R_5$, $R_6$ and $R_7$ are optionally substituted with one or more substituents selected from hydrogen, halogen, CN, oxo, $-C_{1-6}$ alkyl, $-C_{2-6}$ alkenyl, $-C_{2-6}$ alkynyl, - $OR_8$, $-NR_8R_9$, substituted or unsubstituted $-C_{0-6}$ alkyl-$C_{3-6}$ carbocyclyl, substituted or unsubstituted $-C_{0-6}$ alkyl -3-6 membered heterocyclyl, $-C(O)OR_8$ or $-C(O)NR_8R_9$;
$R_5$, $R_6$, $R_7$, $R_8$, $R_9$, $R_{10}$ are each independently selected from the group consisting of hydrogen, halogen, CN, OH, $NH_2$, oxo, $-C_{1-6}$ alkyl, $-C_{1-6}$ alkoxy, $-C_{2-6}$ alkenyl, $-C_{2-6}$ alkynyl, $-C_{0-6}$ alkyl-$C_{3-14}$ carbocyclyl, $-C_{0-6}$ alkyl-3-14 membered heterocyclyl; wherein the $-C_{1-6}$ alkyl, $-C_{1-6}$ alkoxy, $-C_{2-6}$ alkenyl, $-C_{2-6}$ alkynyl, $-C_{0-6}$ alkyl-$C_{3-14}$ carbocyclyl, $-C_{0-6}$ alkyl-3-14 membered heterocyclyl are optionally substituted with one or more substituents selected from hydrogen, halogen, CN, OH, $NH_2$, -COOH, oxo, $-C_{1-6}$ alkyl, $-C_{1-6}$ haloalkyl, $-C_{2-6}$ alkenyl, $-C_{2-6}$ alkynyl, $-C_{1-6}$ alkoxy, $-C_{1-6}$ haloalkoxy, $-NH(C_{1-6}$ alkyl), $-N(C_{1-6}$ alkyl$)_2$, $-COO(C_{1-6}$ alkyl), $-CONH(C_{1-6}$ alkyl), - $CON(C_{1-6}$ alkyl$)_2$, substituted or unsubstituted $-C_{0-6}$ alkyl-$C_{3-6}$ carbocyclyl, substituted or unsubstituted $-C_{0-6}$ alkyl-3-6 membered heterocyclyl;
a is selected from 0, 1, 2, 3, 4, 5 or 6;
e is selected from 0, 1, 2, 3, 4, 5 or 6;
when $X_1$ is selected from CH, $X_2$ is selected from CH, $X_3$ is selected from CH, and $X_4$ is selected from CH,

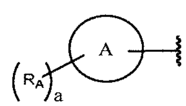

is not phenyl.

2. The compound of claim 1, or a stereoisomer, tautomer, deuterated compound, pharmaceutically acceptable salt, prodrug, chelate, non-covalent complex, or solvate thereof, wherein

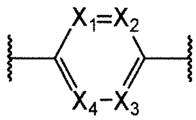

is selected from

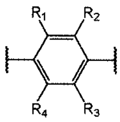, 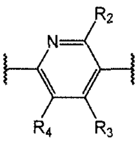, 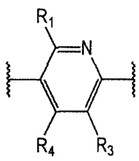,

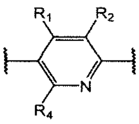, 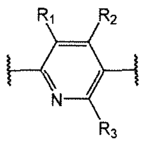, 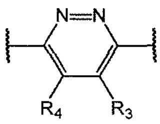, 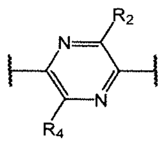, 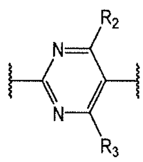, 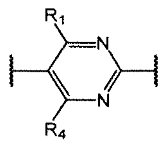,

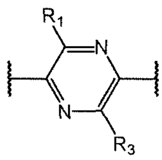 or 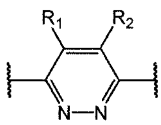.

3. The compound of claim 1 or 2, or a stereoisomer, tautomer, deuterated compound, pharmaceutically acceptable salt, prodrug, chelate, non-covalent complex or solvate thereof, wherein

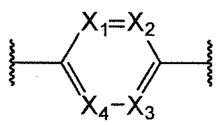

is selected from

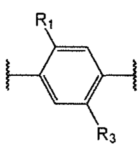, 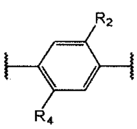,

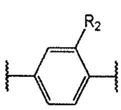, 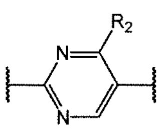, 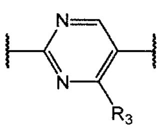, 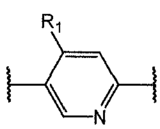, 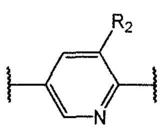, 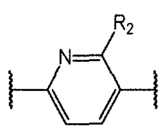,

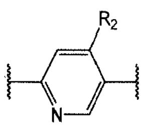 or 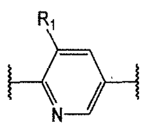.

4. The compound of any one of claims 1-3, or a stereoisomer, tautomer, deuterated compound, pharmaceutically

acceptable salt, prodrug, chelate, non-covalent complex or solvate thereof, wherein $R_1$, $R_3$ are the same or different, and are each independently selected from the group consisting of halogen, $-C_{1-6}$ alkyl, $-OR_5$, $-NR_5R_6$, $-C_{0-6}$ alkyl-$C_{3-8}$ carbocyclyl and $-C_{0-6}$ alkyl-3-8 membered heterocyclyl; wherein the $-C_{1-6}$ alkyl, $-C_{0-6}$ alkyl-$C_{3-8}$ carbocyclyl, $-C_{0-6}$ alkyl-3-8 membered heterocyclyl, $R_5$ and $R_6$ are optionally substituted with one or more substituents selected from hydrogen, halogen, CN, OH, $NH_2$, $-C_{1-6}$ alkyl, $-C_{2-6}$ alkenyl, $-C_{2-6}$ alkynyl, $-C_{1-6}$ alkoxy, $-C_{0-6}$ alkyl-$C_{3-8}$ carbocyclyl or $-C_{0-6}$ alkyl-3-8 membered heterocyclyl.

**5.** The compound of any one of claims 1-4, or a stereoisomer, tautomer, deuterated compound, pharmaceutically acceptable salt, prodrug, chelate, non-covalent complex or solvate thereof, wherein $R_2$, $R_4$ are the same or different, and are each independently selected from the group consisting of halogen, $-C_{1-6}$ alkyl, $-OR_5$, $- NR_5R_6$, $-C_{0-6}$ alkyl-$C_{3-8}$ carbocyclyl and $-C_{0-6}$ alkyl-3-8 membered heterocyclyl; wherein the $-C_{1-6}$ alkyl, $-C_{0-6}$ alkyl-$C_{3-8}$ carbocyclyl, $-C_{0-6}$ alkyl-3-8 membered heterocyclyl, $R_5$ and $R_6$ are optionally substituted with one or more substituents selected from hydrogen, halogen, CN, OH, $NH_2$, $-C_{1-6}$ alkyl, $-C_{2-6}$ alkenyl, $-C_{2-6}$ alkynyl,$-C_{1-6}$ alkoxy, $-C_{0-6}$ alkyl-$C_{3-8}$ carbocyclyl or $-C_{0-6}$ alkyl-3-8 membered heterocyclyl.

**6.** The compound of any one of claims 1-5, or a stereoisomer, tautomer, deuterated compound, pharmaceutically acceptable salt, prodrug, chelate, non-covalent complex or a solvate thereof, wherein $R_1$, $R_2$, $R_3$, $R_4$ are the same or different, and are each independently selected from the group consisting of H, -F, -Cl, -Br, -I, - OH, $-NH_2$, -CN, $-CH_3$, $-CF_3$, $-CH_2CH_3$, $-CH(CH_3)_2$, $-CH_2CH(CH_3)_2$, $-C(CH_3)_3$, $- NHCH(CH_3)_2$, $-NHC(O)CH_3$, $-NHC(O)OCH_2CH_3$,

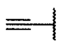, 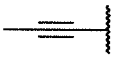, , 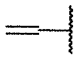, 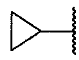, -

$OCH_3$, $-OCH(CH_3)_2$,

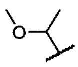, 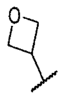, 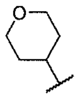, 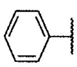, 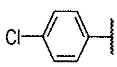, 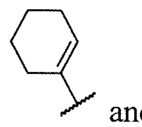 and 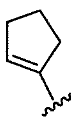.

**7.** The compound of any one of claims 1-6, or a stereoisomer, tautomer, deuterated compound, pharmaceutically acceptable salt, prodrug, chelate, non-covalent complex or solvate thereof, wherein ring A is selected from $-C_6$ carbocyclyl, 6 membered heterocyclyl, $-C_6$ aryl or 6 membered heteroaryl; preferably, ring A is

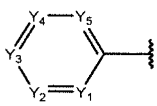 ;

wherein $Y_1$ is selected from $CR_A$ or N; $Y_2$ is selected from $CR_A$ or N; $Y_3$ is selected from $CR_A$ or N; $Y_4$ is selected from $CR_A$ or N; $R_A$ is defined as described in any one of claims 1-6.

**8.** The compound of any one of claims 1-7, or a stereoisomer, tautomer, deuterated compound, pharmaceutically acceptable salt, prodrug, a chelate, non-covalent complex or solvate thereof, wherein ring A is selected from phenyl, pyridyl, furyl, thienyl, pyrrolyl, pyrazolyl, pyrimidinyl, pyridazinyl, diazinyl, cyclopentenyl, pyridin-2(1H)-one or pyrimidine-2(1H )-keto group.

**9.** The compound of any one of claims 1-8, or a stereoisomer, tautomer, deuterated compound, pharmaceutically acceptable salt, prodrug, chelate, non-covalent complex or a solvate thereof, wherein each $R_A$ is the same or different, and is independently selected from the group consisting of hydrogen, oxo, -F, -Cl, -Br, -I, -CN, -OH, $-NH_2$, $-NO_2$, $-CH_3$, $-CF_3$, $-OCF_3$, $-NHCH_3$, $-N(CH_3)_2$, $-OCH_3$, $-SCH_3$, $- CHF_2$, $-OCHF_2$, $-C(O)NH_2$, $-C(O)CH_3$, $-OC(O)NH_2$, $-NHC(O)CH_3$, $-CH_2CH_3$, $- CH(CH_3)_2$,

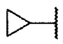, 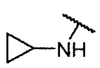,

$-SCH_3$, $-SOCH_3$, $-CH_2SO_2CH_3$, $-SO_2CH_3$, $-NHC(O)NH_2$, - $CH_2OH$, $-CH_2NH_2$, $-NHC(O)OCH_2CH_3$, -NHOH, $-CH_2OCH_3$,

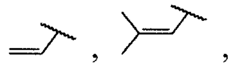

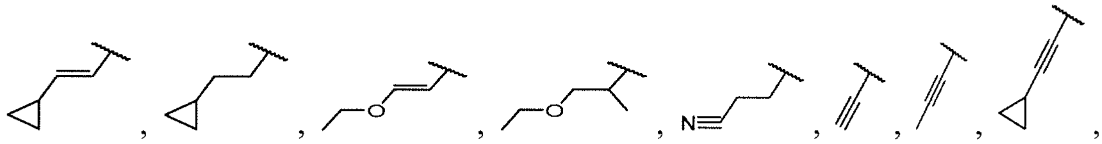

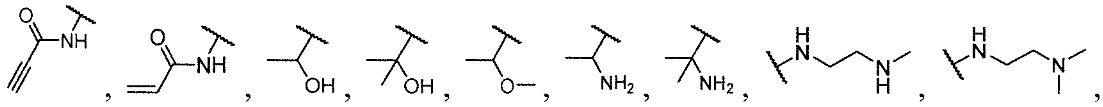

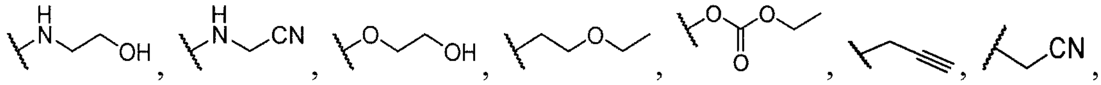

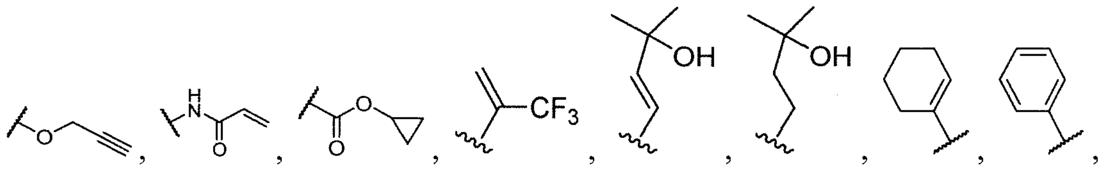

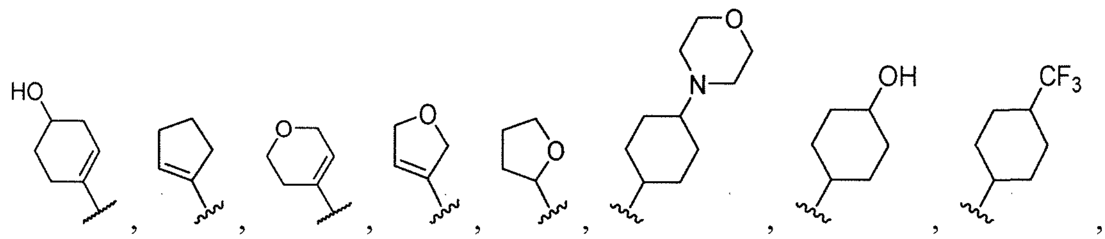

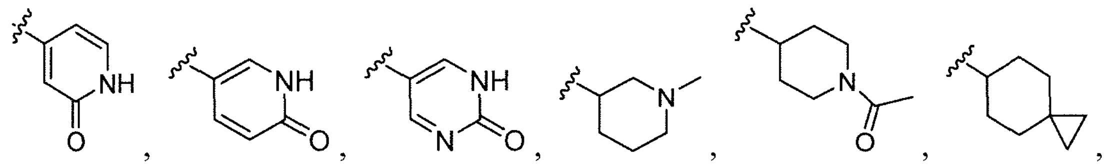

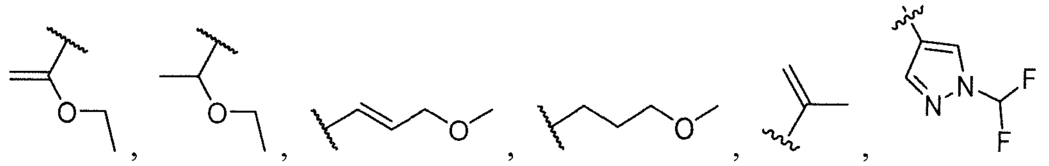

and

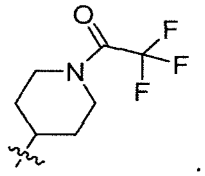.

10. The compound of any one of claims 1-9, or a stereoisomer, tautomer, deuterated compound, pharmaceutically acceptable salt, prodrug, chelate, non-covalent complex or a solvate thereof, wherein ring E is selected from 6-10 membered heterocyclyl or 6-10 membered heteroaryl.

11. The compound of any one of claims 1-10, or stereoisomer, tautomer, deuterated compound, pharmaceutically acceptable salt, prodrug, chelate, non-covalent complex or solvate thereof, wherein ring E is selected from pyridyl, benzo[d]oxazolyl, 3,4-dihydro-2H-benzo[b][l,4]oxazinyl, 1,6-naphthyridinyl, 2,3-dihydro- 1H-pyrido[2,3-b][1,4]oxazinyl, 1H-pyrazolo[4,3-b]pyridyl, 2H-pyrazolo[4,3-b]pyridyl, 1H-pyrazolo[3,4-b]pyridyl, pyrazolo[1,5-a]pyrimidinyl, imidazo[l,2-b]pyridazinyl, thiazolo[5,4-b] pyridyl or 1,5-naphthyridinyl.

12. The compound of any one of claims 1-11, or a stereoisomer, tautomer, deuterated compound, pharmaceutically acceptable salt, prodrug, chelate, non-covalent complex or solvate thereof, wherein each $R_E$ is the same or different, and is independently selected from the group consisting of hydrogen, halogen, oxo, CN, $-C_{1-6}$ alkyl, $-C_{0-6}$ alkyl-5-10 membered heteroaryl, $-C_{0-6}$ alkyl-$C_{3-8}$ carbocyclyl, $-C_{0-6}$ alkyl-3-8 membered heterocyclyl, $-OR_5$, $-NR_5R_6$, $-SR_5$, $-C(O)R_5$, $-S(O)R_5$, $-S(O)_2R_5$ and $-C(O)OR_5$; wherein the $-C_{1-6}$ alkyl, $-C_{0-6}$ alkyl-5-10 membered heteroaryl, $-C_{0-6}$ alkyl-$C_{3-8}$ carbocyclyl and $-C_{0-6}$ alkyl-3-8 membered heterocyclyl are optionally substituted with one or more substituents selected from hydrogen, halogen, CN, oxo, $- C_{1-6}$ alkyl, $-C_{2-6}$ alkenyl, $-C_{2-6}$ alkynyl, $-OR_8$, $-NR_8R_9$, $-C_{0-6}$ alkyl $-C_{3-8}$ carbocyclyl and $-C_{0-6}$ alkyl -3-8 membered heterocyclyl, $-C(O)OR_8$ or $-C(O)NR_8R_9$.

13. The compound of any one of claims 1-12, or a stereoisomer, tautomer, deuterated compound, pharmaceutically acceptable salt, prodrug, chelate, non-covalent complex or solvate thereof, wherein each $R_E$ is the same or different, and is independently selected from the group consisting of hydrogen, oxo, -F, -Cl, -CN, $- CH_3$, $-OCH_3$, $-CH_2OH$, $-CH_2CN$, $-CF_3$, $-C(O)NH_2$, $-C(O)NHCH_3$, $-C(O)NHCH_2CN$, $- C(O)NHCH_2CH_3$, $-C(O)NHCH_2CF_3$, $-C(O)NHCH_2CH_2OCF_3$, $-C(O)NHCH_2CH_2CH_3$, $-OCHF_2$, $-OCF_3$, $-SO_2CH_3$, $-SCH_3$, $-SCF_3$, $-NHC(O)NH_2$, $-NHC(O)CH_3$, $- NHC(O)OCH_3$, $-NHS(O)_2CH_3$,

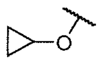, 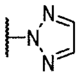, 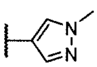, 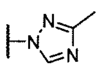, 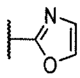, 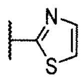, 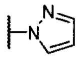,

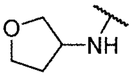, 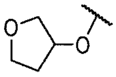, 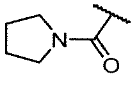, 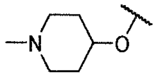, 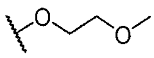, 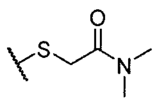, 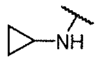,

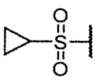, 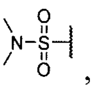, 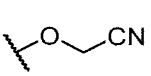, 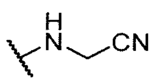, 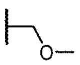, 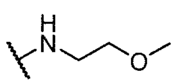, 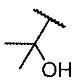, 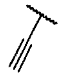,

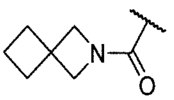, 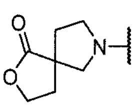, 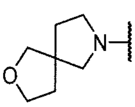, 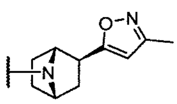, 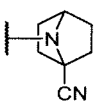, 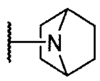, 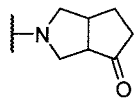,

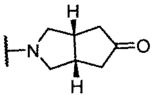, 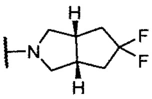, 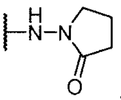, 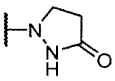, 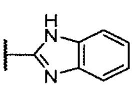, 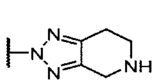, 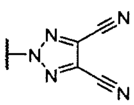,

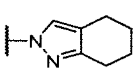, 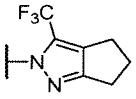, 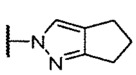, 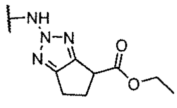, 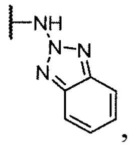, 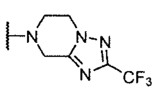, 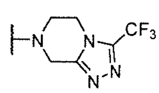,

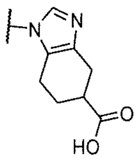, 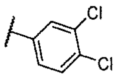, 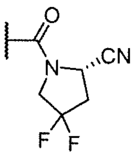, 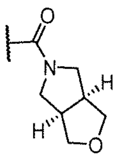, 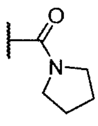, 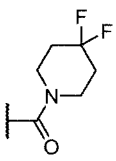, 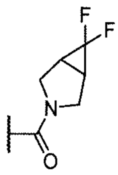, 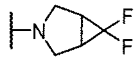,

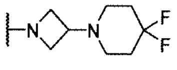, 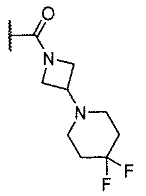, 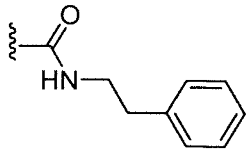, 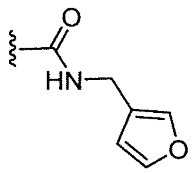, 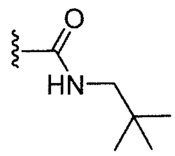,

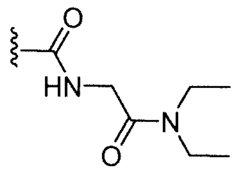, 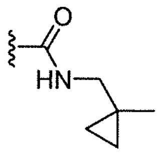, 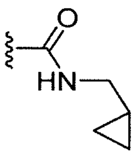, 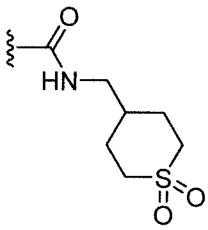, 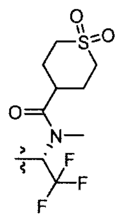,

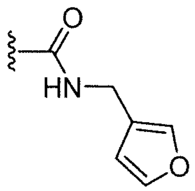, 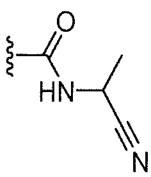, 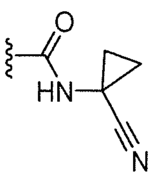, 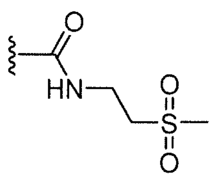, 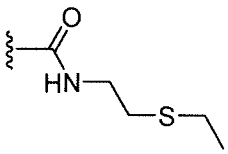 and

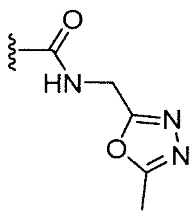.

**14.** The compound of any one of claims 1-13, or a stereoisomer, tautomer, deuterated compound, pharmaceutically acceptable salt, prodrug, chelate, non-covalent complex or solvate thereof, wherein

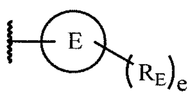

is selected from

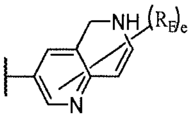, 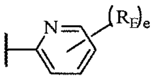, 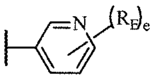, 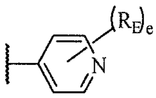, 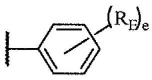, 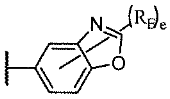,

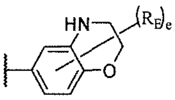, 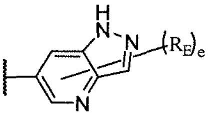, 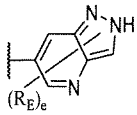, 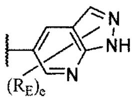, 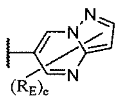, 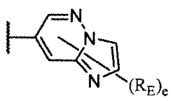,

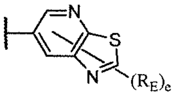, 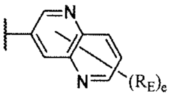 or 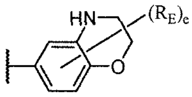;

preferably,

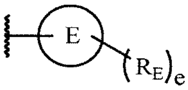

is selected from

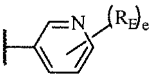, 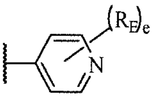 or 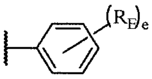;

more preferably,

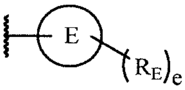 is

selected from

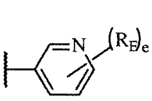 or 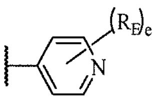.

**15.** The compound of any one of claims 1-14, or a stereoisomer, tautomer, deuterated compound, pharmaceutically acceptable salt, prodrug, chelate, non-covalent complex or solvate thereof, wherein the compound is selected from Formula II:

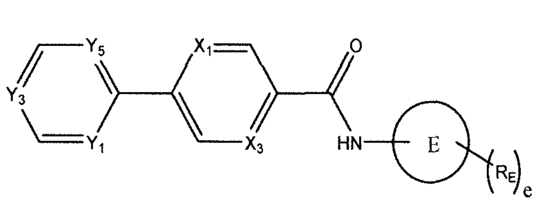

Formula II

wherein, ring E, $X_1$, $X_3$, $Y_1$, $Y_3$, $Y_5$, $R_E$, e are defined as described in any one of claims 1-14.

**16.** The compound of any one of claims 1-14, or a stereoisomer, tautomer, deuterated compound, pharmaceutically acceptable salt, prodrug, chelate, non-covalent complex or solvate thereof, wherein the compound is selected from Formula III:

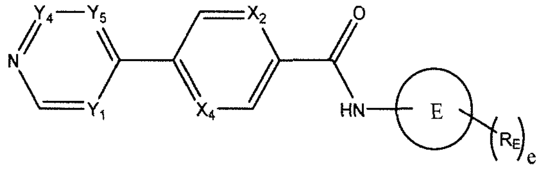

Formula III

wherein, ring E, $X_2$, $X_4$, $Y_1$, $Y_4$, $Y_5$, $R_E$, e are defined as described in any one of claims 1-14.

**17.** The compound of any one of claims 1-16, or a stereoisomer, a tautomer, a deuterated compound, a pharmaceutically acceptable salt, a prodrug, a chelate, a non-covalent complex, or a solvate thereof, wherein the compound is selected from:

N-(6-(2H-1,2,3-triazol-2-yl)-5-(trifluoromethyl)pyridin-3-yl)-5-(2-chloro-4-fluorophenyl)-4-methylpicolinamide;
N-(6-(2H-1,2,3-triazol-2-yl)-5-(trifluoromethyl)pyridin-3-yl)-2-fluoro-4-(3-methylpyridin-2-yl)benzamide;
N-(6-(2H-1,2,3-triazol-2-yl)-5-(trifluoromethyl)pyridin-3-yl)-2'-chloro-4'-fluoro-3-isopropyl-[1,1'-biphenyl]-4-carboxamide;
N-(6-(2H-1,2,3-triazol-2-yl)-5-(trifluoromethyl)pyridin-3-yl)-3,5'-dimethyl-[2,3'-bipyridine]-6'-carboxamide;
N-(6-(2H-1,2,3-triazol-2-yl)-5-(trifluoromethyl)pyridin-3-yl)-2-methoxy-4-(3-methylpyridin-2-yl)benzamide;
N-(6-(2H-1,2,3-triazol-2-yl)-5-(trifluoromethyl)pyridin-3-yl)-2'-chloro-4'-fluoro-3-methoxy-[1,1'-biphenyl]-4-carboxamide;
N-(6-(2H-1,2,3-triazol-2-yl)-5-(trifluoromethyl)pyridin-3-yl)-6-(2-chloro-4-fluorophenyl)-2-methoxynicotinamide;
N-(6-(2H-1,2,3-triazol-2-yl)-5-(trifluoromethyl)pyridin-3-yl)-2-(2-chloro-4-fluorophenyl)-4-methylpyrimidine-5-carboxamide;
N-(6-(2H-1,2,3-triazol-2-yl)-5-(trifluoromethyl)pyridin-3-yl)-4-methyl-2-(4-methylpyridin-3-yl)pyrimidine-5-carboxamide;
N-(6-(2H-1,2,3-triazol-2-yl)-5-(trifluoromethyl)pyridin-3-yl)-2'-chloro-4'-fluoro-3-methyl-[1,1'-biphenyl]-4-carboxamide;
N-(6-(2H-1,2,3-triazol-2-yl)-5-(trifluoromethyl)pyridin-3-yl)-2',3-dichloro-4'-fluoro-[1,1'-biphenyl]-4-carboxamide;
N-(6-(2H-1,2,3-triazol-2-yl)-5-(trifluoromethyl)pyridin-3-yl)-4-chloro-6-(2-chloro-4-fluorophenyl)nicotinamide;
N-(6-(2H-1,2,3-triazol-2-yl)-5-(trifluoromethyl)pyridin-3-yl)-2'-chloro-3-cyclopropyl-4'-fluoro-[1,1'-biphenyl]-4-carboxamide;
N-(6-(2H-1,2,3-triazol-2-yl)-5-(trifluoromethyl)pyridin-3-yl)-2-(4-cyano-2-methylphenyl)-4-methylpyrimidine-5-carboxamide;
N-(6-(2H-1,2,3-triazol-2-yl)-5-(trifluoromethyl)pyridin-3-yl)-5-(2-chloro-4-fluorophenyl)-3-methylpicolinamide;
N-(6-(2H-1,2,3-triazol-2-yl)-5-(trifluoromethyl)pyridin-3-yl)-2-(2-chloro-4-fluorophenyl)-4-(trifluoromethyl)pyrimidine-5-carboxamide;
N-(6-(2H-1,2,3-triazol-2-yl)-5-(trifluoromethyl)pyridin-3-yl)-2'-chloro-3,4'-difluoro-[1,1'-biphenyl]-4-carboxamide;
N-(6-(2H-1,2,3-triazol-2-yl)-5-(trifluoromethyl)pyridin-3-yl)-4-methyl-2-(2-(trifluoromethyl)phenyl)pyrimidine-5-carboxamide;
N-(6-(2H-1,2,3-triazol-2-yl)-5-(trifluoromethyl)pyridin-3-yl)-6-(2-chloro-4-fluorophenyl)-5-methylnicotinamide;
2-(2-chloro-4-fluorophenyl)-N-(5-chloro-6-(2H-1,2,3-triazol-2-yl)pyridin-3-yl)-4-methylpyrimidine-5-carboxamide;
N-(6-(2H-1,2,3-triazol-2-yl)-5-(trifluoromethyl)pyridin-3-yl)-6-(5-amino-2-chlorophenyl)-2-methoxynicotinamide;
N-(6-(2H-1,2,3-triazol-2-yl)-5-(trifluoromethyl)pyridin-3-yl)-6-methoxy-3'-methyl-[2,2'-bipyridine]-5-carboxamide;
N-(6-(2H-1,2,3-triazol-2-yl)-5-(trifluoromethyl)pyridin-3-yl)-6-(2-chloro-4-fluorophenyl)-4-methoxynicotinamide;
3-chloro-N-(5-chloro-6-(2H-1,2,3-triazol-2-yl)pyridin-3-yl)-2'-fluoro-6'-methyl-[1,1'-biphenyl]-4-carboxamide;
3,3'-dichloro-N-(5-chloro-6-(2H-1,2,3-triazol-2-yl)pyridin-3-yl)-2'-methyl-[1,1'-biphenyl]-4-carboxamide;

5-(3-chloro-2-methylphenyl)-N-(5-chloro-6-(2H-1,2,3-triazol-2-yl)pyridin-3-yl)-3-methylpicolinamide;
5-(2-chloro-4-fluorophenyl)-N-(5-chloro-6-(2H-1,2,3-triazol-2-yl)pyridin-3-yl)-3-methylpicolinamide;
N-(6-(2H-1,2,3-triazol-2-yl)-5-(trifluoromethyl)pyridin-3-yl)-2'-chloro-3-methyl-[1,1'-biphenyl]-4-carboxamide;
N-(6-(2H-1,2,3-triazol-2-yl)-5-(trifluoromethyl)pyridin-3-yl)-2'-amino-3-methyl-[1,1'-biphenyl]-4-carboxamide;
N-(6-(2H-1,2,3-triazol-2-yl)-5-(trifluoromethyl)pyridin-3-yl)-4'-chloro-3-methyl-[1,1'-biphenyl]-4-carboxamide;
N-(6-(2H-1,2,3-triazol-2-yl)-5-(trifluoromethyl)pyridin-3-yl)-2'-chloro-3,3'-dimethyl-[1,1'-biphenyl]-4-carboxamide;
N-(6-(2H-1,2,3-triazol-2-yl)-5-(trifluoromethyl)pyridin-3-yl)-2',3'-dichloro-3-methyl-[1,1'-biphenyl]-4-carboxamide;
N-(6-(2H-1,2,3-triazol-2-yl)-5-(trifluoromethyl)pyridin-3-yl)-2'-fluoro-3,6'-dimethyl-[1,1'-biphenyl]-4-carboxamide;
N4'-(6-(2H-1,2,3-triazol-2-yl)-5-(trifluoromethyl)pyridin-3-yl)-3'-methyl-[1,1'-biphenyl]-2,4'-dicarboxamide;
2-chloro-N-(5-chloro-6-(2H-1,2,3-triazol-2-yl)pyridin-3-yl)-4-(3-methylpyridin-4-yl)benzamide;
2',3-dichloro-N-(5-chloro-6-(2H-1,2,3-triazol-2-yl)pyridin-3-yl)-[1,1'-biphenyl]-4-carboxamide;
3-chloro-N-(5-chloro-6-(2H-1,2,3-triazol-2-yl)pyridin-3-yl)-2'-(trifluoromethoxy)-[1,1'-biphenyl]-4-carboxamide;
3-chloro-N-(5-chloro-6-(2H-1,2,3-triazol-2-yl)pyridin-3-yl)-3'-cyano-[1,1'-biphenyl]-4-carboxamide;
N-(5-chloro-6-(2H-1,2,3-triazol-2-yl)pyridin-3-yl)-5-(3-chlorophenyl)-3-methylpicolinamide;
N-(6-(2H-1,2,3-triazol-2-yl)-5-(trifluoromethyl)pyridin-3-yl)-3'-chloro-3-methyl-[1,1'-biphenyl]-4-carboxamide;
N-(6-(2H-1,2,3-triazol-2-yl)-5-(trifluoromethyl)pyridin-3-yl)-2'-cyano-3-methyl-[1,1'-biphenyl]-4-carboxamide;
N-(6-(2H-1,2,3-triazol-2-yl)-5-(trifluoromethyl)pyridin-3-yl)-2'-fluoro-3-methyl-4'-(trifluoromethyl)-[1,1'-biphenyl]-4-carboxamide;
N-(6-(2H-1,2,3-triazol-2-yl)-5-(trifluoromethyl)pyridin-3-yl)-2-methyl-4-(6-(trifluoromethyl)pyridin-3-yl)benzamide;
N-(6-(2H-1,2,3-triazol-2-yl)-5-(trifluoromethyl)pyridin-3-yl)-4-(furan-2-yl)-2-methylbenzamide;
N-(6-(2H-1,2,3-triazol-2-yl)-5-(trifluoromethyl)pyridin-3-yl)-4-(cyclopent-1-en-1-yl)-2-methylbenzamide;
N-(6-(2H-1,2,3-triazol-2-yl)-5-(trifluoromethyl)pyridin-3-yl)-5-(2-cyanophenyl)-3-methylpicolinamide;
N-(6-(2H-1,2,3-triazol-2-yl)-5-(trifluoromethyl)pyridin-3-yl)-2-methyl-4-(1-methyl-1H-pyrazol-5-yl)benzamide;
N-(6-(2H-1,2,3-triazol-2-yl)-5-(trifluoromethyl)pyridin-3-yl)-2'-chloro-3-cyano-[1,1'-biphenyl]-4-carboxamide;
N-(6-(2H-1,2,3-triazol-2-yl)-5-(trifluoromethyl)pyridin-3-yl)-4'-chloro-3'-cyano-3-methyl-[1,1'-biphenyl]-4-carboxamide;
N-(6-(2H-1,2,3-triazol-2-yl)-5-(trifluoromethyl)pyridin-3-yl)-2-methyl-4-(4-methylpyridin-3-yl)benzamide;
N-(6-(2H-1,2,3-triazol-2-yl)-5-(trifluoromethyl)pyridin-3-yl)-2-methyl-4-(1-oxoisoindolin-5-yl)benzamide;
N-(6-(2H-1,2,3-triazol-2-yl)-5-(trifluoromethyl)pyridin-3-yl)-2-methyl-4-(quinolin-4-yl)benzamide;
N4'-(6-(2H-1,2,3-triazol-2-yl)-5-(trifluoromethyl)pyridin-3-yl)-3'-methyl-[1,1'-biphenyl]-3,4'-dicarboxamide;
N-(6-(2H-1,2,3-triazol-2-yl)-5-(trifluoromethyl)pyridin-3-yl)-2'-methoxy-3-(trifluoromethyl)-[1,1'-biphenyl]-4-carboxamide;
N-(6-(2H-1,2,3-triazol-2-yl)-5-(trifluoromethyl)pyridin-3-yl)-2-methyl-4-(1H-pyrrolo[2,3-b]pyridin-5-yl)benzamide;
N-(6-(2H-1,2,3-triazol-2-yl)-5 -(trifluoromethyl)pyridin-3 -yl)-2'-methoxy-3 - methyl-[1,1 '-biphenyl]-4-carboxamide;
N-(6-(2H-1,2,3-triazol-2-y1)-5-(trifluoromethyl)pyridin-3-y1)-4-(6-fluoropyridin-3-yl)-2-methylbenzamide;
N-(6-(2H-l,2,3-triazol-2-yl)-5-(trifluoromethyl)pyridin-3-yl)-4-(6-cyanopyridin-3 -yl)-2-methylbenzamide;
N-(6-(2H-1,2,3-triazol-2-yl)-5-(trifluoromethyl)pyridin-3-yl)-5-chloro-2-fluoro-2'-methoxy-[1,1'-biphenyl]-4-carboxamide;
N-(6-(2H-1,2,3-triazol-2-yl)-5-(trifluoromethyl)pyridin-3-yl)-2'-amino-3-(trifluoromethyl)-[1,1 '-biphenyl]-4-carboxamide;
N-(6-(2H-1,2,3-triazol-2-yl)-5-(trifluoromethyl)pyridin-3-yl)-2'-amino-5-chloro-2-fluoro-[1,1'-biphenyl]-4-carboxamide;
N-(6-(2H-1,2,3-triazol-2-yl)-5-(trifluoromethyl)pyridin-3-yl)-2'-acetamido-3-(trifluoromethyl)-[1,1'-biphenyl]-4-carboxamide;
N-(6-(2H-1,2,3-triazol-2-yl)-5-(trifluoromethyl)pyridin-3-yl)-4-(4-chloropyridin-3-yl)-2-(trifluoromethyl)benzamide;
N-(6-(2H-1,2,3-triazol-2-yl)-5-(trifluoromethyl)pyridin-3-yl)-2'-(dimethylamino)-3-(trifluoromethyl)-[1,1'-biphenyl]-4-carboxamide;
N-(6-(2H-1,2,3-triazol-2-yl)-5-(trifluoromethyl)pyridin-3-yl)-2'-(dimethylamino)-3-methyl-[1,1'-biphenyl]-4-carboxamide;
N-(6-(2H-1,2,3-triazol-2-yl)-5-(trifluoromethyl)pyridin-3-yl)-3-chloro-2'-(dimethylamino)-[1,1'-biphenyl]-4-carboxamide;

N-(6-(2H-1,2,3-triazol-2-yl)-5-(trifluoromethyl)pyridin-3-yl)-3-cyano-2'-(dimethylamino)-[1,1'-biphenyl]-4-carboxamide;
N-(6-(2H-1,2,3-triazol-2-yl)-5-(trifluoromethyl)pyridin-3-yl)-5-chloro-2'-(dimethylamino)-2-fluoro-[1,1'-biphenyl]-4-carboxamide;
N-(5-chloro-6-(2H-1,2,3-triazol-2-yl)pyridin-3-yl)-4',5-dimethyl-[3,3'-bipyridine]-6-carboxamide;
3'-chloro-N4'-(5-chloro-6-(2H-1,2,3-triazol-2-yl)pyridin-3-yl)-[1,1'-biphenyl]-3,4'-dicarboxamide;
2-chloro-N-(5-chloro-6-(2H-1,2,3-triazol-2-yl)pyridin-3-yl)-4-(1-methyl-1H-pyrazol-3 -yl)benzamide;
2'-chloro-N-(5-chloro-6-(2H-1,2,3-triazol-2-yl)pyridin-3-yl)-4'-fluoro-[1,1'-biphenyl]-4-carboxamide;
2'-amino-N-(5-chloro-6-(2H-1,2,3-triazol-2-yl)pyridin-3-yl)-[1,1'-biphenyl]-4-carboxamide;
N-(6-(2H-1,2,3-triazol-2-yl)-5-(trifluoromethyl)pyridin-3-yl)-3-chloro-2'-methoxy-[1,1'-biphenyl]-4-carboxamide;
N-(6-(2H-1,2,3-triazol-2-yl)-5-(trifluoromethyl)pyridin-3-yl)-2'-amino-3-fluoro-[1,1'-biphenyl]-4-carboxamide;
N-(6-(2H-1,2,3-triazol-2-yl)-5-(trifluoromethyl)pyridin-3-yl)-2'-acetamido-3-fluoro-[1,1'-biphenyl]-4-carboxamide;
N-(6-(2H-1,2,3-triazol-2-yl)-5-(trifluoromethyl)pyridin-3-yl)-3-fluoro-2'-methoxy-[1,1'-biphenyl]-4-carboxamide;
N-(6-(2H-1,2,3-triazol-2-yl)-5-(trifluoromethyl)pyridin-3-yl)-3,4'-difluoro-2'-hydroxy-[1,1'-biphenyl]-4-carboxamide;
N-(6-(2H-1,2,3-triazol-2-yl)-5-(trifluoromethyl)pyridin-3-yl)-3-fluoro-2'-hydroxy-[1,1'-biphenyl]-4-carboxamide;
N-(6-(2H-l,2,3-triazol-2-yl)-5-(trifluoromethyl)pyridin-3-yl)-3-cyano-4'-fluoro-2'-hydroxy-[1,1'-biphenyl]-4-carboxamide;
N-(6-(2H-1,2,3-triazol-2-yl)-5-(trifluoromethyl)pyridin-3-yl)-3-cyano-2'-hydroxy-[1,1 '-biphenyl]-4-carboxamide;
3-chloro-N-(5-chloro-6-(2H-1,2,3-triazol-2-yl)pyridin-3-yl)-4'-fluoro-2'-hydroxy-[1,1 '-biphenyl]-4-carboxamide;
3-chloro-N-(5-chloro-6-(2H-1,2,3-triazol-2-yl)pyridin-3-yl)-4'-fluoro-[1,1'-biphenyl]-4-carboxamide;
2-chloro-N-(5-chloro-6-(2H-1,2,3-triazol-2-yl)pyridin-3-yl)-4-(3-chloropyridin-4-yl)benzamide;
3-chloro-N-(5-chloro-6-(2H-1,2,3-triazol-2-yl)pyridin-3-yl)-2',4'-difluoro-[1,1'-biphenyl]-4-carboxamide;
2'-bromo-3-chloro-N-(5-chloro-6-(2H-1,2,3-triazol-2-yl)pyridin-3-yl)-4'-fluoro-[1,1'-biphenyl]-4-carboxamide;
3-chloro-N-(5-chloro-6-(2H-1,2,3-triazol-2-yl)pyridin-3-yl)-4'-fluoro-2'-(methylthio)-[1,1'-biphenyl]-4-carboxamide;
3-chloro-N-(5-chloro-6-(2H-1,2,3-triazol-2-yl)pyridin-3-yl)-4'-fluoro-2'-methoxy-[1,1'-biphenyl]-4-carboxamide;
N-(6-(2H-1,2,3-triazol-2-yl)-5-(trifluoromethyl)pyridin-3-yl)-2',5-dichloro-2,4'-difluoro-[1,1'-biphenyl]-4-carboxamide;
N-(6-(2H-1,2,3-triazol-2-yl)-5-(trifluoromethyl)pyridin-3-yl)-2',5-dichloro-2-fluoro-[1,1 '-biphenyl]-4-carboxamide;
2'-amino-5-chloro-N-(5-chloro-6-(2H-1,2,3-triazol-2-yl)pyridin-3-yl)-2-fluoro-[1,1'-biphenyl]-4-carboxamide;
N-(6-(2H-1,2,3-triazol-2-yl)-5-(trifluoromethyl)pyridin-3-yl)-2'-amino-2-chloro-5-fluoro-[1,1 '-biphenyl]-4-carboxamide;
N-(6-(2H-1,2,3-triazol-2-yl)-5-(trifluoromethyl)pyridin-3-yl)-2'-amino-2,5-dichloro-[1,1'-biphenyl]-4-carboxamide;
N-(6-(2H-1,2,3-triazol-2-yl)-5-(trifluoromethyl)pyridin-3-yl)-5-chloro-2,4'-difluoro-2'-methoxy-[1,1'-biphenyl]-4-carboxamide;
2'-amino-N-(5-chloro-6-(2H-1,2,3-triazol-2-yl)pyridin-3-yl)-2,4'-difluoro-5-methyl-[1,1'-biphenyl]-4-carboxamide;
2'-bromo-N-(5-chloro-6-(2H-1,2,3-triazol-2-yl)pyridin-3-yl)-2,4'-difluoro-5-methyl-[1,1'-biphenyl]-4-carboxamide;
2'-chloro-N-(5-chloro-6-(2H-1,2,3-triazol-2-yl)pyridin-3-yl)-2,4'-difluoro-5-methyl-[1,1'-biphenyl]-4-carboxamide;
N-(5-chloro-6-(2H-1,2,3-triazol-2-yl)pyridin-3-yl)-2,2',4'-trifluoro-5-methyl-[1,1'-biphenyl]-4-carboxamide;
N-(5-chloro-6-(2H-1,2,3-triazol-2-yl)pyridin-3-yl)-2-fluoro-2'-methoxy-5-methyl-[1,1'-biphenyl]-4-carboxamide;
N-(5-chloro-6-(2H-1,2,3-triazol-2-yl)pyridin-3-yl)-2,4'-difluoro-2'-methoxy-5-methyl-[1,1'-biphenyl]-4-carboxamide;
2'-amino-2-chloro-N-(5-chloro-6-(2H-1,2,3-triazol-2-yl)pyridin-3-yl)-5-fluoro-[1,1'-biphenyl]-4-carboxamide;
2'-amino-2-chloro-N-(5-chloro-6-(2H-1,2,3-triazol-2-yl)pyridin-3-yl)-4',5-difluoro-[1,1'-biphenyl]-4-carboxamide;
2'-amino-5-chloro-N-(5-chloro-6-(2H-1,2,3-triazol-2-yl)pyridin-3-yl)-2,4'-difluoro-[1,1'-biphenyl]-4-carboxamide;
N-(6-(2H-1,2,3-triazol-2-yl)-5-(trifluoromethyl)pyridin-3-yl)-2'-amino-5-chloro-2,4'-difluoro-[1,1'-biphenyl]-4-carboxamide;
N-(6-(2H-1,2,3-triazol-2-yl)-5-(trifluoromethyl)pyridin-3-yl)-2'-amino-2-chloro-4',5-difluoro-[1,1'-biphenyl]-4-carboxamide;

4-(3-aminopyridin-4-yl)-5-chloro-N-(5-chloro-6-(2H-1,2,3-triazol-2-yl)pyridin-3-yl)-2-fluorobenzamide formate;
4-(3-aminopyridin-4-yl)-N-(5-chloro-6-(2H-1,2,3-triazol-2-yl)pyridin-3-yl)-5-fluoro-2-methylbenzamide;
N-(5-chloro-6-(2H-1,2,3-triazol-2-yl)pyridin-3-yl)-4-(3-chloropyridin-4-yl)-5-fluoro-2-methylbenzamide;
2-chloro-N-(5-chloro-6-(2H-1,2,3-triazol-2-yl)pyridin-3-yl)-4',5-difluoro-2'-(methylamino)-[1,1'-biphenyl]-4-carboxamide;
N-(6-(2H-1,2,3-triazol-2-yl)-5-(trifluoromethyl)pyridin-3-yl)-4-(3-amino-5-fluoropyridin-2-yl)-2-chloro-5-fluorobenzamide;
N-(6-(2H-1,2,3-triazol-2-yl)-5-(trifluoromethyl)pyridin-3-yl)-3-chloro-2'-(difluoromethoxy)-4'-fluoro-[1,1'-biphenyl]-4-carboxamide;
4-(4-aminopyrimidin-5-yl)-2-chloro-N-(5-chloro-6-(2H-1,2,3-triazol-2-yl)pyridin-3 -yl)-5-fluorobenzamide;
4-(3-aminopyridazin-4-yl)-2-chloro-N-(5-chloro-6-(2H-1,2,3-triazol-2-yl)pyridin-3 -yl)-5-fluorobenzamide;
2-chloro-N-(5-chloro-6-(2H-1,2,3-triazol-2-yl)pyridin-3-yl)-4-(3-chloropyridazin-4-yl)-5-fluorobenzamide;
4-(6-amino-2-oxo-1,2-dihydropyrimidin-5-yl)-2-chloro-N-(5-chloro-6-(2H-1,2,3-triazol-2-yl)pyridin-3-yl)-5-fluorobenzamide;
2'-amino-N-(5-chloro-6-(2H-1,2,3-triazol-2-yl)pyridin-3-yl)-4',5-difluoro-2-methoxy-[1,1'-biphenyl]-4-carboxamide;
4-(3-aminopyridin-4-yl)-N-(5-chloro-6-(2H-1,2,3-triazol-2-yl)pyridin-3-yl)-2-fluoro-5-methoxybenzamide;
2'-bromo-N-(5-chloro-6-(2H-1,2,3-triazol-2-yl)pyridin-3-yl)-4',5-difluoro-2-methoxy-[1,1'-biphenyl]-4-carboxamide;
N-(6-(2H-1,2,3-triazol-2-yl)-5-(trifluoromethyl)pyridin-3-yl)-3-chloro-4'-fluoro-2'-iodo-[1,1 '-biphenyl] -4-carboxamide;
N-(6-(2H-1,2,3-triazol-2-yl)-5-(trifluoromethyl)pyridin-3-yl)-2',3-dibromo-4'-fluoro-[1,1 '-biphenyl] -4-carboxamide;
N-(6-(2H-1,2,3-triazol-2-yl)-5-(trifluoromethyl)pyridin-3-yl)-2'-amino-3-bromo-4'-fluoro-[1,1 '-biphenyl] -4-carboxamide;
N-(6-(2H-1,2,3-triazol-2-yl)-5-(trifluoromethyl)pyridin-3-yl)-5-chloro-2,4'-difluoro-2'-iodo-[1,1'-biphenyl]-4-carboxamide;
N-(6-(2H-1,2,3-triazol-2-yl)-5-(trifluoromethyl)pyridin-3-yl)-3-bromo-4'-fluoro-2'-iodo-[1,1'-biphenyl]-4-carboxamide;
N-(6-(2H-1,2,3-triazol-2-yl)-5-(trifluoromethyl)pyridin-3-yl)-2'-amino-5-bromo-2,4'-difluoro-[1,1'-biphenyl]-4-carboxamide;
N-(6-(2H-1,2,3-triazol-2-yl)-5-(trifluoromethyl)pyridin-3-yl)-2-(2-amino-4-fluorophenyl)-4-methylpyrimidine-5-carboxamide;
N-(6-(2H-l,2,3-triazol-2-yl)-5-(trifluoromethyl)pyridin-3-yl)-5-bromo-2,4'-difluoro-2'-iodo-[1,1 '-biphenyl]-4-carboxamide;
N-(6-(2H-1,2,3-triazol-2-yl)-5-(trifluoromethyl)pyridin-3-yl)-2'-amino-4'-fluoro-3-iodo-[1,1'-biphenyl]-4-carboxamide;
N-(6-(2H-1,2,3-triazol-2-yl)-5-(trifluoromethyl)pyridin-3-yl)-2'-bromo-5-chloro-2,4'-difluoro-[1,1'-biphenyl]-4-carboxamide;
N-(6-(2H-1,2,3 -triazol-2-yl)-5 -(trifluoromethyl)pyridin-3 -yl)-2,4'-difluoro-2',5 - diiodo-[1,1'-biphenyl]-4-carboxamide;
N-(6-(2H-l,2,3-triazol-2-yl)-5-(trifluoromethyl)pyridin-3-yl)-2'-amino-2,4'-difluoro-5-iodo-[1,1'-biphenyl]-4-carboxamide;
N-(6-(2H-1,2,3-triazol-2-yl)-5-(trifluoromethyl)pyridin-3-yl)-2'-amino-2,4',5-trifluoro-[1,1'-biphenyl]-4-carboxamide;
N-(6-(2H-1,2,3-triazol-2-yl)-5-(trifluoromethyl)pyridin-3-yl)-2'-chloro-2,4',5-trifluoro-[1,1'-biphenyl]-4-carboxamide;
N-(6-(2H-1,2,3-triazol-2-yl)-5-(trifluoromethyl)pyridin-3-yl)-4-(3-aminopyridin-4-yl)-2,5-difluorobenzamide;
N-(6-(2H-1,2,3-triazol-2-yl)-5-(trifluoromethyl)pyridin-3-yl)-2'-amino-2-chloro-4',5-difluoro-[1,1'-biphenyl]-4-carboxamide;
2'-amino-2-bromo-N-(5-chloro-6-(2H-1,2,3-triazol-2-yl)pyridin-3-yl)-4',5-difluoro-[1,1 '-biphenyl]-4-carboxamide;
2'-bromo-2-chloro-N-(5-chloro-6-(2H-1,2,3-triazol-2-yl)pyridin-3-yl)-4',5-difluoro-[1,1 '-biphenyl] -4-carboxamide;
2'-amino-N-(5-chloro-6-(2H-1,2,3-triazol-2-yl)pyridin-3-yl)-4',5-difluoro-2-iodo-[1,1'-biphenyl]-4-carboxamide;
2-chloro-N-(5-chloro-6-(2H-l,2,3-triazol-2-yl)pyridin-3-yl)-4',5-difluoro-2'-iodo-[1,1'-biphenyl]-4-carboxamide;
N-(6-(2H-1,2,3-triazol-2-yl)-5-(trifluoromethyl)pyridin-3-yl)-4-(3-bromopyridin-4-yl)-2-chlorobenzamide;
N-(6-(2H-1,2,3-triazol-2-yl)-5-(trifluoromethyl)pyridin-3-yl)-4-(3-aminopyridin-4-yl)-2-bromo-5-fluorobenzamide;
N-(6-(2H-1,2,3-triazol-2-yl)-5-(trifluoromethyl)pyridin-3-yl)-4-(3-bromopyridin-4-yl)-2-chloro-5-fluorobenzamide;
N-(6-(2H-1,2,3-triazol-2-yl)-5-(trifluoromethyl)pyridin-3-yl)-4-(3-aminopyridin-4-yl)-5-fluoro-2-iodobenzamide;
N-(6-(2H-1,2,3-triazol-2-yl)-5-(trifluoromethyl)pyridin-3-yl)-2-chloro-5-fluoro-4-(3-iodopyridin-4-yl)benzamide;
N-(6-(2H-1,2,3-triazol-2-yl)-5-(trifluoromethyl)pyridin-3-yl)-4-(3-aminopyridin-4-yl)-5-fluoro-2-methylbenzamide;
N-(6-(2H-1,2,3-triazol-2-yl)-5-(trifluoromethyl)pyridin-3-yl)-4-(3-aminopyridin-4-yl)-2-chloro-5-fluorobenzamide;
N-(6-(2H-1,2,3-triazol-2-yl)-5-(trifluoromethyl)pyridin-3-yl)-2-chloro-5-fluoro-4-(3-methoxypyridin-4-yl)benzamide;
N-(6-(2H-1,2,3-triazol-2-yl)-5-(trifluoromethyl)pyridin-3-yl)-4-(3-chloropyridin-4-yl)-2,5-difluorobenzamide;
N-(6-(2H-1,2,3-triazol-2-yl)-5-(trifluoromethyl)pyridin-3-yl)-2-chloro-4-(3-chloropyridin-4-yl)-5-fluorobenzamide;
2,2'-dibromo-N-(5-chloro-6-(2H-1,2,3-triazol-2-yl)pyridin-3-yl)-4',5-difluoro-[1,1'-biphenyl]-4-carboxamide;
4-(3-aminopyridin-4-yl)-5-chloro-N-(5-chloro-6-(2H-1,2,3-triazol-2-yl)pyridin-3-yl)-2-fluorobenzamide;
4-(3-bromopyridin-4-yl)-5-chloro-N-(5-chloro-6-(2H-1,2,3-triazol-2-yl)pyridin-3-yl)-2-fluorobenzamide;
5-chloro-N-(5-chloro-6-(2H-1,2,3-triazol-2-yl)pyridin-3-yl)-4-(3-chloropyridin-4-yl)-2-fluorobenzamide;
(S)-4-(3-aminopyridin-4-yl)-N-(5-chloro-6-(2H-1,2,3-triazol-2-yl)pyridin-3-yl)-2-fluoro-5-(1-methoxyethyl)benzamide;
(S)-4-(3-amino-5-fluoropyridin-2-yl)-N-(5-chloro-6-(2H-1,2,3-triazol-2-yl)pyridin-3-yl)-2-fluoro-5-(1-methoxyethyl)benzamide;
2'-amino-N-(5-chloro-6-(2H-1,2,3-triazol-2-yl)pyridin-3-yl)-4',5-difluoro-2-isopropoxy-[1,1'-biphenyl]-4-carboxamide;
2'-bromo-N-(5-chloro-6-(2H-1,2,3-triazol-2-yl)pyridin-3-yl)-4',5-difluoro-2-isopropoxy-[1,1'-biphenyl]-4-carboxamide;
4-(3-aminopyridin-4-yl)-N-(5-chloro-6-(2H-1,2,3-triazol-2-yl)pyridin-3-yl)-2-fluoro-5-methylbenzamide;
4-(3-aminopyridin-4-yl)-N-(5-chloro-6-(2H-1,2,3-triazol-2-yl)pyridin-3-yl)-2-fluoro-5-isopropylbenzamide;
2'-amino-N-(5-chloro-6-(2H-1,2,3-triazol-2-yl)pyridin-3-yl)-4',5-difluoro-2-isobutyl-[1,1'-biphenyl]-4-carboxamide;
2'-bromo-N-(5-chloro-6-(2H-1,2,3-triazol-2-yl)pyridin-3-yl)-4',5-difluoro-2-isobutyl-[1,1'-biphenyl]-4-carboxamide;
4-(3-aminopyridin-4-yl)-N-(5-chloro-6-(2H-1,2,3-triazol-2-yl)pyridin-3 -yl)-5 - cyclopropyl-2-fluorobenzamide;
4-(3-aminopyridin-4-yl)-N-(5-chloro-6-(2H-1,2,3-triazol-2-yl)pyridin-3-yl)-2-fluoro-5-isopropoxybenzamide;
2'-amino-N-(5-chloro-6-(2H-l,2,3-triazol-2-yl)pyridin-3-yl)-2-cyclopropyl-4',5-difluoro-[1,1 '-biphenyl]-4-carboxamide;
2'-bromo-N-(5-chloro-6-(2H-1,2,3-triazol-2-yl)pyridin-3-yl)-2-cyclopropyl-4',5-difluoro-[1,1'-biphenyl]-4-carboxamide;
4-(3-aminopyridin-4-yl)-N-(5-chloro-6-(2H-1,2,3-triazol-2-yl)pyridin-3-yl)-2-fluoro-5-(tetrahydro-2H-pyran-4-yl)benzamide;
4-(3-aminopyridin-4-yl)-N-(5-chloro-6-(2H-1,2,3-triazol-2-yl)pyridin-3-yl)-5-ethynyl-2-fluorobenzamide;
2'-amino-N-(5-chloro-6-(2H-1,2,3-triazol-2-yl)pyridin-3-yl)-2-ethynyl-4',5-difluoro-[1,1'-biphenyl]-4-carboxamide;
2'-bromo-N-(5-chloro-6-(2H-1,2,3-triazol-2-yl)pyridin-3-yl)-2-ethynyl-4',5-difluoro-[1,1'-biphenyl]-4-carboxamide;
4-(3-aminopyridin-4-yl)-5-(tert-butyl)-N-(5-chloro-6-(2H-1,2,3-triazol-2-yl)pyridin-3-yl)-2-fluorobenzamide;
4-(3-aminopyridin-4-yl)-N-(5-chloro-6-(2H-1,2,3-triazol-2-yl)pyridin-3-yl)-2-fluoro-5-isobutylbenzamide;
2'-bromo-N-(5-chloro-6-(2H-1,2,3-triazol-2-yl)pyridin-3-yl)-4',5-difluoro-2-(oxetan-3-yl)-[1,1'-biphenyl]-4-carboxamide;
4-(3-aminopyridin-4-yl)-N-(5-chloro-6-(2H-1,2,3-triazol-2-yl)pyridin-3-yl)-2-fluoro-5-(oxetan-3 -yl)benzamide;
4-(3-aminopyridin-4-yl)-N-(5-chloro-6-(2H-1,2,3-triazol-2-yl)pyridin-3-yl)-5-ethyl-2-fluorobenzamide;
2'-amino-N-(5-chloro-6-(2H-1,2,3-triazol-2-yl)pyridin-3-yl)-4',5-difluoro-2-isopropyl-[1,1'-biphenyl]-4-carboxamide;
2'-bromo-N-(5-chloro-6-(2H-1,2,3-triazol-2-yl)pyridin-3-yl)-4',5-difluoro-2-isopropyl-[1,1'-biphenyl]-4-carboxamide;
5-chloro-N-(5-chloro-6-(2H-1,2,3-triazol-2-yl)pyridin-3-yl)-2'-(difluoromethyl)-2,4'-difluoro-[1,1'-biphenyl]-4-carboxamide;
5-chloro-N-(5-chloro-6-(2H-1,2,3-triazol-2-yl)pyridin-3-yl)-2'-(difluoromethoxy)-2,4'-difluoro-[1,1'-biphenyl]-4-carboxamide;
2-chloro-N-(5-chloro-6-(2H-1,2,3-triazol-2-yl)pyridin-3-yl)-5-fluoro-4-(6-oxo-1,6-dihydropyridin-3-yl)benzamide;
2-chloro-N-(5-chloro-6-(2H-1,2,3-triazol-2-yl)pyridin-3-yl)-5-fluoro-4-(1-methyl-6-oxo-1,6-dihydropyridin-3 -yl)benzamide;
2-chloro-N-(5-chloro-6-(2H-1,2,3-triazol-2-yl)pyridin-3-yl)-5-fluoro-4-(5-fluoro-1-methyl-6-oxo-1,6-dihydropyridin-3-yl)benzamide;
N-(5-chloro-6-(2H-1,2,3-triazol-2-yl)pyridin-3-yl)-2,4'-difluoro-2'-hydroxy-5-methyl-[1,1 '-biphenyl]-4-carboxamide;
N-(5-chloro-6-(2H-1,2,3-triazol-2-yl)pyridin-3-yl)-5-fluoro-4-(3-hydroxypyridin-4-yl)-2-methylbenzamide;
4-(5-aminopyrimidin-4-yl)-N-(5-chloro-6-(2H-1,2,3-triazol-2-yl)pyridin-3-yl)-5-fluoro-2-methylbenzamide;
2-chloro-N-(5-chloro-6-(2H-1,2,3-triazol-2-yl)pyridin-3-yl)-5-fluoro-4-(5-fluoro-3-methylpyridin-2-yl)benzamide;
5-chloro-N-(5-chloro-6-(2H-1,2,3-triazol-2-yl)pyridin-3-yl)-2'-(difluoromethyl)-2,4'-difluoro-[1,1'-biphenyl]-4-carboxamide;
2-chloro-N-(5-chloro-6-(2H-1,2,3-triazol-2-yl)pyridin-3-yl)-5-fluoro-4-(6-oxo-1,6-dihydropyridin-3-yl)benzamide;
N-(5-chloro-6-(2H-1,2,3-triazol-2-yl)pyridin-3-yl)-2,4'-difluoro-2'-hydroxy-5-methyl-[1,1'-biphenyl]-4-carboxamide;
5-chloro-N-(5-chloro-6-(2H-1,2,3-triazol-2-yl)pyridin-3-yl)-2'-cyano-2,4'-difluoro-[1,1 '-biphenyl]-4-carboxamide;
5-chloro-N-(5-chloro-6-(2H-1,2,3-triazol-2-yl)pyridin-3-yl)-2'-(difluoromethoxy)-2,4'-difluoro-[1,1'-biphenyl]-4-carboxamide;
2-chloro-N-(5-chloro-6-(2H-1,2,3-triazol-2-yl)pyridin-3-yl)-5-fluoro-4-(1-methyl-6-oxo-1,6-dihydropyridin-3-yl)benzamide;
N-(5-chloro-6-(2H-1,2,3-triazol-2-yl)pyridin-3-yl)-5-fluoro-4-(3-hydroxypyridin-4-yl)-2-methylbenzamide;
2-chloro-N-(5-chloro-6-(2H-1,2,3-triazol-2-yl)pyridin-3-yl)-4-(3-cyanopyridin-4-yl)-5-fluorobenzamide;
N-(6-(2H-1,2,3-triazol-2-yl)-5-(trifluoromethyl)pyridin-3-yl)-2'-amino-5-chloro-4'-fluoro-2-(trifluoromethyl)-[1,1'-biphenyl]-4-carboxamide;
N-(6-(2H-1,2,3-triazol-2-yl)-5-(trifluoromethyl)pyridin-3-yl)-2,2',3,4',5,6-hexafluoro-[1,1 '-biphenyl] -4-carboxamide;
2'-chloro-N-(5-chloro-6-(2H-l,2,3-triazol-2-yl)pyridin-3-yl)-2,3,4',5,6-pentafluoro-[1,1'-biphenyl]-4-carboxamide;
N-(6-(2H-1,2,3-triazol-2-yl)-5-(trifluoromethyl)pyridin-3-yl)-2',5-dichloro-4'-fluoro-2-(trifluoromethyl)-[1,1'-biphenyl]-4-carboxamide;
N-(6-(2H-l,2,3-triazol-2-yl)-5-(trifluoromethyl)pyridin-3-yl)-5-chloro-2',4'-difluoro-2-(trifluoromethyl)-[1,1 '-biphenyl]-4-carboxamide;
2'-amino-5-chloro-N-(5-chloro-6-(2H-1,2,3-triazol-2-yl)pyridin-3-yl)-2-(trifluoromethyl)-[1,1'-biphenyl]-4-carboxamide;
5-chloro-N-(5-chloro-6-(2H-1,2,3-triazol-2-yl)pyridin-3-yl)-2,2',4'-trifluoro-[1,1'-biphenyl]-4-carboxamide;
2'-bromo-5-chloro-N-(5-chloro-6-(2H-1,2,3-triazol-2-yl)pyridin-3-yl)-2,4'-difluoro-[1,l'-biphenyl]-4-carboxamide;
2'-amino-5-chloro-N-(5-chloro-6-(2H-1,2,3-triazol-2-yl)pyridin-3-yl)-4'-fluoro-2-(trifluoromethyl)-[1,1'-biphenyl]-4-carboxamide;
2'-amino-5-chloro-N-(5-chloro-6-(2H-1,2,3-triazol-2-yl)pyridin-3-yl)-2-cyclopropyl-4'-fluoro- [1,1'-biphenyl]-4-carboxamide;
5-chloro-N-(5-chloro-6-(2H-1,2,3-triazol-2-yl)pyridin-3-yl)-2'-(difluoromethyl)-2,4'-difluoro-[1,1 '-biphenyl]-4-carboxamide;
5-chloro-N-(5-chloro-6-(2H-1,2,3 -triazol-2-yl)pyridin-3 -yl)-2,4'-difluoro-2'-(trifluoromethyl)-[1,1 '-biphenyl]-4-carboxamide;
5-chloro-N-(5-chloro-6-(2H-1,2,3-triazol-2-yl)pyridin-3-yl)-2'-cyclopropyl-2,4'-difluoro-[1,1 '-biphenyl]-4-carboxamide;
2'-amino-5-chloro-N-(5-chloro-6-(2H-1,2,3-triazol-2-yl)pyridin-3-yl)-4'-fluoro-2-methyl-[1,1 '-biphenyl]-4-carboxamide;
4-(3-aminopyridin-4-yl)-2-chloro-N-(5-chloro-6-(2H-1,2,3-triazol-2-yl)pyridin-3-yl)-5-(trifluoromethyl)benzamide;
2-chloro-N-(5-chloro-6-(2H-1,2,3-triazol-2-yl)pyridin-3-yl)-4-(3-chloropyridin-4-yl)-5-(trifluoromethyl)benzamide;
4-(3-bromopyridin-4-yl)-2-chloro-N-(5-chloro-6-(2H-1,2,3-triazol-2-yl)pyridin-3 -yl)-5 -(trifluoromethyl)benzamide;

2'-amino-5-chloro-N-(5-chloro-6-(2H-1,2,3-triazol-2-yl)pyridin-3-yl)-2-ethynyl-4'-fluoro-[1,1 '-biphenyl]-4-carboxamide;
2',5-dichloro-N-(5-chloro-6-(2H-1,2,3-triazol-2-yl)pyridin-3-yl)-4'-fluoro-2-(trifluoromethyl)-[1,1 '-biphenyl] -4-carboxamide;
2'-bromo-5-chloro-N-(5-chloro-6-(2H-1,2,3-triazol-2-yl)pyridin-3-yl)-4'-fluoro-2-(trifluoromethyl)-[1,1 '-biphenyl]-4-carboxamide;
5-chloro-N-(5-chloro-6-(2H-1,2,3-triazol-2-yl)pyridin-3-yl)-4'-fluoro-2'-methyl-2-(trifluoromethyl)-[1,1 '-biphenyl]-4-carboxamide;
2'-amino-2,5-dichloro-N-(5-chloro-6-(2H-1,2,3-triazol-2-yl)pyridin-3-yl)-4'-fluoro-[1,1'-biphenyl]-4-carboxamide;
2',5-dichloro-2,4'-difluoro-N-(6-methyl-5-oxo-5,6-dihydro-1,6-naphthyridin-3-yl)-[1,1 '-biphenyl]-4-carboxamide;
2',5-dichloro-N-(7-chloro-2-methylbenzo[d]oxazol-5-yl)-2,4'-difluoro-[1,1'-biphenyl]-4-carboxamide;
2',5-dichloro-N-(8-chloro-3-oxo-3,4-dihydro-2H-benzo[b][1,4]oxazin-6-yl)-2,4'-difluoro-[1,1 '-biphenyl]-4-carboxamide;
2',5-dichloro-2,4'-difluoro-N-(1-methyl-1H-pyrazolo[4,3-b]pyridin-6-yl)-[1,1'-biphenyl]-4-carboxamide;
2',5-dichloro-2,4'-difluoro-N-(2-methyl-2H-pyrazolo[4,3-b]pyridin-6-yl)-[1,1'-biphenyl]-4-carboxamide;
2',5-dichloro-2,4'-difluoro-N-(1-methyl-1H-pyrazolo[3,4-b]pyridin-5-yl)-[1,1'-biphenyl]-4-carboxamide;
2',5-dichloro-N-(2-chloropyrazolo[1,5-a]pyrimidin-6-yl)-2,4'-difluoro-[1,1'-biphenyl]-4-carboxamide;
2',5-dichloro-2,4'-difluoro-N-(2-methylimidazo[1,2-b]pyridazin-7-yl)-[1,1'-biphenyl] -4-carboxamide;
2',5-dichloro-N-(2-chlorothiazolo[5,4-b]pyridin-6-yl)-2,4'-difluoro-[1,1'-biphenyl]-4-carboxamide;
2',5-dichloro-N-(6-chloro-1,5-naphthyridin-3-yl)-2,4'-difluoro-[1,1'-biphenyl]-4-carboxamide;
2',5-dichloro-2,4'-difluoro-N-(2-(trifluoromethyl)pyridin-4-yl)-[1,1'-biphenyl]-4-carboxamide;
2',5-dichloro-N-(2-cyanopyridin-4-yl)-2,4'-difluoro-[1,1'-biphenyl]-4-carboxamide;
2',5-dichloro-N-(2-chloropyridin-4-yl)-2,4'-difluoro-[1,1'-biphenyl]-4-carboxamide;
2',5-dichloro-N-(5-chloro-6-methoxypyridin-3-yl)-2,4'-difluoro-[1,1'-biphenyl]-4-carboxamide;
2',5-dichloro-N-(6-cyano-5-(trifluoromethyl)pyridin-3-yl)-2,4'-difluoro-[1,1'-biphenyl]-4-carboxamide;
2',5-dichloro-N-(6-cyano-5-fluoropyridin-3-yl)-2,4'-difluoro-[1,1'-biphenyl]-4-carboxamide;
2',5-dichloro-2,4'-difluoro-N-(2-methyl-6-(trifluoromethyl)pyridin-4-yl)-[1,1'-biphenyl]-4-carboxamide;
2',5-dichloro-N-(5-chloro-6-(1-methyl-1H-pyrazol-4-yl)pyridin-3-yl)-2,4'-difluoro-[1,1 '-biphenyl]-4-carboxamide;
2',5-dichloro-N-(5-chloro-6-(3-methyl-1H-1,2,4-triazol-1-yl)pyridin-3-yl)-2,4'-difluoro-[1,1 '-biphenyl]-4-carboxamide;
2',5-dichloro-N-(5-chloro-6-(oxazol-2-yl)pyridin-3-yl)-2,4'-difluoro-[1,1'-biphenyl]-4-carboxamide;
2',5-dichloro-N-(5-chloro-6-(thiazol-2-yl)pyridin-3-yl)-2,4'-difluoro-[1,1'-biphenyl]-4-carboxamide;
2',5-dichloro-N-(5-chloro-6-(1H-pyrazol-1-yl)pyridin-3-yl)-2,4'-difluoro-[1,1'-biphenyl]-4-carboxamide;
2',5-dichloro-N-(5-cyano-6-(2H-1,2,3-triazol-2-yl)pyridin-3-yl)-2,4'-difluoro-[1,1'-biphenyl]-4-carboxamide;
2',5-dichloro-N-(5-cyano-6-cyclopropoxypyridin-3-yl)-2,4'-difluoro-[1,1'-biphenyl]-4-carboxamide;
2',5-dichloro-N-(5-cyano-6-(difluoromethoxy)pyridin-3-yl)-2,4'-difluoro-[1,1'-biphenyl]-4-carboxamide;
4-chloro-6-(2-chloro-4-fluorophenyl)-N-(5-chloro-6-(2H-1,2,3-triazol-2-yl)pyridin-3-yl)nicotinamide;
6-(2-amino-4-fluorophenyl)-4-chloro-N-(5-chloro-6-(2H-1,2,3-triazol-2-yl)pyridin-3-yl)nicotinamide;
2-(2-amino-4-fluorophenyl)-4-chloro-N-(5-chloro-6-(2H-1,2,3-triazol-2-yl)pyridin-3-yl)pyrimidine-5-carboxamide;
6-(2-amino-4-fluorophenyl)-2-chloro-N-(5-chloro-6-(2H-1,2,3-triazol-2-yl)pyridin-3 -yl)nicotinamide;
4-chloro-6-(2-chloro-4-fluorophenyl)-N-(5-chloro-6-(2H-1,2,3-triazol-2-yl)pyridin-3-yl)pyridazine-3-carboxamide;
3-chloro-5-(2-chloro-4-fluorophenyl)-N-(5-chloro-6-(2H-1,2,3-triazol-2-yl)pyridin-3-yl)pyrazine-2-carboxamide;
N-(6-(2H-1,2,3-triazol-2-yl)-5-(trifluoromethyl)pyridin-3-yl)-4-(3-bromopyridin-4-yl)-2-chloro-5-(trifluoromethyl)benzamide;
2-amino-2',5-dichloro-N-(5-chloro-6-(2H-1,2,3-triazol-2-yl)pyridin-3-yl)-4'-fluoro-[1,1 '-biphenyl]-4-carboxamide;
2-acetamido-2',5-dichloro-N-(5-chloro-6-(2H-l,2,3-triazol-2-yl)pyridin-3-yl)-4'-fluoro-[1,1 '-biphenyl]-4-carboxamide;
ethyl(2',5-dichloro-4-((5-chloro-6-(2H-1,2,3-triazol-2-yl)pyridin-3-yl)aminoformyl)-4'-fluoro-[1 ,1'-biphenyl]-2-yl)aminoformate;
2',5-dichloro-N-(5-chloro-6-(2H-l,2,3-triazol-2-yl)pyridin-3-yl)-4'-fluoro-2-(isopropylamino)-[1,1'-biphenyl]-4-carboxamide;
N-(6-(2H-1,2,3-triazol-2-yl)-5-(trifluoromethyl)pyridin-3-yl)-5-chloro-2,2',4'-trifluoro-[1,1'-biphenyl]-4-carboxamide;
5-chloro-N-(5-chloro-6-(2H-1,2,3-triazol-2-yl)pyridin-3-yl)-2,2',4'-trifluoro-[1,1'-biphenyl]-4-carboxamide;
N-(5-chloro-6-(2H-1,2,3-triazol-2-yl)pyridin-3-yl)-2,2',4',5-tetrafluoro-[1,1'-biphenyl]-4-carboxamide;
N-(6-(2H-1,2,3-triazol-2-yl)-5-(trifluoromethyl)pyridin-3-yl)-2,2',4',5-tetrafluoro-[1,1'-biphenyl]-4-carboxamide;
4-(3-bromopyridin-4-yl)-2-chloro-N-(5-chloro-6-(2H-1,2,3-triazol-2-y1)pyridin-3-yl)-5-fluorobenzamide;
4-(2-amino-6-fluoropyridin-3-yl)-N-(5-chloro-6-(2H-1,2,3-triazol-2-yl)pyridin-3-yl)- 2-Fluoro-5-isopropylbenzamide;
5-chloro-N-(5-chloro-6-(2H-1,2,3-triazol-2-yl)pyridin-3-yl)-2'-(cyclopropylamino)-2,4'-difluoro-[1,1'-biphenyl]-4-carboxamide;
5-chloro-N-(5-chloro-6-(2H-1,2,3-triazol-2-yl)pyridin-3-yl)-2,4'-difluoro-2'-(methylthio)-[1,1'-biphenyl]-4-carboxamide;
5-chloro-N-(5-chloro-6-(2H-1,2,3-triazol-2-yl)pyridin-3-yl)-2,4'-difluoro-2'-(methylsulfonyl)-[1,1'-biphenyl]-4-carboxamide;
5-chloro-N-(5-chloro-6-(2H-1,2,3-triazol-2-yl)pyridin-3-yl)-2,4'-difluoro-2'-ureido-[1,1'-biphenyl]-4-carboxamide;
2'-acetamido-5-chloro-N-(5-chloro-6-(2H-1,2,3-triazol-2-yl)pyridin-3-yl)-2,4'-difluoro-[1,1'-biphenyl]-4-carboxamide;
5-chloro-N-(5-chloro-6-(2H-1,2,3-triazol-2-yl)pyridin-3-yl)-2,4'-difluoro-2'-(hydroxymethyl)-[1,1'-biphenyl]-4-carboxamide;
4-(3-amino-5-ethynylpyridin-4-yl)-2-chloro-N-(5-chloro-6-(2H-1,2,3-triazol-2-yl)pyridin-3-yl)-5-fluorobenzamide;
5-chloro-N-(5-chloro-6-(2H-1,2,3-triazol-2-yl)pyridin-3-yl)-2,4'-difluoro-2'-(methylsulfinyl)-[1,1 '-biphenyl]-4-carboxamide;
5-chloro-N-(5-chloro-6-(2H-1,2,3-triazol-2-yl)pyridin-3-yl)-2,4'-difluoro-2'-((methylsulfonyl)methyl)-[1,1'-biphenyl] -4-carboxamide;
ethyl (5'-chloro-4'-((5-chloro-6-(2H-1,2,3-triazol-2-yl)pyridin-3-yl)carbamoyl)-2',4-difluoro-[1,1'-biphenyl]-2-yl)carbamate;
5-chloro-N-(5-chloro-6-(2H-1,2,3-triazol-2-yl)pyridin-3-yl)-2,4'-difluoro-2'-vinyl-[1,1 '-biphenyl]-4-carboxamide;
5-chloro-N-(5-chloro-6-(2H-1,2,3-triazol-2-yl)pyridin-3-yl)-2,4'-difluoro-2'-(1-hydroxyethyl)-[1,1 '-biphenyl]-4-carboxamide
5-chloro-N-(5-chloro-6-(2H-1,2,3-triazol-2-yl)pyridin-3-yl)-2,4'-difluoro-2'-(1-methoxyethyl)-[1,1 '-biphenyl]-4-carboxamide;
5-chloro-N-(5-chloro-6-(2H-1,2,3-triazol-2-yl)pyridin-3-yl)-2,4'-difluoro-2'-((2-(methylamino)ethyl)amino)-[1,1'-biphenyl]-4-carboxamide;
5-chloro-N-(5-chloro-6-(2H-1,2,3-triazol-2-yl)pyridin-3-yl)-2,4'-difluoro-2'-((2-hydroxyethyl)amino)-[1,1'-biphenyl]-4-carboxamide;
5-chloro-N-(5-chloro-6-(2H-1,2,3-triazol-2-yl)pyridin-3-yl)-2,4'-difluoro-2'-(2-hydroxyethoxy)-[1,1'-biphenyl]-4-carboxamide;
5-chloro-N-(5-chloro-6-(2H-1,2,3-triazol-2-yl)pyridin-3-yl)-2,4'-difluoro-2'-(hydroxyamino)-[1,1'-biphenyl]-4-carboxamide;
5-chloro-N-(5-chloro-6-(2H-1,2,3-triazol-2-yl)pyridin-3-yl)-2,4'-difluoro-2'-(2-hydroxypropan-2-yl)-[1,1 '-biphenyl]-4-carboxamide;
5'-chloro-4'-((5-chloro-6-(2H-1,2,3-triazol-2-yl)pyridin-3-yl)carbamoyl)-2',4-difluoro-[l,l'-biphenyl]-2-yl ethyl carbonate;
5-chloro-N-(5-chloro-6-(2H-1,2,3-triazol-2-yl)pyridin-3-yl)-2'-((2-(dimethylamino)ethyl)amino)-2,4'-difluoro-[1,1'-biphenyl]-4-carboxamide;
5-chloro-N-(5-chloro-6-(2H-1,2,3-triazol-2-yl)pyridin-3-yl)-2,4'-difluoro-2'-((2-methoxyethyl)amino)-[1,1 '-biphenyl]-4-carboxamide;
5-chloro-N-(5-chloro-6-(2H-1,2,3-triazol-2-yl)pyridin-3-yl)-2,4'-difluoro-2'-(2-methoxyethoxy)-[1,1 '-biphenyl] -4-carboxamide;
5-chloro-N-(5-chloro-6-(2H-1,2,3-triazol-2-yl)pyridin-3-yl)-2,4'-difluoro-2'-(prop-2-yn-1-ylamino)-[1,1'-biphenyl]-4-carboxamide;
5-chloro-N-(5-chloro-6-(2H-1,2,3-triazol-2-yl)pyridin-3-yl)-2'-((cyanomethyl)amino)-2,4'-difluoro-[1,1'-biphenyl]-4-carboxamide;
2'-(aminomethyl)-5-chloro-N-(5-chloro-6-(2H-1,2,3-triazol-2-yl)pyridin-3-yl)-2,4'-difluoro-[1,1'-biphenyl]-4-carboxamide;
5-chloro-N-(5-chloro-6-(2H-1,2,3-triazol-2-yl)pyridin-3-yl)-2,4'-difluoro-2'-(prop-2-yn-1-yl)-[1,1'-biphenyl]-4-carboxamide;

2'-acrylamido-5-chloro-N-(5-chloro-6-(2H-1,2,3-triazol-2-yl)pyridin-3-yl)-2,4'-difluoro-[1,1 '-biphenyl]-4-carboxamide;
5'-chloro-N4'-(5-chloro-6-(2H-1,2,3-triazol-2-yl)pyridin-3-yl)-2',4-difluoro-[1,1'-biphenyl]-2,4'-dicarboxamide;
5-chloro-N-(5-chloro-6-(2H-1,2,3-triazol-2-yl)pyridin-3-yl)-2,4'-difluoro-2'-(prop-2-yn-1-yloxy)-[1,1'-biphenyl]-4-carboxamide;
5-chloro-N-(5-chloro-6-(2H-1,2,3-triazol-2-yl)pyridin-3-yl)-2'-(cyanomethoxy)-2,4'-difluoro-[1,1 '-biphenyl]-4-carboxamide;
2'-(1 -aminoethyl)-5 -chloro-N-(5-chloro-6-(2H-1,2,3-triazol-2-yl)pyridin-3 -yl)-2,4'-difluoro-[1,1'-biphenyl]-4-carboxamide;
5-chloro-N-(5-chloro-6-(2H-1,2,3-triazol-2-yl)pyridin-3-yl)-2'-(cyanomethyl)-2,4'-difluoro-[1,1 '-biphenyl]-4-carboxamide;
2'-amino-5-chloro-N-(5-chloro-6-(2H-1,2,3-triazol-2-yl)pyridin-3-yl)-6'-ethynyl-2,4'-difluoro-[1,1'-biphenyl]-4-carboxamide;
cyclopropyl 5'-chloro-4'-((5-chloro-6-(2H-1,2,3-triazol-2-yl)pyridin-3-yl)carbamoyl)-2',4-difluoro-[1,1'-biphenyl]-2-carboxylate;
2',5-dichloro-N-(5-chloro-6-(2H-1,2,3-triazol-2-yl)pyridin-3-yl)-6'-ethynyl-2,4'-difluoro-[1,1 '-biphenyl]-4-carboxamide;
2'-(2-aminopropan-2-yl)-5-chloro-N-(5-chloro-6-(2H-1,2,3-triazol-2-yl)pyridin-3-yl)-2,4'-difluoro-[1,1'-biphenyl]-4-carboxamide;
5-chloro-N-(5-chloro-6-(2H-1,2,3-triazol-2-yl)pyridin-3-yl)-2,4'-difluoro-2'-(methoxymethyl)-[1,1 '-biphenyl]-4-carboxamide;
5'-chloro-4'-((5-chloro-6-(2H-1,2,3-triazol-2-yl)pyridin-3-yl)carbamoyl)-2',4-difluoro-[1,1'-biphenyl]-2-yl carbamate;
2',5-dichloro-N-(5-chloro-6-(2H-1,2,3-triazol-2-yl)pyridin-3-yl)-2,4'-difluoro-6'-(methoxymethyl)-[1,1 '-biphenyl]-4-carboxamide;
N-(6-(2H-1,2,3-triazol-2-yl)-5-(trifluoromethyl)pyridin-3-yl)-5-chloro-2,4'-difluoro-2'-vinyl-[1,1'-biphenyl]-4-carboxamide;
N-(6-(2H-1,2,3-triazol-2-yl)-5-(trifluoromethyl)pyridin-3-yl)-5-chloro-2'-ethynyl-2,4'-difluoro-[1,1'-biphenyl]-4-carboxamide;
2-chloro-N-(5-chloro-6-(2H-1,2,3-triazol-2-yl)pyridin-3-yl)-4-(2-ethynyl-6-fluoropyridin-3-yl)-5-fluorobenzamide;
2-chloro-N-(5-chloro-6-(2H-1,2,3-triazol-2-yl)pyridin-3-yl)-4-(3-ethynylpyridin-4-yl)-5-fluorobenzamide;
5-chloro-2'-ethynyl-2,4'-difluoro-N-(2-(trifluoromethyl)pyridin-4-yl)-[1,1'-biphenyl]-4-carboxamide;
5-chloro-N-(8-chloro-3-oxo-3,4-dihydro-2H-benzo[b][1,4]oxazin-6-yl)-2'-ethynyl-2,4'-difluoro-[1,1'-biphenyl]-4-carboxamide;
5 -chloro-N-(6-cyano-5 -(trifluoromethyl)pyridin-3 -yl)-2'-ethynyl-2,4'-difluoro-[1,1'-biphenyl]-4-carboxamide;
N-(6-(2H-1,2,3-triazol-2-yl)-5-(trifluoromethyl)pyridin-3-yl)-2-chloro-4-(2-ethynyl-6-fluoropyridin-3-yl)-5-fluorobenzamide;
2-chloro-4-(3-ethynylpyridin-4-yl)-5-fluoro-N-(6-((tetrahydrofuran-3-yl)amino)-5-(trifluoromethyl)pyridin-3-yl)benzamide;
2-chloro-4-(3-ethynyl-5-fluoropyridin-2-yl)-5-fluoro-N-(2-(trifluoromethyl)pyridin-4-yl)benzamide;
2-chloro-N-(8-chloro-3-oxo-3,4-dihydro-2H-benzo[b][1,4]oxazin-6-yl)-4-(3-ethynyl-5-fluoropyridin-2-yl)-5-fluorobenzamide;
2-chloro-N-(6-cyano-5-(trifluoromethyl)pyridin-3-yl)-4-(3-ethynyl-5-fluoropyridin-2-yl)-5-fluorobenzamide;
N-(6-(2H-1,2,3-triazol-2-yl)-5-(trifluoromethyl)pyridin-3-yl)-2-chloro-4-(3-ethynyl-5-fluoropyridin-2-yl)-5-fluorobenzamide;
5-chloro-N-(5-cyano-6-(trifluoromethoxy)pyridin-3-yl)-2'-ethynyl-2,4'-difluoro-[1,1'-biphenyl]-4-carboxamide;
5-chloro-N-(5-chloro-6-(difluoromethoxy)pyridin-3-yl)-2'-ethynyl-2,4'-difluoro-[1,1'-biphenyl]-4-carboxamide;
5-chloro-N-(5-cyano-6-((1-methylpiperidin-4-yl)oxy)pyridin-3-yl)-2'-ethynyl-2,4'-difluoro-[1,1'-biphenyl]-4-carboxamide;
N-(6-(2H-l,2,3-triazol-2-yl)-5-(trifluoromethyl)pyridin-3-yl)-2-chloro-4-(3-ethynylpyridin-4-yl)-5-fluorobenzamide;
N-(5-chloro-6-(2H-1,2,3-triazol-2-yl)pyridin-3-yl)-2,3,4',5,6-pentafluoro-2'-propiolamido-[1,1'-biphenyl]-4-carboxamide;
N-(5-chloro-6-(2H-1,2,3-triazol-2-yl)pyridin-3-yl)-2,3,4',5,6-pentafluoro-2'-propiolamido-[1,1'-biphenyl]-4-carboxamide;
N-(5-chloro-6-(2H-1,2,3-triazol-2-yl)pyridin-3-yl)-4-(3-ethynylpyridin-4-yl)-2,3,5,6-tetrafluorobenzamide;
2-chloro-N-(3-chloro-4-(2-methoxyethoxy)phenyl)-4-(3-ethynylpyridin-4-yl)-5-fluorobenzamide;

2-chloro-N-(5-chloro-6-(2H-1,2,3-triazol-2-yl)pyridin-3-yl)-4-(3-ethynyl-5-fluoropyridin-2-yl)-5-fluorobenzamide;
6-chloro-N-(5-chloro-6-(2H-1,2,3-triazol-2-yl)pyridin-3-yl)-3'-ethynyl-[2,4'-bipyridine]-5-carboxamide;
2-chloro-N-(5-chloro-6-(2H-1,2,3-triazol-2-yl)pyridin-3-yl)-5-fluoro-4-(3-propiolamidopyridin-4-yl)benzamide;
2-chloro-N-(3-chloro-4-methoxyphenyl)-4-(3-ethynylpyridin-4-yl)-5-fluorobenzamide;
2-chloro-4-(3-ethynyl-5-fluoropyridin-2-yl)-5-fluoro-N-(6-((tetrahydrofuran-3-yl)amino)-5-(trifluoromethyl)pyridin-3-yl)benzamide;
N-(5-chloro-6-(2H-1,2,3-triazol-2-yl)pyridin-3-yl)-4-(3-ethynyl-5-fluoropyridin-2-yl)-2,3,5,6-tetrafluorobenzamide;
N-(5-chloro-6-(2H-1,2,3-triazol-2-yl)pyridin-3-yl)-2,3,5,6-tetrafluoro-4-(5-fluoro-3-propiolamidopyridin-2-yl)benzamide;
4-(3-acrylamidopyridin-4-yl)-2-chloro-N-(5-chloro-6-(2H-1,2,3-triazol-2-yl)pyridin-3-yl)-5-fluorobenzamide;
N-(3-chloro-4-(methylsulfonyl)phenyl)-4-(3-ethynylpyridin-4-yl)-2,3,5,6-tetrafluorobenzamide;
N-(3-chloro-4-((trifluoromethyl)thio)phenyl)-4-(3-ethynylpyridin-4-yl)-2,3,5,6-tetrafluorobenzamide;
N-(3-chloro-4-((2-(dimethylamino)-2-oxoethyl)thio)phenyl)-4-(3-ethynylpyridin-4-yl)-2,3,5,6-tetrafluorobenzamide;
N-(6-(2H-1,2,3-triazol-2-yl)-5-(trifluoromethyl)pyridin-3-yl)-2-chloro-5-fluoro-4-(3-vinylpyridin-4-yl)benzamide;
2'-amino-5-chloro-2,4'-difluoro-N-(6-(2-methoxyethoxy)-5-(trifluoromethyl)pyridin-3-yl)-[1,1'-biphenyl]-4-carboxamide;
2'-amino-5-chloro-N-(6-(cyclopropylamino)-5-(trifluoromethyl)pyridin-3-yl)-2,4'-difluoro-[1,1'-biphenyl]-4-carboxamide;
2'-amino-5-chloro-N-(6-(cyanomethoxy)-5-(trifluoromethyl)pyridin-3-yl)-2,4'-difluoro-[1,1'-biphenyl]-4-carboxamide;
2'-amino-5-chloro-N-(6-((cyanomethyl)amino)-5-(trifluoromethyl)pyridin-3-yl)-2,4'-difluoro-[1,1 '-biphenyl]-4-carboxamide;
2'-amino-5-chloro-2,4'-difluoro-N-(6-(methoxymethyl)-5-(trifluoromethyl)pyridin-3-yl)-[1,1 '-biphenyl]-4-carboxamide;
2'-amino-5-chloro-2,4'-difluoro-N-(6-((2-methoxyethyl)amino)-5-(trifluoromethyl)pyridin-3-yl)-[1,1 '-biphenyl]-4-carboxamide;
methyl (4-(2'-amino-5-chloro-2,4'-difluoro-[1,1'-biphenyl]-4-carboxamido)-2-(trifluoromethyl)phenyl)carbamate;
N-(4-acetamido-3-(trifluoromethyl)phenyl)-2'-amino-5-chloro-2,4'-difluoro-[1,1'-biphenyl]-4-carboxamide;
2'-amino-5-chloro-2,4'-difluoro-N-(3-(trifluoromethyl)-4-ureidophenyl)-[1,1'-biphenyl]-4-carboxamide;
2'-amino-5-chloro-2,4'-difluoro-N-(4-(methylsulfonamido)-3-(trifluoromethyl)phenyl)-[1,1 '-biphenyl]-4-carboxamide;
2'-amino-5-chloro-2,4'-difluoro-N-(6-(hydroxymethyl)-5-(trifluoromethyl)pyridin-3-yl)-[1, 1'-biphenyl]-4-carboxamide;
2'-amino-5-chloro-N-(6-(cyanomethyl)-5-(trifluoromethyl)pyridin-3-yl)-2,4'-difluoro-[1,1 '-biphenyl]-4-carboxamide;
2'-amino-5-chloro-2,4'-difluoro-N-(4-(methylthio)-3-(trifluoromethyl)phenyl)-[1,1'-biphenyl]-4-carboxamide;
2'-amino-5-chloro-2,4'-difluoro-N-(4-(methylsulfonyl)-3-(trifluoromethyl)phenyl)-[1,1'-biphenyl]-4-carboxamide;
2'-amino-5-chloro-2,4'-difluoro-N-(6-((tetrahydrofuran-3-yl)oxy)-5-(trifluoromethyl)pyridin-3-yl)-[1, 1'-biphenyl]-4-carboxamide;
2'-amino-5-chloro-2,4'-difluoro-N-(6-(2-hydroxypropan-2-yl)-5-(trifluoromethyl)pyridin-3-yl)-[1,1'-biphenyl]-4-carboxamide;
5-(2'-amino-5-chloro-2,4'-difluoro-[1,1'-biphenyl]-4-carboxamido)-3-(trifluoromethyl)picolinamide;
2'-amino-5-chloro-N-(6-ethynyl-5-(trifluoromethyl)pyridin-3-yl)-2,4'-difluoro-[1,1'-biphenyl]-4-carboxamide;
2'-amino-5-chloro-2,4'-difluoro-N-(6-((tetrahydrofuran-3-yl)amino)-5-(trifluoromethyl)pyridin-3-yl)-[1,1'-biphenyl]-4-carboxamide;
2'-amino-5-chloro-N-(4-(cyclopropylsulfonyl)-3-(trifluoromethyl)phenyl)-2,4'-difluoro-[1,1 '-biphenyl]-4-carboxamide;
2'-amino-5-chloro-2,4'-difluoro-N-(6-(pyrrolidine-1-carbonyl)-5-(trifluoromethyl)pyridin-3-yl)-[1,1'-biphenyl]-4-carboxamide;
2'-amino-5-chloro-N-(4-(N,N-dimethylsulfamoyl)-3-(trifluoromethyl)phenyl)-2,4'-difluoro-[1,1'-biphenyl]-4-carboxamide;
N-(4-(2-azaspiro[3.3]heptane-2-carbonyl)-3-(trifluoromethyl)phenyl)-2'-amino-5-chloro-2,4'-difluoro-[1,1'-biphenyl]-4-carboxamide;
2-chloro-N-(5-chloro-6-(1-oxo-2-oxa-7-azaspiro[4.4]nonan-7-yl)pyridin-3-yl)-4-(3-ethynylpyridin-4-yl)-5-fluorobenzamide;
2-chloro-N-(5-chloro-6-(2-oxa-7-azaspiro[4.4]nonan-7-yl)pyridin-3-yl)-4-(3-ethynylpyridin-4-yl)-5-fluorobenzamide;
2-chloro-N-(5-chloro-6-((1R,2S,4S)-2-(3-methylisoxazol-5-yl)-7-azabicyclo[2.2.1]heptan-7-yl)pyridin-3-yl)-4-(3-ethynylpyridin-4-yl)-5-fluorobenzamide;
2-chloro-N-(5-chloro-6-(1-cyano-7-azabicyclo[2.2.1]heptan-7-yl)pyridin-3-yl)-4-(3-ethynylpyridin-4-yl)-5-fluorobenzamide;
N-(6-(7-azabicyclo[2.2.1]heptan-7-yl)-5-chloropyridin-3-yl)-2-chloro-4-(3-ethynylpyridin-4-yl)-5-fluorobenzamide;
2-chloro-N-(5-chloro-6-(4-oxohexahydrocyclopenta[c]pyrrol-2(1H)-yl)pyridin-3-yl)-4-(3-ethynylpyridin-4-yl)-5-fluorobenzamide;
2-chloro-N-(5-chloro-6-((3aR,6aS)-5-oxohexahydrocyclopenta[c]pyrrol-2(1H)-yl)pyridin-3-yl)-4-(3-ethynylpyridin-4-yl)-5-fluorobenzamide;
2-chloro-N-(5-chloro-6-((3aR,6aS)-5,5-difluorohexahydrocyclopenta[c]pyrrol-2(1H)-yl)pyridin-3-yl)-4-(3-ethynylpyridin-4-yl)-5-fluorobenzamide;
2-chloro-N-(5-chloro-6-((2-oxopyrrolidin-1-yl)amino)pyridin-3-yl)-4-(3-ethynylpyridin-4-yl)-5-fluorobenzamide;
2-chloro-N-(5-chloro-6-(3-oxopyrazolidin-1-yl)pyridin-3-yl)-4-(3-ethynylpyridin-4-yl)-5-fluorobenzamide;
N-(6-(1H-benzo[d]imidazol-2-yl)-5-chloropyridin-3-yl)-2-chloro-4-(3-ethynylpyridin-4-yl)-5-fluorobenzamide;
2-chloro-N-(5-chloro-6-(4,5,6,7-tetrahydro-2H-[1,2,3]triazolo[4,5-c]pyridin-2-yl)pyridin-3-yl)-4-(3-ethynylpyridin-4-yl)-5-fluorobenzamide;
2-chloro-N-(5-chloro-6-(4,5-dicyano-2H-1,2,3-triazol-2-yl)pyridin-3-yl)-4-(3-ethynylpyridin-4-yl)-5-fluorobenzamide;
2-chloro-N-(5-chloro-6-(4,5,6,7-tetrahydro-2H-indazol-2-yl)pyridin-3-yl)-4-(3-ethynylpyridin-4-yl)-5-fluorobenzamide;
2-chloro-N-(5-chloro-6-(5,6-dihydrocyclopenta[c]pyrazol-2(4H)-yl)pyridin-3-yl)-4-(3-ethynylpyridin-4-yl)-5-fluorobenzamide;
2-chloro-N-(5-chloro-6-(3-(trifluoromethyl)-5,6-dihydrocyclopenta[c]pyrazol-2(4H)-yl)pyridin-3-yl)-4-(3-ethynylpyridin-4-yl)-5-fluorobenzamide;
ethyl 2-((3-chloro-5-(2-chloro-4-(3-ethynylpyridin-4-yl)-5-fluorobenzamido)pyridin-2-yl)amino)-2,4,5,6-tetrahydrocyclopenta[d][1,2,3]triazole-4-carboxylate;
N-(6-((2H-benzo[d][1,2,3]triazol-2-yl)amino)-5-chloropyridin-3-yl)-2-chloro-4-(3-ethynylpyridin-4-yl)-5-fluorobenzamide;
2-chloro-N-(5-chloro-6-(2-(trifluoromethyl)-5,6-dihydro-[1,2,4]triazolo[1,5-a]pyrazin-7(8H)-yl)pyridin-3-yl)-4-(3-ethynylpyridin-4-yl)-5-fluorobenzamide;
2-chloro-N-(5-chloro-6-(3-(trifluoromethyl)-5,6-dihydro-[1,2,4]triazolo[4,3-a]pyrazin-7(8H)-yl)pyridin-3-yl)-4-(3-ethynylpyridin-4-yl)-5-fluorobenzamide;
1-(3-chloro-5-(2-chloro-4-(3-ethynylpyridin-4-yl)-5-fluorobenzamido)pyridin-2-yl)-4,5,6,7-tetrahydro-1H-benzo[d]imidazole-5-carboxylic acid;
2-chloro-N-(2-(3,4-dichlorophenyl)benzo[d]oxazol-5-yl)-4-(3-ethynylpyridin-4-yl)-5-fluorobenzamide;
(S)-2-chloro-N-(5-chloro-6-(2-cyano-4,4-difluoropyrrolidine-1-carbonyl)pyridin-3-yl)-4-(3-ethynylpyridin-4-yl)-5-fluorobenzamide;
2-chloro-N-(5-chloro-6-((3aR,6aS)-hexahydro-1H-furo[3,4-c]pyrrole-5-carbonyl)pyridin-3-yl)-4-(3-ethynylpyridin-4-yl)-5-fluorobenzamide;
2-chloro-N-(5-chloro-6-(pyrrolidine-1-carbonyl)pyridin-3-yl)-4-(3-ethynylpyridin-4-yl)-5-fluorobenzamide;
2-chloro-N-(5-chloro-6-(4,4-difluoropiperidine-1-carbonyl)pyridin-3-yl)-4-(3-ethynylpyridin-4-yl)-5-fluorobenzamide;
2-chloro-N-(5-chloro-6-(6,6-difluoro-3-azabicyclo[3.1.0]hexane-3-carbonyl)pyridin-3-yl)-4-(3-ethynylpyridin-4-yl)-5-fluorobenzamide;
2-chloro-N-(5-chloro-6-(6,6-difluoro-3-azabicyclo[3.1.0]hexan-3-yl)pyridin-3-yl)-4-(3-ethynylpyridin-4-yl)-5-fluorobenzamide;
2-chloro-N-(5-chloro-6-(3-(4,4-difluoropiperidin-1-yl)azetidin-1-yl)pyridin-3-yl)-4-(3-ethynylpyridin-4-yl)-5-fluorobenzamide;
2-chloro-N-(5-chloro-6-(3-(4,4-difluoropiperidin-1-yl)azetidine-1-carbonyl)pyridin-3-yl)-4-(3-ethynylpyridin-4-yl)-5-fluorobenzamide;
4,4'-dichloro-N-(5-chloro-6-(2H-1,2,3-triazol-2-yl)pyridin-3-yl)-6-(3-ethynylpyridin-4-yl)-[1,1'-biphenyl]-3-carboxamide;
2-amino-4",5'-dichloro-N-(5-chloro-6-(2H-1,2,3-triazol-2-yl)pyridin-3-yl)-4-fluoro-[1,1':2',1 "-terphenyl]-4'-carboxamide;

N-(6-(2H-1,2,3-triazol-2-yl)-5-(trifluoromethyl)pyridin-3-yl)-5'-methyl-[1,1':2',1"-terphenyl]-4'-carboxamide;
2-chloro-N-(5 -chloro-6-(2H-1,2,3 -triazol-2-yl)pyridin-3 -yl)-5 -ethynyl-4-(3 - ethynylpyridin-4-yl)benzamide;
N-(5-chloro-6-(2H-1,2,3-triazol-2-yl)pyridin-3-yl)-5-ethynyl-4-(3-ethynylpyridin-4-yl)-2-methylbenzamide;
N-(5-chloro-6-(2H-1,2,3-triazol-2-yl)pyridin-3-yl)-5-cyclopropyl-4-(3-ethynylpyridin-4-yl)-2-methylbenzamide;
4-(3-amino-5-fluoropyridin-2-yl)-N-(5-chloro-6-(2H-1,2,3-triazol-2-yl)pyridin-3-yl)-5-cyclopropyl-2-methylbenzamide;
2-chloro-N-(5-chloro-6-(2H-1,2,3-triazol-2-yl)pyridin-3-yl)-5-cyclopropyl-4-(3-ethynylpyridin-4-yl)benzamide;
4-(3-amino-5-fluoropyridin-2-yl)-2-chloro-N-(5-chloro-6-(2H-1,2,3-triazol-2-yl)pyridin-3-yl)-5-cyclopropylbenzamide;
2'-amino-N-(5-chloro-6-(2H-1,2,3-triazol-2-yl)pyridin-3-yl)-2-ethynyl-4'-fluoro-5-methyl-[1,1'-biphenyl]-4-carboxamide;
N-(6-(2H-1,2,3-triazol-2-yl)-5-(trifluoromethyl)pyridin-3-yl)-4-(3-amino-5-ethynylpyridin-4-yl)-2-chloro-5-fluorobenzamide;
4-(3-amino-5-ethynylpyridin-4-yl)-2-chloro-N-(5-chloro-6-(2H-1,2,3-triazol-2-yl)pyridin-3-yl)-5-fluorobenzamide;
2'-amino-5-chloro-N-(5-chloro-6-(2H-1,2,3-triazol-2-yl)pyridin-3-yl)-6'-ethynyl-2,4'-difluoro-[1,1 '-biphenyl]-4-carboxamide;
N-(6-(2H-1,2,3-triazol-2-yl)-5-(trifluoromethyl)pyridin-3-yl)-2',5-dichloro-6'-ethynyl-2-fluoro-[1,1'-biphenyl]-4-carboxamide;
2'-chloro-N-(5-chloro-6-(2H-1,2,3-triazol-2-yl)pyridin-3-yl)-2-ethynyl-4'-fluoro-5-methyl-[1,1'-biphenyl]-4-carboxamide;
N-(5-chloro-6-(2H-1,2,3-triazol-2-yl)pyridin-3-yl)-2,2'-diethynyl-4'-fluoro-5-methyl-[1,1'-biphenyl]-4-carboxamide;
4-(2-amino-6-fluoropyridin-3-yl)-N-(5-chloro-6-(2H-1,2,3-triazol-2-yl)pyridin-3-yl)-5-ethynyl-2-methylbenzamide;
N-(5-chloro-6-(2H-1,2,3-triazol-2-yl)pyridin-3-yl)-5-ethynyl-4-(3-methoxypyridin-4-yl)-2-methylbenzamide;
2-amino-5'-chloro-N-(5-chloro-6-(2H-1,2,3-triazol-2-yl)pyridin-3-yl)-4-fluoro-2",3",4",5"-tetrahydro-[1,1':2',1 "-terphenyl]-4'-carboxamide;
2-chloro-N-(5-chloro-6-(2H-1,2,3-triazol-2-yl)pyridin-3-yl)-4-fluoro-5'-methyl-[1,1':2',1"-terphenyl]-4'-carboxamide;
2'-amino-N-(5-chloro-6-(2H-1,2,3-triazol-2-yl)pyridin-3-yl)-2-(cyclopent-1-en-1-yl)-4'-fluoro-5-methyl-[1,1'-biphenyl]-4-carboxamide;
2-amino-N-(5-chloro-6-(2H-1,2,3-triazol-2-yl)pyridin-3-yl)-4-fluoro-5'-methyl-[1,1':2',1"-terphenyl]-4'-carboxamide;
N-(5-chloro-6-(2H-1,2,3-triazol-2-yl)pyridin-3-yl)-2-ethynyl-4-fluoro-5'-methyl-[1,1':2',1"-terphenyl]-4'-carboxamide;
2'-amino-N-(5-chloro-6-(2H-1,2,3-triazol-2-yl)pyridin-3-yl)-4'-fluoro-5-methyl-2-(prop-1-yn-1-yl)-[1,1'-biphenyl]-4-carboxamide;
2'-amino-5-chloro-N-(5-chloro-6-(2H-1,2,3-triazol-2-yl)pyridin-3-yl)-4'-fluoro-2-vinyl-[1,1'-biphenyl]-4-carboxamide;
2'-amino-N-(5-chloro-6-(2H-1,2,3-triazol-2-yl)pyridin-3-yl)-4'-fluoro-5-methyl-2-vinyl-[1,1'-biphenyl]-4-carboxamide;
3-chloro-5-(2-chloro-4-(3-ethynylpyridin-4-yl)-5-fluorobenzamido)-N-methylpicolinamide;
3-chloro-5-(2-chloro-4-(3-ethynylpyridin-4-yl)-5-fluorobenzamido)-N-(cyanomethyl)picolinamide;
3-chloro-5-(2-chloro-4-(3-ethynylpyridin-4-yl)-5-fluorobenzamido)-N-ethylpicolinamide;
3-chloro-5-(2-chloro-4-(3-ethynylpyridin-4-yl)-5-fluorobenzamido)-N-(2,2,2-trifluoroethyl)picolinamide;
3-chloro-5-(2-chloro-4-(3-ethynylpyridin-4-yl)-5-fluorobenzamido)-N-propylpicolinamide;
3-chloro-5-(2-chloro-4-(3-ethynylpyridin-4-yl)-5-fluorobenzamido)-N-(2-(trifluoromethoxy)ethyl)picolinamide;
3-chloro-5-(2-chloro-4-(3-ethynylpyridin-4-yl)-5-fluorobenzamido)-N-phenethylpicolinamide;
3-chloro-5-(2-chloro-4-(3-ethynylpyridin-4-yl)-5-fluorobenzamido)-N-(furan-3-ylmethyl)picolinamide;
3-chloro-5-(2-chloro-4-(3-ethynylpyridin-4-yl)-5-fluorobenzamido)-N-neopentylpicolinamide;
3-chloro-5-(2-chloro-4-(3-ethynylpyridin-4-yl)-5-fluorobenzamido)-N-(2-(diethylamino)-2-oxoethyl)picolinamide;
5-(2'-amino-5-chloro-2,4'-difluoro-[1,1'-biphenyl]-4-carboxamido)-3-chloro-N-((1-methylcyclopropyl)methyl)picolinamide;
5-(2'-amino-5-chloro-2,4'-difluoro-[1,1'-biphenyl]-4-carboxamido)-3-chloro-N-((1,1-dioxidotetrahydro-2H-thiopyran-4-yl)methyl)picolinamide;
5-(2'-amino-5-chloro-2,4'-difluoro-[1,1'-biphenyl]-4-carboxamido)-N-butyl-3-chloropicolinamide;

5-(2'-amino-5-chloro-2,4'-difluoro-[1,1'-biphenyl]-4-carboxamido)-3-chloro-N-(prop-2-yn-1-yl)picolinamide;
N-(6-(2H-1,2,3-triazol-2-yl)-5-(trifluoromethyl)pyridin-3-yl)-4-(3-bromo-5-nitropyridin-4-yl)-2-chloro-5-fluorobenzamide;
5-(2'-amino-5-chloro-2,4'-difluoro-[1,1'-biphenyl] -4-carboxamido)-3 -chloro-N-(cyanomethyl)picolinamide;
5-(2'-amino-5-chloro-2,4'-difluoro-6'-methyl-[1,1'-biphenyl]-4-carboxamido)-3-chloro-N-(cyanomethyl)picolinamide;
5-(2'-amino-5-chloro-2,4'-difluoro-[1,1'-biphenyl]-4-carboxamido)-3-chloro-N-(2,2,2-trifluoroethyl)picolinamide;
5-(2'-amino-5-chloro-2,4'-difluoro-[1,1'-biphenyl]-4-carboxamido)-3-chloro-N-ethylpicolinamide;
2-chloro-N-(5-chloro-6-(2H-1,2,3-triazol-2-yl)pyridin-3-yl)-4-(3-cyclopropylpyridin-4-yl)-5-fluorobenzamide;
5-(2'-amino-5-chloro-2,4'-difluoro-[1,1'-biphenyl] -4-carboxamido)-3 -chloro-N-neopentylpicolinamide;
5-(2'-amino-5-chloro-2,4'-difluoro-[1,1'-biphenyl]-4-carboxamido)-3-chloro-N-(furan-3-ylmethyl)picolinamide;
5-(2'-amino-5-chloro-2,4'-difluoro-[1,1'-biphenyl]-4-carboxamido)-3-chloro-N-propylpicolinamide;
5-(2'-amino-5-chloro-2,4'-difluoro-[1,1'-biphenyl]-4-carboxamido)-3-chloro-N-(2-methoxyethyl)picolinamide;
5-(2'-amino-5-chloro-2,4'-difluoro-[1,1'-biphenyl]-4-carboxamido)-3-chloro-N-(1-cyanoethyl)picolinamide;
5-(2'-amino-5-chloro-2,4'-difluoro-[1,1'-biphenyl] -4-carboxamido)-3 -chloro-N-((3,3-difluorocyclobutyl)methyl)picolinamide;
N-((1,3,4-oxadiazol-2-yl)methyl)-5-(2'-amino-5-chloro-2,4'-difluoro-[1,1'-biphenyl]-4-carboxamido)-3-chloropicolinamide;
5-(2'-amino-5-chloro-2,4'-difluoro-[1,1'-biphenyl] -4-carboxamido)-3 -chloro-N-(2-(dimethylamino)ethyl)picolinamide;
5-(2'-amino-5-chloro-2,4'-difluoro-[1,1'-biphenyl]-4-carboxamido)-3-chloro-N-(cyclobutylmethyl)picolinamide;
5-(2'-ainino-5-chloro-2,4'-difluoro-[1,1'-biphenyl]-4-carboxamido)-3-chloro-N-((1 -methyl-1H-pyrazol-3 -yl)methyl)picolinamide;
5-(4-(2-amino-6-fluoropyridin-3-yl)-2-chloro-5-fluorobenzamido)-3-chloro-N-(cyanomethyl)picolinamide;
5-(2'-amino-5-chloro-2,4'-difluoro-[1,1'-biphenyl]-4-carboxamido)-3-chloro-N-(2,2-difluoro-2λ3-ethyl)picolinamide;
5-(2'-amino-5-chloro-2,4'-difluoro-[1,1'-biphenyl]-4-carboxamido)-3-chloro-N-(2,2,3,3,4,4,4-heptafluorobutyl)picolinamide;
5-(2'-amino-5-chloro-2,4'-difluoro-[1,1'-biphenyl]-4-carboxamido)-3-chloro-N-(2-(trifluoromethoxy)ethyl)picolinamide;
2,2'-diamino-5-chloro-N-(5-chloro-6-(2H-1,2,3-triazol-2-yl)pyridin-3-yl)-4'-fluoro-[1,1'-biphenyl] -4-carboxamide;
5-(2'-amino-5-chloro-2,4'-difluoro-[1,1'-biphenyl]-4-carboxamido)-3-chloro-N-(2-fluoroethyl)picolinamide;
5-(2'-amino-5-chloro-2,4'-difluoro-[1,1'-biphenyl]-4-carboxamido)-3-chloro-N-((5-methyl-1,3,4-oxadiazol-2-yl)methyl)picolinamide;
N-((1H-1,2,4-triazol-3-yl)methyl)-5-(2'-amino-5-chloro-2,4'-difluoro-[1,1'-biphenyl] -4-carboxamido)-3 -chloropicolinamide;
5-(2'-amino-5-chloro-2,4'-difluoro-[1,1'-biphenyl]-4-carboxamido)-3-chloro-N-(2-(ethylthio)ethyl)picolinamide;
N-(2-amino-2-oxoethyl)-5-(2'-amino-5-chloro-2,4'-difluoro-[1,1'-biphenyl]-4-carboxamido)-3-chloropicolinamide;
5-(2'-amino-5-chloro-2,4'-difluoro-[1,1'-biphenyl]-4-carboxamido)-3-chloro-N-((1-cyanocyclopropyl)methyl)picolinamide;
5-(2'-amino-5-chloro-2,4'-difluoro-[1,1'-biphenyl]-4-carboxamido)-3-chloro-N-(2,2,3,3,3-pentafluoropropyl)picolinamide;
5-(2'-amino-5-chloro-2,4'-difluoro-[1,1'-biphenyl]-4-carboxamido)-3-chloro-N-((1-(trifluoromethyl)cyclopropyl)methyl)picolinamide;
5-(2'-amino-5-chloro-2,4'-difluoro-[1,1'-biphenyl]-4-carboxamido)-3-chloro-N-(2-(methylsulfonyl)ethyl)picolinamide;
5-(4-(3-amino-5-ethynylpyridin-4-yl)-2-chloro-5-fluorobenzamido)-3-chloro-N-(2,2,2-trifluoroethyl)picolinamide;
5-(4-(3-amino-5-ethynylpyridin-4-yl)-2-chloro-5-fluorobenzamido)-3-chloro-N-(2,2-difluoroethyl)picolinamide;
5-(4-(3-amino-5-ethynylpyridin-4-yl)-2-chloro-5-fluorobenzamido)-3-chloro-N-(2-fluoroethyl)picolinamide;
5-(4-(3-amino-5-ethynylpyridin-4-yl)-2-chloro-5-fluorobenzamido)-3-chloro-N-((1,1-dioxidotetrahydro-2H-thiopyran-4-yl)methyl)picolinamide;
5-(4-(3-amino-5-ethynylpyridin-4-yl)-2-chloro-5-fluorobenzamido)-3-chloro-N-(furan-3-ylmethyl)picolinamide;
N-(6-(2H-1,2,3 -triazol-2-yl)-5 -(trifluoromethyl)pyridin-3 -yl)-4-(3 -amino-5 - cyclopropylpyridin-4-yl)-2-chloro-5-fluorobenzamide;
5-(4-(3-amino-5-ethynylpyridin-4-yl)-2-chloro-5-fluorobenzamido)-3-chloro-N-(1-cyanoethyl)picolinamide;

5-(4-(3-amino-5-ethynylpyridin-4-yl)-2-chloro-5-fluorobenzamido)-3-chloro-N-(cyclopropylmethyl)picolinamide;
5-(4-(3-amino-5-ethynylpyridin-4-yl)-2-chloro-5-fluorobenzamido)-3-chloro-N-neopentylpicolinamide;
5-(4-(3-amino-5-ethynylpyridin-4-yl)-2-chloro-5-fluorobenzamido)-3-chloro-N-ethylpicolinamide;
5-(4-(3-amino-5-ethynylpyridin-4-yl)-2-chloro-5-fluorobenzamido)-3-chloro-N-(1-cyanocyclopropyl)picolinamide;
5-(4-(3-amino-5-ethynylpyridin-4-yl)-2-chloro-5-fluorobenzamido)-3-chloro-N-((1-cyanocyclopropyl)methyl)picolinamide;
5-(4-(3-amino-5-ethynylpyridin-4-yl)-2-chloro-5-fluorobenzamido)-3-chloro-N-(2-(methylsulfonyl)ethyl)picolinamide;
5-(4-(3-amino-5-ethynylpyridin-4-yl)-2-chloro-5-fluorobenzamido)-3-chloro-N-(2-(ethylthio)ethyl)picolinamide;
5-(4-(3-amino-5-ethynylpyridin-4-yl)-2-chloro-5-fluorobenzamido)-3-chloro-N-((5-methyl-1,3,4-oxadiazol-2-yl)methyl)picolinamide;
4-(3-amino-5-ethynyl-2-fluoropyridin-4-yl)-2-chloro-N-(5-chloro-6-(2H-1,2,3-triazol-2-yl)pyridin-3-yl)-5-fluorobenzamide;
4-(5-amino-3-ethynyl-2-fluoropyridin-4-yl)-2-chloro-N-(5-chloro-6-(2H-1,2,3-triazol-2-yl)pyridin-3 -yl)-5 -fluorobenzamide;
N-(6-(2H-1,2,3-triazol-2-yl)-5-(trifluoromethyl)pyridin-3-yl)-4-(3-amino-5-fluoropyridin-2-yl)-2-chloro-5-ethynylbenzamide;
N-(6-(2H-1,2,3-triazol-2-yl)-5-(trifluoromethyl)pyridin-3-yl)-4-(3-amino-5-(2-methylprop-1-en-1-yl)pyridin-4-yl)-2-chloro-5-fluorobenzamide;
N-(6-(2H-1 ,2,3 -triazol-2-yl)-5 -(trifluoromethyl)pyridin-3 -yl)-4-(3-amino-5-(1-ethoxyvinyl)pyridin-4-yl)-2-chloro-5-fluorobenzamide;
N-(6-(2H-1,2,3-triazol-2-yl)-5-(trifluoromethyl)pyridin-3-yl)-4-(3-amino-5-(1-ethoxyethyl)pyridin-4-yl)-2-chloro-5-fluorobenzamide;
N-(6-(2H-1,2,3-triazol-2-yl)-5-(trifluoromethyl)pyridin-3-yl)-4-(3-amino-5-(1-hydroxyethyl)pyridin-4-yl)-2-chloro-5-fluorobenzamide;
N-(6-(2H-1,2,3-triazol-2-yl)-5-(trifluoromethyl)pyridin-3-yl)-4-(3-amino-5-(2-cyanoethyl)pyridin-4-yl)-2-chloro-5-fluorobenzamide;
N-(6-(2H-l,2,3-triazol-2-yl)-5-(trifluoromethyl)pyridin-3-yl)-4-(3-amino-5-(2-ethoxyethyl)pyridin-4-yl)-2-chloro-5-fluorobenzamide;
N-(6-(2H-1,2,3-triazol-2-yl)-5-(trifluoromethyl)pyridin-3-yl)-4-(3-acetyl-5-aminopyridin-4-yl)-2-chloro-5-fluorobenzamide;
N-(6-(2H-1,2,3-triazol-2-yl)-5-(trifluoromethyl)pyridin-3-yl)-4-(3-amino-5-(3,3,3-trifluoroprop-1-en-2-yl)pyridin-4-yl)-2-chloro-5-fluorobenzamide;
(E)-N-(6-(2H-1,2,3-triazol-2-yl)-5-(trifluoromethyl)pyridin-3-yl)-4-(3-amino-5-(3-hydroxy-3-methylbut-1-en-1-yl)pyridin-4-yl)-2-chloro-5-fluorobenzamide;
(E)-N-(6-(2H-1,2,3-triazol-2-yl)-5-(trifluoromethyl)pyridin-3-yl)-4-(3-amino-5-(2-cyclopropylvinyl)pyridin-4-yl)-2-chloro-5-fluorobenzamide;
N-(6-(2H-1,2,3-triazol-2-yl)-5-(trifluoromethyl)pyridin-3-yl)-4-(3-amino-5-(2-cyclopropylethyl)pyridin-4-yl)-2-chloro-5-fluorobenzamide;
N-(6-(2H-1,2,3-triazol-2-yl)-5-(trifluoromethyl)pyridin-3-yl)-4-(3-amino-5-(3-hydroxy-3-methylbutyl)pyridin-4-yl)-2-chloro-5-fluorobenzamide;
(E)-N-(6-(2H-1,2,3-triazol-2-yl)-5-(trifluoromethyl)pyridin-3-yl)-4-(3-amino-5-(3-methoxyprop-1-en-1-yl)pyridin-4-yl)-2-chloro-5-fluorobenzamide;
N-(6-(2H-1,2,3-triazol-2-yl)-5-(trifluoromethyl)pyridin-3-yl)-4-(3-amino-5-(3-methoxypropyl)pyridin-4-yl)-2-chloro-5-fluorobenzamide;
5-(4-(3-amino-5-ethynylpyridin-4-yl)-2-chloro-5-fluorobenzamido)-3-chloro-N-(2,2,2-trifluoroethyl)picolinamide;
4-(3-amino-5-(1-(difluoromethyl)-1H-pyrazol-4-yl)pyridin-4-yl)-2-chloro-N-(5-chloro-6-(2H-1,2,3-triazol-2-yl)pyridin-3-yl)-5-fluorobenzamide;
4-(3-amino-5-(prop-1-en-2-yl)pyridin-4-yl)-2-chloro-N-(5-chloro-6-(2H-1,2,3-triazol-2-yl)pyridin-3-yl)-5-fluorobenzamide;
4-(3-amino-5-ethynylpyridin-4-yl)-2-chloro-N-(5-ethynyl-6-(2H-1,2,3-triazol-2-yl)pyridin-3-yl)-5-fluorobenzamide;
4-(3-amino-5-cyclopropylpyridin-4-yl)-2-chloro-N-(5-chloro-6-(2H-1,2,3-triazol-2-yl)pyridin-3-yl)-5-fluorobenzamide;
4-(3-amino-5-vinylpyridin-4-yl)-2-chloro-N-(5-chloro-6-(2H-1,2,3-triazol-2-yl)pyridin-3-yl)-5-fluorobenzamide;

4-(3-amino-5-(prop-1-yn-1-yl)pyridin-4-yl)-2-chloro-N-(5-chloro-6-(2H-1,2,3-triazol-2-yl)pyridin-3 -yl)-5-fluorobenzamide formate;
4-(3-amino-5-(cyclohex-1-en-1-yl)pyridin-4-yl)-2-chloro-N-(5-chloro-6-(2H-1,2,3-triazol-2-yl)pyridin-3-yl)-5-fluorobenzamide;
N-(6-(2H-1,2,3-triazol-2-yl)-5-(trifluoromethyl)pyridin-3-yl)-4-(3-amino-5-bromo-2-ethynylpyridin-4-yl)-2-chloro-5-fluorobenzamide;
N-(6-(2H-1,2,3-triazol-2-yl)-5-(trifluoromethyl)pyridin-3-yl)-4-(3-amino-2,5-diethynylpyridin-4-yl)-2-chloro-5-fluorobenzamide;
4-(3-amino-5-(cyclopent-1-en-1-yl)pyridin-4-yl)-2-chloro-N-(5-chloro-6-(2H-1,2,3-triazol-2-yl)pyridin-3-yl)-5-fluorobenzamide;
4-(3-amino-5-ethynylpyridin-4-yl)-2-chloro-5-fluoro-N-(6-(trifluoromethyl)pyridin-3-yl)benzamide;
4-(3-amino-5-(cyclopropylethynyl)pyridin-4-yl)-2-chloro-N-(5-chloro-6-(2H-1,2,3-triazol-2-yl)pyridin-3-yl)-5-fluorobenzamide;
N-(6-(2H-1,2,3-triazol-2-yl)-5-(trifluoromethyl)pyridin-3-yl)-4-(3-amino-5-ethynylpyridin-4-yl)-3-fluorobenzamide;
5-(4-(3-amino-5-cyclopropylpyridin-4-yl)-2-chloro-5-fluorobenzamido)-3-chloro-N-(2,2,2-trifluoroethyl)picolinamide;
N-(6-(2H-1,2,3-triazol-2-yl)-5-(trifluoromethyl)pyridin-3-yl)-4-(3-amino-5-bromopyridin-4-yl)-2-chloro-5-fluorobenzamide;
4-(3-amino-5-ethynylpyridin-4-yl)-2-chloro-5-fluoro-N-(2-(trifluoromethyl)pyridin-4-yl)benzamide;
5-(4-(3-amino-5-cyclopropylpyridin-4-yl)-2-chloro-5-fluorobenzamido)-3-cyclopropyl-N-(2,2,2-trifluoroethyl)picolinamide;
N-(6-(2H-1,2,3-triazol-2-yl)-5-(trifluoromethyl)pyridin-3-yl)-4-(5-amino-3-ethynyl-2-fluoropyridin-4-yl)-2-chloro-5-fluorobenzamide;
N-(6-(2H-1,2,3-triazol-2-yl)-5-(trifluoromethyl)pyridin-3-yl)-4-(5-amino-3-bromo-2-fluoropyridin-4-yl)-2-chloro-5-fluorobenzamide;
N-(6-(2H-1,2,3-triazol-2-yl)-5-(trifluoromethyl)pyridin-3-yl)-4-(3-amino-5-(3,6-dihydro-2H-pyran-4-yl)pyridin-4-yl)-2-chloro-5-fluorobenzamide;
N-(6-(2H-1,2,3 -triazol-2-yl)-5 -(trifluoromethyl)pyridin-3 -yl)-4-(3 -amino-5-(3,6-dihydro-2H-pyran-4-yl)pyridin-4-yl)-3-fluorobenzamide;
4-(3-amino-5-cyclopropylpyridin-4-yl)-2-chloro-5-fluoro-N-(2-(trifluoromethyl)pyridin-4-yl)benzamide;
2'-amino-5-chloro-N-(5-cyclopropyl-6-(2H-1,2,3-triazol-2-yl)pyridin-3-yl)-2,4'-difluoro-[1,1 '-biphenyl]-4-carboxamide;
2-chloro-4-(3-cyclopropyl-5-(methylamino)pyridin-4-yl)-5-fluoro-N-(2-(trifluoromethyl)pyridin-4-yl)benzamide;
N-(6-(2H-1,2,3-triazol-2-yl)-5-(trifluoromethyl)pyridin-3-yl)-2'-amino-5-chloro-6'-cyclopropyl-2,4'-difluoro-[1,1'-biphenyl]-4-carboxamide;
N-(6-(2H-1,2,3-triazol-2-yl)-5-(trifluoromethyl)pyridin-3-yl)-4-(3-amino-5-(2,5-dihydrofuran-3-yl)pyridin-4-yl)-2-chloro-5-fluorobenzamide;
4-(3-acetyl-5-aminopyridin-4-yl)-2-chloro-N-(5-chloro-6-(2H-1,2,3-triazol-2-yl)pyridin-3-yl)-5-fluorobenzamide;
N-(6-(2H-1,2,3-triazol-2-yl)-5-(trifluoromethyl)pyridin-3-yl)-4-(3-amino-5-(4-hydroxycyclohex-1-en-1 -yl)pyridin-4-yl)-2-chloro-5-fluorobenzamide;
N-(6-(2H-1,2,3-triazol-2-yl)-5-(trifluoromethyl)pyridin-3-yl)-4-(3-amino-5-phenylpyridin-4-yl)-2-chloro -5 - fluorobenzamide;
2'-amino-5-chloro-6'-(2-ethoxyethyl)-2,4'-difluoro-N-(2-(trifluoromethyl)pyridin-4-yl)-[1,1 '-biphenyl] -4-carboxamide;
2'-amino-5-chloro-2,4'-difluoro-6'-(tetrahydrofuran-2-yl)-N-(2-(trifluoromethyl)pyridin-4-yl)-[1,1 '-biphenyl]-4-carboxamide;
2'-amino-5-chloro-2,4'-difluoro-6'-(4-morpholinocyclohexyl)-N-(2-(trifluoromethyl)pyridin-4-yl)-[1,1'-biphenyl]-4-carboxamide;
2'-amino-5-chloro-2,4'-difluoro-6'-(4-hydroxycyclohexyl)-N-(2-(trifluoromethyl)pyridin-4-yl)-[1,1 '-biphenyl]-4-carboxamide;
2'-amino-5-chloro-2,4'-difluoro-6'-(2-oxo-1,2-dihydropyridin-4-yl)-N-(2-(trifluoromethyl)pyridin-4-yl)-[1,1'-biphenyl]-4-carboxamide;
2'-amino-5-chloro-2,4'-difluoro-6'-(6-oxo-1,6-dihydropyridin-3-yl)-N-(2-(trifluoromethyl)pyridin-4-yl)-[1,1'-biphenyl]-4-carboxamide;
2'-amino-5-chloro-2,4'-difluoro-6'-(l-methylpiperidin-3-yl)-N-(2-(trifluoromethyl)pyridin-4-yl)-[1,1'-biphenyl]-4-carboxamide;
2'-(1-acetylpiperidin-4-yl)-6'-amino-5-chloro-2,4'-difluoro-N-(2-(trifluoromethyl)pyridin-4-yl)-[1,1 '-biphenyl] -4-carboxamide;
2'-amino-5-chloro-2,4'-difluoro-6'-(spiro[2.5]octan-6-yl)-N-(2-(trifluoromethyl)pyridin-4-yl)-[1,1 '-biphenyl]-4-carboxamide;
2'-amino-5-chloro-2,4'-difluoro-6'-(2-oxo-1,2-dihydropyrimidin-5-yl)-N-(2-(trifluoromethyl)pyridin-4-yl)-[1,1'-biphenyl]-4-carboxamide;
2'-amino-5-chloro-2,4'-difluoro-6'-(1-(2,2,2-trifluoroacetyl)piperidin-4-yl)-N-(2-(trifluoromethyl)pyridin-4-yl)-[1,1 '-biphenyl] -4-carboxamide;
2'-amino-5-chloro-2,4'-difluoro-6'-(4-(trifluoromethyl)cyclohexyl)-N-(2-(trifluoromethyl)pyridin-4-yl)-[1,1 '-biphenyl]-4-carboxamide;
2'-amino-5-chloro-2,4'-difluoro-6'-(1-hydroxyethyl)-N-(2-(trifluoromethyl)pyridin-4-yl)-[1, 1'-biphenyl]-4-carboxamide;
4-(3-amino-5-bromo-2-chloropyridin-4-yl)-2-chloro-5-fluoro-N-(2-(trifluoromethyl)pyridin-4-yl)benzamide;
4-(3-amino-2,5-dimethylpyridin-4-yl)-2-chloro-5-fluoro-N-(2-(trifluoromethyl)pyridin-4-yl)benzamide;
4-(3-amino-5-bromo-2-methoxypyridin-4-yl)-2-chloro-5-fluoro-N-(2-(trifluoromethyl)pyridin-4-yl)benzamide;
2-chloro-4-(2,3-diamino-5-ethynylpyridin-4-yl)-5-fluoro-N-(2-(trifluoromethyl)pyridin-4-yl)benzamide;
4-(2-amino-5-ethynyl-3-iodopyridin-4-yl)-2-chloro-5-fluoro-N-(2-(trifluoromethyl)pyridin-4-yl)benzamide;
4-(3-amino-5-ethynyl-2-methylpyridin-4-yl)-2-chloro-5-fluoro-N-(2-(trifluoromethyl)pyridin-4-yl)benzamide;
N-(6-(2H-1,2,3-triazol-2-yl)-5-(trifluoromethyl)pyridin-3-yl)-4-(3-amino-5-ethynyl-2-methylpyridin-4-yl)-2-chloro-5-fluorobenzamide;
N-(6-(2H-1,2,3-triazol-2-yl)-5-(trifluoromethyl)pyridin-3-yl)-4-(3-amino-5-ethynyl-2-fluoropyridin-4-yl)-2-chloro-5-fluorobenzamide;
N-(6-(2H-1,2,3-triazol-2-yl)-5-(trifluoromethyl)pyridin-3-yl)-4-(3-amino-2-chloro-5-ethynylpyridin-4-yl)-2-chloro-5-fluorobenzamide;
N-(6-(2H-1,2,3-triazol-2-yl)-5-(trifluoromethyl)pyridin-3-yl)-4-(3-amino-2-cyano-5-ethynylpyridin-4-yl)-2-chloro-5-fluorobenzamide;
N-(6-(2H-1,2,3-triazol-2-yl)-5-(trifluoromethyl)pyridin-3-yl)-4-(3-amino-5-ethynyl-2-methoxypyridin-4-yl)-2-chloro-5-fluorobenzamide;
N-(6-(2H-1,2,3-triazol-2-yl)-5-(trifluoromethyl)pyridin-3-yl)-4-(3-amino-5-ethynyl-6-fluoro-2-methylpyridin-4-yl)-2-chloro-5-fluorobenzamide;
N-(6-(2H-1,2,3-triazol-2-yl)-5-(trifluoromethyl)pyridin-3-yl)-4-(3-amino-5-ethynyl-2,6-difluoropyridin-4-yl)-2-chloro-5-fluorobenzamide;
N-(6-(2H-1,2,3-triazol-2-yl)-5-(trifluoromethyl)pyridin-3-yl)-4-(3-amino-5-bromo-2-chloropyridin-4-yl)-2-chloro-5-fluorobenzamide;
N-(6-(2H-1,2,3-triazol-2-yl)-5-(trifluoromethyl)pyridin-3-yl)-4-(3-amino-5-ethynyl-2-hydroxypyridin-4-yl)-2-chloro-5-hydroxybenzamide;
N-(6-(2H-1,2,3-triazol-2-yl)-5-(trifluoromethyl)pyridin-3-yl)-4-(3-amino-5-bromo-2-hydroxypyridin-4-yl)-2-chloro-5-hydroxybenzamide;
4-(4-((6-(2H-1,2,3-triazol-2-yl)-5-(trifluoromethyl)pyridin-3-yl)carbamoyl)-5-chloro-2-fluorophenyl)-3-amino-5-ethynylpicolinamide;
N-(6-(2H-1,2,3-triazol-2-yl)-5-(trifluoromethyl)pyridin-3-yl)-4-(3-amino-5-ethynylpyridazin-4-yl)-2-chloro-5-fluorobenzamide;
N-(6-(2H-1,2,3-triazol-2-yl)-5-(trifluoromethyl)pyridin-3-yl)-4-(2-amino-4-ethynyl-6-fluoropyridin-3-yl)-2-chloro-5-fluorobenzamide;
N-(6-(2H-1,2,3 -triazol-2-yl)-5-(trifluoromethyl)pyridin-3-yl)-4-(2-amino-4-ethynylpyridin-3-yl)-2-chloro-5-fluorobenzamide;
N-((1S)-1-(4-(4-(3-amino-5-ethynylpyridin-4-yl)-2-chloro-5-fluorobenzamide)phenyl)-2,2,2-trifluoroethyl)-N-methyltetrahydro2H-thiopyran-4-carboxamide 1,1 -dioxide;
4-(3-amino-2-chloro-5-ethynylpyridin-4-yl)-2-chloro-N-(5-chloro-6-(2H-1,2,3-triazol-2-yl)pyridin-3-yl)-5-fluorobenzamide;
4-(3-amino-2-chloro-5-ethynylpyridin-4-yl)-2-chloro-5-fluoro-N-(2-(trifluoromethyl)pyridin-4-yl)benzamido;
4-(3-amino-5-ethynylpyridin-4-yl)-2-chloro-5-fluoro-N-(5-hydroxy-6-(2H-1,2,3-triazol-2-yl)pyridin-3-yl)benzamido;
4-(3-amino-5-ethynylpyridin-4-yl)-2-chloro-5-fluoro-N-(5-methoxy-6-(2H-1,2,3-triazol-2-yl)pyridin-3-yl)benzamido;
4-(3-amino-5-ethynylpyridin-4-yl)-2-chloro-N-(5-(difluoromethoxy)-6-(2H-1,2,3-triazol-2-yl)pyridin-3-yl)-5-fluorobenzamide; or
N-(6-(2H-1,2,3-triazol-2-yl)-5-(trifluoromethyl)pyridin-3-yl)-4-(5-amino-3-ethynyl-2-fluoropyridin-4-yl)-2-chloro-5-fluorobenzamide.

**18.** A pharmaceutical composition comprising a therapeutically effective amount of at least a compound of any one of claims 1-17, or a stereoisomer, tautomer, deuterated compound, pharmaceutically acceptable salt, prodrug, chelate, non-covalent complex or solvate thereof as an active ingredient, and at least one pharmaceutically acceptable ingredient, such as carrier or excipient.

**19.** Use of a compound of any one of claims 1-17, or a stereoisomer, tautomer, deuterated compound, pharmaceutically acceptable salt, prodrug, chelate, non-covalent complex or solvate thereof, or a pharmaceutical composition of claim 18 for the manufacture of a medicament for the treatment of diseases, syndromes, disorders or obstacles.

**20.** The use of claim 19, wherein the diseases, syndromes, disorders and obstacles are affected by MALT1 inhibition.

**21.** The use of claim 19 or 20, wherein the diseases, syndromes, disorders and obstacles are selected from diffuse large B-cell lymphoma (DLBCL), mantle cell lymphoma (MCL), follicular lymphoma (FL), mucosa-associated lymphoid tissue (MALT) lymphoma, rheumatoid arthritis (RA), psoriatic arthritis (PsA), psoriasis (Pso), ulcerative colitis (UC), Crohn's disease, systemic lupus erythematosus (SLE), asthma or chronic obstructive pulmonary disease (COPD).

**22.** A method of treating diseases, syndromes, disorders or obstacles, wherein the diseases, syndromes, disorders and obstacles are affected by MALT1 inhibition, comprising administering to a patient in need the compound of any one of claims 1-17, or a stereoisomer, tautomer, deuterated compound, pharmaceutically acceptable salt, prodrug, chelate, non-covalent complex or solvate thereof, or the pharmaceutical composition of claim 18.

**23.** The method of claim 22, wherein the diseases, syndromes, disorders and obstacles are selected from diffuse large B-cell lymphoma (DLBCL), mantle cell lymphoma (MCL), follicular lymphoma (FL), mucosa-associated lymphoid tissue (MALT) lymphoma, rheumatoid arthritis (RA), psoriatic arthritis (PsA), psoriasis (Pso), ulcerative colitis (UC), Crohn's disease, systemic lupus erythematosus (SLE), asthma or chronic obstructive pulmonary disease (COPD).

**INTERNATIONAL SEARCH REPORT**

International application No. **PCT/CN2022/101146**

**A. CLASSIFICATION OF SUBJECT MATTER**

C07D 401/04(2006.01)i; C07D 401/12(2006.01)i; C07D 401/14(2006.01)i; C07D 413/14(2006.01)i; C07D 403/12(2006.01)i; C07D 409/14(2006.01)i; A61K 31/165(2006.01)i; A61K 31/4192(2006.01)i; A61P 35/00(2006.01)i

According to International Patent Classification (IPC) or to both national classification and IPC

**B. FIELDS SEARCHED**

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

CNPAT, CNTXT, CNKI, WPI, EPODOC, WOTXT, EPTXT, USTXT, ISI, CAPLUS(STN), REGISTRY(STN): 贝达, 付邦, 李因龙, 任伟, 申其超, 杜国龙, 周小军, 孙云, 周瑜珍, 孙晓, 蔡卫东, 刘湘永, 丁列明, 王家炳, 淋巴, 瘤, 癌, mucosa-associated lymphoid tissue, MALT1, tumor, cancer, STN结构式检索, STN structural formula search

**C. DOCUMENTS CONSIDERED TO BE RELEVANT**

| Category* | Citation of document, with indication, where appropriate, of the relevant passages | Relevant to claim No. |
|---|---|---|
| PX | WO 2021138298 A1 (RHEOS MEDICINES, INC.) 08 July 2021 (2021-07-08) description, paragraphs 3-115, embodiments 1-2, and tables 1-2 | 1-23 |
| PX | REGISTRY. "RN: 2776345-09-4" *STN,* 23 June 2022 (2022-06-23), p. 1 | 1-6, 8-9, 12-14 |
| X | REGISTRY. "RN: 2637183-50-5, 2637006-62-1, 2419393-29-4, 1624465-83-3, 1572752-51-2, 1572502-88-5, 1445347-09-0, 1384733-96-3, 1302540-29-9, 1299920-08-3, 1298364-79-0, 1298361-80-4, 1296941-30-4, 1296796-39-8, 1294983-77-9, 1289283-46-0, 1277726-68-7" *STN,* 23 April 2021 (2021-04-23), pp. 1-9 | 1-16 |
| A | CN 111171020 A (SHANGHAI ENNOVABIO PHARMACEUTICALS CO., LTD.) 19 May 2020 (2020-05-19) description, paragraphs 5-53, and embodiments 1-5 | 1-23 |
| A | CN 110214136 A (JANSSEN PHARMACEUTICA NV) 06 September 2019 (2019-09-06) description, paragraphs 11-53, and embodiments 1-445 | 1-23 |

☑ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **13 September 2022** | **21 September 2022** |
| Name and mailing address of the ISA/CN<br>**China National Intellectual Property Administration (ISA/CN)**<br>**No. 6, Xitucheng Road, Jimenqiao, Haidian District, Beijing 100088, China**<br>Facsimile No. **(86-10)62019451** | Authorized officer<br><br>Telephone No. |

Form PCT/ISA/210 (second sheet) (January 2015)

INTERNATIONAL SEARCH REPORT

International application No.
PCT/CN2022/101146

C. DOCUMENTS CONSIDERED TO BE RELEVANT

| Category* | Citation of document, with indication, where appropriate, of the relevant passages | Relevant to claim No. |
|---|---|---|
| A | CN 1805923 A (ASTELLAS PHARMA INC.) 19 July 2006 (2006-07-19)<br>description, p. 3, paragraph 5 to p. 12, paragraph 12, and embodiments 1-232 | 1-23 |

Form PCT/ISA/210 (second sheet) (January 2015)

INTERNATIONAL SEARCH REPORT

International application No.
PCT/CN2022/101146

**Box No. II Observations where certain claims were found unsearchable (Continuation of item 2 of first sheet)**

This international search report has not been established in respect of certain claims under Article 17(2)(a) for the following reasons:

1. [✓] Claims Nos.: **22-23**
because they relate to subject matter not required to be searched by this Authority, namely:

[1] Claims 22-23 relate to a method for treating or preventing diseases of a human or animal body. However, the present search report was conducted on the basis of a use of a compound or a pharmaceutically acceptable salt thereof in the preparation of a drug for treating a corresponding disease.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2022/101146**

| Patent document cited in search report | Publication date (day/month/year) | Patent family member(s) | Publication date (day/month/year) |
|---|---|---|---|
| WO 2021138298 A1 | 08 July 2021 | None | |
| CN 111171020 A | 19 May 2020 | CN 113166141 A | 23 July 2021 |
| | | WO 2020098723 A1 | 22 May 2020 |
| | | AU 2019379213 A1 | 01 July 2021 |
| | | CA 3120430 A1 | 22 May 2020 |
| | | US 2022017512 A1 | 20 January 2022 |
| | | EP 3882247 A1 | 22 September 2021 |
| CN 110214136 A | 06 September 2019 | JP 2020514267 A | 21 May 2020 |
| | | US 2018170909 A1 | 21 June 2018 |
| | | UY 37537 A | 29 June 2018 |
| | | WO 2018119036 A1 | 28 June 2018 |
| | | IL 267343 A | 29 August 2019 |
| | | BR 112019012355 A2 | 26 November 2019 |
| | | CA 3048027 A1 | 28 June 2018 |
| | | AU 2017382185 A1 | 13 June 2019 |
| | | TW 201835067 A | 01 October 2018 |
| | | AU 2022201352 A1 | 24 March 2022 |
| | | EP 3558969 A1 | 30 October 2019 |
| | | MA 47125 A | 28 April 2021 |
| | | EA 201991503 A1 | 29 November 2019 |
| | | MX 2019007540 A | 24 October 2019 |
| | | PH 12019501212 A1 | 16 December 2019 |
| | | PE 20191755 A1 | 12 December 2019 |
| | | AR 110421 A1 | 27 March 2019 |
| | | CO 2019006622 A2 | 28 June 2019 |
| | | CL 2019001709 A1 | 04 October 2019 |
| | | KR 20190093669 A | 09 August 2019 |
| | | TN 2019000192 A1 | 05 October 2020 |
| CN 1805923 A | 19 July 2006 | None | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **JAWORSKI et al.** *Cell Mol Life Sci,* 2016, vol. 73, 459-473
- **STAUDT.** *ColdSpring Harb Perspect Biol,* 2010, vol. 2
- **LIM et al.** *Immunol Rev,* 2012, vol. 246, 359-378
- **FONTAN et al.** *Cancer Cell,* 2012, vol. 22, 812-824
- **NAGEL et al.** *Cancer ell,* 2012, vol. 22, 825-837
- **JAWORSKI M et al.** *Cell Mol Life Sci.,* 2016
- **MC GUIRE et al.** *J.Neuro inflammation,* 2014, vol. 11, 124
- **ARIANNA BERTOSSI et al.** *EMBO J,* 2014, vol. 33, 2740-2742
- Design of Prodrugs. Elsevier, 1985